(19) Europäisches Patentamt / European Patent Office / Office européen des brevets

(11) EP 1 511 768 B1

(12) **EUROPEAN PATENT SPECIFICATION**

(45) Date of publication and mention of the grant of the patent: **11.04.2007 Bulletin 2007/15**

(21) Application number: **03757056.1**

(22) Date of filing: **06.06.2003**

(51) Int Cl.:
***C07K 14/315*** (2006.01) ***A61K 39/385*** (2006.01)
***C12N 5/10*** (2006.01) ***C12N 15/62*** (2006.01)
***C12N 15/63*** (2006.01) ***A61K 38/16*** (2006.01)

(86) International application number: **PCT/EP2003/006096**

(87) International publication number: **WO 2003/104272 (18.12.2003 Gazette 2003/51)**

(54) **IMMUNOGENIC COMPOSITIONS**

IMMUNOGENE ZUSAMMENSETZUNGEN

COMPOSITIONS IMMUNOGENES

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **11.06.2002 GB 0213365**
**15.01.2003 GB 0300914**

(43) Date of publication of application: **09.03.2005 Bulletin 2005/10**

(73) Proprietors:
- **GlaxoSmithKline Biologicals s.a. 1330 Rixensart Brussels (BE)**
- **GLAXO GROUP LIMITED Greenford, Middlesex UB6 ONN (GB)**

(72) Inventors:
- **CABEZON SILVA, T.E.V., GlaxoSmithKline Biolog. SA 1330 Rixensart Brussels (BE)**
- **ELLIS, Jonathan H., GlaxoSmithKline Stevenage, Hertfordshire SG1 2NY (GB)**
- **GERARD, C.M.G., GlaxoSmithKline Biologicals s.a. 1330 Rixensart Brussels (BE)**
- **HAMBLIN, Paul A., GlaxoSmithKline Stevenage, Hertfordshire SG1 2NY (GB)**
- **PALMANTIER, R.M., GlaxoSmithKline Biologicals s.a. 1330 Rixensart Brussels (BE)**
- **VINALS Y DE BASSOLS, C., GlaxoSmithKline Biolog.sa 1330 Rixensart Brussels (BE)**

(74) Representative: **Lovatt, Victoria Jayne et al GlaxoSmithKline, Corporate Intellectual Property CN925.1, 980 Great West Road Brentford, Middlesex TW8 9GS (GB)**

(56) References cited:
**WO-A-99/67293** **US-A- 6 090 388**

- **SANCHEZ-PUELLES J M ET AL: "IMMOBILIZATION AND SINGLE-STEP PURIFICATION OF FUSION PROTEINS USING DEAE-CELLULOSE" EUROPEAN JOURNAL OF BIOCHEMISTRY, vol. 203, no. 1-2, 1992, pages 153-160, XP001155694 ISSN: 0014-2956**
- **CAUBIN J ET AL: "Choline-binding domain as a novel affinity tag for purification of fusion proteins produced in Pichia pastoris" BIOTECHNOLOGY AND BIOENGINEERING, vol. 74, no. 2, 20 July 2001 (2001-07-20), pages 164-171, XP001155696 ISSN: 0006-3592**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- KJERRULF M ET AL: "TANDEM REPEATS OF T HELPER EPITOPES ENHANCE IMMUNOGENICITY OF FUSION PROTEINS BY PROMOTING PROCESSING AND PRESENTATION" MOLECULAR IMMUNOLOGY, ELMSFORD, NY, US, vol. 34, no. 8/9, June 1997 (1997-06), pages 599-608, XP000857056 ISSN: 0161-5890
- ASTORI M ET AL: "RECOMBINATION FUSION PEPTIDES CONTAINING SINGLE OR MULTIPLE REPEATSOF A UBIQUITOUS T-HELPER EPITOPE ARE HIGHLY IMMUNOGENIC" MOLECULAR IMMUNOLOGY, ELMSFORD, NY, US, vol. 33, no. 13, 1996, pages 1017-1024, XP001028964 ISSN: 0161-5890
- OLSZEWSKA WIESLAWA ET AL: "Protection against measles virus-induced encephalitis by anti-mimotope antibodies: The role of antibody affinity" VIROLOGY, vol. 272, no. 1, 30 June 2000 (2000-06-30), pages 98-105, XP002259121 ISSN: 0042-6822

SEQUENCE LISTING

<110> GlaxosmithKline Biologicals sa
Glaxo Group Ltd

<120> Immunogenic compositions

<130> B45311

<160> 52

<170> FastSEQ for Windows version 4.0

<210> 1
<211> 15
<212> PRT
<213> Streptococcus pneumoniae

<400> 1

```
Gly Trp Gln Lys Asn Asp Thr Gly Tyr Trp Tyr Val His Ser Asp
 1               5                  10                  15
```

<210> 2

<211> 21
<212> PRT
<213> Streptococcus pneumoniae

<400> 2

```
Gly Ser Tyr Pro Lys Asp Lys Phe Glu Lys Ile Asn Gly Thr Trp Tyr
 1               5                  10                  15
Tyr Phe Asp Ser Ser
            20
```

<210> 3
<211> 22
<212> PRT
<213> Streptococcus pneumoniae

<400> 3

```
Gly Tyr Met Leu Ala Asp Arg Trp Arg Lys His Thr Asp Gly Asn Trp
 1               5                  10                  15
Tyr Trp Phe Asp Asn Ser
            20
```

<210> 4
<211> 20
<212> PRT
<213> Streptococcus pneumoniae

<400> 4

```
Gly Glu Met Ala Thr Gly Trp Lys Lys Ile Ala Asp Lys Trp Tyr Tyr
 1               5                  10                  15
Phe Asn Glu Glu
            20
```

<210> 5
<211> 21
<212> PRT
<213> Streptococcus pneumoniae

<400> 5

```
Gly Ala Met Lys Thr Gly Trp Val Lys Tyr Lys Asp Thr Trp Tyr Tyr
 1               5                  10                  15
Leu Asp Ala Lys Glu
            20
```

<210> 6
<211> 23
<212> PRT
<213> Streptococcus pneumoniae

<400> 6

Gly Ala Met Val Ser Asn Ala Phe Ile Gln Ser Ala Asp Gly Thr Gly
1               5                   10                  15
Trp Tyr Tyr Leu Lys Pro Asp
            20

<210> 7
<211> 142
<212> PRT
<213> Streptococcus pneumoniae

<400> 7

Gly Trp Gln Lys Asn Asp Thr Gly Tyr Trp Tyr Val His Ser Asp Gly
1               5                   10                  15
Ser Tyr Pro Lys Asp Lys Phe Glu Lys Ile Asn Gly Thr Trp Tyr Tyr
            20                  25                  30
Phe Asp Ser Ser Gly Tyr Met Leu Ala Asp Arg Trp Arg Lys His Thr
        35                  40                  45
Asp Gly Asn Trp Tyr Trp Phe Asp Asn Ser Gly Glu Met Ala Thr Gly
    50                  55                  60
Trp Lys Lys Ile Ala Asp Lys Trp Tyr Tyr Phe Asn Glu Glu Gly Ala
65                  70                  75                  80
Met Lys Thr Gly Trp Val Lys Tyr Lys Asp Thr Trp Tyr Tyr Leu Asp
                85                  90                  95
Ala Lys Glu Gly Ala Met Val Ser Asn Ala Phe Ile Gln Ser Ala Asp
            100                 105                 110
Gly Thr Gly Trp Tyr Tyr Leu Lys Pro Asp Gly Thr Leu Ala Asp Arg
        115                 120                 125
Pro Glu Phe Thr Val Glu Pro Asp Gly Leu Ile Thr Val Lys
    130                 135                 140

<210> 8
<211> 112
<212> PRT
<213> Streptococcus pneumoniae

<400> 8

Tyr Val His Ser Asp Gly Ser Tyr Pro Lys Asp Lys Phe Glu Lys Ile
1               5                   10                  15
Asn Gly Thr Trp Tyr Tyr Phe Asp Ser Ser Gly Tyr Met Leu Ala Asp
            20                  25                  30
Arg Trp Arg Lys His Thr Asp Gly Asn Trp Tyr Trp Phe Asp Asn Ser
        35                  40                  45

Gly Glu Met Ala Thr Gly Trp Lys Lys Ile Ala Asp Lys Trp Tyr Tyr
    50                  55                  60
Phe Asn Glu Glu Gly Ala Met Lys Thr Gly Trp Val Lys Tyr Lys Asp
65                  70                  75                  80
Thr Trp Tyr Tyr Leu Asp Ala Lys Glu Gly Ala Met Val Ser Asn Ala
                85                  90                  95
Phe Ile Gln Ser Ala Asp Gly Thr Gly Trp Tyr Tyr Leu Lys Pro Asp
            100                 105                 110

<210> 9
<211> 45
<212> DNA
<213> Streptococcus pneumoniae

<400> 9
ggctggcaga agaatgacac tggctactgg tacgtacatt cagac 45

<210> 10
<211> 63
<212> DNA
<213> Streptococcus pneumoniae

<400> 10

ggctcttatc caaaagacaa gtttgagaaa atcaatggca cttggtacta ctttgacagt 60
tca 63

<210> 11
<211> 66
<212> DNA
<213> Streptococcus pneumoniae

<400> 11

ggctatatgc ttgcagaccg ctggaggaag cacacagacg gcaactggta ctggttcgac 60
aactca 66

<210> 12
<211> 60
<212> DNA
<213> Streptococcus pneumoniae

<400> 12
ggcgaaatgg ctacaggctg gaagaaaatc gctgataagt ggtactattt caacgaagaa 60

<210> 13
<211> 63
<212> DNA
<213> Streptococcus pneumoniae

<400> 13

ggtgccatga agacaggctg ggtcaagtac aaggacactt ggtactactt agacgctaaa 60
gaa 63

<210> 14
<211> 69
<212> DNA
<213> Streptococcus pneumoniae

<400> 14

ggcgccatgg tatcaaatgc ctttatccag tcagcggacg gaacaggctg gtactacctc 60

aaaccagac 69

<210> 15
<211> 429
<212> DNA
<213> Streptococcus pneumoniae

<400> 15

ggctggcaga agaatgacac tggctactgg tacgtacatt cagacggctc ttatccaaaa 60
gacaagtttg agaaaatcaa tggcacttgg tactactttg acagttcagg ctatatgctt 120
gcagaccgct ggaggaagca cacagacggc aactggtact ggttcgacaa ctcaggcgaa 180
atggctacag gctggaagaa aatcgctgat aagtggtact atttcaacga agaaggtgcc 240
atgaagacag gctgggtcaa gtacaaggac acttggtact acttagacgc taaagaaggc 300
gccatggtat caaatgcctt tatccagtca gcggacggaa caggctggta ctacctcaaa 360
ccagacggaa cactggcaga caggccagaa ttcacagtag agccagatgg cttgattaca 420
gtaaaataa 429

<210> 16
<211> 336
<212> DNA
<213> Streptococcus pneumoniae

<400> 16

tacgtacatt ccgacggctc ttatccaaaa gacaagtttg agaaaatcaa tggcacttgg 60
tactactttg acagttcagg ctatatgctt gcagaccgct ggaggaagca cacagacggc 120
aactggtact ggttcgacaa ctcaggcgaa atggctacag gctggaagaa aatcgctgat 180
aagtggtact atttcaacga agaaggtgcc atgaagacag gctgggtcaa gtacaaggac 240
acttggtact acttagacgc taaagaaggc gccatggtat caaatgcctt tatccagtca 300
gcggacggaa caggctggta ctacctcaaa ccagac 336

<210> 17
<211> 1674
<212> DNA
<213> Homo sapiens

<400> 17

```
gccaccatgg tccagaggct gtgggtgagc cgcctgctgc ggcaccggaa agcccagctc 60
ttgctggtca acctgctaac ctttggcctg gaggtgtgtt tggccgcagg catcacctat 120
gtgccgcctc tgctgctgga agtgggggta gaggagaagt tcatgaccat ggtgctgggc 180
attggtccag tgctgggcct ggtctgtgtc ccgctcctag gctcagccag tgaccactgg 240
cgtggacgct atggccgccg ccggcccttc atctgggcac tgtccttggg catcctgctg 300
agcctctttc tcatcccaag ggccggctgg ctagcagggc tgctgtgccc ggatcccagg 360
cccctggagc tggcactgct catcctgggc gtggggctgc tggacttctg tggccaggtg 420
tgcttcactc cactggaggc cctgctctct gacctcttcc gggacccgga ccactgtcgc 480
caggcctact ctgtctatgc cttcatgatc agtcttgggg gctgcctggg ctacctcctg 540
cctgccattg actgggacac cagtgccctg gccccctacc tgggcaccca ggaggagtgc 600
ctctttggcc tgctcaccct catcttcctc acctgcgtag cagccacact gctggtggct 660
gaggaggcag cgctgggccc caccgagcca gcagaagggc tgtcggcccc ctccttgtcg 720
ccccactgct gtccatgccg ggcccgcttg gctttccgga acctgggcgc cctgcttccc 780
cggctgcacc agctgtgctg ccgcatgccc cgcaccctgc gccggctctt cgtggctgag 840
ctgtgcagct ggatggcact catgaccttc acgctgtttt acacggattt cgtgggcgag 900
gggctgtacc agggcgtgcc cagagctgag ccgggcaccg aggcccggag acactatgat 960
gaaggcgttc ggatgggcag cctggggctg ttcctgcagt gcgccatctc cctggtcttc 1020
tctctggtca tggaccggct ggtgcagcga ttcggcactc gagcagtcta tttggccagt 1080
gtggcagctt tccctgtggc tgccggtgcc acatgcctgt cccacagtgt ggccgtggtg 1140
acagcttcag ccgccctcac cgggttcacc ttctcagccc tgcagatcct gccctacaca 1200
ctggcctccc tctaccaccg ggagaagcag gtgttcctgc ccaaataccg aggggacact 1260
ggaggtgcta gcagtgagga cagcctgatg accagcttcc tgccaggccc taagcctgga 1320
gctcccttcc ctaatggaca cgtgggtgct ggaggcagtg gcctgctccc acctccaccc 1380
gcgctctgcg ggcctctgc ctgtgatgtc tccgtacgtg tggtggtggg tgagcccacc 1440
gaggccaggg tggttccggg ccggggcatc tgcctggacc tcgccatcct ggatagtgcc 1500

ttcctgctgt cccaggtggc cccatccctg tttatgggct ccattgtcca gctcagccag 1560
tctgtcactg cctatatggt gtctgccgca ggcctgggtc tggtcgccat ttactttgct 1620
acacaggtag tatttgacaa gagcgacttg gccaaatact cagcgtaggt cgag       1674
```

<210> 18
<211> 1947
<212> DNA
<213> Artificial Sequence

<220>
<223> Hybrid gene between St. pneum. C-LytA, P2 T helper epitope and human P501S.

<400> 18

```
gccaccatgg cggccgctta cgtacattcc gacggctctt atccaaaaga caagtttgag 60
aaaatcaatg gcacttggta ctactttgac agttcaggct atatgcttgc agaccgctgg 120
aggaagcaca cagacggcaa ctggtactgg ttcgacaact caggcgaaat ggctacaggc 180
tggaagaaaa tcgctgataa gtggtactat ttcaacgaag aaggtgccat gaagacaggc 240
tgggtcaagt acaaggacac ttggtactac ttagacgcta aagaaggcgc catgcaatac 300
atcaaggcta actctaagtt cattggtatc actgaaggcg tcatggtatc aaatgccttt 360
atccagtcag cggacggaac aggctggtac tacctcaaac cagacggaac actggcagac 420
aggccagaaa agttcatgta catggtgctg ggcattggtc cagtgctggg cctggtctgt 480
gtcccgctcc taggctcagc cagtgaccac tggcgtggac gctatggccg ccgccggccc 540
ttcatctggg cactgtcctt gggcatcctg ctgagcctct ttctcatccc aagggccggc 600
tggctagcag ggctgctgtg cccggatccc aggcccctgg agctggcact gctcatcctg 660
ggcgtggggc tgctggactt ctgtggccag gtgtgcttca ctccactgga ggccctgctc 720
tctgacctct tccgggaccc ggaccactgt cgccaggcct actctgtcta tgccttcatg 780
atcagtcttg gggctgcct gggctacctc ctgcctgcca ttgactggga caccagtgcc 840
ctggcccccct acctgggcac ccaggaggag tgcctctttg gcctgctcac cctcatcttc 900
ctcacctgcg tagcagccac actgctggtg gctgaggagg cagcgctggg ccccaccgag 960
ccagcagaag ggctgtcggc cccctccttg tcgccccact gctgtccatg ccgggcccgc 1020
ttggctttcc ggaacctggg cgccctgctt ccccggctgc accagctgtg ctgccgcatg 1080
ccccgcaccc tgcgccggct cttcgtggct gagctgtgca gctggatggc actcatgacc 1140
ttcacgctgt tttacacgga tttcgtgggc gaggggctgt accagggcgt gcccagagct 1200
gagccgggca ccgaggcccg gagacactat gatgaaggcg ttcggatggg cagcctgggg 1260
ctgttcctgc agtgcgccat ctccctggtc ttctctctgg tcatggaccg gctggtgcag 1320
cgattcggca ctcgagcagt ctatttggcc agtgtggcag ctttccctgt ggctgccggt 1380
gccacatgcc tgtcccacag tgtggccgtg gtgacagctt cagccgccct caccgggttc 1440
accttctcag ccctgcagat cctgccctac acactggcct ccctctacca ccgggagaag 1500
caggtgttcc tgcccaaata ccgaggggac actggaggtg ctagcagtga ggacagcctg 1560
atgaccagct tcctgccagg ccctaagcct ggagctccct tccctaatgg acacgtgggt 1620
gctggaggca gtggcctgct cccacctcca cccgcgctct gcggggcctc tgcctgtgat 1680
gtctccgtac gtgtggtggt gggtgagccc accgaggcca gggtggttcc gggccggggc 1740
atctgcctgg acctcgccat cctggatagt gccttcctgc tgtcccaggt ggccccatcc 1800
ctgtttatgg gctccattgt ccagctcagc cagtctgtca ctgcctatat ggtgtctgcc 1860
gcaggcctgg gtctggtcgc catttacttt gctacacagg tagtatttga caagagcgac 1920
ttggccaaat actcagcgta ggtcgag                                     1947
```

<210> 19
<211> 1662
<212> DNA
<213> Artificial Sequence

<220>
<223> Codon optimised human P501S

<400> 19

```
atggtgcagc ggctctgggt gagccgcctc ctgcggcatc gcaaggccca gctcctgctg 60
gtgaatctgc tcacattcgg cctggaggtg tgcctggccg ccggcatcac ctacgtgccc 120
cccctcctgc tggaggtggg agtcgaggag aagttcatga ccatggtgct gggcattggg 180
cccgtcctgg gcctcgtgtg cgtgcctctc ctcggcagcg cttccgacca ttggcgcggc 240
```

```
cggtatggcc gcaggagacc cttcatctgg gctctgagtc tcggcatcct gctgagcctg 300
ttcctgatcc ctcgggccgg ctggctggcc gggctgctgt gccccgatcc tcggcccctg 360
gagctggccc tgctgatcct cggcgtgggc ctgctggact tctgcggcca ggtgtgcttc 420
acgcccctgg aggcactgct gagcgacctg ttccgggacc ccgaccattg ccgccaggcg 480
tacagcgtgt acgccttcat gatctccctg ggaggctgcc tgggctacct gctccccgcc 540
atcgattggg acaccagcgc actcgccccc tatctcggaa cacaggagga atgcctgttc 600
ggattgttga cgctcatctt cctcacgtgc gtcgcggcca ccctgttggt ggccgaggag 660
gccgccctgg ggcccaccga gccggccgag ggactgagcg ccccgagcct gagtccacac 720
tgctgccctt gccgggcccg cctggccttc cgtaatctgg gcgccctcct gcctcggctc 780
catcagctgt gttgcagaat gcctaggacg ctgcggcgcc tgttcgtcgc tgagttgtgc 840
tcctggatgg ctctcatgac cttcaccctg ttttatacgg acttcgtcgg ggagggcctg 900
taccaggggg tgccgcgcgc cgagcccggg acagaggcgc gccgccacta cgacgaggga 960
gtgcgtatgg gctccctggg cctcttcttg cagtgcgcca tcagtctggt tttctctctg 1020
gtcatggaca ggctggtgca gcgcttcgga acccgggcgg tgtacctggc gagcgtggcc 1080
gccttccccg tggctgccgg cgccacctgc ctctctcact cggtggccgt ggtcaccgcc 1140
agcgccgccc tgaccgggtt caccttctct gccctgcaga ttctgcctta caccctggcc 1200
agcctgtacc atcgcgagaa acaggtgttt ctccccaagt acagaggcga caccgggggc 1260
gcctccagcg aggacagcct catgacctcc ttcctgcctg gccccaagcc cggcgcccct 1320
ttccccaacg ggcacgtggg cgccggcggg agtgggctcc tgcccccccc tcctgcgctg 1380
tgcggggcca gcgcctgcga cgtgagcgtg cgcgtggtgg tgggcgagcc caccgaggcc 1440
cgcgtggtgc cgggcagagg catttgtctg gacctggcca tcctcgactc cgccttcctc 1500
ctcagccagg tggccccgtc cctcttcatg ggctctatcg tccagctgtc tcagagcgtc 1560
accgcttaca tggtgtccgc tgctggactg ggcttggtgg ctatttattt cgccacccag 1620
gtggtgttcg acaagagcga cctggccaaa tactccgcct ga                   1662
```

<210> 20
<211> 1662
<212> DNA
<213> Artificial Sequence

<220>
<223> Codon optimised human P501S

<400> 20

atggtgcagc ggctgtgggt gtcccggctg ctgcgccata gaaaggccca gttgctgctg 60
gtgaacctgc tgactttcgg actggaggtg tgcctggctg ccgggatcac gtacgtgccc 120
cccctgctgc tggaggtggg cgtggaggag aagttcatga caatggtgct gggcatcggc 180
cccgtcctgg gcctcgtgtg tgtgcccctc ctcgggagtg cgtccgatca ttggcggggc 240
cgctacggcc gccgcagacc gttcatctgg gccctgagcc tggggatcct gctctctctc 300
ttcctgatcc cccgggccgg ctggctggcc ggcctgctgt gtcccgaccc ccgccctctg 360
gagctggccc tcctgatcct gggcgtgggc ttgttggact tctgcggcca ggtgtgtttc 420
actcccctgg aggctctgct ctccgacctc ttccgcgacc ccgaccactg taggcaggct 480
tacagcgtgt acgccttcat gatcagtctg gggggatgcc tgggctatct gctgcccgct 540
atcgactggg acaccagcgc cctggccccc tacctgggga ctcaggagga gtgcctgttc 600
ggcctgctca ccttgatctt cctgacgtgc gtcgccgcca ccctgctggt ggccgaggag 660
gcggccctgg ggcccaccga gcccgccgag ggcctgagcg ctcccagcct gagcccccat 720
tgctgcccgt gcagggctag gctcgccttc aggaatctgg gcgctttgct gccccgcctg 780
catcagctgt gctgtcgcat gcctcgcacc ctgcgccgcc tgttcgtcgc tgagctctgt 840
tcctggatgg ccctgatgac gttcaccctc ttctacaccg acttcgtggg ggagggcctg 900
taccagggcg tgcccagggc cgagcccggc accgaggcta ggcgccatta cgacgagggc 960
gtcaggatgg gctctctggg cctcttcctg cagtgcgcca tcagtctggt gttctctctg 1020
gtgatggacc ggctggtgca gcgcttcggc acccgggccg tgtacctcgc ctctgtggcg 1080
gctttccccg tcgccgccgg cgcgacctgc ctgtctcatt ctgtcgccgt ggtgaccgcc 1140
agcgccgccc tgaccggctt caccttcagt gcgctccaga ttctgcccta caccctggcg 1200
tctctgtacc atcgcgagaa gcaggtgttc ctgcccaagt accgcgggga cacaggggga 1260
gcttcctctg aggacagcct gatgaccagc ttcttgcccg gccccaagcc gggggcccct 1320
ttccccaacg gccatgtcgg ggcgggcggc agcggcctgc tccctccccc ccccgccctg 1380
tgcggcgcta gtgcctgcga cgtgagcgtg cgggtggtgg tgggggagcc caccgaggct 1440
agggtcgtgc ctggccgggg gatctgcctg gacctggcca tcctcgactc cgccttcctg 1500
ctctcccagg tggcgcccag cctgttcatg ggcagtatcg tgcagctgag ccagagcgtg 1560
accgcctaca tggtgagcgc cgccggcctg gggttggtgg ccatctactt tgccacccag 1620

gtcgtgttcg acaagagcga tctcgccaag tatagcgcct ga 1662

<210> 21
<211> 1688
<212> DNA
<213> Artificial Sequence

<220>
<223> Codon optimised human P501S

<400> 21

```
gacggctagc gccaccatgg tgcagcggct ctgggtgagc cgcctcctgc ggcatcgcaa 60
ggcccagctc ctgctggtga atctgctcac attcggcctg gaggtgtgcc tggccgccgg 120
catcacctac gtgccccccc tcctgctgga ggtgggagtc gaggagaagt tcatgaccat 180
ggtgctgggc attgggcccg tcctgggcct cgtgtgcgtg cctctcctcg gcagcgcttc 240
cgaccattgg cgcggccggt atggccgcag gagacccttc atctgggctc tgagtctcgg 300
catcctgctg agcctgttcc tgatccctcg ggccggctgg ctggccgggc tgctgtgccc 360
cgatcctcgg cccctggagc tggccctgct gatcctcggc gtgggcctgc tggacttctg 420
cggccaggtg tgcttcacgc ccctggaggc actgctgagc gacctgttcc gggaccccga 480
ccattgccgc caggcgtaca gcgtgtacgc cttcatgatc tccctgggag gctgcctggg 540
ctacctgctc cccgccatcg attgggacac cagcgcactc gcccccctatc tcggaacaca 600
ggaggaatgc ctgttcggac tgctgacgct catcttcctc acgtgcgtcg cggccaccct 660
gttggtggcc gaggaggccg ccctggggcc caccgagccg gccgagggac tgagcgcccc 720
gagcctgagt ccacactgct gcccttgccg ggcccgcctg gccttccgta atctgggcgc 780
cctcctgcct cggctccatc agctgtgttg cagaatgcct aggacgctgc ggcgcctgtt 840
cgtcgctgag ttgtgctcct ggatggctct catgaccttc accctgtttt atacggactt 900
cgtcggggag ggcctgtacc agggggtgcc gcgcgccgag cccgggacag aggcgcgccg 960
ccactacgac gagggagtgc gtatgggctc cctgggcctc ttcttgcagt gcgccatcag 1020
tctggttttc tctctggtca tggacaggct ggtgcagcgc ttcggaaccc gggcggtgta 1080
cctggcgagc gtggccgcct tccccgtggc tgccggcgcc acctgcctct ctcactcggt 1140
ggccgtggtc accgccagcg ccgccctgac cgggttcacc ttctctgccc tgcagattct 1200
gccttacacc ctggccagcc tgtaccatcg cgagaaacag gtgtttctcc ccaagtacag 1260
aggcgacacc gggggcgcct ccagcgagga cagcctcatg acctccttcc tgcctggccc 1320
caagcccggc gcccctttcc ccaacgggca cgtgggcgcc ggcgggagtg ggctcctgcc 1380
ccccccctcct gcgctgtgcg gggccagcgc ctgcgacgtg agcgtgcgcg tggtggtggg 1440
cgagcccacc gaggcccgcg tggtgccggg cagaggcatt tgtctggacc tggccatcct 1500
cgactccgcc ttcctcctca gccaggtggc cccgtccctc ttcatgggct ctatcgtcca 1560
gctgtctcag agcgtcaccg cttacatggt gtccgctgct ggactgggct tggtggctat 1620
ttatttcgcc acccaggtgg tgttcgacaa gagcgacctg gccaaatact ccgcctgact 1680
cgaggcag                                                          1688
```

<210> 22
<211> 1688
<212> DNA
<213> Artificial Sequence

<220>
<223> Codon optimised human P501S

<400> 22

```
gacggctagc gccaccatgg tgcagcggct gtgggtgtcc cggctgctgc gccatagaaa 60
ggcccagttg ctgctggtga acctgctgac tttcggactg gaggtgtgcc tggctgccgg 120
gatcacgtac gtgccccccc tgctgctgga ggtgggcgtg gaggagaagt tcatgacaat 180
ggtgctgggc atcggccccg tcctgggcct cgtgtgtgtg cccctcctcg ggagtgcgtc 240
cgatcattgg cggggccgct acggccgccg cagaccgttc atctgggccc tgagcctggg 300
catcctgctc tctctcttcc tgatcccccg ggccggctgg ctggccggcc tgctgtgtcc 360
cgacccccgc cctctggagc tggccctcct gatcctgggc gtgggcctgc tggacttctg 420
cggccaggtg tgtttcactc ccctggaggc tctgctctcc gacctcttcc gcgaccccga 480
ccactgtagg caggcttaca gcgtgtacgc cttcatgatc agtctggggg gatgcctggg 540
ctatctgctg cccgctatcg actgggacac cagcgccctg gcccccctacc tggggactca 600
ggaggagtgc ctgttcggcc tgctcacctt gatcttcctg acgtgcgtcg ccgccaccct 660
```

```
gctggtggcc gaggaggcgg ccctggggcc caccgagccc gccgagggcc tgagcgctcc 720
cagcctgagc ccccattgct gcccgtgcag ggctaggctc gccttcagga atctgggcgc 780
tttgctgccc cgcctgcatc agctgtgctg tcgcatgcct cgcaccctgc gccgcctgtt 840
cgtcgctgag ctctgttcct ggatggccct gatgacgttc accctcttct acaccgactt 900
cgtgggggag ggcctgtacc agggcgtgcc cagggccgag cccggcaccg aggctaggcg 960
ccattacgac gagggcgtca ggatgggctc tctgggcctc ttcctgcagt gcgccatcag 1020
tctggtgttc tctctggtga tggaccggct ggtgcagcgc ttcggcaccc gggccgtgta 1080
cctcgcctct gtggcggctt tccccgtcgc cgccggcgcg acctgcctgt ctcattctgt 1140
cgccgtggtg accgccagcg ccgccctgac cggcttcacc ttcagtgcgc tccagattct 1200
gccctacacc ctggcgtctc tgtaccatcg cgagaagcag gtgttcctgc ccaagtaccg 1260
cggggacaca gggggagctt cctctgagga cagcctgatg accagcttct tgcccggccc 1320
caagccgggg gcccctttcc ccaacggcca tgtcggggcg ggcggcagcg gcctgctccc 1380
tcccccccc gccctgtgcg gcgctagtgc ctgcgacgtg agcgtgcggg tggtggtggg 1440
ggagcccacc gaggctaggg tcgtgcctgg ccgggggatc tgcctggacc tggccatcct 1500
cgactccgcc ttcctgctct cccaggtggc gcccagcctg ttcatgggca gtatcgtgca 1560
gctgagccag agcgtgaccg cctacatggt gagcgccgcc ggcctggggt tggtggccat 1620
ctactttgcc acccaggtcg tgttcgacaa gagcgatctc gccaagtata gcgcctgact 1680
cgaggcag                                                          1688
```

<210> 23
<211> 435
<212> DNA
<213> Artificial Sequence

<220>
<223> Hybrid gene between St. pneum. C-LytA, P2 T helper epitope and a small portion of the 5' end of human P501S

<400> 23

```
atggcggccg cttacgtaca ttccgacggc tcttatccaa aagacaagtt tgagaaaatc 60
aatggcactt ggtactactt tgacagttca ggctatatgc ttgcagaccg ctggaggaag 120
cacacagacg gcaactggta ctggttcgac aactcaggcg aaatggctac aggctggaag 180
aaaatcgctg ataagtggta ctatttcaac gaagaaggtg ccatgaagac aggctgggtc 240
aagtacaagg acacttggta ctacttagac gctaaagaag gcgccatgca atacatcaag 300
gctaactcta agttcattgg tatcactgaa ggcgtcatgg tatcaaatgc ctttatccag 360
tcagcggacg gaacaggctg gtactacctc aaaccagacg gaacactggc agacaggcca 420
gaaaagttca tgtac                                                  435
```

<210> 24
<211> 435
<212> DNA
<213> Artificial Sequence

<220>
<223> Hybrid gene between St. pneum. C-LytA, P2 T helper epitope and a small portion of the 5' end of human P501S - codon-optimised

<400> 24

```
atggccgccg cctacgtgca tagcgacggg agctacccca aggacaagtt cgagaagatc 60
aacgggacat ggtactactt cgactcctcc ggctacatgc tcgccgaccg ctggcggaag 120
cacaccgacg gcaactggta ctggttcgat aactcgggag agatggccac cggctggaag 180
aagatcgcgg acaagtggta ctatttcaac gaggagggcg ccatgaagac cggctgggtg 240
aagtataagg acacctggta ctacctcgac gccaaggagg gcgccatgca gtatatcaag 300
gccaacagca agttcatcgg catcaccgag ggagtgatgg tcagcaacgc ctttatccag 360
agcgccgacg gcaccggatg gtactacttg aagccggacg gcaccctcgc ggatcggccc 420
gagaagttca tgtac                                                  435
```

<210> 25
<211> 435
<212> DNA
<213> Artificial Sequence

<220>
<223> Hybrid gene between St. pneum. C-LytA, P2 T helper epitope and a small portion of the 5' end of human P501S - codon-optimised

<400> 25

```
atggccgccg cctacgtgca cagcgacggg tcctacccaa aggacaagtt cgagaagatc 60
aacggcacgt ggtactattt cgacagcagc ggctacatgc tcgccgatcg ctggcgcaag 120
cacaccgacg ggaactggta ctggttcgac aactctggcg agatggctac ggggtggaag 180
aagatcgccg acaagtggta ctacttcaac gaggagggcg ccatgaagac cgggtgggtg 240
aagtacaagg acacctggta ctacctggac gctaaggagg gcgccatgca gtacatcaag 300
gccaactcga agttcatcgg gatcaccgag ggcgtgatgg tcagtaacgc tttcatccag 360
agcgcggacg gcacaggctg gtattacctg aagcccgatg gcaccctggc ggacagacct 420
gagaaattca tgtac                                                  435
```

<210> 26
<211> 464
<212> DNA
<213> Artificial Sequence

<220>
<223> Hybrid gene between St. pneum. C-LytA, P2 T helper epitope and a small portion of the 5' end of human P501S - codon-optimised

<400> 26

```
gacggctagc gccaccatgg ccgccgccta cgtgcatagc gacgggagct accccaagga 60
caagttcgag aagatcaacg ggacatggta ctacttcgac tcctccggct acatgctcgc 120
cgaccgctgg cggaagcaca ccgacggcaa ctggtactgg ttcgataact cgggagagat 180
ggccaccggc tggaagaaga tcgcggacaa gtggtactat ttcaacgagg agggcgccat 240
gaagaccggc tgggtgaagt ataaggacac ctggtactac ctcgacgcca aggagggcgc 300
catgcagtat atcaaggcca acagcaagtt catcggcatc accgagggag tgatggtcag 360
caacgccttt atccagagcg ccgacggcac cggatggtac tacttgaagc cggacggcac 420
cctcgcggat cggcccgaga agttcatgta ctgactcgag gcag                  464
```

<210> 27
<211> 652
<212> PRT
<213> Artificial Sequence

<220>

<223> Hybrid protein between St. pneum. C-LytA, P2 T helper epitope and amino acids 51-553 of human P501S

<400> 27

```
Met Ala Ala Ala Tyr Val His Ser Asp Gly Ser Tyr Pro Lys Asp Lys
 1               5                  10                  15
Phe Glu Lys Ile Asn Gly Thr Trp Tyr Tyr Phe Asp Ser Ser Gly Tyr
            20                  25                  30
Met Leu Ala Asp Arg Trp Arg Lys His Thr Asp Gly Asn Trp Tyr Trp
        35                  40                  45
Phe Asp Asn Ser Gly Glu Met Ala Thr Gly Trp Lys Lys Ile Ala Asp
    50                  55                  60
Lys Trp Tyr Tyr Phe Asn Glu Glu Gly Ala Met Lys Thr Gly Trp Val
65                  70                  75                  80
Lys Tyr Lys Asp Thr Trp Tyr Tyr Leu Asp Ala Lys Glu Gly Ala Met
                85                  90                  95
```

```
Gln Tyr Ile Lys Ala Asn Ser Lys Phe Ile Gly Ile Thr Glu Gly Val
            100                 105                 110
Met Val Ser Asn Ala Phe Ile Gln Ser Ala Asp Gly Thr Gly Trp Tyr
        115                 120                 125
Tyr Leu Lys Pro Asp Gly Thr Leu Ala Asp Arg Pro Glu Lys Phe Met
    130                 135                 140
Tyr Met Val Leu Gly Ile Gly Pro Val Leu Gly Leu Val Cys Val Pro
145                 150                 155                 160
Leu Leu Gly Ser Ala Ser Asp His Trp Arg Gly Arg Tyr Gly Arg Arg
                165                 170                 175
Arg Pro Phe Ile Trp Ala Leu Ser Leu Gly Ile Leu Leu Ser Leu Phe
            180                 185                 190
Leu Ile Pro Arg Ala Gly Trp Leu Ala Gly Leu Leu Cys Pro Asp Pro
        195                 200                 205
Arg Pro Leu Glu Leu Ala Leu Leu Ile Leu Gly Val Gly Leu Leu Asp
    210                 215                 220
Phe Cys Gly Gln Val Cys Phe Thr Pro Leu Glu Ala Leu Leu Ser Asp
225                 230                 235                 240
Leu Phe Arg Asp Pro Asp His Cys Arg Gln Ala Tyr Ser Val Tyr Ala
                245                 250                 255
Phe Met Ile Ser Leu Gly Gly Cys Leu Gly Tyr Leu Leu Pro Ala Ile
            260                 265                 270
Asp Trp Asp Thr Ser Ala Leu Ala Pro Tyr Leu Gly Thr Gln Glu Glu
        275                 280                 285
Cys Leu Phe Gly Leu Leu Thr Leu Ile Phe Leu Thr Cys Val Ala Ala
    290                 295                 300
Thr Leu Leu Val Ala Glu Glu Ala Ala Leu Gly Pro Thr Glu Pro Ala
305                 310                 315                 320
Glu Gly Leu Ser Ala Pro Ser Leu Ser Pro His Cys Cys Pro Cys Arg
                325                 330                 335
Ala Arg Leu Ala Phe Arg Asn Leu Gly Ala Leu Leu Pro Arg Leu His
            340                 345                 350
Gln Leu Cys Cys Arg Met Pro Arg Thr Leu Arg Arg Leu Phe Val Ala
        355                 360                 365
Glu Leu Cys Ser Trp Met Ala Leu Met Thr Phe Thr Leu Phe Tyr Thr
    370                 375                 380
Asp Phe Val Gly Glu Gly Leu Tyr Gln Gly Val Pro Arg Ala Glu Pro
385                 390                 395                 400
Gly Thr Glu Ala Arg Arg His Tyr Asp Glu Gly Val Arg Met Gly Ser
                405                 410                 415
Leu Gly Leu Phe Leu Gln Cys Ala Ile Ser Leu Val Phe Ser Leu Val
            420                 425                 430
Met Asp Arg Leu Val Gln Arg Phe Gly Thr Arg Ala Val Tyr Leu Ala
        435                 440                 445
Ser Val Ala Ala Phe Pro Val Ala Ala Gly Ala Thr Cys Leu Ser His
    450                 455                 460
Ser Val Ala Val Val Thr Ala Ser Ala Ala Leu Thr Gly Phe Thr Phe
465                 470                 475                 480
Ser Ala Leu Gln Ile Leu Pro Tyr Thr Leu Ala Ser Leu Tyr His Arg
                485                 490                 495
Glu Lys Gln Val Phe Leu Pro Lys Tyr Arg Gly Asp Thr Gly Gly Ala
            500                 505                 510
Ser Ser Glu Asp Ser Leu Met Thr Ser Phe Leu Pro Gly Pro Lys Pro
        515                 520                 525
Gly Ala Pro Phe Pro Asn Gly His Val Gly Ala Gly Gly Ser Gly Leu
    530                 535                 540
Leu Pro Pro Pro Pro Ala Leu Cys Gly Ala Ser Ala Cys Asp Val Ser
545                 550                 555                 560
Val Arg Val Val Val Gly Glu Pro Thr Glu Ala Arg Val Val Pro Gly
                565                 570                 575
Arg Gly Ile Cys Leu Asp Leu Ala Ile Leu Asp Ser Ala Phe Leu Leu
```

```
                  580                     585                     590
Ser Gln Val Ala Pro Ser Leu Phe Met Gly Ser Ile Val Gln Leu Ser
            595                     600                     605
Gln Ser Val Thr Ala Tyr Met Val Ser Ala Ala Gly Leu Gly Leu Val
        610                     615                     620
Ala Ile Tyr Phe Ala Thr Gln Val Val Phe Asp Lys Ser Asp Leu Ala
625                     630                     635                     640
Lys Tyr Ser Ala Gly Gly His His His His His His
                    645                     650
```

<210> 28
<211> 1959
<212> DNA
<213> Artificial Sequence

<220>
<223> DNA encoding the Hybrid protein between St. pneum. C-LytA, P2 T helper epitope and amino acids 51-553 of human P501S

<400> 28

```
atggcggccg cttacgtaca ttccgacggc tcttatccaa aagacaagtt tgagaaaatc 60
aatggcactt ggtactactt tgacagttca ggctatatgc ttgcagaccg ctggaggaag 120
cacacagacg gcaactggta ctggttcgac aactcaggcg aaatggctac aggctggaag 180
aaaatcgctg ataagtggta ctatttcaac gaagaaggtg ccatgaagac aggctgggtc 240
aagtacaagg acacttggta ctacttagac gctaaagaag gcgccatgca atacatcaag 300
gctaactcta agttcattgg tatcactgaa ggcgtcatgg tatcaaatgc ctttatccag 360
tcagcggacg gaacaggctg gtactacctc aaaccagacg gaacactggc agacaggcca 420
gaaaagttca tgtacatggt gctgggcatt ggtccagtgc tgggcctggt ctgtgtcccg 480
ctcctaggct cagccagtga ccactggcgt ggacgctatg gccgccgccg gcccttcatc 540
tgggcactgt ccttgggcat cctgctgagc ctctttctca tcccaagggc cggctggcta 600
gcagggctgc tgtgcccgga tcccaggccc ctggagctgg cactgctcat cctgggcgtg 660
gggctgctgg acttctgtgg ccaggtgtgc ttcactccac tggaggccct gctctctgac 720
ctcttccggg acccggacca ctgtcgccag gcctactctg tctatgcctt catgatcagt 780
cttgggggct gcctgggcta cctcctgcct gccattgact gggacaccag tgccctggcc 840
ccctacctgg gcacccagga ggagtgcctc tttggcctgc tcaccctcat cttcctcacc 900
tgcgtagcag ccacactgct ggtggctgag gaggcagcgc tgggccccac cgagccagca 960
gaagggctgt cggcccccctc cttgtcgccc cactgctgtc catgccgggc ccgcttggct 1020
ttccggaacc tgggcgccct gcttccccgg ctgcaccagc tgtgctgccg catgccccgc 1080
accctgcgcc ggctcttcgt ggctgagctg tgcagctgga tggcactcat gaccttcacg 1140
ctgttttaca cggatttcgt gggcgagggg ctgtaccagg gcgtgcccag agctgagccg 1200
ggcaccgagg cccggagaca ctatgatgaa ggcgttcgga tgggcagcct ggggctgttc 1260
ctgcagtgcg ccatctccct ggtcttctct ctggtcatgg accggctggt gcagcgattc 1320
ggcactcgag cagtctattt ggccagtgtg gcagctttcc ctgtggctgc cggtgccaca 1380
tgcctgtccc acagtgtggc cgtggtgaca gcttcagccg ccctcaccgg gttcaccttc 1440
tcagccctgc agatcctgcc ctacacactg gcctccctct accaccggga gaagcaggtg 1500
ttcctgccca aataccgagg ggacactgga ggtgctagca gtgaggacag cctgatgacc 1560
agcttcctgc caggccctaa gcctggagct cccttcccta atggacacgt gggtgctgga 1620
ggcagtggcc tgctcccacc tccacccgcg ctctgcgggg cctctgcctg tgatgtctcc 1680
gtacgtgtgg tggtgggtga gcccaccgag gccagggtgg ttccgggccg gggcatctgc 1740
ctggacctcg ccatcctgga tagtgccttc ctgctgtccc aggtggcccc atccctgttt 1800
atgggctcca ttgtccagct cagccagtct gtcactgcct atatggtgtc tgccgcaggc 1860
ctgggtctgg tcgccattta ctttgctaca caggtagtat ttgacaagag cgacttggcc 1920
aaatactcag cgggtggaca ccatcaccat caccattaa                        1959
```

<210> 29

<211> 507
<212> PRT
<213> Artificial Sequence

<220>
<223> Human P501S (amino acids 55-553) fused to 6 histidine residues

<400> 29

```
Met Val Leu Gly Ile Gly Pro Val Leu Gly Leu Val Cys Val Pro Leu
 1               5                  10                  15
Leu Gly Ser Ala Ser Asp His Trp Arg Gly Arg Tyr Gly Arg Arg Arg
            20                  25                  30
Pro Phe Ile Trp Ala Leu Ser Leu Gly Ile Leu Leu Ser Leu Phe Leu
        35                  40                  45
Ile Pro Arg Ala Gly Trp Leu Ala Gly Leu Leu Cys Pro Asp Pro Arg
    50                  55                  60
Pro Leu Glu Leu Ala Leu Leu Ile Leu Gly Val Gly Leu Leu Asp Phe
65                  70                  75                  80
Cys Gly Gln Val Cys Phe Thr Pro Leu Glu Ala Leu Leu Ser Asp Leu
                85                  90                  95
Phe Arg Asp Pro Asp His Cys Arg Gln Ala Tyr Ser Val Tyr Ala Phe
            100                 105                 110
Met Ile Ser Leu Gly Gly Cys Leu Gly Tyr Leu Leu Pro Ala Ile Asp
        115                 120                 125
Trp Asp Thr Ser Ala Leu Ala Pro Tyr Leu Gly Thr Gln Glu Glu Cys
    130                 135                 140
Leu Phe Gly Leu Leu Thr Leu Ile Phe Leu Thr Cys Val Ala Ala Thr
145                 150                 155                 160
Leu Leu Val Ala Glu Glu Ala Ala Leu Gly Pro Thr Glu Pro Ala Glu
                165                 170                 175
Gly Leu Ser Ala Pro Ser Leu Ser Pro His Cys Cys Pro Cys Arg Ala
            180                 185                 190
Arg Leu Ala Phe Arg Asn Leu Gly Ala Leu Leu Pro Arg Leu His Gln
        195                 200                 205
Leu Cys Cys Arg Met Pro Arg Thr Leu Arg Arg Leu Phe Val Ala Glu
    210                 215                 220
Leu Cys Ser Trp Met Ala Leu Met Thr Phe Thr Leu Phe Tyr Thr Asp
225                 230                 235                 240
Phe Val Gly Glu Gly Leu Tyr Gln Gly Val Pro Arg Ala Glu Pro Gly
                245                 250                 255
Thr Glu Ala Arg Arg His Tyr Asp Glu Gly Val Arg Met Gly Ser Leu
            260                 265                 270
Gly Leu Phe Leu Gln Cys Ala Ile Ser Leu Val Phe Ser Leu Val Met
        275                 280                 285
Asp Arg Leu Val Gln Arg Phe Gly Thr Arg Ala Val Tyr Leu Ala Ser
    290                 295                 300
Val Ala Ala Phe Pro Val Ala Ala Gly Ala Thr Cys Leu Ser His Ser
305                 310                 315                 320
Val Ala Val Val Thr Ala Ser Ala Ala Leu Thr Gly Phe Thr Phe Ser
                325                 330                 335
Ala Leu Gln Ile Leu Pro Tyr Thr Leu Ala Ser Leu Tyr His Arg Glu
            340                 345                 350
Lys Gln Val Phe Leu Pro Lys Tyr Arg Gly Asp Thr Gly Gly Ala Ser
        355                 360                 365
Ser Glu Asp Ser Leu Met Thr Ser Phe Leu Pro Gly Pro Lys Pro Gly
    370                 375                 380
Ala Pro Phe Pro Asn Gly His Val Gly Ala Gly Gly Ser Gly Leu Leu
385                 390                 395                 400
Pro Pro Pro Pro Ala Leu Cys Gly Ala Ser Ala Cys Asp Val Ser Val
                405                 410                 415
Arg Val Val Val Gly Glu Pro Thr Glu Ala Arg Val Val Pro Gly Arg
            420                 425                 430
Gly Ile Cys Leu Asp Leu Ala Ile Leu Asp Ser Ala Phe Leu Leu Ser
        435                 440                 445
```

```
Gln Val Ala Pro Ser Leu Phe Met Gly Ser Ile Val Gln Leu Ser Gln
    450                 455                 460
Ser Val Thr Ala Tyr Met Val Ser Ala Ala Gly Leu Gly Leu Val Ala
465                 470                 475                 480
Ile Tyr Phe Ala Thr Gln Val Val Phe Asp Lys Ser Asp Leu Ala Lys
                485                 490                 495
Tyr Ser Ala Gly Gly His His His His His His
            500                 505
```

<210> 30
<211> 1524
<212> DNA
<213> Artificial Sequence

<220>
<223> DNA encoding Human P501S (amino acids 55-553) fused to 6 histidine residues

<400> 30

```
atggtgctgg gcattggtcc agtgctgggc ctggtctgtg tcccgctcct aggctcagcc 60
agtgaccact ggcgtggacg ctatggccgc cgccggccct tcatctgggc actgtccttg 120
ggcatcctgc tgagcctctt tctcatccca agggccggct ggctagcagg gctgctgtgc 180
ccggatccca ggcccctgga gctggcactg ctcatcctgg gcgtggggct gctggacttc 240
tgtggccagg tgtgcttcac tccactggag gccctgctct ctgacctctt ccgggacccg 300
gaccactgtc gccaggccta ctctgtctat gccttcatga tcagtcttgg gggctgcctg 360
ggctacctcc tgcctgccat tgactgggac accagtgccc tggcccccta cctgggcacc 420
caggaggagt gcctctttgg cctgctcacc ctcatcttcc tcacctgcgt agcagccaca 480
ctgctggtgg ctgaggaggc agcgctgggc cccaccgagc cagcagaagg gctgtcggcc 540
ccctccttgt cgccccactg ctgtccatgc cgggcccgct tggctttccg gaacctgggc 600
gccctgcttc cccggctgca ccagctgtgc tgccgcatgc cccgcaccct gcgccggctc 660
ttcgtggctg agctgtgcag ctggatggca ctcatgacct tcacgctgtt ttacacggat 720
ttcgtgggcg aggggctgta ccagggcgtg cccagagctg agccgggcac cgaggcccgg 780
agacactatg atgaaggcgt tcggatgggc agcctggggc tgttcctgca gtgcgccatc 840
tccctggtct tctctctggt catggaccgg ctggtgcagc gattcggcac tcgagcagtc 900
tatttggcca gtgtggcagc tttccctgtg gctgccggtg ccacatgcct gtcccacagt 960
gtggccgtgg tgacagcttc agccgccctc accgggttca ccttctcagc cctgcagatc 1020
ctgccctaca cactggcctc cctctaccac cgggagaagc aggtgttcct gcccaaatac 1080
cgaggggaca ctggaggtgc tagcagtgag gacagcctga tgaccagctt cctgccaggc 1140
cctaagcctg gagctccctt ccctaatgga cacgtgggtg ctggaggcag tggcctgctc 1200
ccacctccac ccgcgctctg cggggcctct gcctgtgatg tctccgtacg tgtggtggtg 1260
ggtgagccca ccgaggccag ggtggttccg ggccggggca tctgcctgga cctcgccatc 1320
ctggatagtg ccttcctgct gtcccaggtg gccccatccc tgtttatggg ctccattgtc 1380
cagctcagcc agtctgtcac tgcctatatg gtgtctgccg caggcctggg tctggtcgcc 1440
atttactttg ctacacaggt agtatttgac aagagcgact tggccaaata ctcagcgggt 1500
ggacaccatc accatcacca ttaa                                        1524
```

<210> 31
<211> 685
<212> PRT
<213> Artificial Sequence

<220>
<223> Human P501S (amino acids 1-34 fused to 55-553) fused to 6 histidine residues

<400> 31

```
Met Ala Ala Val Gln Arg Leu Trp Val Ser Arg Leu Leu Arg His Arg
 1              5                   10                  15
Lys Ala Gln Leu Leu Leu Val Asn Leu Leu Thr Phe Gly Leu Glu Val
            20                  25                  30
```

```
Cys Leu Ala Ala Ala Tyr Val His Ser Asp Gly Ser Tyr Pro Lys Asp
        35                  40                  45
Lys Phe Glu Lys Ile Asn Gly Thr Trp Tyr Tyr Phe Asp Ser Ser Gly
    50                  55                  60
Tyr Met Leu Ala Asp Arg Trp Arg Lys His Thr Asp Gly Asn Trp Tyr
65                  70                  75                  80
Trp Phe Asp Asn Ser Gly Glu Met Ala Thr Gly Trp Lys Lys Ile Ala
                85                  90                  95
Asp Lys Trp Tyr Tyr Phe Asn Glu Glu Gly Ala Met Lys Thr Gly Trp
            100                 105                 110
Val Lys Tyr Lys Asp Thr Trp Tyr Tyr Leu Asp Ala Lys Glu Gly Ala
        115                 120                 125
Met Gln Tyr Ile Lys Ala Asn Ser Lys Phe Ile Gly Ile Thr Glu Gly
    130                 135                 140
Val Met Val Ser Asn Ala Phe Ile Gln Ser Ala Asp Gly Thr Gly Trp
145                 150                 155                 160
Tyr Tyr Leu Lys Pro Asp Gly Thr Leu Ala Asp Arg Pro Glu Lys Phe
                165                 170                 175
Met Tyr Met Val Leu Gly Ile Gly Pro Val Leu Gly Leu Val Cys Val
            180                 185                 190
Pro Leu Leu Gly Ser Ala Ser Asp His Trp Arg Gly Arg Tyr Gly Arg
        195                 200                 205
Arg Arg Pro Phe Ile Trp Ala Leu Ser Leu Gly Ile Leu Leu Ser Leu
    210                 215                 220
Phe Leu Ile Pro Arg Ala Gly Trp Leu Ala Gly Leu Leu Cys Pro Asp
225                 230                 235                 240
Pro Arg Pro Leu Glu Leu Ala Leu Leu Ile Leu Gly Val Gly Leu Leu
                245                 250                 255
Asp Phe Cys Gly Gln Val Cys Phe Thr Pro Leu Glu Ala Leu Leu Ser
            260                 265                 270
Asp Leu Phe Arg Asp Pro Asp His Cys Arg Gln Ala Tyr Ser Val Tyr
        275                 280                 285
Ala Phe Met Ile Ser Leu Gly Gly Cys Leu Gly Tyr Leu Leu Pro Ala
    290                 295                 300
Ile Asp Trp Asp Thr Ser Ala Leu Ala Pro Tyr Leu Gly Thr Gln Glu
305                 310                 315                 320
Glu Cys Leu Phe Gly Leu Leu Thr Leu Ile Phe Leu Thr Cys Val Ala
                325                 330                 335
Ala Thr Leu Leu Val Ala Glu Glu Ala Ala Leu Gly Pro Thr Glu Pro
            340                 345                 350
Ala Glu Gly Leu Ser Ala Pro Ser Leu Ser Pro His Cys Cys Pro Cys
        355                 360                 365
Arg Ala Arg Leu Ala Phe Arg Asn Leu Gly Ala Leu Leu Pro Arg Leu
    370                 375                 380
His Gln Leu Cys Cys Arg Met Pro Arg Thr Leu Arg Arg Leu Phe Val
385                 390                 395                 400
Ala Glu Leu Cys Ser Trp Met Ala Leu Met Thr Phe Thr Leu Phe Tyr
                405                 410                 415
Thr Asp Phe Val Gly Glu Gly Leu Tyr Gln Gly Val Pro Arg Ala Glu
            420                 425                 430
Pro Gly Thr Glu Ala Arg Arg His Tyr Asp Glu Gly Val Arg Met Gly
        435                 440                 445
Ser Leu Gly Leu Phe Leu Gln Cys Ala Ile Ser Leu Val Phe Ser Leu
    450                 455                 460
Val Met Asp Arg Leu Val Gln Arg Phe Gly Thr Arg Ala Val Tyr Leu
465                 470                 475                 480
Ala Ser Val Ala Ala Phe Pro Val Ala Ala Gly Ala Thr Cys Leu Ser
                485                 490                 495
His Ser Val Ala Val Val Thr Ala Ser Ala Ala Leu Thr Gly Phe Thr
            500                 505                 510
Phe Ser Ala Leu Gln Ile Leu Pro Tyr Thr Leu Ala Ser Leu Tyr His
```

```
        515                 520                 525
Arg Glu Lys Gln Val Phe Leu Pro Lys Tyr Arg Gly Asp Thr Gly Gly
    530                 535                 540
Ala Ser Ser Glu Asp Ser Leu Met Thr Ser Phe Leu Pro Gly Pro Lys
545                 550                 555                 560
Pro Gly Ala Pro Phe Pro Asn Gly His Val Gly Ala Gly Gly Ser Gly
                565                 570                 575
Leu Leu Pro Pro Pro Pro Ala Leu Cys Gly Ala Ser Ala Cys Asp Val
            580                 585                 590
Ser Val Arg Val Val Val Gly Glu Pro Thr Glu Ala Arg Val Val Pro
        595                 600                 605
Gly Arg Gly Ile Cys Leu Asp Leu Ala Ile Leu Asp Ser Ala Phe Leu
    610                 615                 620
Leu Ser Gln Val Ala Pro Ser Leu Phe Met Gly Ser Ile Val Gln Leu
625                 630                 635                 640
Ser Gln Ser Val Thr Ala Tyr Met Val Ser Ala Ala Gly Leu Gly Leu
                645                 650                 655
Val Ala Ile Tyr Phe Ala Thr Gln Val Val Phe Asp Lys Ser Asp Leu
            660                 665                 670
Ala Lys Tyr Ser Ala Gly Gly His His His His His His
        675                 680                 685
```

<210> 32
<211> 2058
<212> DNA
<213> Artificial Sequence

<220>
<223> DNA encoding Human P501S (amino acids 1-34 fused to 55-553) fused to 6 histidine residues

<400> 32

```
atggcggccg tgcagaggct atgggtatcg agactgctaa gacaccgcaa agctcagttg 60
ttgttggtta acttgttgac cttcgggctg gaagtctgtt tggcggccgc ttacgtacat 120
tccgacggct cttatccaaa agacaagttt gagaaaatca atggcacttg gtactacttt 180
gacagttcag gctatatgct tgcagaccgc tggaggaagc acacagacgg caactggtac 240
tggttcgaca actcaggcga aatggctaca ggctggaaga aaatcgctga taagtggtac 300
tatttcaacg aagaaggtgc catgaagaca ggctgggtca agtacaagga cacttggtac 360
tacttagacg ctaaagaagg cgccatgcaa tacatcaagg ctaactctaa gttcattggt 420
atcactgaag gcgtcatggt atcaaatgcc tttatccagt cagcggacgg aacaggctgg 480
tactacctca aaccagacgg aacactggca gacaggccag aaaagttcat gtacatggtg 540
ctgggcattg gtccagtgct gggcctggtc tgtgtcccgc tcctaggctc agccagtgac 600
cactggcgtg gacgctatgg ccgccgccgg cccttcatct gggcactgtc cttgggcatc 660
ctgctgagcc tctttctcat cccaagggcc ggctggctag cagggctgct gtgcccggat 720
cccaggcccc tggagctggc actgctcatc ctgggcgtgg ggctgctgga cttctgtggc 780
caggtgtgct tcactccact ggaggccctg ctctctgacc tcttccggga cccggaccac 840
tgtcgccagg cctactctgt ctatgccttc atgatcagtc ttgggggctg cctgggctac 900
ctcctgcctg ccattgactg ggacaccagt gccctggccc cctacctggg cacccaggag 960
gagtgcctct ttggcctgct caccctcatc ttcctcacct gcgtagcagc cacactgctg 1020
gtggctgagg aggcagcgct gggccccacc gagccagcag aagggctgtc ggccccctcc 1080
ttgtcgcccc actgctgtcc atgccgggcc cgcttggctt tccggaacct gggcgccctg 1140
cttccccggc tgcaccagct gtgctgccgc atgccccgca ccctgcgccg gctcttcgtg 1200
gctgagctgt gcagctggat ggcactcatg accttcacgc tgttttacac ggatttcgtg 1260
ggcgaggggc tgtaccaggg cgtgcccaga gctgagccgg gcaccgaggc ccggagacac 1320
tatgatgaag gcgttcggat gggcagcctg gggctgttcc tgcagtgcgc catctccctg 1380
gtcttctctc tggtcatgga ccggctggtg cagcgattcg gcactcgagc agtctatttg 1440
gccagtgtgg cagctttccc tgtggctgcc ggtgccacat gcctgtccca cagtgtggcc 1500
gtggtgacag cttcagccgc cctcaccggg ttcaccttct cagccctgca gatcctgccc 1560
tacacactgg cctccctcta ccaccgggag aagcaggtgt tcctgcccaa ataccgaggg 1620
gacactggag gtgctagcag tgaggacagc ctgatgacca gcttcctgcc aggccctaag 1680

cctggagctc ccttccctaa tggacacgtg ggtgctggag gcagtggcct gctcccacct 1740
ccacccgcgc tctgcggggc ctctgcctgt gatgtctccg tacgtgtggt ggtgggtgag 1800
cccaccgagg ccagggtggt tccgggccgg ggcatctgcc tggacctcgc catcctggat 1860
agtgccttcc tgctgtccca ggtggcccca tccctgttta tgggctccat tgtccagctc 1920
agccagtctg tcactgccta tatggtgtct gccgcaggcc tgggtctggt cgccatttac 1980
tttgctacac aggtagtatt tgacaagagc gacttggcca aatactcagc gggtggacac 2040
catcaccatc accattaa                                               2058
```

<210> 33
<211> 671
<212> PRT
<213> Artificial Sequence

<220>
<223> St. pneum. C-LytA portion fused to P2 T helper epitope fused to Human P501S (amino acids 55-553) fused to 6 histidine residues downstream of yeast alphaprepro signal sequence

<400> 33

```
Met Ala Ala Arg Phe Pro Ser Ile Phe Thr Ala Val Leu Phe Ala Ala
 1               5                  10                  15
Ser Ser Ala Leu Ala Ala Ala Tyr Val His Ser Asp Gly Ser Tyr Pro
            20                  25                  30
Lys Asp Lys Phe Glu Lys Ile Asn Gly Thr Trp Tyr Tyr Phe Asp Ser
        35                  40                  45
Ser Gly Tyr Met Leu Ala Asp Arg Trp Arg Lys His Thr Asp Gly Asn
    50                  55                  60
Trp Tyr Trp Phe Asp Asn Ser Gly Glu Met Ala Thr Gly Trp Lys Lys
65                  70                  75                  80
Ile Ala Asp Lys Trp Tyr Tyr Phe Asn Glu Glu Gly Ala Met Lys Thr
                85                  90                  95
Gly Trp Val Lys Tyr Lys Asp Thr Trp Tyr Tyr Leu Asp Ala Lys Glu
            100                 105                 110
Gly Ala Met Gln Tyr Ile Lys Ala Asn Ser Lys Phe Ile Gly Ile Thr
        115                 120                 125
Glu Gly Val Met Val Ser Asn Ala Phe Ile Gln Ser Ala Asp Gly Thr
    130                 135                 140
Gly Trp Tyr Tyr Leu Lys Pro Asp Gly Thr Leu Ala Asp Arg Pro Glu
145                 150                 155                 160
Lys Phe Met Tyr Met Val Leu Gly Ile Gly Pro Val Leu Gly Leu Val
                165                 170                 175
Cys Val Pro Leu Leu Gly Ser Ala Ser Asp His Trp Arg Gly Arg Tyr
            180                 185                 190
Gly Arg Arg Arg Pro Phe Ile Trp Ala Leu Ser Leu Gly Ile Leu Leu
        195                 200                 205
Ser Leu Phe Leu Ile Pro Arg Ala Gly Trp Leu Ala Gly Leu Leu Cys
    210                 215                 220
Pro Asp Pro Arg Pro Leu Glu Leu Ala Leu Leu Ile Leu Gly Val Gly
225                 230                 235                 240
Leu Leu Asp Phe Cys Gly Gln Val Cys Phe Thr Pro Leu Glu Ala Leu
                245                 250                 255
Leu Ser Asp Leu Phe Arg Asp Pro Asp His Cys Arg Gln Ala Tyr Ser
            260                 265                 270
Val Tyr Ala Phe Met Ile Ser Leu Gly Gly Cys Leu Gly Tyr Leu Leu
        275                 280                 285
Pro Ala Ile Asp Trp Asp Thr Ser Ala Leu Ala Pro Tyr Leu Gly Thr
    290                 295                 300
Gln Glu Glu Cys Leu Phe Gly Leu Leu Thr Leu Ile Phe Leu Thr Cys
305                 310                 315                 320
Val Ala Ala Thr Leu Leu Val Ala Glu Glu Ala Ala Leu Gly Pro Thr
```

```
                        325                 330                 335
Glu Pro Ala Glu Gly Leu Ser Ala Pro Ser Leu Ser Pro His Cys Cys
            340                 345                 350
Pro Cys Arg Ala Arg Leu Ala Phe Arg Asn Leu Gly Ala Leu Leu Pro
        355                 360                 365
Arg Leu His Gln Leu Cys Cys Arg Met Pro Arg Thr Leu Arg Arg Leu
    370                 375                 380
Phe Val Ala Glu Leu Cys Ser Trp Met Ala Leu Met Thr Phe Thr Leu
385                 390                 395                 400
Phe Tyr Thr Asp Phe Val Gly Glu Gly Leu Tyr Gln Gly Val Pro Arg
                405                 410                 415
Ala Glu Pro Gly Thr Glu Ala Arg Arg His Tyr Asp Glu Gly Val Arg
            420                 425                 430
Met Gly Ser Leu Gly Leu Phe Leu Gln Cys Ala Ile Ser Leu Val Phe
        435                 440                 445
Ser Leu Val Met Asp Arg Leu Val Gln Arg Phe Gly Thr Arg Ala Val
    450                 455                 460
Tyr Leu Ala Ser Val Ala Ala Phe Pro Val Ala Ala Gly Ala Thr Cys
465                 470                 475                 480
Leu Ser His Ser Val Ala Val Val Thr Ala Ser Ala Ala Leu Thr Gly
                485                 490                 495
Phe Thr Phe Ser Ala Leu Gln Ile Leu Pro Tyr Thr Leu Ala Ser Leu
            500                 505                 510
Tyr His Arg Glu Lys Gln Val Phe Leu Pro Lys Tyr Arg Gly Asp Thr
        515                 520                 525
Gly Gly Ala Ser Ser Glu Asp Ser Leu Met Thr Ser Phe Leu Pro Gly
    530                 535                 540
Pro Lys Pro Gly Ala Pro Phe Pro Asn Gly His Val Gly Ala Gly Gly
545                 550                 555                 560
Ser Gly Leu Leu Pro Pro Pro Pro Ala Leu Cys Gly Ala Ser Ala Cys
                565                 570                 575
Asp Val Ser Val Arg Val Val Val Gly Glu Pro Thr Glu Ala Arg Val
            580                 585                 590
Val Pro Gly Arg Gly Ile Cys Leu Asp Leu Ala Ile Leu Asp Ser Ala
        595                 600                 605
Phe Leu Leu Ser Gln Val Ala Pro Ser Leu Phe Met Gly Ser Ile Val
    610                 615                 620
Gln Leu Ser Gln Ser Val Thr Ala Tyr Met Val Ser Ala Ala Gly Leu
625                 630                 635                 640
Gly Leu Val Ala Ile Tyr Phe Ala Thr Gln Val Val Phe Asp Lys Ser
                645                 650                 655
Asp Leu Ala Lys Tyr Ser Ala Gly Gly His His His His His His
            660                 665                 670
```

<210> 34
<211> 2477
<212> DNA
<213> Artificial Sequence

<220>
<223> DNA encoding St. pneum. C-LytA portion fused to P2 T helper epitope fused to Human P501S (amino acids 55-553) fused to 6 histidine residues downstream of yeast alphaprepro signal sequence

<400> 34

```
tacgtacatt ccgacggctc ttatccaaaa gacaagtttg agaaaatcaa tggcacttgg 60
tactactttg acagttcagg ctatatgctt gcagaccgct ggaggaagca cacagacggc 120
aactggtact ggttcgacaa ctcaggcgaa atggctacag gctggaagaa aatcgctgat 180
aagtggtact atttcaacga agaaggtgcc atgaagacag gctgggtcaa gtacaaggac 240

acttggtact acttagacgc taaagaaggc gccatgcaat acatcaaggc taactctaag 300
ttcattggta tcactgaagg cgtcatggta tcaaatgcct ttatccagtc agcggacgga 360
acaggctggt actacctcaa accagacgga acactggcag acaggccaga aatggcggcc 420
agatttcctt caatttttac tgcagtttta ttcgcagcat cctccgcatt agcggccgct 480
tacgtacatt ccgacggctc ttatccaaaa gacaagtttg agaaaatcaa tggcacttgg 540
tactactttg acagttcagg ctatatgctt gcagaccgct ggaggaagca cacagacggc 600
aactggtact ggttcgacaa ctcaggcgaa atggctacag gctggaagaa aatcgctgat 660
aagtggtact atttcaacga agaaggtgcc atgaagacag gctgggtcaa gtacaaggac 720
acttggtact acttagacgc taaagaaggc gccatgcaat acatcaaggc taactctaag 780
ttcattggta tcactgaagg cgtcatggta tcaaatgcct ttatccagtc agcggacgga 840
acaggctggt actacctcaa accagacgga acactggcag acaggccaga agctggtatt 900
acttacgttc caccattgtt gttggaagtt ggtgttgaag aaaagttcat gtacatggtg 960
ctgggcattg gtccagtgct gggcctggtc tgtgtcccgc tcctaggctc agccagtgac 1020
cactggcgtg gacgctatgg ccgccgccgg cccttcatct gggcactgtc cttgggcatc 1080
ctgctgagcc tctttctcat cccaagggcc ggctggctag cagggctgct gtgcccggat 1140
cccaggcccc tggagctggc actgctcatc ctgggcgtgg ggctgctgga cttctgtggc 1200
caggtgtgct tcactccact ggaggccctg ctctctgacc tcttccggga cccggaccac 1260
tgtcgccagg cctactctgt ctatgcttca tgatcagtct tggggctgc ctgggctacc 1320
tcctgcctgc cattgactgg gacaccagtg ccctggcccc ctacctgggc acccaggagg 1380
agtgcctctt tggcctgctc accctcatct tcctcacctg cgtagcagcc acactgctgg 1440
tggctgagga ggcagcgctg ggccccaccg agccagcaga agggctgtcg gccccctcct 1500
tgtcgcccca ctgctgtcca tgccgggccc gcttggcttt ccggaacctg ggcgccctgc 1560
ttccccggct gcaccagctg tgctgccgca tgccccgcac cctgcgccgg ctcttcgtgg 1620
ctgagctgtg cagctggatg gcactcatga ccttcacgct gttttacacg gatttcgtgg 1680
gcgaggggct gtaccagggc gtgcccagag ctgagccggg caccgaggcc cggagacact 1740
atgatgaagg cgttcggatg ggcagcctgg ggctgttcct gcagtgcgcc atctccctgg 1800
tcttctctct ggtcatggac cggctggtgc agcgattcgg cactcgagca gtctatttgg 1860
ccagtgtggc agctttccct gtggctgccg gtgccacatg cctgtcccac agtgtggccg 1920
tggtgacagc ttcagccgcc ctcaccgggt tcaccttctc agccctgcag atcctgccct 1980
acacactggc ctccctctac caccgggaga agcaggtgtt cctgcccaaa taccgagggg 2040
acactggagg tgctagcagt gaggacagcc tgatgaccag cttcctgcca ggccctaagc 2100
ctggagctcc cttccctaat ggacacgtgg gtgctggagg cagtggcctg ctcccacctc 2160
cacccgcgct ctgcggggcc tctgcctgtg atgtctccgt acgtgtggtg gtgggtgagc 2220
ccaccgaggc cagggtggtt ccgggccggg gcatctgcct ggacctcgcc atcctggata 2280
gtgccttcct gctgtcccag gtggccccat ccctgtttat gggctccatt gtccagctca 2340
gccagtctgt cactgcctat atggtgtctg ccgcaggcct gggtctggtc gccatttact 2400
ttgctacaca ggtagtattt gacaagagcg acttggccaa atactcagcg ggtggacacc 2460
atcaccatca ccattaa                                                2477
```

<210> 35
<211> 595
<212> PRT
<213> Artificial Sequence

<220>
<223> Human P501S (amino acids 55-553) fused to 6 histidine residues downstream of yeast alphaprepro signal sequence

<400> 35

```
Met Ser Phe Leu Asn Phe Thr Ala Val Leu Phe Ala Ala Ser Ser Ala
 1               5                  10                  15
Leu Ala Ala Pro Val Asn Thr Thr Thr Glu Asp Glu Thr Ala Gln Ile
            20                  25                  30
Pro Ala Glu Ala Val Ile Gly Tyr Ser Asp Leu Glu Gly Asp Phe Asp
        35                  40                  45
Val Ala Val Leu Pro Phe Ser Asn Ser Thr Asn Asn Gly Leu Leu Phe
    50                  55                  60
Ile Asn Thr Thr Ile Ala Ser Ile Ala Ala Lys Glu Glu Gly Val Ser
65                  70                  75                  80
Leu Glu Lys Arg Glu Ala Glu Ala Met Val Leu Gly Ile Gly Pro Val
```

85 90 95
Leu Gly Leu Val Cys Val Pro Leu Leu Gly Ser Ala Ser Asp His Trp
100 105 110
Arg Gly Arg Tyr Gly Arg Arg Arg Pro Phe Ile Trp Ala Leu Ser Leu
115 120 125
Gly Ile Leu Leu Ser Leu Phe Leu Ile Pro Arg Ala Gly Trp Leu Ala
130 135 140
Gly Leu Leu Cys Pro Asp Pro Arg Pro Leu Glu Leu Ala Leu Leu Ile
145 150 155 160
Leu Gly Val Gly Leu Leu Asp Phe Cys Gly Gln Val Cys Phe Thr Pro
165 170 175
Leu Glu Ala Leu Leu Ser Asp Leu Phe Arg Asp Pro Asp His Cys Arg
180 185 190
Gln Ala Tyr Ser Val Tyr Ala Phe Met Ile Ser Leu Gly Gly Cys Leu
195 200 205
Gly Tyr Leu Leu Pro Ala Ile Asp Trp Asp Thr Ser Ala Leu Ala Pro
210 215 220
Tyr Leu Gly Thr Gln Glu Glu Cys Leu Phe Gly Leu Leu Thr Leu Ile
225 230 235 240
Phe Leu Thr Cys Val Ala Ala Thr Leu Leu Val Ala Glu Glu Ala Ala
245 250 255
Leu Gly Pro Thr Glu Pro Ala Glu Gly Leu Ser Ala Pro Ser Leu Ser
260 265 270
Pro His Cys Cys Pro Cys Arg Ala Arg Leu Ala Phe Arg Asn Leu Gly
275 280 285
Ala Leu Leu Pro Arg Leu His Gln Leu Cys Cys Arg Met Pro Arg Thr
290 295 300
Leu Arg Arg Leu Phe Val Ala Glu Leu Cys Ser Trp Met Ala Leu Met
305 310 315 320
Thr Phe Thr Leu Phe Tyr Thr Asp Phe Val Gly Glu Gly Leu Tyr Gln
325 330 335
Gly Val Pro Arg Ala Glu Pro Gly Thr Glu Ala Arg Arg His Tyr Asp
340 345 350
Glu Gly Val Arg Met Gly Ser Leu Gly Leu Phe Leu Gln Cys Ala Ile
355 360 365
Ser Leu Val Phe Ser Leu Val Met Asp Arg Leu Val Gln Arg Phe Gly
370 375 380
Thr Arg Ala Val Tyr Leu Ala Ser Val Ala Ala Phe Pro Val Ala Ala
385 390 395 400
Gly Ala Thr Cys Leu Ser His Ser Val Ala Val Val Thr Ala Ser Ala
405 410 415
Ala Leu Thr Gly Phe Thr Phe Ser Ala Leu Gln Ile Leu Pro Tyr Thr
420 425 430
Leu Ala Ser Leu Tyr His Arg Glu Lys Gln Val Phe Leu Pro Lys Tyr
435 440 445
Arg Gly Asp Thr Gly Gly Ala Ser Ser Glu Asp Ser Leu Met Thr Ser
450 455 460
Phe Leu Pro Gly Pro Lys Pro Gly Ala Pro Phe Pro Asn Gly His Val
465 470 475 480
Gly Ala Gly Gly Ser Gly Leu Leu Pro Pro Pro Pro Ala Leu Cys Gly
485 490 495
Ala Ser Ala Cys Asp Val Ser Val Arg Val Val Val Gly Glu Pro Thr
500 505 510
Glu Ala Arg Val Val Pro Gly Arg Gly Ile Cys Leu Asp Leu Ala Ile
515 520 525
Leu Asp Ser Ala Phe Leu Leu Ser Gln Val Ala Pro Ser Leu Phe Met
530 535 540
Gly Ser Ile Val Gln Leu Ser Gln Ser Val Thr Ala Tyr Met Val Ser
545 550 555 560
Ala Ala Gly Leu Gly Leu Val Ala Ile Tyr Phe Ala Thr Gln Val Val
565 570 575

Phe Asp Lys Ser Asp Leu Ala Lys Tyr Ser Ala Gly Gly His His His
580 585 590
His His His
595

<210> 36
<211> 1788
<212> DNA
<213> Artificial Sequence

<220>
<223> DNA encoding Human P501S (amino acids 55-553) fused to 6 histidine residues downstream of yeast alphaprepro signal sequence

<400> 36

```
atgagtttcc tcaattttac tgcagtttta ttcgcagcat cctccgcatt agctgctcca 60
gtcaacacta caacagaaga tgaaacggca caaattccgg ctgaagctgt catcggttac 120
tcagatttag aaggggattt cgatgttgct gttttgccat tttccaacag cacaaataac 180
gggttattgt ttataaatac tactattgcc agcattgctg ctaaagaaga aggggtatct 240
ctcgagaaaa gagaggctga agccatggtg ctgggcattg gtccagtgct gggcctggtc 300
tgtgtcccgc tcctaggctc agccagtgac cactggcgtg gacgctatgg ccgccgccgg 360
cccttcatct gggcactgtc cttgggcatc ctgctgagcc tctttctcat cccaagggcc 420
ggctggctag cagggctgct gtgcccggat cccaggcccc tggagctggc actgctcatc 480
ctgggcgtgg ggctgctgga cttctgtggc caggtgtgct tcactccact ggaggccctg 540
ctctctgacc tcttccggga cccggaccac tgtcgccagg cctactctgt ctatgccttc 600
atgatcagtc ttgggggctg cctgggctac ctcctgcctg ccattgactg ggacaccagt 660
gccctggccc cctacctggg cacccaggag gagtgcctct ttggcctgct caccctcatc 720
ttcctcacct gcgtagcagc cacactgctg gtggctgagg aggcagcgct gggccccacc 780
gagccagcag aagggctgtc ggccccctcc ttgtcgcccc actgctgtcc atgccgggcc 840
cgcttggctt tccggaacct gggcgccctg cttccccggc tgcaccagct gtgctgccgc 900
atgccccgca ccctgcgccg gctcttcgtg gctgagctgt gcagctggat ggcactcatg 960
accttcacgc tgttttacac ggatttcgtg ggcgaggggc tgtaccaggg cgtgcccaga 1020
gctgagccgg gcaccgaggc ccggagacac tatgatgaag gcgttcggat gggcagcctg 1080
gggctgttcc tgcagtgcgc catctccctg gtcttctctc tggtcatgga ccggctggtg 1140
cagcgattcg gcactcgagc agtctatttg gccagtgtgg cagctttccc tgtggctgcc 1200
ggtgccacat gcctgtccca cagtgtggcc gtggtgacag cttcagccgc cctcaccggg 1260
ttcaccttct cagccctgca gatcctgccc tacacactgg cctccctcta ccaccgggag 1320
aagcaggtgt tcctgcccaa ataccgaggg gacactggag gtgctagcag tgaggacagc 1380
ctgatgacca gcttcctgcc aggccctaag cctggagctc ccttccctaa tggacacgtg 1440
ggtgctggag gcagtggcct gctcccacct ccacccgcgc tctgcggggc ctctgcctgt 1500
gatgtctccg tacgtgtggt ggtgggtgag cccaccgagg ccagggtggt tccgggccgg 1560
ggcatctgcc tggacctcgc catcctggat agtgccttcc tgctgtccca ggtggcccca 1620
tccctgttta tgggctccat tgtccagctc agccagtctg tcactgccta tatggtgtct 1680
gccgcaggcc tgggtctggt cgccatttac tttgctacac aggtagtatt tgacaagagc 1740
gacttggcca aatactcagc gggtggacac catcaccatc accattaa 1788
```

<210> 37
<211> 1955
<212> DNA
<213> Artificial Sequence

<220>
<223> DNA encoding codon-optimised Human P501S (amino acids 51-553) fused to St.pneum. C-LytA P2 helper epitope C-Lyta

<400> 37

```
gcggccgcgc caccatggcc gccgcctacg tgcatagcga cgggagctac cccaaggaca 60
agttcgagaa gatcaacggg acatggtact acttcgactc ctccggctac atgctcgccg 120
```

```
accgctggcg gaagcacacc gacggcaact ggtactggtt cgataactcg ggagagatgg 180
ccaccggctg gaagaagatc gcggacaagt ggtactattt caacgaggag ggcgccatga 240
agaccggctg ggtgaagtat aaggacacct ggtactacct cgacgccaag gagggcgcca 300
tgcagtatat caaggccaac agcaagttca tcggcatcac cgagggagtg atggtcagca 360
acgcctttat ccagagcgcc gacggcaccg gatggtacta cttgaagccg gacggcaccc 420
tcgcggatcg gcccgagaag ttcatgtaca tggtgctggg catcggcccc gtcctgggcc 480
tcgtgtgtgt gcccctcctc gggagtgcgt ccgatcattg gcggggccgc tacggccgcc 540
gcagaccgtt catctgggcc ctgagcctgg gcatcctgct ctctctcttc ctgatccccc 600
gggccggctg gctggccggc ctgctgtgtc ccgacccccg ccctctggag ctggccctcc 660
tgatcctggg cgtgggcctg ctggacttct gcggccaggt gtgtttcact cccctggagg 720
ctctgctctc cgacctcttc cgcgaccccg accactgtag gcaggcttac agcgtgtacg 780
ccttcatgat cagtctgggg ggatgcctgg gctatctgct gcccgctatc gactgggaca 840
ccagcgccct ggccccctac ctggggactc aggaggagtg cctgttcggc ctgctcacct 900
tgatcttcct gacgtgcgtc gccgccaccc tgctggtggc cgaggaggcg gccctggggc 960
ccaccgagcc cgccgagggc ctgagcgctc ccagcctgag cccccattgc tgcccgtgca 1020
gggctaggct cgccttcagg aatctgggcg ctttgctgcc ccgcctgcat cagctgtgct 1080
gtcgcatgcc tcgcaccctg cgccgcctgt tcgtcgctga gctctgttcc tggatggccc 1140
tgatgacgtt caccctcttc tacaccgact tcgtggggga gggcctgtac cagggcgtgc 1200
ccagggccga gcccggcacc gaggctaggc gccattacga cgagggcgtc aggatggggct 1260
ctctgggcct cttcctgcag tgcgccatca gtctggtgtt ctctctggtg atggaccggc 1320
tggtgcagcg cttcggcacc cgggccgtgt acctcgcctc tgtggcggct ttccccgtcg 1380
ccgccggcgc gacctgcctg tctcattctg tcgccgtggt gaccgccagc gccgccctga 1440
ccggcttcac cttcagtgcg ctccagattc tgccctacac cctggcgtct ctgtaccatc 1500
gcgagaagca ggtgttcctg cccaagtacc gcggggacac agggggagct tcctctgagg 1560
acagcctgat gaccagcttc ttgcccggcc ccaagccggg ggcccctttc cccaacggcc 1620
atgtcggggc gggcggcagc ggcctgctcc ctcccccccc cgccctgtgc ggcgctagtg 1680
cctgcgacgt gagcgtgcgg gtggtggtgg gggagcccac cgaggctagg gtcgtgcctg 1740
gccgggggat ctgcctggac ctggccatcc tcgactccgc cttcctgctc tcccaggtgg 1800
cgcccagcct gttcatgggc agtatcgtgc agctgagcca gagcgtgacc gcctacatgg 1860
tgagcgccgc cggcctgggg ttggtggcca tctactttgc cacccaggtc gtgttcgaca 1920
agagcgatct cgccaagtat agcgcctgag gatcc                            1955
```

<210> 38
<211> 2045
<212> DNA
<213> Artificial Sequence

<220>
<223> DNA encoding codon-optimised Human P501S (amino acids 1-553) fused to St.pneum. C-LytA P2 helper epitope C-Lyta

<400> 38

```
gcggccgcgc caccatggcc gccgcctacg tgcatagcga cgggagctac cccaaggaca 60
agttcgagaa gatcaacggg acatggtact acttcgactc ctccggctac atgctcgccg 120
accgctggcg gaagcacacc gacggcaact ggtactggtt cgataactcg ggagagatgg 180
ccaccggctg gaagaagatc gcggacaagt ggtactattt caacgaggag ggcgccatga 240
agaccggctg ggtgaagtat aaggacacct ggtactacct cgacgccaag gagggcgcca 300
tgcagtatat caaggccaac agcaagttca tcggcatcac cgagggagtg atggtcagca 360
acgcctttat ccagagcgcc gacggcaccg gatggtacta cttgaagccg gacggcaccc 420
tcgcggatcg gcccgagatg gtgcagcggc tgtgggtgtc ccggctgctg cgccatagaa 480
aggcccagtt gctgctggtg aacctgctga ctttcggact ggaggtgtgc ctggctgccg 540
tggtgctggg catcggcccc gtcctgggcc tcgtgtgtgt gcccctcctc gggagtgcgt 600
ccgatcattg gcggggccgc tacggccgcc gcagaccgtt catctgggcc ctgagcctgg 660
gcatcctgct ctctctcttc ctgatccccc gggccggctg gctggccggc ctgctgtgtc 720
ccgacccccg ccctctggag ctggccctcc tgatcctggg cgtgggcctg ctggacttct 780
gcggccaggt gtgtttcact cccctggagg ctctgctctc cgacctcttc cgcgaccccg 840
accactgtag gcaggcttac agcgtgtacg ccttcatgat cagtctgggg ggatgcctgg 900
gctatctgct gcccgctatc gactgggaca ccagcgccct ggccccctac ctggggactc 960
aggaggagtg cctgttcggc ctgctcacct tgatcttcct gacgtgcgtc gccgccaccc 1020
tgctggtggc cgaggaggcg gccctggggc ccaccgagcc cgccgagggc ctgagcgctc 1080
ccagcctgag cccccattgc tgcccgtgca gggctaggct cgccttcagg aatctgggcg 1140

ctttgctgcc ccgcctgcat cagctgtgct gtcgcatgcc tcgcaccctg cgccgcctgt 1200
tcgtcgctga gctctgttcc tggatggccc tgatgacgtt caccctcttc tacaccgact 1260
tcgtgggggga gggcctgtac cagggcgtgc ccagggccga gcccggcacc gaggctaggc 1320
gccattacga cgagggcgtc aggatgggct ctctgggcct cttcctgcag tgcgccatca 1380
gtctggtgtt ctctctggtg atggaccggc tggtgcagcg cttcggcacc cgggccgtgt 1440
acctcgcctc tgtggcggct ttccccgtcg ccgccggcgc gacctgcctg tctcattctg 1500
tcgccgtggt gaccgccagc gccgccctga ccggcttcac cttcagtgcg ctccagattc 1560
tgccctacac cctggcgtct ctgtaccatc gcgagaagca ggtgttcctg cccaagtacc 1620
gcgggggacac agggggagct tcctctgagg acagcctgat gaccagcttc ttgcccggcc 1680
ccaagccggg ggcccctttc cccaacggcc atgtcggggc gggcggcagc ggcctgctcc 1740
ctcccccccc cgccctgtgc ggcgctagtg cctgcgacgt gagcgtgcgg gtggtggtgg 1800
gggagcccac cgaggctagg gtcgtgcctg gccgggggat ctgcctggac ctggccatcc 1860
tcgactccgc cttcctgctc tcccaggtgg cgcccagcct gttcatgggc agtatcgtgc 1920
agctgagcca gagcgtgacc gcctacatgg tgagcgccgc cggcctgggg ttggtggcca 1980
tctactttgc cacccaggtc gtgttcgaca agagcgatct cgccaagtat agcgcctgag 2040
gatcc                                                              2045
```

<210> 39

<211> 2105

<212> DNA

<213> Artificial Sequence

<220>

<223> DNA encoding St.pneum. C-LytA P2 helper epitope C-Lyta fused to Human P501S (amino acids 51-553) fused to Human P501S (amino acids 1-50) - Codon-optimised

<400> 39

```
gcggccgcgc caccatggcc gccgcctacg tgcatagcga cgggagctac cccaaggaca 60
agttcgagaa gatcaacggg acatggtact acttcgactc ctccggctac atgctcgccg 120
accgctggcg gaagcacacc gacggcaact ggtactggtt cgataactcg ggagagatgg 180
ccaccggctg gaagaagatc gcggacaagt ggtactattt caacgaggag ggcgccatga 240
agaccggctg ggtgaagtat aaggacacct ggtactacct cgacgccaag gagggcgcca 300
tgcagtatat caaggccaac agcaagttca tcggcatcac cgagggagtg atggtcagca 360
acgcctttat ccagagcgcc gacggcaccg gatggtacta cttgaagccg gacggcaccc 420
tcgcggatcg gcccgagaag ttcatgtaca tggtgctggg catcggcccc gtcctgggcc 480
tcgtgtgtgt gcccctcctc gggagtgcgt ccgatcattg gcggggccgc tacggccgcc 540
gcagaccgtt catctgggcc ctgagcctgg gcatcctgct ctctctcttc ctgatccccc 600
gggccggctg gctggccggc ctgctgtgtc ccgacccccg ccctctggag ctggccctcc 660
tgatcctggg cgtgggcctg ctggacttct gcggccaggt gtgtttcact cccctggagg 720
ctctgctctc cgacctcttc cgcgaccccg accactgtag gcaggcttac agcgtgtacg 780
ccttcatgat cagtctgggg ggatgcctgg gctatctgct gcccgctatc gactgggaca 840
ccagcgccct ggccccctac ctggggactc aggaggagtg cctgttcggc ctgctcacct 900
tgatcttcct gacgtgcgtc gccgccaccc tgctggtggc cgaggaggcg gccctggggc 960
ccaccgagcc cgccgagggc ctgagcgctc ccagcctgag cccccattgc tgcccgtgca 1020
gggctaggct cgccttcagg aatctgggcg ctttgctgcc ccgcctgcat cagctgtgct 1080
gtcgcatgcc tcgcaccctg cgccgcctgt tcgtcgctga gctctgttcc tggatggccc 1140
tgatgacgtt caccctcttc tacaccgact tcgtggggga gggcctgtac cagggcgtgc 1200
ccagggccga gcccggcacc gaggctaggc gccattacga cgagggcgtc aggatgggct 1260
ctctgggcct cttcctgcag tgcgccatca gtctggtgtt ctctctggtg atggaccggc 1320
tggtgcagcg cttcggcacc cgggccgtgt acctcgcctc tgtggcggct ttccccgtcg 1380
ccgccggcgc gacctgcctg tctcattctg tcgccgtggt gaccgccagc gccgccctga 1440
ccggcttcac cttcagtgcg ctccagattc tgccctacac cctggcgtct ctgtaccatc 1500
gcgagaagca ggtgttcctg cccaagtacc gcggggacac agggggagct tcctctgagg 1560
acagcctgat gaccagcttc ttgcccggcc ccaagccggg ggcccctttc cccaacggcc 1620
atgtcggggc gggcggcagc ggcctgctcc ctcccccccc cgccctgtgc ggcgctagtg 1680
cctgcgacgt gagcgtgcgg gtggtggtgg gggagcccac cgaggctagg gtcgtgcctg 1740
gccgggggat ctgcctggac ctggccatcc tcgactccgc cttcctgctc tcccaggtgg 1800
cgcccagcct gttcatgggc agtatcgtgc agctgagcca gagcgtgacc gcctacatgg 1860
tgagcgccgc cggcctgggg ttggtggcca tctactttgc cacccaggtc gtgttcgaca 1920

agagcgatct cgccaagtat agcgccatgg tgcagcggct gtgggtgtcc cggctgctgc 1980
gccatagaaa ggcccagttg ctgctggtga acctgctgac tttcggactg gaggtgtgcc 2040
tggctgccgg gatcacgtac gtgccccccc tgctgctgga ggtgggcgtg gaggagtgag 2100
gatcc                                                             2105
```

<210> 40
<211> 2105
<212> DNA
<213> Artificial Sequence

<220>
<223> DNA encoding Human P501S (amino acids 1-50) fused to St.pneum. C-LytA P2 helper epitope C-Lyta fused to Human P501S (amino acids 51-553) - Codon-optimised

<400> 40

```
gcggccgcgc caccatggtg cagcggctgt gggtgtcccg gctgctgcgc catagaaagg 60
cccagttgct gctggtgaac ctgctgactt tcggactgga ggtgtgcctg gctgccggga 120
tcacgtacgt gcccccccctg ctgctggagg tgggcgtgga ggagatggcc gccgcctacg 180
tgcatagcga cgggagctac cccaaggaca agttcgagaa gatcaacggg acatggtact 240
acttcgactc ctccggctac atgctcgccg accgctggcg gaagcacacc gacggcaact 300
ggtactggtt cgataactcg ggagagatgg ccaccggctg gaagaagatc gcggacaagt 360
ggtactattt caacgaggag ggcgccatga agaccggctg ggtgaagtat aaggacacct 420
ggtactacct cgacgccaag gagggcgcca tgcagtatat caaggccaac agcaagttca 480
tcggcatcac cgagggagtg atggtcagca acgcctttat ccagagcgcc gacggcaccg 540
gatggtacta cttgaagccg gacggcaccc tcgcggatcg gcccgagaag ttcatgtaca 600
tggtgctggg catcggcccc gtcctgggcc tcgtgtgtgt gcccctcctc gggagtgcgt 660
ccgatcattg gcggggccgc tacggccgcc gcagaccgtt catctgggcc ctgagcctgg 720
gcatcctgct ctctctcttc ctgatccccc gggccggctg gctggccggc ctgctgtgtc 780
ccgacccccg ccctctggag ctggccctcc tgatcctggg cgtgggcctg ctggacttct 840
gcggccaggt gtgtttcact cccctggagg ctctgctctc cgacctcttc cgcgaccccg 900
accactgtag gcaggcttac agcgtgtacg ccttcatgat cagtctgggg ggatgcctgg 960
gctatctgct gcccgctatc gactgggaca ccagcgccct ggccccctac ctggggactc 1020
aggaggagtg cctgttcggc ctgctcacct tgatcttcct gacgtgcgtc gccgccaccc 1080
tgctggtggc cgaggaggcg gccctggggc ccaccgagcc cgccgagggc ctgagcgctc 1140
ccagcctgag cccccattgc tgcccgtgca gggctaggct cgccttcagg aatctgggcg 1200
ctttgctgcc ccgcctgcat cagctgtgct gtcgcatgcc tcgcaccctg cgccgcctgt 1260
tcgtcgctga gctctgttcc tggatggccc tgatgacgtt caccctcttc tacaccgact 1320
tcgtggggga gggcctgtac cagggcgtgc ccagggccga gcccggcacc gaggctaggc 1380
gccattacga cgagggcgtc aggatgggct ctctgggcct cttcctgcag tgcgccatca 1440
gtctggtgtt ctctctggtg atggaccggc tggtgcagcg cttcggcacc cgggccgtgt 1500
acctcgcctc tgtggcggct ttccccgtcg ccgccggcgc gacctgcctg tctcattctg 1560
tcgccgtggt gaccgccagc gccgccctga ccggcttcac cttcagtgcg ctccagattc 1620
tgccctacac cctggcgtct ctgtaccatc gcgagaagca ggtgttcctg cccaagtacc 1680
gcggggacac agggggagct tcctctgagg acagcctgat gaccagcttc ttgcccggcc 1740
ccaagccggg ggcccctttc cccaacggcc atgtcggggc gggcggcagc ggcctgctcc 1800
ctcccccccc cgccctgtgc ggcgctagtg cctgcgacgt gagcgtgcgg gtggtggtgg 1860
gggagcccac cgaggctagg gtcgtgcctg gccgggggat ctgcctggac ctggccatcc 1920
tcgactccgc cttcctgctc tcccaggtgg cgcccagcct gttcatgggc agtatcgtgc 1980
agctgagcca gagcgtgacc gcctacatgg tgagcgccgc cggcctgggg ttggtggcca 2040
tctactttgc cacccaggtc gtgttcgaca agagcgatct cgccaagtat agcgcctgag 2100
gatcc                                                             2105
```

<210> 41
<211> 652
<212> PRT
<213> Artificial Sequence

<220>
<223> St.pneum. C-LytA P2 helper epitope C-Lyta fused to Human P501S

<400> 41

Met Ala Ala Ala Tyr Val His Ser Asp Gly Ser Tyr Pro Lys Asp Lys
1 5 10 15
Phe Glu Lys Ile Asn Gly Thr Trp Tyr Tyr Phe Asp Ser Ser Gly Tyr
20 25 30
Met Leu Ala Asp Arg Trp Arg Lys His Thr Asp Gly Asn Trp Tyr Trp
35 40 45
Phe Asp Asn Ser Gly Glu Met Ala Thr Gly Trp Lys Lys Ile Ala Asp
50 55 60
Lys Trp Tyr Tyr Phe Asn Glu Glu Gly Ala Met Lys Thr Gly Trp Val
65 70 75 80
Lys Tyr Lys Asp Thr Trp Tyr Tyr Leu Asp Ala Lys Glu Gly Ala Met
85 90 95
Gln Tyr Ile Lys Ala Asn Ser Lys Phe Ile Gly Ile Thr Glu Gly Val
100 105 110
Met Val Ser Asn Ala Phe Ile Gln Ser Ala Asp Gly Thr Gly Trp Tyr
115 120 125
Tyr Leu Lys Pro Asp Gly Thr Leu Ala Asp Arg Pro Glu Lys Phe Met
130 135 140
Tyr Met Val Leu Gly Ile Gly Pro Val Leu Gly Leu Val Cys Val Pro
145 150 155 160
Leu Leu Gly Ser Ala Ser Asp His Trp Arg Gly Arg Tyr Gly Arg Arg
165 170 175
Arg Pro Phe Ile Trp Ala Leu Ser Leu Gly Ile Leu Leu Ser Leu Phe
180 185 190
Leu Ile Pro Arg Ala Gly Trp Leu Ala Gly Leu Leu Cys Pro Asp Pro
195 200 205
Arg Pro Leu Glu Leu Ala Leu Leu Ile Leu Gly Val Gly Leu Leu Asp
210 215 220
Phe Cys Gly Gln Val Cys Phe Thr Pro Leu Glu Ala Leu Leu Ser Asp
225 230 235 240
Leu Phe Arg Asp Pro Asp His Cys Arg Gln Ala Tyr Ser Val Tyr Ala
245 250 255
Phe Met Ile Ser Leu Gly Gly Cys Leu Gly Tyr Leu Leu Pro Ala Ile
260 265 270
Asp Trp Asp Thr Ser Ala Leu Ala Pro Tyr Leu Gly Thr Gln Glu Glu
275 280 285
Cys Leu Phe Gly Leu Leu Thr Leu Ile Phe Leu Thr Cys Val Ala Ala
290 295 300
Thr Leu Leu Val Ala Glu Glu Ala Ala Leu Gly Pro Thr Glu Pro Ala
305 310 315 320
Glu Gly Leu Ser Ala Pro Ser Leu Ser Pro His Cys Cys Pro Cys Arg
325 330 335
Ala Arg Leu Ala Phe Arg Asn Leu Gly Ala Leu Leu Pro Arg Leu His
340 345 350
Gln Leu Cys Cys Arg Met Pro Arg Thr Leu Arg Arg Leu Phe Val Ala
355 360 365
Glu Leu Cys Ser Trp Met Ala Leu Met Thr Phe Thr Leu Phe Tyr Thr
370 375 380
Asp Phe Val Gly Glu Gly Leu Tyr Gln Gly Val Pro Arg Ala Glu Pro
385 390 395 400
Gly Thr Glu Ala Arg Arg His Tyr Asp Glu Gly Val Arg Met Gly Ser
405 410 415
Leu Gly Leu Phe Leu Gln Cys Ala Ile Ser Leu Val Phe Ser Leu Val
420 425 430
Met Asp Arg Leu Val Gln Arg Phe Gly Thr Arg Ala Val Tyr Leu Ala
435 440 445
Ser Val Ala Ala Phe Pro Val Ala Ala Gly Ala Thr Cys Leu Ser His
450 455 460

```
Ser Val Ala Val Val Thr Ala Ser Ala Ala Leu Thr Gly Phe Thr Phe
465                 470                 475                 480
Ser Ala Leu Gln Ile Leu Pro Tyr Thr Leu Ala Ser Leu Tyr His Arg
                485                 490                 495
Glu Lys Gln Val Phe Leu Pro Lys Tyr Arg Gly Asp Thr Gly Gly Ala
            500                 505                 510
Ser Ser Glu Asp Ser Leu Met Thr Ser Phe Leu Pro Gly Pro Lys Pro
        515                 520                 525
Gly Ala Pro Phe Pro Asn Gly His Val Gly Ala Gly Gly Ser Gly Leu
    530                 535                 540
Leu Pro Pro Pro Pro Ala Leu Cys Gly Ala Ser Ala Cys Asp Val Ser
545                 550                 555                 560
Val Arg Val Val Val Gly Glu Pro Thr Glu Ala Arg Val Val Pro Gly
                565                 570                 575
Arg Gly Ile Cys Leu Asp Leu Ala Ile Leu Asp Ser Ala Phe Leu Leu
            580                 585                 590
Ser Gln Val Ala Pro Ser Leu Phe Met Gly Ser Ile Val Gln Leu Ser
        595                 600                 605
Gln Ser Val Thr Ala Tyr Met Val Ser Ala Ala Gly Leu Gly Leu Val
    610                 615                 620
Ala Ile Tyr Phe Ala Thr Gln Val Val Phe Asp Lys Ser Asp Leu Ala
625                 630                 635                 640
Lys Tyr Ser Ala Gly Gly His His His His His His
                645                 650
```

<210> 42
<211> 1959
<212> DNA
<213> Artificial Sequence

<220>
<223> DNA encoding St.pneum. C-LytA P2 helper epitope C-Lyta fused to Human P501S (plus his tag)

<400> 42

```
atggcggccg cttacgtaca ttccgacggc tcttatccaa aagacaagtt tgagaaaatc 60
aatggcactt ggtactactt tgacagttca ggctatatgc ttgcagaccg ctggaggaag 120
cacacagacg gcaactggta ctggttcgac aactcaggcg aaatggctac aggctggaag 180
aaaatcgctg ataagtggta ctatttcaac gaagaaggtg ccatgaagac aggctgggtc 240
aagtacaagg acacttggta ctacttagac gctaaagaag gcgccatgca atacatcaag 300
gctaactcta agttcattgg tatcactgaa ggcgtcatgg tatcaaatgc ctttatccag 360
tcagcggacg gaacaggctg gtactacctc aaaccagacg gaacactggc agacaggcca 420
gaaaagttca tgtacatggt gctgggcatt ggtccagtgc tgggcctggt ctgtgtcccg 480
ctcctaggct cagccagtga ccactggcgt ggacgctatg gccgccgccg gcccttcatc 540
tgggcactgt ccttgggcat cctgctgagc ctctttctca tcccaagggc cggctggcta 600
gcagggctgc tgtgcccgga tcccaggccc ctggagctgg cactgctcat cctgggcgtg 660
gggctgctgg acttctgtgg ccaggtgtgc ttcactccac tggaggccct gctctctgac 720
ctcttccggg acccggacca ctgtcgccag gcctactctg tctatgcctt catgatcagt 780
cttgggggct gcctgggcta cctcctgcct gccattgact gggacaccag tgccctggcc 840
ccctacctgg gcacccagga ggagtgcctc tttggcctgc tcaccctcat cttcctcacc 900
tgcgtagcag ccacactgct ggtggctgag gaggcagcgc tgggccccac cgagccagca 960
gaagggctgt cggccccctc cttgtcgccc cactgctgtc catgccgggc ccgcttggct 1020
ttccggaacc tgggcgccct gcttccccgg ctgcaccagc tgtgctgccg catgccccgc 1080
accctgcgcc ggctcttcgt ggctgagctg tgcagctgga tggcactcat gaccttcacg 1140
ctgttttaca cggatttcgt gggcgagggg ctgtaccagg gcgtgcccag agctgagccg 1200
ggcaccgagg cccggagaca ctatgatgaa ggcgttcgga tgggcagcct ggggctgttc 1260
ctgcagtgcg ccatctccct ggtcttctct ctggtcatgg accggctggt gcagcgattc 1320
ggcactcgag cagtctattt ggccagtgtg gcagctttcc ctgtggctgc cggtgccaca 1380
tgcctgtccc acagtgtggc cgtggtgaca gcttcagccg ccctcaccgg gttcaccttc 1440
tcagccctgc agatcctgcc ctacacactg gcctccctct accaccggga gaagcaggtg 1500

ttcctgccca aataccgagg ggacactgga ggtgctagca gtgaggacag cctgatgacc 1560
agcttcctgc caggccctaa gcctggagct cccttcccta atggacacgt gggtgctgga 1620
ggcagtggcc tgctcccacc tccacccgcg ctctgcgggg cctctgcctg tgatgtctcc 1680
gtacgtgtgg tggtgggtga gcccaccgag gccagggtgg ttccgggccg gggcatctgc 1740
ctggacctcg ccatcctgga tagtgccttc ctgctgtccc aggtggcccc atccctgttt 1800
atgggctcca ttgtccagct cagccagtct gtcactgcct atatggtgtc tgccgcaggc 1860
ctgggtctgg tcgccattta ctttgctaca caggtagtat ttgacaagag cgacttggcc 1920
aaatactcag cgggtggaca ccatcaccat caccattaa                      1959
```

<210> 43
<211> 553
<212> PRT
<213> Homo sapiens

<400> 43

```
Met Val Gln Arg Leu Trp Val Ser Arg Leu Leu Arg His Arg Lys Ala
1               5                   10                  15
Gln Leu Leu Leu Val Asn Leu Leu Thr Phe Gly Leu Glu Val Cys Leu
            20                  25                  30
Ala Ala Gly Ile Thr Tyr Val Pro Pro Leu Leu Leu Glu Val Gly Val
        35                  40                  45
Glu Glu Lys Phe Met Thr Met Val Leu Gly Ile Gly Pro Val Leu Gly
    50                  55                  60
Leu Val Cys Val Pro Leu Leu Gly Ser Ala Ser Asp His Trp Arg Gly
65                  70                  75                  80
Arg Tyr Gly Arg Arg Arg Pro Phe Ile Trp Ala Leu Ser Leu Gly Ile
                85                  90                  95
Leu Leu Ser Leu Phe Leu Ile Pro Arg Ala Gly Trp Leu Ala Gly Leu
            100                 105                 110
Leu Cys Pro Asp Pro Arg Pro Leu Glu Leu Ala Leu Leu Ile Leu Gly
        115                 120                 125
Val Gly Leu Leu Asp Phe Cys Gly Gln Val Cys Phe Thr Pro Leu Glu
    130                 135                 140
Ala Leu Leu Ser Asp Leu Phe Arg Asp Pro Asp His Cys Arg Gln Ala
145                 150                 155                 160
Tyr Ser Val Tyr Ala Phe Met Ile Ser Leu Gly Gly Cys Leu Gly Tyr
                165                 170                 175
Leu Leu Pro Ala Ile Asp Trp Asp Thr Ser Ala Leu Ala Pro Tyr Leu
            180                 185                 190
Gly Thr Gln Glu Glu Cys Leu Phe Gly Leu Leu Thr Leu Ile Phe Leu
        195                 200                 205
Thr Cys Val Ala Ala Thr Leu Leu Val Ala Glu Glu Ala Ala Leu Gly
    210                 215                 220
Pro Thr Glu Pro Ala Glu Gly Leu Ser Ala Pro Ser Leu Ser Pro His
225                 230                 235                 240
Cys Cys Pro Cys Arg Ala Arg Leu Ala Phe Arg Asn Leu Gly Ala Leu
                245                 250                 255
Leu Pro Arg Leu His Gln Leu Cys Cys Arg Met Pro Arg Thr Leu Arg
            260                 265                 270
Arg Leu Phe Val Ala Glu Leu Cys Ser Trp Met Ala Leu Met Thr Phe
        275                 280                 285
Thr Leu Phe Tyr Thr Asp Phe Val Gly Glu Gly Leu Tyr Gln Gly Val
    290                 295                 300
Pro Arg Ala Glu Pro Gly Thr Glu Ala Arg Arg His Tyr Asp Glu Gly
305                 310                 315                 320
Val Arg Met Gly Ser Leu Gly Leu Phe Leu Gln Cys Ala Ile Ser Leu
                325                 330                 335
Val Phe Ser Leu Val Met Asp Arg Leu Val Gln Arg Phe Gly Thr Arg
            340                 345                 350
Ala Val Tyr Leu Ala Ser Val Ala Ala Phe Pro Val Ala Ala Gly Ala
        355                 360                 365
```

```
Thr Cys Leu Ser His Ser Val Ala Val Val Thr Ala Ser Ala Ala Leu
    370                 375                 380
Thr Gly Phe Thr Phe Ser Ala Leu Gln Ile Leu Pro Tyr Thr Leu Ala
385                 390                 395                 400
Ser Leu Tyr His Arg Glu Lys Gln Val Phe Leu Pro Lys Tyr Arg Gly
                405                 410                 415
Asp Thr Gly Gly Ala Ser Ser Glu Asp Ser Leu Met Thr Ser Phe Leu
            420                 425                 430
Pro Gly Pro Lys Pro Gly Ala Pro Phe Pro Asn Gly His Val Gly Ala
        435                 440                 445
Gly Gly Ser Gly Leu Leu Pro Pro Pro Pro Ala Leu Cys Gly Ala Ser
    450                 455                 460
Ala Cys Asp Val Ser Val Arg Val Val Val Gly Glu Pro Thr Glu Ala
465                 470                 475                 480
Arg Val Val Pro Gly Arg Gly Ile Cys Leu Asp Leu Ala Ile Leu Asp
                485                 490                 495
Ser Ala Phe Leu Leu Ser Gln Val Ala Pro Ser Leu Phe Met Gly Ser
            500                 505                 510
Ile Val Gln Leu Ser Gln Ser Val Thr Ala Tyr Met Val Ser Ala Ala
        515                 520                 525
Gly Leu Gly Leu Val Ala Ile Tyr Phe Ala Thr Gln Val Val Phe Asp
    530                 535                 540
Lys Ser Asp Leu Ala Lys Tyr Ser Ala
545                 550
```

<210> 44
<211> 644
<212> PRT
<213> Artificial Sequence

<220>
<223> St.pneum. C-LytA P2 helper epitope C-Lyta fused to Human P501S

<400> 44

```
Met Ala Ala Ala Tyr Val His Ser Asp Gly Ser Tyr Pro Lys Asp Lys
 1               5                  10                  15
Phe Glu Lys Ile Asn Gly Thr Trp Tyr Tyr Phe Asp Ser Ser Gly Tyr
            20                  25                  30
Met Leu Ala Asp Arg Trp Arg Lys His Thr Asp Gly Asn Trp Tyr Trp
        35                  40                  45
Phe Asp Asn Ser Gly Glu Met Ala Thr Gly Trp Lys Lys Ile Ala Asp
    50                  55                  60
Lys Trp Tyr Tyr Phe Asn Glu Glu Gly Ala Met Lys Thr Gly Trp Val
65                  70                  75                  80
Lys Tyr Lys Asp Thr Trp Tyr Tyr Leu Asp Ala Lys Glu Gly Ala Met
                85                  90                  95
Gln Tyr Ile Lys Ala Asn Ser Lys Phe Ile Gly Ile Thr Glu Gly Val
            100                 105                 110
Met Val Ser Asn Ala Phe Ile Gln Ser Ala Asp Gly Thr Gly Trp Tyr
        115                 120                 125
Tyr Leu Lys Pro Asp Gly Thr Leu Ala Asp Arg Pro Glu Lys Phe Met
    130                 135                 140
Tyr Met Val Leu Gly Ile Gly Pro Val Leu Gly Leu Val Cys Val Pro
145                 150                 155                 160
Leu Leu Gly Ser Ala Ser Asp His Trp Arg Gly Arg Tyr Gly Arg Arg
                165                 170                 175
Arg Pro Phe Ile Trp Ala Leu Ser Leu Gly Ile Leu Leu Ser Leu Phe
            180                 185                 190
Leu Ile Pro Arg Ala Gly Trp Leu Ala Gly Leu Leu Cys Pro Asp Pro
```

```
        195                     200                     205
Arg Pro Leu Glu Leu Ala Leu Leu Ile Leu Gly Val Gly Leu Leu Asp
    210                     215                     220
Phe Cys Gly Gln Val Cys Phe Thr Pro Leu Glu Ala Leu Leu Ser Asp
225                     230                     235                     240
Leu Phe Arg Asp Pro Asp His Cys Arg Gln Ala Tyr Ser Val Tyr Ala
                245                     250                     255
Phe Met Ile Ser Leu Gly Gly Cys Leu Gly Tyr Leu Leu Pro Ala Ile
            260                     265                     270
Asp Trp Asp Thr Ser Ala Leu Ala Pro Tyr Leu Gly Thr Gln Glu Glu
        275                     280                     285
Cys Leu Phe Gly Leu Leu Thr Leu Ile Phe Leu Thr Cys Val Ala Ala
    290                     295                     300
Thr Leu Leu Val Ala Glu Glu Ala Ala Leu Gly Pro Thr Glu Pro Ala
305                     310                     315                     320
Glu Gly Leu Ser Ala Pro Ser Leu Ser Pro His Cys Cys Pro Cys Arg
                325                     330                     335
Ala Arg Leu Ala Phe Arg Asn Leu Gly Ala Leu Leu Pro Arg Leu His
            340                     345                     350
Gln Leu Cys Cys Arg Met Pro Arg Thr Leu Arg Arg Leu Phe Val Ala
        355                     360                     365
Glu Leu Cys Ser Trp Met Ala Leu Met Thr Phe Thr Leu Phe Tyr Thr
    370                     375                     380
Asp Phe Val Gly Glu Gly Leu Tyr Gln Gly Val Pro Arg Ala Glu Pro
385                     390                     395                     400
Gly Thr Glu Ala Arg Arg His Tyr Asp Glu Gly Val Arg Met Gly Ser
                405                     410                     415
Leu Gly Leu Phe Leu Gln Cys Ala Ile Ser Leu Val Phe Ser Leu Val
            420                     425                     430
Met Asp Arg Leu Val Gln Arg Phe Gly Thr Arg Ala Val Tyr Leu Ala
        435                     440                     445
Ser Val Ala Ala Phe Pro Val Ala Ala Gly Ala Thr Cys Leu Ser His
    450                     455                     460
Ser Val Ala Val Val Thr Ala Ser Ala Ala Leu Thr Gly Phe Thr Phe
465                     470                     475                     480
Ser Ala Leu Gln Ile Leu Pro Tyr Thr Leu Ala Ser Leu Tyr His Arg
                485                     490                     495
Glu Lys Gln Val Phe Leu Pro Lys Tyr Arg Gly Asp Thr Gly Gly Ala
            500                     505                     510
Ser Ser Glu Asp Ser Leu Met Thr Ser Phe Leu Pro Gly Pro Lys Pro
        515                     520                     525
Gly Ala Pro Phe Pro Asn Gly His Val Gly Ala Gly Gly Ser Gly Leu
    530                     535                     540
Leu Pro Pro Pro Pro Ala Leu Cys Gly Ala Ser Ala Cys Asp Val Ser
545                     550                     555                     560
Val Arg Val Val Val Gly Glu Pro Thr Glu Ala Arg Val Val Pro Gly
                565                     570                     575
Arg Gly Ile Cys Leu Asp Leu Ala Ile Leu Asp Ser Ala Phe Leu Leu
            580                     585                     590
Ser Gln Val Ala Pro Ser Leu Phe Met Gly Ser Ile Val Gln Leu Ser
        595                     600                     605
Gln Ser Val Thr Ala Tyr Met Val Ser Ala Ala Gly Leu Gly Leu Val
    610                     615                     620
Ala Ile Tyr Phe Ala Thr Gln Val Val Phe Asp Lys Ser Asp Leu Ala
625                     630                     635                     640
Lys Tyr Ser Ala
```

<210> 45
<211> 644
<212> PRT
<213> Artificial Sequence

<220>
<223> Codon-optimised hybrid protein between St.pneum. C-LytA P2 helper epitope C-Lyta fused to Human P501S amino acids 51-553)

<400> 45

Met Ala Ala Ala Tyr Val His Ser Asp Gly Ser Tyr Pro Lys Asp Lys
1 5 10 15
Phe Glu Lys Ile Asn Gly Thr Trp Tyr Tyr Phe Asp Ser Ser Gly Tyr
20 25 30
Met Leu Ala Asp Arg Trp Arg Lys His Thr Asp Gly Asn Trp Tyr Trp
35 40 45
Phe Asp Asn Ser Gly Glu Met Ala Thr Gly Trp Lys Lys Ile Ala Asp
50 55 60
Lys Trp Tyr Tyr Phe Asn Glu Glu Gly Ala Met Lys Thr Gly Trp Val
65 70 75 80
Lys Tyr Lys Asp Thr Trp Tyr Tyr Leu Asp Ala Lys Glu Gly Ala Met
85 90 95
Gln Tyr Ile Lys Ala Asn Ser Lys Phe Ile Gly Ile Thr Glu Gly Val
100 105 110
Met Val Ser Asn Ala Phe Ile Gln Ser Ala Asp Gly Thr Gly Trp Tyr
115 120 125
Tyr Leu Lys Pro Asp Gly Thr Leu Ala Asp Arg Pro Glu Lys Phe Met
130 135 140
Tyr Met Val Leu Gly Ile Gly Pro Val Leu Gly Leu Val Cys Val Pro
145 150 155 160
Leu Leu Gly Ser Ala Ser Asp His Trp Arg Gly Arg Tyr Gly Arg Arg
165 170 175
Arg Pro Phe Ile Trp Ala Leu Ser Leu Gly Ile Leu Leu Ser Leu Phe
180 185 190
Leu Ile Pro Arg Ala Gly Trp Leu Ala Gly Leu Leu Cys Pro Asp Pro
195 200 205
Arg Pro Leu Glu Leu Ala Leu Leu Ile Leu Gly Val Gly Leu Leu Asp
210 215 220
Phe Cys Gly Gln Val Cys Phe Thr Pro Leu Glu Ala Leu Leu Ser Asp
225 230 235 240
Leu Phe Arg Asp Pro Asp His Cys Arg Gln Ala Tyr Ser Val Tyr Ala
245 250 255
Phe Met Ile Ser Leu Gly Gly Cys Leu Gly Tyr Leu Leu Pro Ala Ile
260 265 270
Asp Trp Asp Thr Ser Ala Leu Ala Pro Tyr Leu Gly Thr Gln Glu Glu
275 280 285
Cys Leu Phe Gly Leu Leu Thr Leu Ile Phe Leu Thr Cys Val Ala Ala
290 295 300
Thr Leu Leu Val Ala Glu Glu Ala Ala Leu Gly Pro Thr Glu Pro Ala
305 310 315 320
Glu Gly Leu Ser Ala Pro Ser Leu Ser Pro His Cys Cys Pro Cys Arg
325 330 335
Ala Arg Leu Ala Phe Arg Asn Leu Gly Ala Leu Leu Pro Arg Leu His
340 345 350
Gln Leu Cys Cys Arg Met Pro Arg Thr Leu Arg Arg Leu Phe Val Ala
355 360 365
Glu Leu Cys Ser Trp Met Ala Leu Met Thr Phe Thr Leu Phe Tyr Thr
370 375 380
Asp Phe Val Gly Glu Gly Leu Tyr Gln Gly Val Pro Arg Ala Glu Pro
385 390 395 400
Gly Thr Glu Ala Arg Arg His Tyr Asp Glu Gly Val Arg Met Gly Ser
405 410 415

```
Leu Gly Leu Phe Leu Gln Cys Ala Ile Ser Leu Val Phe Ser Leu Val
            420                 425                 430
Met Asp Arg Leu Val Gln Arg Phe Gly Thr Arg Ala Val Tyr Leu Ala
        435                 440                 445
Ser Val Ala Ala Phe Pro Val Ala Ala Gly Ala Thr Cys Leu Ser His
    450                 455                 460
Ser Val Ala Val Val Thr Ala Ser Ala Ala Leu Thr Gly Phe Thr Phe
465                 470                 475                 480
Ser Ala Leu Gln Ile Leu Pro Tyr Thr Leu Ala Ser Leu Tyr His Arg
                485                 490                 495
Glu Lys Gln Val Phe Leu Pro Lys Tyr Arg Gly Asp Thr Gly Gly Ala
            500                 505                 510
Ser Ser Glu Asp Ser Leu Met Thr Ser Phe Leu Pro Gly Pro Lys Pro
        515                 520                 525
Gly Ala Pro Phe Pro Asn Gly His Val Gly Ala Gly Gly Ser Gly Leu
    530                 535                 540
Leu Pro Pro Pro Pro Ala Leu Cys Gly Ala Ser Ala Cys Asp Val Ser
545                 550                 555                 560
Val Arg Val Val Val Gly Glu Pro Thr Glu Ala Arg Val Val Pro Gly
                565                 570                 575
Arg Gly Ile Cys Leu Asp Leu Ala Ile Leu Asp Ser Ala Phe Leu Leu
            580                 585                 590
Ser Gln Val Ala Pro Ser Leu Phe Met Gly Ser Ile Val Gln Leu Ser
        595                 600                 605
Gln Ser Val Thr Ala Tyr Met Val Ser Ala Ala Gly Leu Gly Leu Val
    610                 615                 620
Ala Ile Tyr Phe Ala Thr Gln Val Val Phe Asp Lys Ser Asp Leu Ala
625                 630                 635                 640
Lys Tyr Ser Ala
```

<210> 46
<211> 694
<212> PRT
<213> Artificial Sequence

<220>
<223> St.pneum. C-LytA P2 helper epitope C-Lyta fused to Human P501S (amino acids 1-553)- codon optimised

<400> 46

```
Met Ala Ala Ala Tyr Val His Ser Asp Gly Ser Tyr Pro Lys Asp Lys
 1               5                  10                  15
Phe Glu Lys Ile Asn Gly Thr Trp Tyr Tyr Phe Asp Ser Ser Gly Tyr
            20                  25                  30
Met Leu Ala Asp Arg Trp Arg Lys His Thr Asp Gly Asn Trp Tyr Trp
        35                  40                  45
Phe Asp Asn Ser Gly Glu Met Ala Thr Gly Trp Lys Lys Ile Ala Asp
    50                  55                  60
Lys Trp Tyr Tyr Phe Asn Glu Glu Gly Ala Met Lys Thr Gly Trp Val
65                  70                  75                  80
Lys Tyr Lys Asp Thr Trp Tyr Tyr Leu Asp Ala Lys Glu Gly Ala Met
                85                  90                  95
Gln Tyr Ile Lys Ala Asn Ser Lys Phe Ile Gly Ile Thr Glu Gly Val
            100                 105                 110
Met Val Ser Asn Ala Phe Ile Gln Ser Ala Asp Gly Thr Gly Trp Tyr
        115                 120                 125
Tyr Leu Lys Pro Asp Gly Thr Leu Ala Asp Arg Pro Glu Met Val Gln
    130                 135                 140
Arg Leu Trp Val Ser Arg Leu Leu Arg His Arg Lys Ala Gln Leu Leu
```

145 150 155 160
Leu Val Asn Leu Leu Thr Phe Gly Leu Glu Val Cys Leu Ala Ala Gly
165 170 175
Ile Thr Tyr Val Pro Pro Leu Leu Leu Glu Val Gly Val Glu Glu Lys
180 185 190
Phe Met Thr Met Val Leu Gly Ile Gly Pro Val Leu Gly Leu Val Cys
195 200 205
Val Pro Leu Leu Gly Ser Ala Ser Asp His Trp Arg Gly Arg Tyr Gly
210 215 220
Arg Arg Arg Pro Phe Ile Trp Ala Leu Ser Leu Gly Ile Leu Leu Ser
225 230 235 240
Leu Phe Leu Ile Pro Arg Ala Gly Trp Leu Ala Gly Leu Leu Cys Pro
245 250 255
Asp Pro Arg Pro Leu Glu Leu Ala Leu Leu Ile Leu Gly Val Gly Leu
260 265 270
Leu Asp Phe Cys Gly Gln Val Cys Phe Thr Pro Leu Glu Ala Leu Leu
275 280 285
Ser Asp Leu Phe Arg Asp Pro Asp His Cys Arg Gln Ala Tyr Ser Val
290 295 300
Tyr Ala Phe Met Ile Ser Leu Gly Gly Cys Leu Gly Tyr Leu Leu Pro
305 310 315 320
Ala Ile Asp Trp Asp Thr Ser Ala Leu Ala Pro Tyr Leu Gly Thr Gln
325 330 335
Glu Glu Cys Leu Phe Gly Leu Leu Thr Leu Ile Phe Leu Thr Cys Val
340 345 350
Ala Ala Thr Leu Leu Val Ala Glu Glu Ala Ala Leu Gly Pro Thr Glu
355 360 365
Pro Ala Glu Gly Leu Ser Ala Pro Ser Leu Ser Pro His Cys Cys Pro
370 375 380
Cys Arg Ala Arg Leu Ala Phe Arg Asn Leu Gly Ala Leu Leu Pro Arg
385 390 395 400
Leu His Gln Leu Cys Cys Arg Met Pro Arg Thr Leu Arg Arg Leu Phe
405 410 415
Val Ala Glu Leu Cys Ser Trp Met Ala Leu Met Thr Phe Thr Leu Phe
420 425 430
Tyr Thr Asp Phe Val Gly Glu Gly Leu Tyr Gln Gly Val Pro Arg Ala
435 440 445
Glu Pro Gly Thr Glu Ala Arg Arg His Tyr Asp Glu Gly Val Arg Met
450 455 460
Gly Ser Leu Gly Leu Phe Leu Gln Cys Ala Ile Ser Leu Val Phe Ser
465 470 475 480
Leu Val Met Asp Arg Leu Val Gln Arg Phe Gly Thr Arg Ala Val Tyr
485 490 495
Leu Ala Ser Val Ala Ala Phe Pro Val Ala Ala Gly Ala Thr Cys Leu
500 505 510
Ser His Ser Val Ala Val Val Thr Ala Ser Ala Ala Leu Thr Gly Phe
515 520 525
Thr Phe Ser Ala Leu Gln Ile Leu Pro Tyr Thr Leu Ala Ser Leu Tyr
530 535 540
His Arg Glu Lys Gln Val Phe Leu Pro Lys Tyr Arg Gly Asp Thr Gly
545 550 555 560
Gly Ala Ser Ser Glu Asp Ser Leu Met Thr Ser Phe Leu Pro Gly Pro
565 570 575
Lys Pro Gly Ala Pro Phe Pro Asn Gly His Val Gly Ala Gly Gly Ser
580 585 590
Gly Leu Leu Pro Pro Pro Pro Ala Leu Cys Gly Ala Ser Ala Cys Asp
595 600 605
Val Ser Val Arg Val Val Val Gly Glu Pro Thr Glu Ala Arg Val Val
610 615 620
Pro Gly Arg Gly Ile Cys Leu Asp Leu Ala Ile Leu Asp Ser Ala Phe
625 630 635 640

```
Leu Leu Ser Gln Val Ala Pro Ser Leu Phe Met Gly Ser Ile Val Gln
                645                 650                 655
Leu Ser Gln Ser Val Thr Ala Tyr Met Val Ser Ala Ala Gly Leu Gly
            660                 665                 670
Leu Val Ala Ile Tyr Phe Ala Thr Gln Val Val Phe Asp Lys Ser Asp
        675                 680                 685
Leu Ala Lys Tyr Ser Ala
    690
```

<210> 47
<211> 694
<212> PRT
<213> Artificial Sequence

<220>
<223> St.pneum. C-LytA P2 helper epitope C-Lyta fused to Human P501S (amino acids 51-553) fused to Human P501S (amino acids 1-50) - codon-optimised

<400> 47

```
Met Ala Ala Ala Tyr Val His Ser Asp Gly Ser Tyr Pro Lys Asp Lys
 1               5                  10                  15
Phe Glu Lys Ile Asn Gly Thr Trp Tyr Tyr Phe Asp Ser Ser Gly Tyr
            20                  25                  30
Met Leu Ala Asp Arg Trp Arg Lys His Thr Asp Gly Asn Trp Tyr Trp
        35                  40                  45
Phe Asp Asn Ser Gly Glu Met Ala Thr Gly Trp Lys Lys Ile Ala Asp
    50                  55                  60
Lys Trp Tyr Tyr Phe Asn Glu Glu Gly Ala Met Lys Thr Gly Trp Val
65                  70                  75                  80
Lys Tyr Lys Asp Thr Trp Tyr Tyr Leu Asp Ala Lys Glu Gly Ala Met
                85                  90                  95
Gln Tyr Ile Lys Ala Asn Ser Lys Phe Ile Gly Ile Thr Glu Gly Val
            100                 105                 110
Met Val Ser Asn Ala Phe Ile Gln Ser Ala Asp Gly Thr Gly Trp Tyr
        115                 120                 125
Tyr Leu Lys Pro Asp Gly Thr Leu Ala Asp Arg Pro Glu Lys Phe Met
    130                 135                 140
Tyr Met Val Leu Gly Ile Gly Pro Val Leu Gly Leu Val Cys Val Pro
145                 150                 155                 160
Leu Leu Gly Ser Ala Ser Asp His Trp Arg Gly Arg Tyr Gly Arg Arg
                165                 170                 175
Arg Pro Phe Ile Trp Ala Leu Ser Leu Gly Ile Leu Leu Ser Leu Phe
            180                 185                 190
Leu Ile Pro Arg Ala Gly Trp Leu Ala Gly Leu Leu Cys Pro Asp Pro
        195                 200                 205
Arg Pro Leu Glu Leu Ala Leu Leu Ile Leu Gly Val Gly Leu Leu Asp
    210                 215                 220
Phe Cys Gly Gln Val Cys Phe Thr Pro Leu Glu Ala Leu Leu Ser Asp
225                 230                 235                 240
Leu Phe Arg Asp Pro Asp His Cys Arg Gln Ala Tyr Ser Val Tyr Ala
                245                 250                 255
Phe Met Ile Ser Leu Gly Gly Cys Leu Gly Tyr Leu Leu Pro Ala Ile
            260                 265                 270
Asp Trp Asp Thr Ser Ala Leu Ala Pro Tyr Leu Gly Thr Gln Glu Glu
        275                 280                 285
Cys Leu Phe Gly Leu Leu Thr Leu Ile Phe Leu Thr Cys Val Ala Ala
    290                 295                 300
Thr Leu Leu Val Ala Glu Glu Ala Ala Leu Gly Pro Thr Glu Pro Ala
305                 310                 315                 320
```

```
Glu Gly Leu Ser Ala Pro Ser Leu Ser Pro His Cys Cys Pro Cys Arg
                325                 330                 335
Ala Arg Leu Ala Phe Arg Asn Leu Gly Ala Leu Leu Pro Arg Leu His
            340                 345                 350
Gln Leu Cys Cys Arg Met Pro Arg Thr Leu Arg Arg Leu Phe Val Ala
        355                 360                 365
Glu Leu Cys Ser Trp Met Ala Leu Met Thr Phe Thr Leu Phe Tyr Thr
    370                 375                 380
Asp Phe Val Gly Glu Gly Leu Tyr Gln Gly Val Pro Arg Ala Glu Pro
385                 390                 395                 400
Gly Thr Glu Ala Arg Arg His Tyr Asp Glu Gly Val Arg Met Gly Ser
                405                 410                 415
Leu Gly Leu Phe Leu Gln Cys Ala Ile Ser Leu Val Phe Ser Leu Val
            420                 425                 430
Met Asp Arg Leu Val Gln Arg Phe Gly Thr Arg Ala Val Tyr Leu Ala
        435                 440                 445
Ser Val Ala Ala Phe Pro Val Ala Ala Gly Ala Thr Cys Leu Ser His
    450                 455                 460
Ser Val Ala Val Val Thr Ala Ser Ala Ala Leu Thr Gly Phe Thr Phe
465                 470                 475                 480
Ser Ala Leu Gln Ile Leu Pro Tyr Thr Leu Ala Ser Leu Tyr His Arg
                485                 490                 495
Glu Lys Gln Val Phe Leu Pro Lys Tyr Arg Gly Asp Thr Gly Gly Ala
            500                 505                 510
Ser Ser Glu Asp Ser Leu Met Thr Ser Phe Leu Pro Gly Pro Lys Pro
        515                 520                 525
Gly Ala Pro Phe Pro Asn Gly His Val Gly Ala Gly Gly Ser Gly Leu
    530                 535                 540
Leu Pro Pro Pro Pro Ala Leu Cys Gly Ala Ser Ala Cys Asp Val Ser
545                 550                 555                 560
Val Arg Val Val Val Gly Glu Pro Thr Glu Ala Arg Val Val Pro Gly
                565                 570                 575
Arg Gly Ile Cys Leu Asp Leu Ala Ile Leu Asp Ser Ala Phe Leu Leu
            580                 585                 590
Ser Gln Val Ala Pro Ser Leu Phe Met Gly Ser Ile Val Gln Leu Ser
        595                 600                 605
Gln Ser Val Thr Ala Tyr Met Val Ser Ala Ala Gly Leu Gly Leu Val
    610                 615                 620
Ala Ile Tyr Phe Ala Thr Gln Val Val Phe Asp Lys Ser Asp Leu Ala
625                 630                 635                 640
Lys Tyr Ser Ala Met Val Gln Arg Leu Trp Val Ser Arg Leu Leu Arg
                645                 650                 655
His Arg Lys Ala Gln Leu Leu Leu Val Asn Leu Leu Thr Phe Gly Leu
            660                 665                 670
Glu Val Cys Leu Ala Ala Gly Ile Thr Tyr Val Pro Pro Leu Leu Leu
        675                 680                 685
Glu Val Gly Val Glu Glu
    690
```

<210> 48
<211> 694
<212> PRT
<213> Artificial Sequence

<220>
<223> Human P501S (amino acids 1-50) fused to St.pneum. C-LytA P2 helper epitope C-Lyta fused to Human P501S (amino acids 51-553) - codon optimised

<400> 48

```
Met Val Gln Arg Leu Trp Val Ser Arg Leu Leu Arg His Arg Lys Ala
 1               5                  10                  15
Gln Leu Leu Leu Val Asn Leu Leu Thr Phe Gly Leu Glu Val Cys Leu
            20                  25                  30
Ala Ala Gly Ile Thr Tyr Val Pro Pro Leu Leu Leu Glu Val Gly Val
        35                  40                  45
Glu Glu Met Ala Ala Ala Tyr Val His Ser Asp Gly Ser Tyr Pro Lys
    50                  55                  60
Asp Lys Phe Glu Lys Ile Asn Gly Thr Trp Tyr Tyr Phe Asp Ser Ser
65                  70                  75                  80
Gly Tyr Met Leu Ala Asp Arg Trp Arg Lys His Thr Asp Gly Asn Trp
                85                  90                  95
Tyr Trp Phe Asp Asn Ser Gly Glu Met Ala Thr Gly Trp Lys Lys Ile
            100                 105                 110
Ala Asp Lys Trp Tyr Tyr Phe Asn Glu Glu Gly Ala Met Lys Thr Gly
        115                 120                 125
Trp Val Lys Tyr Lys Asp Thr Trp Tyr Tyr Leu Asp Ala Lys Glu Gly
    130                 135                 140
Ala Met Gln Tyr Ile Lys Ala Asn Ser Lys Phe Ile Gly Ile Thr Glu
145                 150                 155                 160
Gly Val Met Val Ser Asn Ala Phe Ile Gln Ser Ala Asp Gly Thr Gly
                165                 170                 175
Trp Tyr Tyr Leu Lys Pro Asp Gly Thr Leu Ala Asp Arg Pro Glu Lys
            180                 185                 190
Phe Met Tyr Met Val Leu Gly Ile Gly Pro Val Leu Gly Leu Val Cys
        195                 200                 205
Val Pro Leu Leu Gly Ser Ala Ser Asp His Trp Arg Gly Arg Tyr Gly
    210                 215                 220
Arg Arg Arg Pro Phe Ile Trp Ala Leu Ser Leu Gly Ile Leu Leu Ser
225                 230                 235                 240
Leu Phe Leu Ile Pro Arg Ala Gly Trp Leu Ala Gly Leu Leu Cys Pro
                245                 250                 255
Asp Pro Arg Pro Leu Glu Leu Ala Leu Leu Ile Leu Gly Val Gly Leu
            260                 265                 270
Leu Asp Phe Cys Gly Gln Val Cys Phe Thr Pro Leu Glu Ala Leu Leu
        275                 280                 285
Ser Asp Leu Phe Arg Asp Pro Asp His Cys Arg Gln Ala Tyr Ser Val
    290                 295                 300
Tyr Ala Phe Met Ile Ser Leu Gly Gly Cys Leu Gly Tyr Leu Leu Pro
305                 310                 315                 320
Ala Ile Asp Trp Asp Thr Ser Ala Leu Ala Pro Tyr Leu Gly Thr Gln
                325                 330                 335
Glu Glu Cys Leu Phe Gly Leu Leu Thr Leu Ile Phe Leu Thr Cys Val
            340                 345                 350
Ala Ala Thr Leu Leu Val Ala Glu Glu Ala Ala Leu Gly Pro Thr Glu
        355                 360                 365
Pro Ala Glu Gly Leu Ser Ala Pro Ser Leu Ser Pro His Cys Cys Pro
    370                 375                 380
Cys Arg Ala Arg Leu Ala Phe Arg Asn Leu Gly Ala Leu Leu Pro Arg
385                 390                 395                 400
Leu His Gln Leu Cys Cys Arg Met Pro Arg Thr Leu Arg Arg Leu Phe
                405                 410                 415
Val Ala Glu Leu Cys Ser Trp Met Ala Leu Met Thr Phe Thr Leu Phe
            420                 425                 430
Tyr Thr Asp Phe Val Gly Glu Gly Leu Tyr Gln Gly Val Pro Arg Ala
        435                 440                 445
Glu Pro Gly Thr Glu Ala Arg Arg His Tyr Asp Glu Gly Val Arg Met
    450                 455                 460
Gly Ser Leu Gly Leu Phe Leu Gln Cys Ala Ile Ser Leu Val Phe Ser
465                 470                 475                 480
Leu Val Met Asp Arg Leu Val Gln Arg Phe Gly Thr Arg Ala Val Tyr
```

```
                    485                     490                     495
Leu Ala Ser Val Ala Ala Phe Pro Val Ala Ala Gly Ala Thr Cys Leu
                500                     505                     510
Ser His Ser Val Ala Val Val Thr Ala Ser Ala Ala Leu Thr Gly Phe
            515                     520                     525
Thr Phe Ser Ala Leu Gln Ile Leu Pro Tyr Thr Leu Ala Ser Leu Tyr
        530                     535                     540
His Arg Glu Lys Gln Val Phe Leu Pro Lys Tyr Arg Gly Asp Thr Gly
545                     550                     555                     560
Gly Ala Ser Ser Glu Asp Ser Leu Met Thr Ser Phe Leu Pro Gly Pro
                    565                     570                     575
Lys Pro Gly Ala Pro Phe Pro Asn Gly His Val Gly Ala Gly Gly Ser
                580                     585                     590
Gly Leu Leu Pro Pro Pro Pro Ala Leu Cys Gly Ala Ser Ala Cys Asp
            595                     600                     605
Val Ser Val Arg Val Val Val Gly Glu Pro Thr Glu Ala Arg Val Val
        610                     615                     620
Pro Gly Arg Gly Ile Cys Leu Asp Leu Ala Ile Leu Asp Ser Ala Phe
625                     630                     635                     640
Leu Leu Ser Gln Val Ala Pro Ser Leu Phe Met Gly Ser Ile Val Gln
                    645                     650                     655
Leu Ser Gln Ser Val Thr Ala Tyr Met Val Ser Ala Ala Gly Leu Gly
                660                     665                     670
Leu Val Ala Ile Tyr Phe Ala Thr Gln Val Val Phe Asp Lys Ser Asp
            675                     680                     685
Leu Ala Lys Tyr Ser Ala
        690
```

<210> 49
<211> 1971
<212> DNA
<213> Artificial Sequence

<220>
<223> DNA encoding Human MUC-1 fused to St.pneum. C-LytA P2 helper epitope C-Lyta

<400> 49

```
atgacaccgg gcacccagtc tcctttcttc ctgctgctgc tcctcacagt gcttacagtt 60
gttacaggtt ctggtcatgc aagctctacc ccaggtggag aaaaggagac ttcggctacc 120
cagagaagtt cagtgcccag ctctactgag aagaatgctg tgagtatgac cagcagcgta 180
ctctccagcc acagccccgg ttcaggctcc tccaccactc agggacagga tgtcactctg 240
gccccggcca cggaaccagc ttcaggttca gctgccacct ggggacagga tgtcacctcg 300
gtcccagtca ccaggccagc cctgggctcc accaccccgc cagcccacga tgtcacctca 360
gccccggaca acaagccagc cccgggctcc accgcccccc cagcccacgg tgtcacctcg 420
gccccggaca ccaggccgcc cccgggctcc accgcccccc cagcccacgg tgtcacctcg 480
gccccggaca ccaggccgcc cccgggctcc accgcgcccg cagcccacgg tgtcacctcg 540
gccccggaca ccaggccggc cccgggctcc accgcccccc cagcccatgg tgtcacctcg 600
gccccggaca acaggcccgc cttggcgtcc accgcccctc cagtccacaa tgtcacctcg 660
gcctcaggct ctgcatcagg ctcagcttct actctggtgc acaacggcac ctctgccagg 720
gctaccacaa ccccagccag caagagcact ccattctcaa ttcccagcca ccactctgat 780
actcctacca cccttgccag ccatagcacc aagactgatg ccagtagcac tcaccatagc 840
acggtacctc ctctcacctc ctccaatcac agcacttctc cccagttgtc tactggggtc 900
tctttctttt tcctgtcttt tcacatttca aacctccagt ttaattcctc tctggaagat 960
cccagcaccg actactacca agagctgcag agagacattt ctgaaatgtt tttgcagatt 1020
tataaacaag gggttttctt gggcctctcc aatattaagt tcaggccagg atctgtggtg 1080
gtacaattga ctctggcctt ccgagaaggt accatcaatg tccacgacgt ggagacacag 1140
ttcaatcagt ataaaacgga agcagcctct cgatataacc tgacgatctc agacgtcagc 1200
gtgagtgatg tgccatttcc tttctctgcc cagtctgggg ctggggtgcc aggctggggc 1260
atcgcgctgc tggtgctggt ctgtgttctg gttgcgctgg ccattgtcta tctcattgcc 1320

ttggctgtct gtcagtgccg ccgaaagaac tacgggcagc tggacatctt tccagcccgg 1380
gatacctacc atcctatgag cgagtacccc acctaccaca cccatgggcg ctatgtgccc 1440
cctagcagta ccgatcgtag cccctatgag aaggtttctg caggtaatgg tggcagcagc 1500
ctctcttaca caaacccagc agtggcagcc acttctgcca acttgatggc ggccgcttac 1560
gtacattccg acggctctta tccaaaagac aagtttgaga aaatcaatgg cacttggtac 1620
tactttgaca gttcaggcta tatgcttgca gaccgctgga ggaagcacac agacggcaac 1680
tggtactggt tcgacaactc aggcgaaatg gctacaggct ggaagaaaat cgctgataag 1740
tggtactatt tcaacgaaga aggtgccatg aagacaggct gggtcaagta caaggacact 1800
tggtactact tagacgctaa agaaggcgcc atgcaataca tcaaggctaa ctctaagttc 1860
attggtatca ctgaaggcgt catggtatca aatgccttta tccagtcagc ggacggaaca 1920
ggctggtact acctcaaacc agacggaaca ctggcagaca ggccagaatg a          1971
```

<210> 50
<211> 656
<212> PRT
<213> Artificial Sequence

<220>
<223> Human MUC-1 fused to St.pneum. C-LytA P2 helper epitope C-Lyta

<400> 50

```
Met Thr Pro Gly Thr Gln Ser Pro Phe Phe Leu Leu Leu Leu Leu Thr
 1               5                  10                  15
Val Leu Thr Val Val Thr Gly Ser Gly His Ala Ser Ser Thr Pro Gly
            20                  25                  30
Gly Glu Lys Glu Thr Ser Ala Thr Gln Arg Ser Ser Val Pro Ser Ser
        35                  40                  45
Thr Glu Lys Asn Ala Val Ser Met Thr Ser Ser Val Leu Ser Ser His
    50                  55                  60
Ser Pro Gly Ser Gly Ser Ser Thr Thr Gln Gly Gln Asp Val Thr Leu
65                  70                  75                  80
Ala Pro Ala Thr Glu Pro Ala Ser Gly Ser Ala Ala Thr Trp Gly Gln
                85                  90                  95
Asp Val Thr Ser Val Pro Val Thr Arg Pro Ala Leu Gly Ser Thr Thr
            100                 105                 110
Pro Pro Ala His Asp Val Thr Ser Ala Pro Asp Asn Lys Pro Ala Pro
        115                 120                 125
Gly Ser Thr Ala Pro Pro Ala His Gly Val Thr Ser Ala Pro Asp Thr
    130                 135                 140
Arg Pro Pro Pro Gly Ser Thr Ala Pro Pro Ala His Gly Val Thr Ser
145                 150                 155                 160
Ala Pro Asp Thr Arg Pro Pro Pro Gly Ser Thr Ala Pro Ala Ala His
                165                 170                 175
Gly Val Thr Ser Ala Pro Asp Thr Arg Pro Ala Pro Gly Ser Thr Ala
            180                 185                 190
Pro Pro Ala His Gly Val Thr Ser Ala Pro Asp Asn Arg Pro Ala Leu
        195                 200                 205
Ala Ser Thr Ala Pro Pro Val His Asn Val Thr Ser Ala Ser Gly Ser
    210                 215                 220
Ala Ser Gly Ser Ala Ser Thr Leu Val His Asn Gly Thr Ser Ala Arg
225                 230                 235                 240
Ala Thr Thr Thr Pro Ala Ser Lys Ser Thr Pro Phe Ser Ile Pro Ser
                245                 250                 255
His His Ser Asp Thr Pro Thr Thr Leu Ala Ser His Ser Thr Lys Thr
            260                 265                 270
Asp Ala Ser Ser Thr His His Ser Thr Val Pro Pro Leu Thr Ser Ser
        275                 280                 285
Asn His Ser Thr Ser Pro Gln Leu Ser Thr Gly Val Ser Phe Phe Phe
    290                 295                 300
Leu Ser Phe His Ile Ser Asn Leu Gln Phe Asn Ser Ser Leu Glu Asp
```

```
305                 310                 315                 320
Pro Ser Thr Asp Tyr Tyr Gln Glu Leu Gln Arg Asp Ile Ser Glu Met
                325                 330                 335
Phe Leu Gln Ile Tyr Lys Gln Gly Gly Phe Leu Gly Leu Ser Asn Ile
            340                 345                 350
Lys Phe Arg Pro Gly Ser Val Val Val Gln Leu Thr Leu Ala Phe Arg
        355                 360                 365
Glu Gly Thr Ile Asn Val His Asp Val Glu Thr Gln Phe Asn Gln Tyr
    370                 375                 380
Lys Thr Glu Ala Ala Ser Arg Tyr Asn Leu Thr Ile Ser Asp Val Ser
385                 390                 395                 400
Val Ser Asp Val Pro Phe Pro Phe Ser Ala Gln Ser Gly Ala Gly Val
                405                 410                 415
Pro Gly Trp Gly Ile Ala Leu Leu Val Leu Val Cys Val Leu Val Ala
            420                 425                 430
Leu Ala Ile Val Tyr Leu Ile Ala Leu Ala Val Cys Gln Cys Arg Arg
        435                 440                 445
Lys Asn Tyr Gly Gln Leu Asp Ile Phe Pro Ala Arg Asp Thr Tyr His
    450                 455                 460
Pro Met Ser Glu Tyr Pro Thr Tyr His Thr His Gly Arg Tyr Val Pro
465                 470                 475                 480
Pro Ser Ser Thr Asp Arg Ser Pro Tyr Glu Lys Val Ser Ala Gly Asn
                485                 490                 495
Gly Gly Ser Ser Leu Ser Tyr Thr Asn Pro Ala Val Ala Ala Thr Ser
            500                 505                 510
Ala Asn Leu Met Ala Ala Ala Tyr Val His Ser Asp Gly Ser Tyr Pro
        515                 520                 525
Lys Asp Lys Phe Glu Lys Ile Asn Gly Thr Trp Tyr Tyr Phe Asp Ser
    530                 535                 540
Ser Gly Tyr Met Leu Ala Asp Arg Trp Arg Lys His Thr Asp Gly Asn
545                 550                 555                 560
Trp Tyr Trp Phe Asp Asn Ser Gly Glu Met Ala Thr Gly Trp Lys Lys
                565                 570                 575
Ile Ala Asp Lys Trp Tyr Tyr Phe Asn Glu Glu Gly Ala Met Lys Thr
            580                 585                 590
Gly Trp Val Lys Tyr Lys Asp Thr Trp Tyr Tyr Leu Asp Ala Lys Glu
        595                 600                 605
Gly Ala Met Gln Tyr Ile Lys Ala Asn Ser Lys Phe Ile Gly Ile Thr
    610                 615                 620
Glu Gly Val Met Val Ser Asn Ala Phe Ile Gln Ser Ala Asp Gly Thr
625                 630                 635                 640
Gly Trp Tyr Tyr Leu Lys Pro Asp Gly Thr Leu Ala Asp Arg Pro Glu
                645                 650                 655
```

<210> 51
<211> 2037
<212> DNA
<213> Artificial Sequence

<220>
<223> DNA encoding St.pneum. C-LytA P2 helper epitope C-Lyta fused to Human MUC-1

<400> 51

```
atgggatgga gctgtatcat cctcttcttg gtagcaacag ctacaggtgt ccactcccag 60
gtccaaatgg cggccgctta cgtacattcc gacggctctt atccaaaaga caagtttgag 120
aaaatcaatg gcacttggta ctactttgac agttcaggct atatgcttgc agaccgctgg 180
aggaagcaca cagacggcaa ctggtactgg ttcgacaact caggcgaaat ggctacaggc 240
tggaagaaaa tcgctgataa gtggtactat ttcaacgaag aaggtgccat gaagacaggc 300
tgggtcaagt acaaggacac ttggtactac ttagacgcta aagaaggcgc catgcaatac 360
```

```
atcaaggcta actctaagtt cattggtatc actgaaggcg tcatggtatc aaatgccttt 420
atccagtcag cggacggaac aggctggtac tacctcaaac cagacggaac actggcagac 480
aggccagaaa tgacaccggg cacccagtct cctttcttcc tgctgctgct cctcacagtg 540
cttacagttg ttacaggttc tggtcatgca agctctaccc caggtggaga aaaggagact 600
tcggctaccc agagaagttc agtgcccagc tctactgaga agaatgctgt gagtatgacc 660
agcagcgtac tctccagcca cagccccggt tcaggctcct ccaccactca gggacaggat 720
gtcactctgg ccccggccac ggaaccagct tcaggttcag ctgccacctg gggacaggat 780
gtcacctcgg tcccagtcac caggccagcc ctgggctcca ccaccccgcc agcccacgat 840
gtcacctcag ccccggacaa caagccagcc ccgggctcca ccgccccccc agcccacggt 900
gtcacctcgg ccccggacac caggccgccc ccgggctcca ccgccccccc agcccacggt 960
gtcacctcgg ccccggacac caggccgccc ccgggctcca ccgcgcccgc agcccacggt 1020
gtcacctcgg ccccggacac caggccggcc ccgggctcca ccgccccccc agcccatggt 1080
gtcacctcgg ccccggacaa caggcccgcc ttggcgtcca ccgcccctcc agtccacaat 1140
gtcacctcgg cctcaggctc tgcatcaggc tcagcttcta ctctggtgca caacggcacc 1200
tctgccaggg ctaccacaac cccagccagc aagagcactc cattctcaat tcccagccac 1260
cactctgata ctcctaccac ccttgccagc catagcacca agactgatgc cagtagcact 1320
caccatagca cggtacctcc tctcacctcc tccaatcaca gcacttctcc ccagttgtct 1380
actggggtct ctttcttttt cctgtctttt cacatttcaa acctccagtt taattcctct 1440
ctggaagatc ccagcaccga ctactaccaa gagctgcaga gagacatttc tgaaatgttt 1500
ttgcagattt ataaacaagg gggttttctg ggcctctcca atattaagtt caggccagga 1560
tctgtggtgg tacaattgac tctggccttc cgagaaggta ccatcaatgt ccacgacgtg 1620
gagacacagt tcaatcagta taaaacggaa gcagcctctc gatataacct gacgatctca 1680
gacgtcagcg tgagtgatgt gccatttcct ttctctgccc agtctggggc tggggtgcca 1740
ggctggggca tcgcgctgct ggtgctggtc tgtgttctgg ttgcgctggc cattgtctat 1800
ctcattgcct tggctgtctg tcagtgccgc cgaaagaact acgggcagct ggacatcttt 1860
ccagcccggg atacctacca tcctatgagc gagtacccca cctaccacac ccatgggcgc 1920
tatgtgcccc ctagcagtac cgatcgtagc ccctatgaga aggtttctgc aggtaatggt 1980
ggcagcagcc tctcttacac aaacccagca gtggcagcca cttctgccaa cttgtag    2037
```

<210> 52
<211> 678
<212> PRT
<213> Artificial Sequence

<220>
<223> St.pneum. C-LytA P2 helper epitope C-Lyta fused to Human MUC-1

<400> 52

```
Met Gly Trp Ser Cys Ile Ile Leu Phe Leu Val Ala Thr Ala Thr Gly
 1               5                  10                  15
Val His Ser Gln Val Gln Met Ala Ala Ala Tyr Val His Ser Asp Gly
            20                  25                  30
Ser Tyr Pro Lys Asp Lys Phe Glu Lys Ile Asn Gly Thr Trp Tyr Tyr
        35                  40                  45
Phe Asp Ser Ser Gly Tyr Met Leu Ala Asp Arg Trp Arg Lys His Thr
    50                  55                  60
Asp Gly Asn Trp Tyr Trp Phe Asp Asn Ser Gly Glu Met Ala Thr Gly
65                  70                  75                  80
Trp Lys Lys Ile Ala Asp Lys Trp Tyr Tyr Phe Asn Glu Glu Gly Ala
                85                  90                  95
Met Lys Thr Gly Trp Val Lys Tyr Lys Asp Thr Trp Tyr Tyr Leu Asp
            100                 105                 110
Ala Lys Glu Gly Ala Met Gln Tyr Ile Lys Ala Asn Ser Lys Phe Ile
        115                 120                 125
Gly Ile Thr Glu Gly Val Met Val Ser Asn Ala Phe Ile Gln Ser Ala
    130                 135                 140
Asp Gly Thr Gly Trp Tyr Tyr Leu Lys Pro Asp Gly Thr Leu Ala Asp
145                 150                 155                 160
Arg Pro Glu Met Thr Pro Gly Thr Gln Ser Pro Phe Phe Leu Leu Leu
                165                 170                 175
```

```
Leu Leu Thr Val Leu Thr Val Val Thr Gly Ser Gly His Ala Ser Ser
            180                 185                 190
Thr Pro Gly Gly Glu Lys Glu Thr Ser Ala Thr Gln Arg Ser Ser Val
        195                 200                 205
Pro Ser Ser Thr Glu Lys Asn Ala Val Ser Met Thr Ser Ser Val Leu
    210                 215                 220
Ser Ser His Ser Pro Gly Ser Gly Ser Ser Thr Thr Gln Gly Gln Asp
225                 230                 235                 240
Val Thr Leu Ala Pro Ala Thr Glu Pro Ala Ser Gly Ser Ala Ala Thr
                245                 250                 255
Trp Gly Gln Asp Val Thr Ser Val Pro Val Thr Arg Pro Ala Leu Gly
            260                 265                 270
Ser Thr Thr Pro Pro Ala His Asp Val Thr Ser Ala Pro Asp Asn Lys
        275                 280                 285
Pro Ala Pro Gly Ser Thr Ala Pro Pro Ala His Gly Val Thr Ser Ala
    290                 295                 300
Pro Asp Thr Arg Pro Pro Pro Gly Ser Thr Ala Pro Pro Ala His Gly
305                 310                 315                 320
Val Thr Ser Ala Pro Asp Thr Arg Pro Pro Pro Gly Ser Thr Ala Pro
                325                 330                 335
Ala Ala His Gly Val Thr Ser Ala Pro Asp Thr Arg Pro Ala Pro Gly
            340                 345                 350
Ser Thr Ala Pro Pro Ala His Gly Val Thr Ser Ala Pro Asp Asn Arg
        355                 360                 365
Pro Ala Leu Ala Ser Thr Ala Pro Pro Val His Asn Val Thr Ser Ala
    370                 375                 380
Ser Gly Ser Ala Ser Gly Ser Ala Ser Thr Leu Val His Asn Gly Thr
385                 390                 395                 400
Ser Ala Arg Ala Thr Thr Thr Pro Ala Ser Lys Ser Thr Pro Phe Ser
                405                 410                 415
Ile Pro Ser His His Ser Asp Thr Pro Thr Thr Leu Ala Ser His Ser
            420                 425                 430
Thr Lys Thr Asp Ala Ser Ser Thr His His Ser Thr Val Pro Pro Leu
        435                 440                 445
Thr Ser Ser Asn His Ser Thr Ser Pro Gln Leu Ser Thr Gly Val Ser
    450                 455                 460
Phe Phe Phe Leu Ser Phe His Ile Ser Asn Leu Gln Phe Asn Ser Ser
465                 470                 475                 480
Leu Glu Asp Pro Ser Thr Asp Tyr Tyr Gln Glu Leu Gln Arg Asp Ile
                485                 490                 495
Ser Glu Met Phe Leu Gln Ile Tyr Lys Gln Gly Gly Phe Leu Gly Leu
            500                 505                 510
Ser Asn Ile Lys Phe Arg Pro Gly Ser Val Val Val Gln Leu Thr Leu
        515                 520                 525
Ala Phe Arg Glu Gly Thr Ile Asn Val His Asp Val Glu Thr Gln Phe
    530                 535                 540
Asn Gln Tyr Lys Thr Glu Ala Ala Ser Arg Tyr Asn Leu Thr Ile Ser
545                 550                 555                 560
Asp Val Ser Val Ser Asp Val Pro Phe Pro Phe Ser Ala Gln Ser Gly
                565                 570                 575
Ala Gly Val Pro Gly Trp Gly Ile Ala Leu Leu Val Leu Val Cys Val
            580                 585                 590
Leu Val Ala Leu Ala Ile Val Tyr Leu Ile Ala Leu Ala Val Cys Gln
        595                 600                 605
Cys Arg Arg Lys Asn Tyr Gly Gln Leu Asp Ile Phe Pro Ala Arg Asp
    610                 615                 620
Thr Tyr His Pro Met Ser Glu Tyr Pro Thr Tyr His Thr His Gly Arg
625                 630                 635                 640
Tyr Val Pro Pro Ser Ser Thr Asp Arg Ser Pro Tyr Glu Lys Val Ser
                645                 650                 655
Ala Gly Asn Gly Gly Ser Ser Leu Ser Tyr Thr Asn Pro Ala Val Ala
```

```
                    660                     665                     670
Ala Thr Ser Ala Asn Leu
                675
```

## Description

The present invention relates to fusion partners which act as immunological fusion partners, as expression enhancers, and preferably to fusion partners having both functions. The invention also relates to fusion proteins containing them, to their manufacture, to their use in vaccines and to their use in medicines. In particular fusion partners are provided that contain a so-called choline binding domain, for example fusions comprising LytA from Streptococcus pneumoniae, or the pneumococcal phage CP1 lysozyme (CPL1) wherein the choline binding domain is modified to include a heterologous T-helper epitope. Such fusion partners are shown to improve the expression level of the heterologous protein attached thereto and also find particular utility when fused to poorly immunogenic proteins or peptides that are otherwise useful as vaccine antigens. More particularly, such fusion partners are useful in constructs comprising self-antigens, eg tumour specific or tissue specific antigens.

## Background to the invention

Streptococcus pneumoniae synthesises an N acetyl-L-alanine amidase, LytA, an autolysin that specifically degrades the peptidoglycan backbone of the cell wall eventually leading to cell lysis. Its polypeptide chain has two domains. The N-terminal domain is responsible for the catalytic activity, whereas the C-terminal domain of LytA is responsible for the affinity to choline and anchorage to the cell wall. This C-terminal domain is known to bind to choline and choline analogues, and will also bind to tertiary amines such as DEAE (diethyl amino ethyl) commonly used in chromatography.
LytA is a 318 amino acid protein, and the C-terminal part comprises a tandem of six imperfect repeats of 20 or 21 amino acids and a short COOH-terminal tail. The repeats are located at the following positions:

R1: 177-191
R2: 192-212
R3: 213-234
R4: 235-254
R5: 255-275
R6: 276-298

These repeats are predicted to be in a beta-turn conformation. The C-terminus is responsible for binding choline. Likewise the C-terminus of CPL1 is responsible for binding affinity and the aromatic residues in the repeat contribute to such binding. These proteins have been used as affinity tags to allow for rapid purification (Sanchez Puelles, Eur J Biochem. 1992, 203, 153-9).
Other proteins with a choline-binding domain have also been studied in Streptococcus pneumoniae.
One of them PspA (or Pneumococcal Surface Protein A), is a virulence factor (Yother J and Briles (1992) J Bacteriol 174(2) p 601). This protein is antigenic and immunogenic. It has a C-terminal domain consisting of 10 repeats of 20 amino acids, homologous with repeats of LytA.
CbpA (or Choline-Binding Protein A) is involved in the adherence of the pneumococcus to human cells (Rosenow et al (1997) Mol Microbiol 25 (5) p 819). It shows 10 repeats of 20 amino acids in the C-terminal domain which are almost identical to those of PspA.
LytB and LytC have a different modular organisation from the above-mentioned proteins as their choline-binding domain, made up of 15 repeats and 11 repeats respectively, is situated at the N-terminal end, not at the C-terminal end (Garcia P Mol Microbiol (1999) 31 (4) p1275 and Garcia P et al (1999) Mol Microbiol 33(1) p128). Sequence comparison shows LytB to have glucosamidase activity. LytC shows in vitro a lysozyme-type activity.
Additionally, three genes called PepA, PepB and PepC were cloned in 1995. Although their function is unknown, these genes also have a variable number of repeats homologous to those of LytA.
In their infection cycle, phages synthesise murein hydrolases facilitating their passage into the bacterium. These hydrolases have a choline-binding domain.
The muramidase CPL1 of the phage Cp-1 has been well studied. It shows 6 repeats of 20 amino acids at the C-terminus involved in the specific recognition of choline (Garica J. L. J. Virol 61 (8) p2573-80; (1987) and Garcia E Prol Natl Acad Sci (1988) p914). A comparison of the LytA and CPL1 repeats enables an initial consensus of those repeats to be made.
The murein hydrolases of phages Dp-1 (Garcia P et al (1983) J Gen Microbiol 129 (2) p489, Cpl-9 (Garcia P et al (1989) Biochem Biophys Res Commun 158(1) p 251, HB-3 Romero et al 1990 J Bacteriol 172 (9) p 5064-5070) and EJ-1 Diaz (1992) J Bacteriol 174 (17) p 5516), also show the characteristics of choline-binding domains.
This property is also shared by the lysozyme encoded by CP-1 a pneumococal phage.
WO 99/10375 describes *inter alia,* human papilloma virus proteins E6, or E7 linked to a His tag and the C-terminal portion of LytA (herein (C-LytA) and the purification of the proteins by differential affinity chromatography.

WO 99/40188 describes *inter alia* fusion proteins comprising MAGE antigens with a His tails and a C-LytA portion at the N-terminus of the molecule.

It has now been surprisingly found that fusion partners according to the present invention, when fused to a heterologous protein were capable of enhancing the immunogenicity of the heterologous proteins attached thereto. It has also been found that the expression level of the heterologous proteins attached thereto can be enhanced. The present invention accordingly provides in a preferred embodiment an improved immunological fusion partner which can also act as an expression enhancer.

## Summary of the invention

Accordingly the present invention comprises a fusion partner molecule comprising a choline binding domain or a fragment thereof or an analogue thereof, and a heterologous promiscuous T helper epitope, preferably a promiscuous MHC Class II T-epitope. Said fusion partner shows a capability of acting as both an immunological fusion partner, or as an expression enhancer and preferably as both an immunological partner and expression enhancer. A promiscuous T-helper epitope is an epitope that binds to more than one MHC Class II allele, preferably more than 3 MHC Class II alleles. In particular such epitopes are capable of eliciting helper T cell response in large numbers of individuals expressing diverse MHC haplotypes. Optionally, the fusion protein may retain its capability to bind to choline.

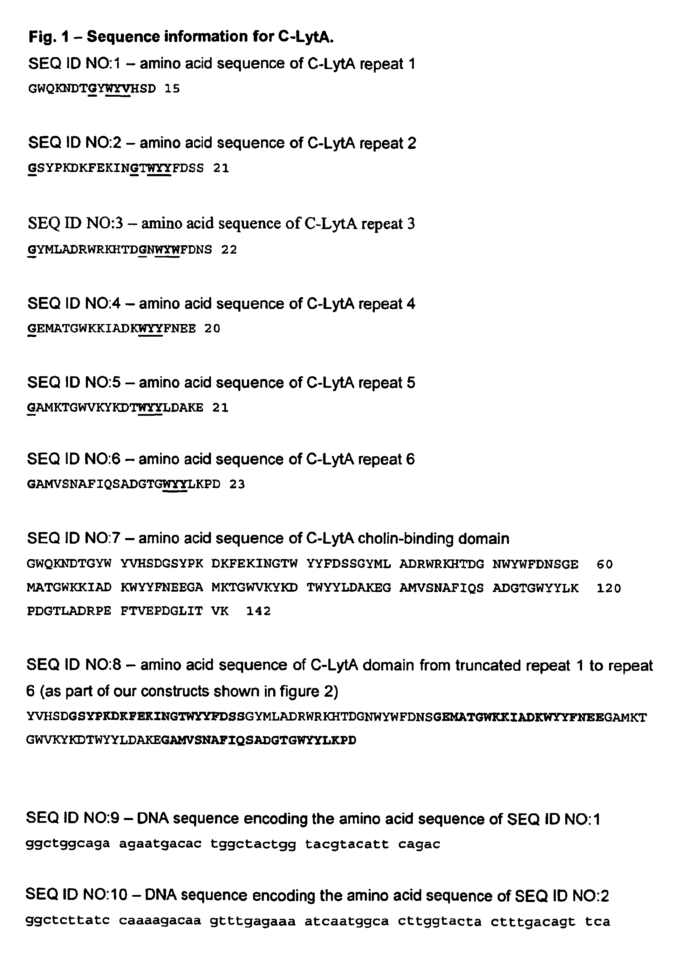

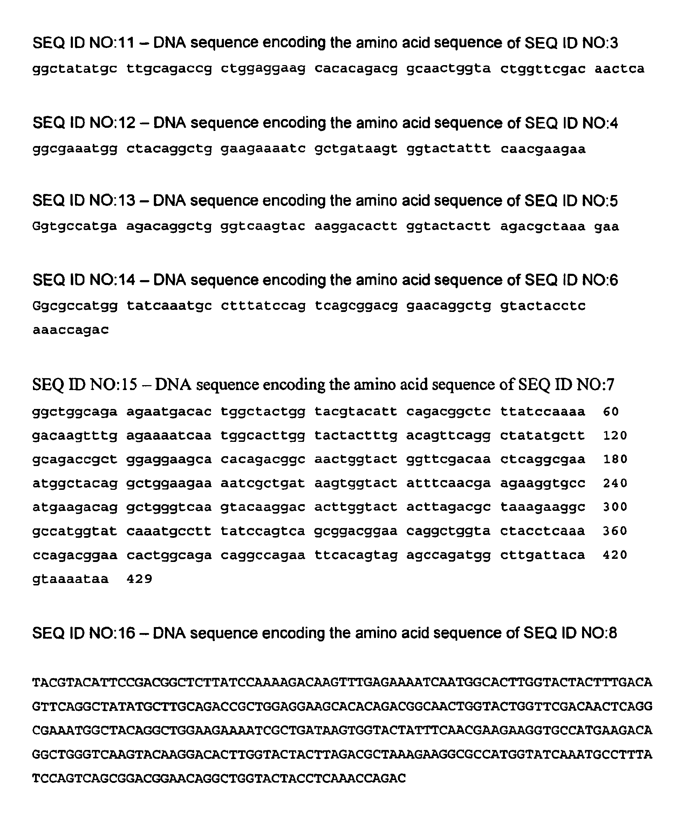

In a preferred embodiment the choline binding moiety is derived from the C terminus of LytA. Preferably the C-LytA or derivatives comprises at least four repeats of any of the repeats R1 to R6 set forth in figure 1 (SEQ ID NO:1 to 6). In a most preferred embodiment, the C-LytA extends from amino acid 177-298 which contains a portion of the first repeat and the complete five others.

In a further aspect of the invention, there is provided a fusion partner as herein defined further comprising a heterologous protein. The heterologous protein may be either chemically conjugated or fused to the fusion partner. Preferably the heterologous protein is a tumour-associated antigen or immunogenic fragment thereof.

In a further aspect of the invention there is provided a nucleic acid sequence encoding the proteins as herein defined. There is also provided an expression vector comprising said nucleic acid, and a host transformed with said nucleic acid or vector.

In a further aspect of the invention there is provided an immunogenic composition comprising a protein or a nucleic acid sequence as herein described, and a pharmaceutically acceptable excipient, diluent or carrier. Preferably the immunogenic composition further comprises a Th-1 inducing adjuvant.

In yet a further embodiment, the invention provides the immunogenic composition or protein and nucleic acids for use in medicine. In particular, there is provided a protein or a nucleic acid of the invention, in the manufacture of a medicament for eliciting an immune response in a patient, or for use in the treatment or prophylaxis of infectious diseases or cancer diseases.

The invention further provides for methods of treating a patient suffering from an infectious disease or a cancer disease, particularly carcinoma of the breast, lung (particularly non - small cell lung carcinoma), colorectal, ovarian, prostate, gastric and other GI (gastrointestinal) by the administration of a safe and effective amount of a composition or nucleic acid as herein described.

In yet a further embodiment the invention provides a method of producing an immunogenic composition as herein described by admixing a nucleic acid or protein of the invention with a pharmaceutically acceptable excipient, diluent or carrier.

## Detailed description of the invention

As described therein, in one embodiment of the present invention the modified choline binding domain (fusion partner) has a capability of acting as an expression enhancer with the resulting fusion protein will be expressed at a higher yield in a host cell as compared to the unfused protein, preferably at a yield greater than about 100% (2-fold higher) or 150% or more, as measured by SDS-PAGE followed by Coomassie blue staining or silver staining, optionally followed by gel scanning. The modified choline binding domain according to the invention has also the capability of acting as an immunological partner with the resulting fusion protein with a heterologous protein will be more immunogenic in a host as compared to the unfused heterologous protein.

In another embodiment of the present invention, the modified choline binding domain has the capability to act as an immunological fusion partner, allowing an enhanced immune response to be obtained with the fusion protein as compared to the heterologous protein alone.

In a preferred embodiment, the modified choline binding domain has a dual function, having the capability to act as both an immunological fusion partner and as an expression enhancer.

In a preferred embodiment the choline binding moiety is derived from the C terminus of LytA. Preferably the C-LytA or derivatives comprises at least two repeats, preferably at least four repeats. In this context, C-LytA derivatives refer to a variant of C-LytA according to the present invention, that is to say variants which have retained both the capability of acting as an immunological partner and an expression enhancer. Preferred variants include, for example, peptides comprising an amino acid sequence having at least 85% identity, preferably at least 90% identity, more preferably at least 95% identity, most preferably at least 97-99% identity, to any of the repeats R1 to R6 set forth in figure 1 (SEQ ID N0:1 to 6), or a peptide comprising an amino acid sequence having at least 15, 20, 30, 40, 50 or 100 contiguous amino acids from the amino acid sequence set forth in figure 1 (SEQ ID NO:1 to 8).

Accordingly, in one aspect of the invention there is provided a fusion partner protein comprising a modified choline binding domain and a heterologous promiscuous T helper epitope, wherein the choline binding domain is selected from the group comprising:

a) the C-terminal domain of LytA as set forth in SEQ ID NO:7;
b) the sequence of SEQ ID NO:8;
c) a peptide sequence comprising an amino acid sequence having at least 85% identity, preferably at least 90% identity, more preferably at least 95% identity, most preferably at least 97-99% identity, to any of SEQ ID NO:1 to 6;
d) a peptide sequence comprising an amino acid sequence having at least 15, 20, 30, 40, 50 or 100 contiguous amino acids from the amino acid sequence of SEQ ID NO:7 or SEQ ID NO:8.

In a most preferred embodiment, the C-LytA extends from amino acid 177-298 which contains a portion of the first repeat and the complete five others, as set forth in figure 1.

The second component of the fusion partner, the heterologous T-cell epitope is preferably selected from the group of epitopes that will bind to a number of individuals expressing more than one MHC II molecules in humans. For example, epitopes that are specifically contemplated are P2 and P30 epitopes from tetanus toxoid, Panina - Bordignon Eur. J. Immunol 19 (12), 2237 (1989). In a preferred embodiment the heterologous T-cell epitope is P2 or P30 from Tetanus toxin.

The P2 epitope has the sequence QYIKANSKFIGITE and corresponds to amino acids 830-843 of the Tetanus toxin. The P30 epitope (residues 947-967 of Tetanus Toxin) has the sequence FNNFTVSFWLRVPKVSASHLE. The FNNFTV sequence may optionally be deleted. Other universal T epitopes can be derived from the circumsporozoite protein from Plasmodium falciparum - in particular the region 378-398 having the sequence DIEKKIAKMEKASSVFNWNS (Alexander J, (1994) Immunity 1 (9), p 751-761). Another epitope is derived from Measles virus fusion protein at residue 288-302 having the sequence LSEIKGVIVHRLEGV (Partidos CD, 1990, J. Gen. Virol 71(9) 2099-2105). Yet another epitope is derived from hepatitis B virus surface antigen, in particular amino acids, having the sequence FFLLTRILTIPQSLD. Another set of epitopes is derived from diphteria toxin. Four of these peptides (amino acids 271-290, 321-340, 331-350, 351-370) map within the T domain of fragment B of the toxin, and the remaining 2 map in the R domain (411-430, 431-450):

PVFAGANYAAWAVNVAQVI
VHHNTEEIVAQSIALSSLMV
QSIALSSLMVAQAIPLVGEL
VDIGFAAYNFVESII NLFQV
QGESGHDIKITAENTPLPIA
GVLLPTIPGKLDVNKSKTHI
(Raju R., Navaneetham D., Okita D., Diethelm-Okita B., McCormick D., Conti-Fine B. M. (1995) Eur. J. Immunol. 25: 3207-14.)

The heterologous T-epitope is preferably fused to C-LytA containing at least 4 repeats, preferably repeat 2 -5 inclusive. One or more subsequent repeats may optionally be fused to the C-terminus of the T-epitope. Alternatively, the heterologous T-epitope is preferably inserted between two consecutive repeats of C-LytA containing a total of at least 4 repeats, or inserted into one of the repeats of C-LytA containing a total of at least 4 repeats. More preferably, the C-LytA contains 6 repeats and the heterologous epitope is inserted within and at the beginning of the sixth repeat of C-LytA.

The present invention further provides, in other aspects, fusion proteins that comprise at least one polypeptide as described above, as well as polynucleotides encoding such fusion proteins, typically in the form of pharmaceutical compositions, e.g., vaccine compositions, comprising a physiologically acceptable carrier and/or an immunostimulant. Thus a self-protein or other poorly immunogenic protein may be fused to either the N or C terminal end of the resulting fusion partner. Alternatively the self protein or poorly immunogenic protein may be inserted into the fusion partner. In an optional embodiment a histidine tag or at least four, preferably more than 6 histidine residues, may be fused to the alternative end of the poorly immunogenic protein. This would allow for the protein to be purified by affinity chromatography steps, as a histidine tail, typically comprising at least four, preferably six or more residues binds to metal ions and therefore is suitable for metal immobilised metal ion affinity chromatography (IMAC).
Typical constructs would therefore comprise:

- Poorly- immunogenic protein - C-LytA repeats$_{1-4}$ -$P_2$ epitope (inserted in or replacing C-LytA repeat$_5$)-C-LytA repeats
- C-LytA repeats$_{1-4}$ -$P_2$ epitope (inserted in or replacing C-LytA repeat$_5$) - C-LytA repeat$_6$-Poorly immunogenic protein
- Poorly immunogenic protein - C-LytA repeat$_{2-5}$ -$P_2$ epitope (inserted into C-LytA repeat$_6$)
- C-LytA$_{2-5}$ -$P_2$epitope (inserted into C-LytA repeat$_6$)- Poorly immunogenic protein.
- Poorly immunogenic protein C-LytA repeats$_{1-5}$-$P_2$ epitope- inserted in C-LytA repeat$_6$
- C-LytA repeats$_{1-5}$-$P_2$ epitope- inserted in C-LytA repeat$_6$- Poorly immunogenic protein
- Poorly immunogenic protein- $P_2$ epitope inserted into C-LytA repeat$_1$-C-LytA repeats$_{2-5}$
- $P_2$ epitope inserted into C-LytA repeat$_1$-C-LytA repeats$_{2-5}$- Poorly immunogenic protein
- Poorly immunogenic protein- $P_2$ epitope inserted into C-LytA repeat$_1$-C-LytA repeats$_{2-6}$
- $P_2$ epitope inserted into C-LytA repeat$_1$-C-LytA repeats$_{2-6}$- Poorly immunogenic protein
- Poorly immunogenic protein-C-LytA repeat$_1$-$P_2$ epitope inserted into C-LytA repeat$_2$-C-LytA repeats$_{3-6}$
- C-LytA repeat$_1$-$P_2$ epitope inserted into C-LytA repeat$_2$-C-LytA repeats$_{3-6}$- Poorly immunogenic protein;

where "inserted into" means at any place into the repeat for example between residue 1 and 2, or between 2 and 3, etc.

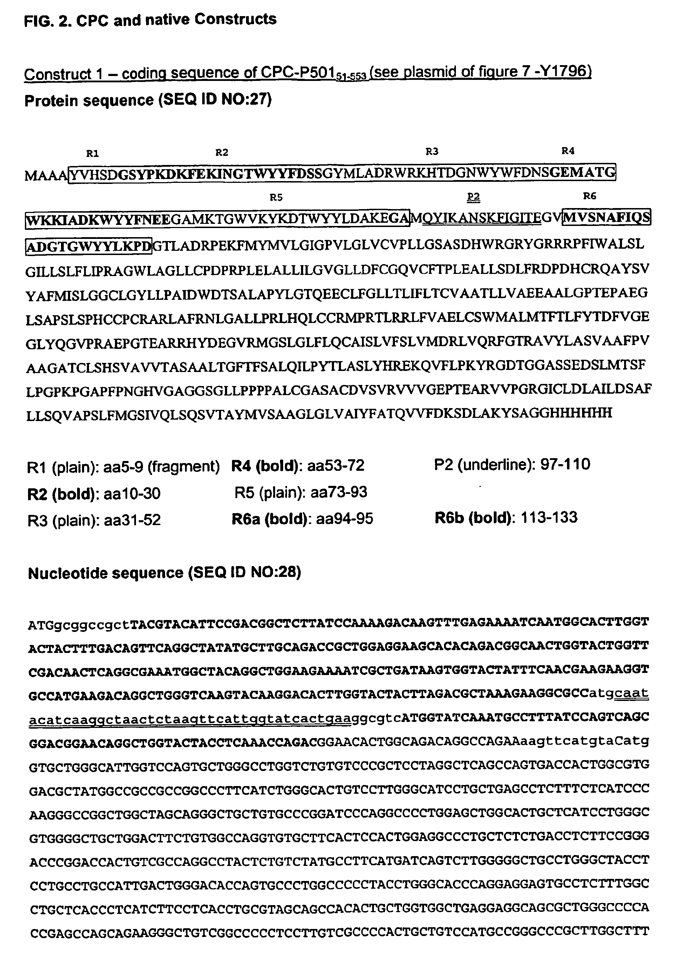

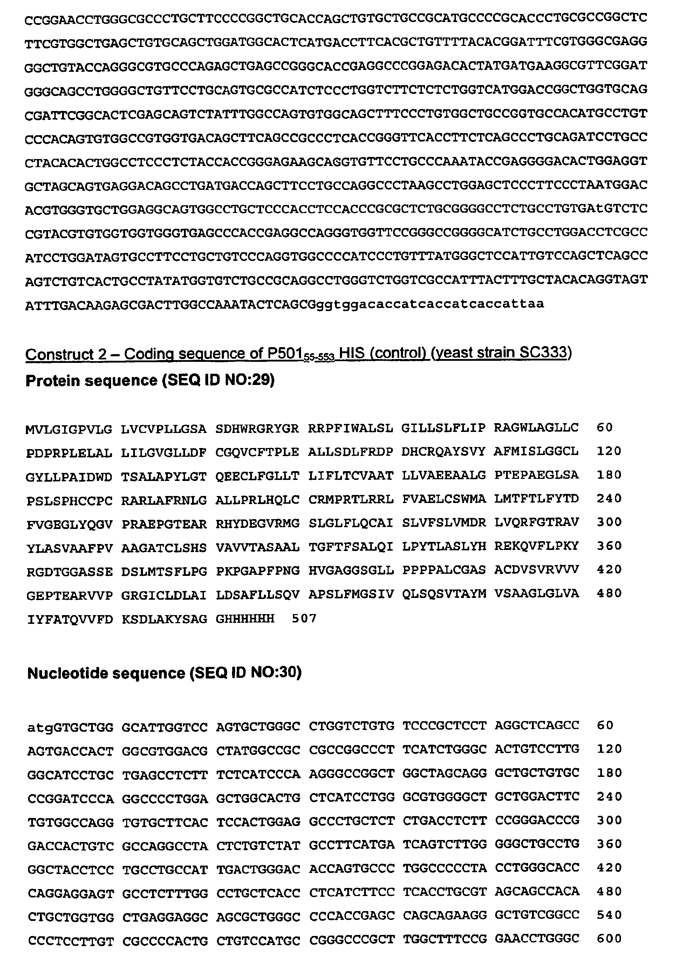

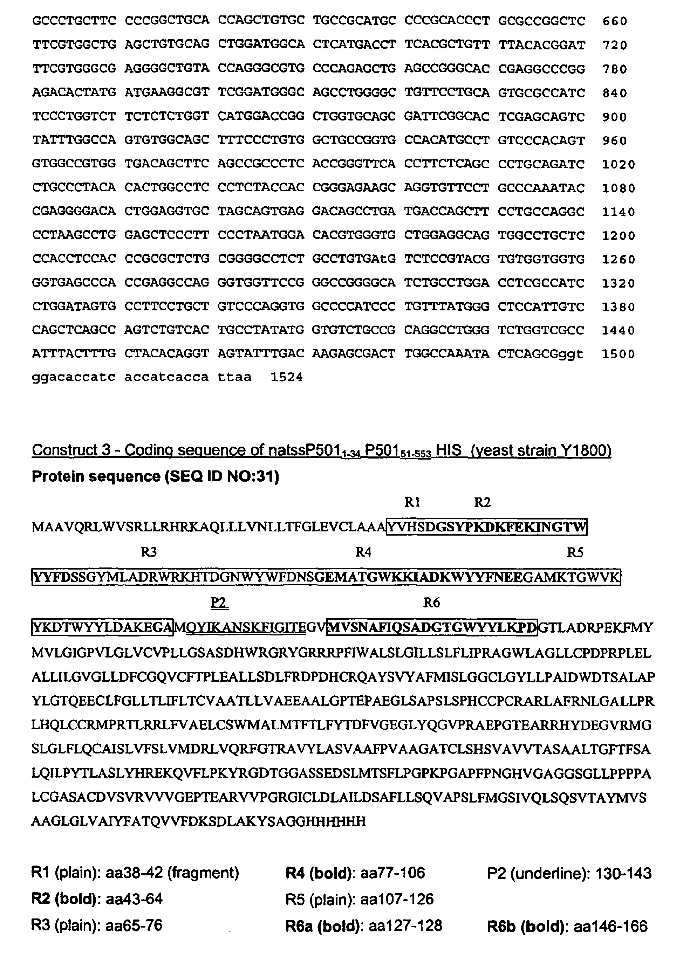

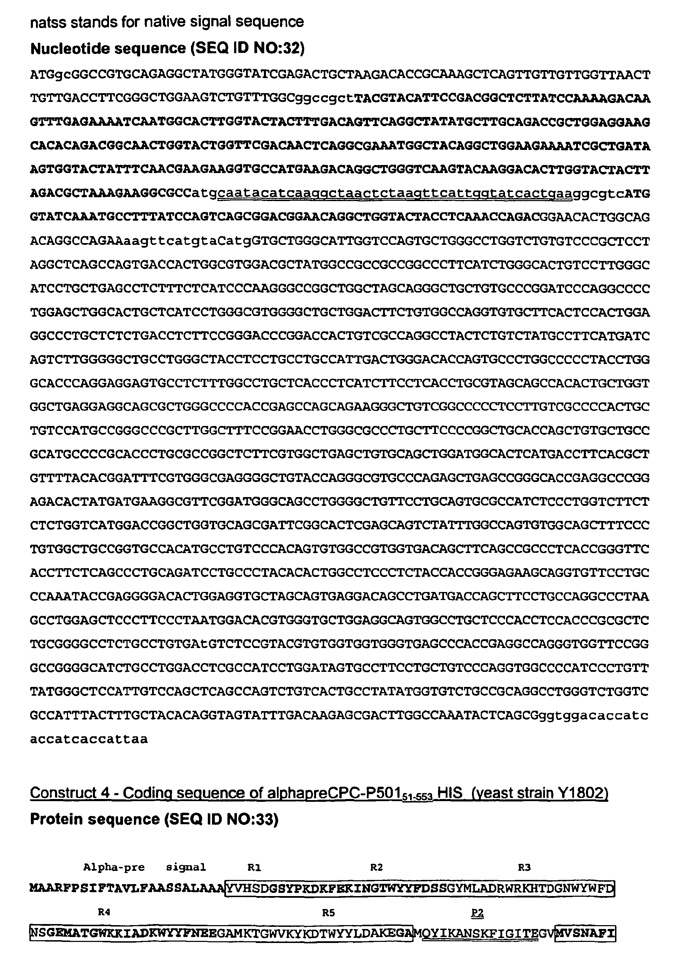

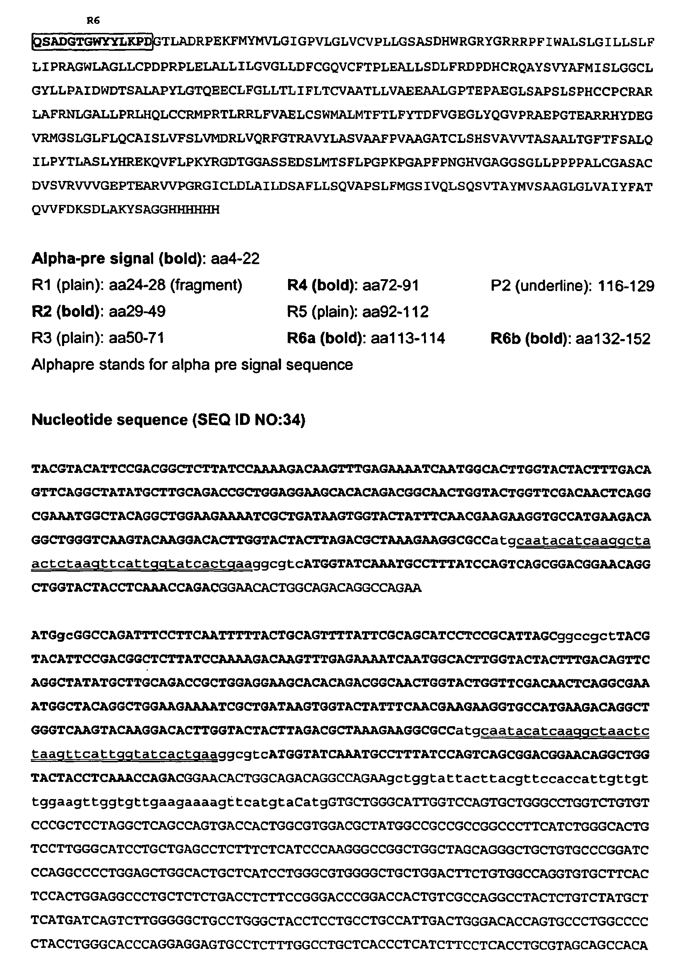

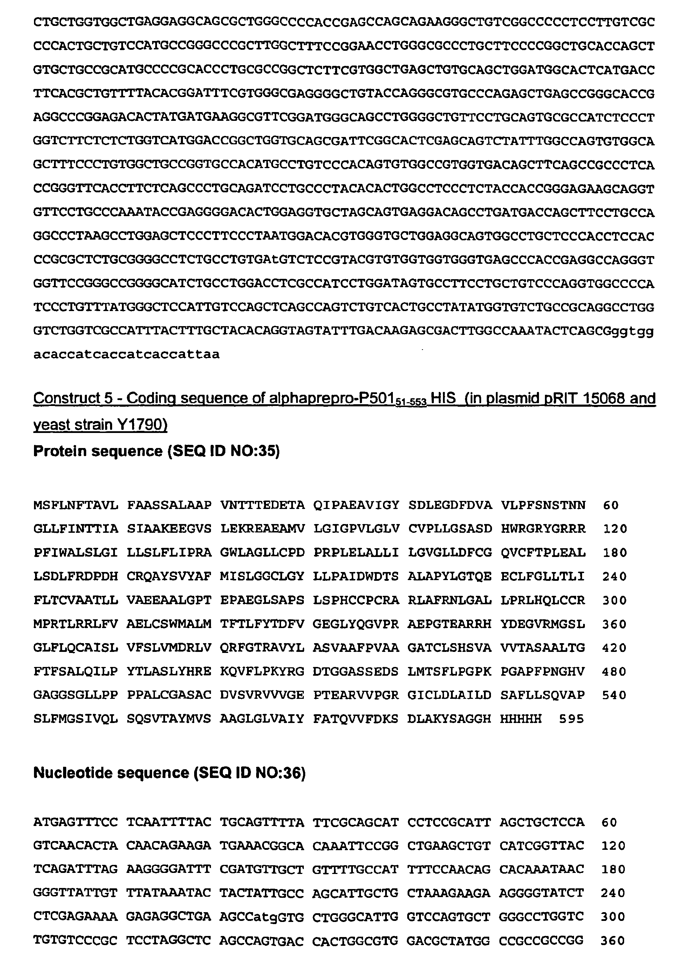

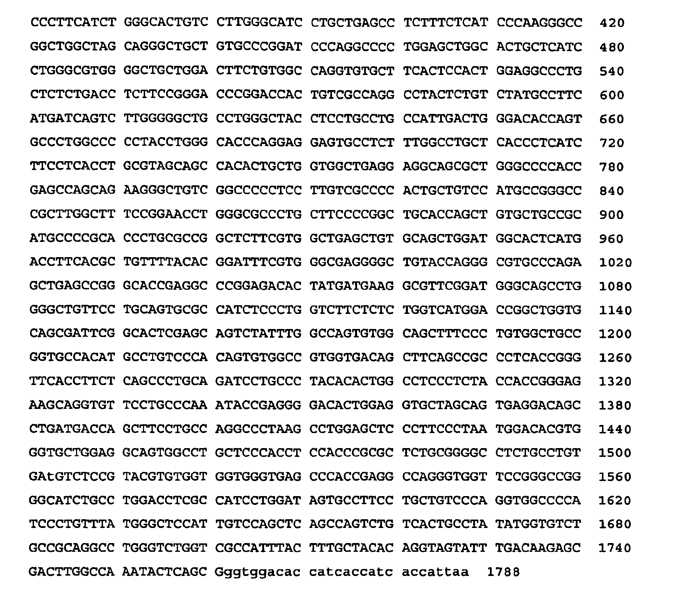

The promiscuous T helper epitope may be inserted within a repeat region for example C-LytA repeats $_{2-5_}$- C-LytA repeat 6a-$P_2$ epitope - C-LytA repeat 6b, where the P2 epitope is inserted within the sixth repeat (see figure 2).

In other preferred embodiments the C-terminal end of CPL1 (C-CPL1) may be used as an alternative to C-LytA.

Alternatively, the P2 epitope in the above constructs may be replaced by other promiscuous T epitopes, for example P30. In an embodiment of the invention, two or more promiscuous epitopes are part of the fusion construct. It is however preferred to keep the fusion partner as small as possible, thus limiting the number of potentially interfering CD8+ and B epitopes. Thus the fusion partner is preferably no bigger than 100-140 amino acids, preferably no bigger than 120 amino acids, typically about 100 amino acid.

The antigen to which the fusion partner is fused may be from bacterial, viral, protozoan, fungal or mammalian, including human, sources.

The fusion partner of the present invention are preferably fused to a self antigen such as a tumour associated or tissue specific antigens such as those for prostrate, breast, colorectal, lung, pancreatic, ovarian, renal or melanoma cancers. Fragments of said self or tumour antigens are expressly contemplated to be fused to the fusion partner of the invention. Typically the fragment will contain at least 20, preferably 50, more preferably 100 contiguous amino acids of the full-length sequence. Typically such fragments will be devoid of one or more transmembrane domains or may have N-terminal or C-terminal deletions of about 3, 5 , 8, 10, 15, 20, 28 , 33, 50, 54 amino acids. Such fragments will, when suitably presented, be able to generate immune responses that recognise the full length protein. Particularly illustrative polypeptides of the present invention comprise a sequence of at least 10 contiguous amino acids, preferably 20, more preferably 30, 40, 50, 60, 70, 80, 90, 100, 110, 120, 130, 140, 150, 160, 170, 180 amino acids of a tumour associated or tissue specific protein fused to the fusion partner.

The polypeptides of the invention are immunogenic, i.e., they react detectably within an immunoassay (such as an ELISA or T-cell stimulation assay) with antisera and/or T-cells from a patient with cripto expressing cancer. Screening for immunogenic activity can be performed using techniques well known to the skilled artisan. For example, such screens can be performed using methods such as those described in Harlow and Lane, *Antibodies: A Laboratory Manual,* Cold Spring Harbor Laboratory, 1988. In one illustrative example, a polypeptide may be immobilised on a solid support and contacted with patient sera to allow binding of antibodies within the sera to the immobilised polypeptide. Unbound sera may then be removed and bound antibodies detected using, for example, $^{125}$I-labeled Protein A. As would be recognised by the skilled artisan, immunogenic portions of tumour associated or tumour specific antigen are also encompassed by the present invention. An "immunogenic portion" as used herein, is a fragment that itself is immunologically reactive (i.e., specifically binds) with the B-cells and/or T-cell surface antigen receptors that recognize the polypeptide. Immunogenic portions may generally be identified using well known techniques, such as those summarized in Paul, *Fundamental Immunology,* 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (i.e., they react with the protein in an ELISA or other immunoassay, and do not react detectably with unrelated proteins). Such antisera and antibodies may be prepared as described herein, and using well-known techniques. In one preferred embodiment, an immunogenic portion of a polypeptide is a portion that reacts with antisera and/or T-cells at a level that is not substantially less than the reactivity of the full-length polypeptide (e.g., in an ELISA and/or T-cell reactivity assay). Preferably, the level of immunogenic activity of the immunogenic portion is at least about 50%, preferably at least about 70% and most preferably greater than about 90% of the immunogenicity for the full-length polypeptide. In some instances, preferred immunogenic portions will be identified that have a level of immunogenic activity greater than that of the corresponding full-length polypeptide, e.g., having greater than about 100% or 150% or more immunogenic activity.

In certain other embodiments, illustrative immunogenic portions may include peptides in which an N-terminal leader sequence and/or transmembrane domain have been deleted. Other illustrative immunogenic portions will contain a small N- and/or C-terminal deletion (e.g., about 1-50 amino acids, preferably about 1-30 amino acids, more preferably about 5-15 amino acids), relative to the mature protein.

Exemplary antigens or fragments derived therefrom include MAGE 1, Mage 3 and MAGE 4 or other MAGE antigens such as disclosed in WO 99/40188, PRAME (WO 96/10577), BAGE, RAGE, LAGE 1 (WO 98/32855), LAGE 2 (also known as NY-ESO-1, WO 98/14464), XAGE (Liu et al, Cancer Res, 2000, 60:4752-4755; WO 02/18584) SAGE, and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma.

In a preferred embodiment prostate antigens are utilised, such as Prostate specific antigen (PSA), PAP, PSCA (PNAS 95(4) 1735 -1740 1998), PSMA or the antigen known as prostase.

In a particularly preferred embodiment, the prostate antigen is P501S or a fragment thereof. P501S, also named prostein (Xu et al., Cancer Res. 61, 2001, 1563-1568), is known as SEQ ID NO. 113 of WO98/37814 and is a 553 amino acid protein. Immunogenic fragments and portions thereof comprising at least 20, preferably 50, more preferably 100 contiguous amino acids as disclosed in the above referenced patent application and are specifically contemplate by the present invention. Preferred fragments are disclosed in WO 98/50567 (PS108 antigen) and as prostate cancer-associated protein (SEQ ID NO: 9 of WO 99/67384). Other preferred fragments are amino acids 51-553, 34-553 or 55-553 of the full-length P501S protein. In particular, construct 1, 2 and 3 (see figure 2, SEQ ID NOs. 27-32) are expressly contemplated, and can be expressed in yeast systems, for example DNA sequences encoding such polypeptides can be expressed in yeast system.

Prostase is a prostate-specific serine protease (trypsin-like), 254 amino acid-long, with a conserved serine protease catalytic triad H-D-S and a amino-terminal pre-propeptide sequence, indicating a potential secretory function (P. Nelson, Lu Gan, C. Ferguson, P. Moss, R. linas, L. Hood & K. Wand, "Molecular cloning and characterisation of prostase, an androgen-regulated serine protease with prostate restricted expression, In Proc. Natl. Acad. Sci. USA (1999) 96, 3114-3119). A putative glycosylation site has been described. The predicted structure is very similar to other known serine proteases, showing that the mature polypeptide folds into a single domain. The mature protein is 224 amino acids-long, with one A2 epitope shown to be naturally processed. Prostase nucleotide sequence and deduced polypeptide sequence and homologous are disclosed in Ferguson, et al. (Proc. Natl. Acad. Sci. USA 1999, 96, 3114-3119) and in International Patent Applications No. WO 98/12302 (and also the corresponding granted patent US 5,955,306), WO 98/20117 (and also the corresponding granted patents US 5,840,871 and US 5,786,148) (prostate-specific kallikrein) and WO 00/04149 (P703P).

Other prostate specific antigens are known from WO98/37418, and WO/004149. Another is STEAP (PNAS 96 14523 14528 7 -12 1999).

Other tumour associated antigens useful in the context of the present invention include: Plu -1 J Biol. Chem 274 (22) 15633 -15645, 1999, HASH -1, HASH-2 (Alders,M. et al., Hum. Mol. Genet. 1997, 6, 859-867). Cripto (Salomon et al Bioessays 199, 21 61 -70,US patent 5654140), CASB616 (WO 00/53216), Criptin (US 5,981,215). Additionally, antigens particularly relevant for vaccines in the therapy of cancer also comprise tyrosinase, telomerase, P53, NY-Br1.1 (WO 01/47959) and fragments thereof such as disclosed in WO 00/43420, B726 (WO 00/60076, SEQ ID nos 469 and 463; WO 01/79286, SEQ ID nos 474 and 475), P510 (WO 01/34802 SEQ ID nos 537 and 538) and survivin.

The present invention is also useful in combination with breast cancer antigens such as Her-2/neu, mammaglobin (US patent 5,668,267), B305D (WO 00/61753 SEQ ID nos 299, 304, 305 and 315), or those disclosed in WO 00/52165, WO 99/33869, WO 99/19479, WO 98/45328. Her-2/neu antigens are disclosed inter alia, in US patent 5,801,005. Preferably the Her-2/neu comprises the entire extracellular domain (comprising approximately amino acid 1-645) or fragments thereof and at least an immunogenic portion of or the entire intracellular domain approximately the C terminal 580 amino acids. In particular, the intracellular portion should comprise the phosphorylation domain or fragments thereof. Such constructs are disclosed in WO 00/44899. A particularly preferred construct is known as ECD-PhD, a second is known as ECD deltaPhD (see WO 00/44899). The Her-2/neu as used herein can be derived from rat, mouse or human.

Certain tumour antigens are small peptide antigens (ie less than about 50 amino acids). These antigens can be chemically conjugated to the modified choline binding protein of the present invention.

Exemplary peptides included Mucin derived peptides such as MUC-1 (see for example US 5,744,144; US 5,827,666; WO 88/05054, US 4,963,484). Specifically contemplated are MUC-1 derived peptides that comprise at least one repeat unit of the MUC-1 peptide, preferably at least two such repeats and which is recognised by the SM3 antibody (US 6,054,438). Other mucin derived peptides include peptide from MUC-5.

Alternatively, said antigen is an interleukin such as IL13 and IL14, which are preferred. Or said antigen maybe a self peptide hormone such as whole length Gonadotrophin hormone releasing hormone (GnRH, WO 95/20600), a short 10 amino acid long peptide, useful in the treatment of many cancers, or in immunocastration.

Other tumour-specific antigens are suitable to be coupled with the modified Choline binding protein of the present invention include, but are not restricted to tumour-specific gangliosides such as GM2, and GM3.

The covalent coupling of the peptide to modified choline binding protein can be carried out in a manner well known in the art. Thus, for example, for direct covalent coupling it is possible to utilise a carbodiimide, glutaraldehyde or (N-[γ-maleimidobutyryloxy] succinimide ester, utilising common commercially available heterobifunctional linkers such as CDAP and SPDP (using manufacturers instructions). After the coupling reaction, the immunogen can easily be isolated and purified by means of a dialysis method, a gel filtration method, a fractionation method etc.

The antigen may also be derived from sources which are pathogenic to humans, such as such as Human Immunodeficiency virus HIV-1 (such as tat, nef, reverse transcriptase, gag, gp120 and gp160), human herpes simplex viruses, such as gD or derivatives thereof or Immediate Early protein such as ICP27 from HSV1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpl, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-bome encephalitis virus, Japanese Encephalitis Virus) or Influenza virus (whole live or inactivated virus, split influenza virus, grown in eggs or MDCK cells, or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp*, including *N. gonorrhea* and *N. meningitidis* (for example capsular polysaccharides and conjugates thereof, transferrin-binding proteins, lactoferrin binding proteins, PIIC, adhesins); *S. pyogenes* (for example M proteins or fragments thereof, C5A protease, lipoteichoic acids), *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C), *M. bovis, M. leprae, M. avium, M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic E. *coli,* enteropathogenic *E. coli* (for example shiga toxin-like toxin or derivatives thereof); *Vibrio spp,* including *V. cholera* (for example cholera toxin or derivatives thereof); *Shigella spp*, including *S. sonnei, S. dysenteriae, S. flexnerii; Yersinia spp,* including *Y*. enterocolitica (for example a Yop protein) , *Y. pestis, Y. pseudotuberculosis; Campylobacter spp,* including *C. jejuni* (for example toxins, adhesins and invasins) and C. *coli, Salmonella spp,* including *S. typhi, S. paratyphi, S. choleraesuis, S. enteritidis; Listeria* spp., including *L. monocytogenes; Helicobacter spp,* including *H*. pylori (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C*. tetani (for example tetanus toxin and derivative thereof), *C. botulinum* (for example botulinum toxin and derivative thereof), *C. difficile* (for example clostridium toxins A or B and derivatives thereof); *Bacillus spp.,* including *B. anthracis* (for example botulinum toxin and derivatives thereof); *Corynebacterium spp.,* including *C. diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC,-DbpA, DbpB), B. *hermsii, Ehrlichia spp.,* including *E. equi* and the agent of the Human Granulocytic Ehrlichiosis; *Rickettsia spp,* including *R. rickettsii; Chlamydia spp., including C. trachomatis* (for example MOMP, heparin-binding proteins), *C. pneumoniae* (for example MOMP, heparin-binding proteins), *C. psittaci; Leptospira spp.,* including *L. interrogans; Treponema spp.*, including *T. pallidum* (for example the rare outer membrane proteins), *T. denticola, T. hyodysenteriae;* or derived from parasites such as *Plasmodium spp.,* including *P. falciparum, Toxoplasma spp.,* including *T. gondii (for example SAG2, SAG3, Tg34); Entamoeba spp.,* including *E. histolytica; Babesia spp.,* including *B. microti; Trypanosoma spp.*, including *T. cruzi; Giardia spp.,* including *G. lamblia; Leshmania spp.,* including *L. major; Pneumocystis spp.,* including *P. carinii; Trichomonas spp.,* including *T. vaginalis; Schisostoma spp.,* including *S. mansoni,* or derived from yeast such as *Candida spp.,* including *C. albicans; Cryptococcus spp.,* including *C. neoformans.*

Other preferred specific antigens for *M. tuberculosis* are for example Tb Ral2, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erdl4-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial antigens are derived from *Streptococcus spp,* including *S. pneumoniae* (for example capsular polysaccharides and conjugates thereof, PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial antigens are derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof.

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred The HBV antigen is HBV polymerase (Ji Hoon Jeong et al, 1996, BBRC 223, 264-271; Lee H.J. et al , Biotechnol. Lett. 15, 821-826). In another preferred aspect the antigen within the fusion is a HIV-1 antigen, gp120, especially when expressed in CHO cells. In a further embodiment, antigen comprises gD2t as hereinabove defined.

In a preferred embodiment of the present invention fusions comprise an antigen derived from the Human Papilloma Virus (HPV 6a, 6b, 11, 16, 18, 31, 33, 35, 39, 45, 51, 52, 56, 58, 59 and 68), in particular those HPV serotypes considered to be responsible for genital warts (HPV 6 or HPV 11 and others), and the HPV viruses responsible for cervical cancer (HPV16, HPV18 and others).

Suitable HPV antigens are E1, E2, E4, E5, E6, E7, L1 and L2. Particularly preferred forms of genital wart prophylactic, or therapeutic, fusions comprise L1 particles or capsomers, and fusion proteins comprising one or more antigens selected from the HPV 6 and HPV 11 proteins E6, E7, L1, and L2.

The most preferred forms of fusion protein are: L2E7 as disclosed in WO 96/26277, and proteinD(1/3)-E7 disclosed in GB 9717953.5 (PCT/EP98/05285).

A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or caposmer structure. Such antigens, virus like particles and capsomer are per se known. See for example WO94/00152, WO94/20137, WO94/05792, and WO93/02184.

Additional early proteins may be included alone or as fusion proteins such as E7, E2 or preferably E5 for example; particularly preferred embodiments of this includes a VLP comprising L1 E7 fusion proteins (WO 96/11272). Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277). Alternatively the HPV 16 or 18 early proteins E6 and E7, may be presented in a single molecule, preferably a Protein D- E6/E7 fusion. Other fusions optionally contain either or both E6 and E7 proteins from HPV 18, preferably in the form of a Protein D - E6 or Protein D - E7 fusion protein or Protein D E6/E7 fusion protein. Fusions may comprise antigens from other HPV strains, preferably from strains HPV 31 or 33.

Fusions according to the present invention comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. Its full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a fusion wherein the antigenic preparation comprises a combination of the RTS, S and TRAP antigens. Other plasmodia antigens that are likely candidates to be components of the fusion are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The present invention also provides a polynucleotide encoding the fusion partner according to the present invention. The invention further relates a polynucleotide that hybridise to the polynucleotide sequence provided herein in figure 1 (SEQ ID NO:9 to 16). In this regard, the invention especially relates to polynucleotides that hybridise under stringent conditions to the polynucleotide described herein. As herein used, the terms "stringent conditions" and "stringent hybridisation conditions" mean hybridisation occurring only if there is at least 95% and preferably at least 97% identity between the sequences. A specific example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml of denatured, sheared salmon sperm DNA, followed by washing the hybridisation support in 0.1x SSC at about 65°C. Hybridisation and wash conditions are well known and exemplified in Sambrook, *et al.,* Molecular Cloning: A Laboratory Manual, Second Edition, Cold Spring Harbor, N.Y., (1989), particularly Chapter 11 therein. Solution hybridisation may also be used with the polynucleotide sequences provided by the invention.

The present invention also provides a polynucleotide encoding the polypeptide comprising the fusion partner according to the present invention fused to a tumour associated antigen or fragment thereof. In particular, the present invention provides for polynucleotide sequences encoding a fusion partner protein comprising a choline binding domain and a heterologous promiscuous T heper epitope, preferably wherein the choline binding domain is derived from the C terminus of LytA. In a more preferred embodiment, the C-LytA moiety of the polynucleotides according to the invention comprise at least four repeats of any of SEQ ID NO.9-14, more preferably comprise the sequence of SEQ ID NO.15, still more preferably the sequence of SEQ ID NO.16. In other related embodiments, the present invention provides for polynucleotide variants having substantial identity to the sequences disclosed herein in SEQ ID NOs:9-16, for example those comprising at least 70% sequence identity, preferably at least 75%, 80%, 85%, 90%, 95%, 96%, 97%, 98%, or 99% or higher, sequence identity compared to a polynucleotide sequence of this invention using conventional methods, e.g., BLAST analysis using standard parameters. In a still further embodiment the polynucleotide as claimed further comprises a heterologous protein.

Such polynucleotide sequences can be inserted into a suitable expression vector and expressed in a suitable host. Vectors may be provided which encode the modified choline binding protein of the invention and which contain a suitable restriction site into which a DNA encoding a poorly immunogenic protein can be inserted to produce a fusion protein.

In other embodiments of the invention, polynucleotide sequences or fragments thereof which encode polypeptide fusions of the invention, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. The DNA code has 4 letters (A, T, C and G) and uses these to spell three letter "codons" which represent the amino acids the proteins encodes in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilisation of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in *E.coli* or a viral gene expressed in mammalian cells will have an inappropriate distribution of codons for efficient expression. It is believed that the presence in a heterologous DNA sequence of clusters of codons which are rarely observed in the host in which expression is to occur, is predictive of low heterologous expression levels in that host.

In consequence, codons preferred by a particular prokaryotic (for example E. *coli* or yeast) or eukaryotic host can be optimised, that is selected to increase the rate of protein expression, to produce a recombinant RNA transcript having desirable properties, such as for example a half-life which is longer than that of a transcript generated from the naturally occurring sequence, or to optimise the immune response in humans. The process of codon optimisation may include any sequence, generated either manually or by computer software, where some or all of the codons of the native sequence are modified. Several methods have been published (Nakamura et.al., Nucleic Acids Research 1996, 24: 214-215; WO98/34640). One preferred method according to this invention is Syngene method, a modification of Calcgene method (R. S. Hale and G Thompson (Protein Expression and Purification Vol. 12 pp.185-188 (1998)).

Accordingly in a preferred embodiment the DNA sequence of the protein has a RSCU (Relative synomons Codon useage (also known as Codon Index CI)) of at least 0.65 and have less than 85% identity to the corresponding wild type region.

This process of codon optimisation and the resulting constructs are advantageous as they may have some or all of the following benefits: 1) to improve expression of the gene product by replacing rare or infrequently used codons with more frequently used codons, 2) to remove or include restriction enzyme sites to facilitate downstream cloning and 3) to reduce the potential for homologous recombination between the insert sequence in the DNA vector and genomic sequences and 4) to improve the immune response in humans by raising a cellular and/or an antibody response (preferably both responses) against the target antigen. The sequences of the present invention advantageously have reduced recombination potential, but express to at least the same level as the wild type sequences. Due to the nature of the algorithms used by the SynGene programme to generate a codon optimised sequence, it is possible to generate an extremely large number of different codon optimised sequences which will perform a similar function. In brief, the codons are assigned using a statistical method to give synthetic gene having a codon frequency closer to that found naturally in highly expressed E.coli and human genes. In brief, the codons are assigned using a statistical method to give synthetic gene having a codon frequency closer to that found naturally in highly expressed human genes such as β-Actin. Illustrative, although non limiting, examples of suitable codon-optimised sequences are given in SEQ ID NOs:19-22 and SEQ ID NOs:24-26.

In the polynucleotides of the present invention, the codon usage pattern is altered from that typical of the target antigen to more closely represent the codon bias of a highly expressed gene in a target organism, for example human 6-actin. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude codons representing < 10% of the codons used for a particular amino acid. A relative synonymous codon usage (RSCU) value is the observed number of codons divided by the number expected if all codons for that amino acid were used equally frequently. A polynucleotide of the present invention will preferably exclude codons with an RSCU value of less than 0.2 in highly expressed genes of the target organism. A polynucleotide of the present invention will generally have a codon usage coefficient for highly expressed human genes of greater than 0.6, preferably greater than 0.65, most preferably greater than 0.7. Codon usage tables for human can also be found in Genbank.

In comparison, a highly expressed beta actin gene has a RSCU of 0.747.

The codon usage table (Table 1) for a homo sapiens is set out below:

**Table 1. Codon usage for human (highly expressed) genes** 1/24/91 (human_high.cod)

| AmAcid | Codon | Number | /1000 | Fraction |
|---|---|---|---|---|
| Gly | GGG | 905.00 | 18.76 | 0.24 |
| Gly | GGA | 525.00 | 10.88 | 0.14 |
| Gly | GGT | 441.00 | 9.14 | 0.12 |
| Gly | GGC | 1867.00 | 38.70 | 0.50 |
| Glu | GAG | 2420.00 | 50.16 | 0.75 |
| Glu | GAA | 792.00 | 16.42 | 0.25 |
| Asp | GAT | 592.00 | 12.27 | 0.25 |
| Asp | GAC | 1821.00 | 37.75 | 0.75 |
| | | | | |
| Val | GTG | 1866.00 | 38.68 | 0.64 |
| Val | GTA | 134.00 | 2.78 | 0.05 |
| Val | GTT | 198.00 | 4.10 | 0.07 |
| Val | GTC | 728.00 | 15.09 | 0.25 |
| | | | | |
| Ala | GCG | 652.00 | 13.51 | 0.17 |
| Ala | GCA | 488.00 | 10.12 | 0.13 |
| Ala | GCT | 654.00 | 13.56 | 0.17 |
| Ala | GCC | 2057.00 | 42.64 | 0.53 |
| | | | | |
| Arg | AGG | 512.00 | 10.61 | 0.18 |
| Arg | AGA | 298.00 | 6.18 | 0.10 |
| Ser | AGT | 354.00 | 7.34 | 0.10 |

(continued)

| AmAcid | Codon | Number | /1000 | Fraction |
|---|---|---|---|---|
| Ser | AGC | 1171.00 | 24.27 | 0.34 |
| | | | | |
| Lys | AAG | 2117.00 | 43.88 | 0.82 |
| Lys | AAA | 471.00 | 9.76 | 0.18 |
| Asn | AAT | 314.00 | 6.51 | 0.22 |
| Asn | AAC | 1120.00 | 23.22 | 0.78 |
| | | | | |
| Met | ATG | 1077.00 | 22.32 | 1.00 |
| Ile | ATA | 88.00 | 1.82 | 0.05 |
| Ile | ATT | 315.00 | 6.53 | 0.18 |
| Ile | ATC | 1369.00 | 28.38 | 0.77 |
| | | | | |
| Thr | ACG | 405.00 | 8.40 | 0.15 |
| Thr | ACA | 373.00 | 7.73 | 0.14 |
| Thr | ACT | 358.00 | 7.42 | 0.14 |
| Thr | ACC | 1502.00 | 31.13 | 0.57 |
| | | | | |
| Trp | TGG | 652.00 | 13.51 | 1.00 |
| End | TGA | 109.00 | 2.26 | 0.55 |
| Cys | TGT | 325.00 | 6.74 | 0.32 |
| Cys | TGC | 706.00 | 14.63 | 0.68 |
| | | | | |
| End | TAG | 42.00 | 0.87 | 0.21 |
| End | TAA | 46.00 | 0.95 | 0.23 |
| Tyr | TAT | 360.00 | 7.46 | 0.26 |
| Tyr | TAC | 1042.00 | 21.60 | 0.74 |
| | | | | |
| Leu | TTG | 313.00 | 6.49 | 0.06 |
| Leu | TTA | 76.00 | 1.58 | 0.02 |
| Phe | TTT | 336.00 | 6.96 | 0.20 |
| Phe | TTC | 1377.00 | 28.54 | 0.80 |
| | | | | |
| Ser | TCG | 325.00 | 6.74 | 0.09 |
| Ser | TCA | 165.00 | 3.42 | 0.05 |
| Ser | TCT | 450.00 | 9.33 | 0.13 |
| Ser | TCC | 958.00 | 19.86 | 0.28 |
| | | | | |
| Arg | CGG | 611.00 | 12.67 | 0.21 |
| Arg | CGA | 183.00 | 3.79 | 0.06 |
| Arg | CGT | 210.00 | 4.35 | 0.07 |
| Arg | CGC | 1086.00 | 22.51 | 0.37 |
| | | | | |
| Gln | CAG | 2020.00 | 41.87 | 0.88 |
| Gln | CAA | 283.00 | 5.87 | 0.12 |
| His | CAT | 234.00 | 4.85 | 0.21 |
| His | CAC | 870.00 | 18.03 | 0.79 |
| | | | | |
| Leu | CTG | 2884.00 | 59.78 | 0.58 |
| Leu | CTA | 166.00 | 3.44 | 0.03 |
| Leu | CTT | 238.00 | 4.93 | 0.05 |

(continued)

| AmAcid | Codon | Number | /1000 | Fraction |
|---|---|---|---|---|
| Leu | CTC | 1276.00 | 26.45 | 0.26 |
| | | | | |
| Pro | CCG | 482.00 | 9.99 | 0.17 |
| Pro | CCA | 456.00 | 9.45 | 0.16 |
| Pro | CCT | 568.00 | 11.77 | 0.19 |
| Pro | CCC | 1410.00 | 29.23 | 0.48 |

A DNA sequence encoding the fusion proteins or modified choline binding protein of the present invention can be synthesised using standard DNA synthesis techniques, such as by enzymatic ligation as described by D.M. Roberts *et al.* in Biochemistry 1985, 24, 5090-5098, by chemical synthesis, by *in vitro* enzymatic polymerisation, or by PCR technology utilising for example a heat stable polymerase, or by a combination of these techniques.

Enzymatic polymerisation of DNA may be carried out *in vitro* using a DNA polymerase such as DNA polymerase I (Klenow fragment) or Taq polymerase in an appropriate buffer containing the nucleoside triphosphates dATP, dCTP, dGTP and dTTP as required at a temperature of 10°-37°C, generally in a volume of 50pi or less. Enzymatic ligation of DNA fragments may be carried out using a DNA ligase such as T4 DNA ligase in an appropriate buffer, such as 0.05M Tris (pH 7.4), 0.01 M $MgCl_2$, 0.01 M dithiothreitol, 1mM spermidine, 1mM ATP and 0.1 mg/ml bovine serum albumin, at a temperature of 4°C to ambient, generally in a volume of 50 μl or less. The chemical synthesis of the DNA polymer or fragments may be carried out by conventional phosphotriester, phosphate or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat, and W. Bannwarth, Tetrahedron Letters, 1983, 24, 5771; M.D. Matteucci and M.H. Caruthers, Tetrahedron Letters, 1980, 21, 719; M.D. Matteucci and M.H. Caruthers, Journal of the American Chemical Society, 1981, 103, 3185; S.P. Adams *et al.,* Journal of the American Chemical Society, 1983, 105, 661; N.D. Sinha, J. Biemat, J. McMannus, and H. Koester, Nucleic Acids Research, 1984, 12, 4539; and H.W.D. Matthes *et al.*, EMBO Journal, 1984, 3, 801.

The process of the invention may be performed by conventional recombinant techniques such as described in Maniatis *et al.,* Molecular Cloning - A Laboratory Manual; Cold Spring Harbor, 1982-1989.

In particular, the process may comprise the steps of :

i) preparing a replicable or integrating expression vector capable, in a host cell, of expressing a DNA polymer comprising a nucleotide sequence that encodes the protein or an immunogenic derivative thereof
ii) transforming a host cell with said vector
iii) culturing said transformed host cell under conditions permitting expression of said DNA polymer to produce said protein; and
iv) recovering said protein

The term 'transforming' is used herein to mean the introduction of foreign DNA into a host cell. This can be achieved for example by transformation, transfection or infection with an appropriate plasmid or viral vector using e.g. conventional techniques as described in Genetic Engineering; Eds. S.M. Kingsman and A.J. Kingsman; Blackwell Scientific Publications; Oxford, England, 1988. The term 'transformed' or 'transformant' will hereafter apply to the resulting host cell containing and expressing the foreign gene of interest.

The expression vectors are novel and also form part of the invention.

The replicable expression vectors may be prepared in accordance with the invention, by cleaving a vector compatible with the host cell to provide a linear DNA segment having an intact replicon, and combining said linear segment with one or more DNA molecules which, together with said linear segment encode the desired product, such as the DNA polymer encoding the protein of the invention, or derivative thereof, under ligating conditions.

Thus, the DNA polymer may be performed or formed during the construction of the vector, as desired.

The choice of vector will be determined in part by the host cell, which may be prokaryotic or eukaryotic but are preferably E. *coli,* yeast or CHO cells. Suitable vectors include plasmids, bacteriophages, cosmids and recombinant viruses. Expression and cloning vectors preferably contain a selectable marker such that only the host cells expressing the marker will survive under selective conditions. Selection genes include but are not limited to the one encoding protein that confer a resistance to ampicillin, tetracyclin or kanamycin. Expression vectors also contain control sequences which are compatible with the designated host. For example, expression control sequences for E. *coli,* and more generally for prokaryotes, include promoters and ribosome binding sites. Promoter sequences may be naturally occurring, such as the β-lactamase (penicillinase) (Weissman 1981, *In Interferon* 3 (ed. L. Gresser), lactose (lac) (Chang et al. Nature, 1977, 198: 1056) and tryptophan (trp) (Goeddel et al. Nucl. Acids Res. 1980, 8, 4057) and lambda-derived $P_L$ promoter system. In addition, synthetic promoters which do not occur in nature also function as bacterial promoters. This is the case for example for the tac synthetic hybrid promoter which is derived from sequences of the trp and lac promoters (De Boer et al., Proc. Natl Acad Sci. USA 1983, 80, 21-26). These systems are particularly suitable with E. *coli.*

Yeast compatible vectors also carry markers that allow the selection of successful transformants by conferring prototrophy to auxotrophic mutants or resistance to heavy metals on wild-type strains. Expression control sequences for yeast vectors include promoters for glycolytic enzymes (Hess et al., J. Adv. Enzyme Reg. 1968, 7, 149), PH05 gene encoding acid phosphatase, CUP1 gene, ARG3 gene, GAL genes promoters and synthetic promoter sequences. Other control elements useful in yeast expression are terminators and mRNA leader sequences. The 5' coding sequence is particularly useful since it typically encodes a signal peptide comprised of hydrophobic amino acids which direct the secretion of the protein from the cell. Suitable signal sequences can be encoded by genes for secreted yeast proteins such as the yeast invertase gene and the a-factor gene, acid phosphatase, killer toxin, the alpha-mating factor gene and recently the heterologous inulinase signal sequence derived from INU1A gene of Kluyveromyces marxianus.. Suitable vectors have been developed for expression in *Pichia pastoris* and *Saccharomyces cerevisiae.*

A variety of *P. pastoris* expression vectors are available based on various inducible or constitutive promoters ( Cereghino and Cregg, FEMS Microbiol. Rev. 2000,24:45-66). For the production of cytosolic and secreted proteins, the most commonly used P. pastoris vectors contain the very strong and tightly regulated alcohol oxidase (AOX1) promoter. The vectors also contain the *P. pastoris* histidinol dehydrogenase (HIS4) gene for selection in his4 hosts. Secretion of foreign protein require the presence of a signal sequence and the *S. cerevisiae* prepro alpha mating factor signal sequence has been widly and successfully used in Pichia expression system. Expression vectors are integrated into the P. pastoris genome to maximize the stability of expression strains. As in *S.cerevisiae,* cleavage of a *P.pastoris* expression vector within a sequence shared by the host genome (AOX1 or HIS4) stimulates homologous recombination events that efficiently target integration of the vector to that genomic locus. In general, a recombinant strain that contains multiple integrated copies of an expression cassette can yield more heterologous protein than single-copy strain. The most effective way to obtain high copy number transformants requires the transformation of Pichia recipient strain by the sphaeroplast technique (Cregg et all 1985, Mol.Cell.Biol. 5: 3376-3385) .

The preparation of the replicable expression vector may be carried out conventionally with appropriate enzymes for restriction, polymerisation and ligation of the DNA, by procedures described in, for example, Maniatis *et al.* cited above.

The recombinant host cell is prepared, in accordance with the invention, by transforming a host cell with a replicable expression vector of the invention under transforming conditions. Suitable transforming conditions are conventional and are described in, for example, Maniatis *et al.* cited above, or "DNA Cloning" Vol. II, D.M. Glover ed., IRL Press Ltd, 1985.

The choice of transforming conditions depends upon the choice of the host cell to be transformed. For example, in vivo transformation using a live viral vector as the transforming agent for the polynucleotides of the invention is described above. Bacterial transformation of a host such as *E. coli* may be done by direct uptake of the polynucleotides (which may be expression vectors containing the desired sequence) after the host has been treated with a solution of $CaCl_2$ (Cohen *et al.,* Proc. Nat. Acad. Sci., 1973, 69, 2110) or with a solution comprising a mixture of rubidium chloride (RbC1), $MnCl_2$, potassium acetate and glycerol, and then with 3-[N-morpholino]-propane-sulphonic acid, RbC1 and glycerol or by electroporation. Transformation of lower eukaryotic organisms such as yeast cells in culture by direct uptake may be carried out for example by using the method of Hinnen et al (Proc. Natl. Acad. Sci. 1978, 75 : 1929-1933). Mammalian cells in culture may be transformed using the calcium phosphate coprecipitation of the vector DNA onto the cells (Graham & Van der Eb, Virology 1978, 52, 546). Other methods for introduction of polynucleotides into mammalian cells include dextran mediated transfection, polybrene mediated transfection, protoplast fusion, electroporation, encapsulation of the polynucleotide(s) into liposomes, and direct microinjection of the polynucleotides into nuclei.

The invention also extends to a host cell transformed with a nucleic acid encoding the protein of the invention or a replicable expression vector of the invention.

Culturing the transformed host cell under conditions permitting expression of the DNA polymer is carried out conventionally, as described in, for example, Maniatis *et al.* and "DNA Cloning" cited above. Thus, preferably the cell is supplied with nutrient and cultured at a temperature below 50°C, preferably between 25°C and 42°C, more preferably between 25°C and 35°C, most preferably at 30°C. The incubation time may vary from a few minutes to a few hours, according to the proportion of the polypeptide in the bacterial cell, as assessed by SDS-PAGE or Western blot.

The product may be recovered by conventional methods according to the host cell and according to the localisation of the expression product (intracellular or secreted into the culture medium or into the cell periplasm). Thus, where the host cell is bacterial, such as *E. coli* it may, for example, be lysed physically, chemically or enzymatically and the protein product isolated from the resulting lysate. Where the host cell is mammalian, the product may generally be isolated from the nutrient medium or from cell free extracts. Where the host cell is a yeast such as *Saccharomyces cerevisiae* or *Pichia pastoris,* the product may generally be isolated from from lysed cells or from the culture medium, and then further purified using conventional techniques. The specificity of the expression system may be assessed by western blot or by ELISA using an antibody directed against the polypeptide of interest.

Conventional protein isolation techniques include selective precipitation, adsorption chromatography, and affinity chromatography including a monoclonal antibody affinity column. When the proteins of the present invention are expressed with a histidine tail (His tag), they can easily be purified by affinity chromatography using an ion metal affinity chromatography column (IMAC) column.The metal ion, may be any suitable ion for example zinc, nickel, iron, magnesium or copper, but is preferably zinc or nickel. Preferably the IMAC buffer contains detergent, preferably an anionic detergent such as SDS, more preferably a non-ionic detergent such as Tween 80, or a zwitterionic detergent such as Empigen BB, as this may result in lower levels of endotoxin in the final product.

Further chromatographic steps include for example a Q-Sepharose step that may be operated either before of after the IMAC column. Preferably the pH is in the range of 7.5 to 10, more preferably from 7.5 to 9.5, optimally between 8 and 9.

The proteins of the invention can thus be purified according to the following protocol. After cell disruption, cell extracts containing the protein can be solubilised in a pH 8.5 Tris buffer containing urea (8.0 M for example), and SDS (from 0.5% to 1% for example). After centrifugation, the resulting supernatant may then be loaded onto on to an IMAC (Nickel) Sepharose FF column equilibrated with a pH 8.5 Tris buffer. The column may then be washed with a high salt containing buffer (eg 0.75 - 1.5m NaC1, 15 mM pH 8.5 Tris buffer). The column may optionally then be washed again with phosphate buffer without salt. The proteins of the invention may be eluated from the column with an imidazole-containing buffered solution. The proteins can then be submitted to an additional chromatographic step, such as to an anion exchange chromatography (Q Sepharose for example).

The proteins of the present invention are provided either soluble in a liquid form or in a lyophilised form, which is the preferred form. It is generally expected that each human dose will comprise 1 to 1000 μg of protein, and preferably 30-300 μg. The purification process can also include a carboxyamidation step whereby the protein is first reduced in the presence of Glutathion and then carboxymethylated in the presence of iodoacetamide. This step offers the advantage of controling the oxidative aggregation of the molecule with itself or with host cell protein contaminants through covalent bridging with disulphide bonds.

The present invention also provides pharmaceutical and immunogenic compositions comprising a protein of the present invention in a pharmaceutically acceptable excipient.

A preferred vaccine composition comprises at least a protein according to the invention. Said protein has, preferably, blocked thiol groups and is highly purified, e.g. has less than 5% host cell contamination. Such vaccine may optionally contain one or more other tumour-associated antigen and derivatives. For example, suitable other associated antigen include prostase, PAP-1, PSA (prostate specific antigen), PSMA (prostate-specific membrane antigen), PSCA (Prostate Stem Cell Antigen), STEAP.

In another embodiment, illustrative immunogenic compositions, such as for example vaccine compositions, of the present invention comprise DNA encoding one or more of the fusion polypeptides as described above, such that the fusion polypeptide is generated *in situ.* As noted above, the polynucleotide may be administered within any of a variety of delivery systems known to those of ordinary skill in the art. Indeed, numerous gene delivery techniques are well known in the art, such as those described by Rolland, *Crit. Rev. Therap. Drug Carrier Systems 15*:143-198, 1998, and references cited therein. Appropriate polynucleotide expression systems will, of course, contain the necessary regulatory DNA regulatory sequences for expression in a patient (such as a suitable promoter and terminating signal). Alternatively, bacterial delivery systems may involve the administration of a bacterium (such as *Bacillus-Calmette-Guerrin)* that expresses an immunogenic portion of the polypeptide on its cell surface or secretes such an epitope.

Therefore, in certain embodiments, polynucleotides encoding immunogenic polypeptides described herein are introduced into suitable mammalian host cells for expression using any of a number of known viral-based systems. In one illustrative embodiment, retroviruses provide a convenient and effective platform for gene delivery systems. A selected nucleotide sequence encoding a polypeptide of the present invention can be inserted into a vector and packaged in retroviral particles using techniques known in the art. The recombinant virus can then be isolated and delivered to a subject. A number of illustrative retroviral systems have been described (e.g., U.S. Pat. No. 5,219,740; Miller and Rosman (1989) BioTechniques 7:980-990; Miller, A. D. (1990) Human Gene Therapy 1:5-14; Scarpa et al. (1991) Virology 180: 849-852; Bums et al. (1993) Proc. Natl. Acad. Sci. USA 90:8033-8037; and Boris-Lawrie and Temin (1993) Cur. Opin. Genet. Develop. 3:102-109.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67: 5911-5921; Mittereder et al. (1994) Human Gene Therapy 5:717-729; Seth et al. (1994) J. Virol. 68:933-940; Barr et al. (1994) Gene Therapy 1:51-58; Berkner, K. L. (1988) BioTechniques 6:616-629; and Rich et al. (1993) Human Gene Therapy 4:461-476). Since humans are sometimes infected by common human adenovirus serotypes such as AdHu5, a significant proportion of the population have a neutralizing antibody response to the adenovirus, which is likley to effect the immune response to a heterologous antigen in a recombinant vaccine based system. Non-human primate adenoviral vectors such as the chimpanzee adenovirus 68 (AdC68, Fitzgerald et al. (2003) J. Immunol 170(3):1416-22)) are may offer an alternative adenoviral system without the disadvantage of a pre-existing neutralising antibody response.

Various adeno-associated virus (AAV) vector systems have also been developed for polynucleotide delivery. AAV vectors can be readily constructed using techniques well known in the art. See, e.g., U.S. Pat. Nos. 5,173,414 and 5,139,941; International Publication Nos. WO 92/01070 and WO 93/03769; Lebkowski et al. (1988) Molec. Cell. Biol. 8: 3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; Kotin, R. M. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the nucleic acid molecules encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK.sup.(-) recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, e.g., WO 91/12882; WO 89/03429; and WO 92/03545.

Any of a number of alphavirus vectors can also be used for delivery of polynucleotide compositions of the present invention, such as those vectors described in U.S. Patent Nos. 5,843,723; 6,015,686; 6,008,035 and 6,015,694. Certain vectors based on Venezuelan Equine Encephalitis (VEE) can also be used, illustrative examples of which can be found in U.S. Patent Nos. 5,505,947 and 5,643,576.

The compositions of the present invention can be delivered by a number of routes such as intramuscularly, subcutaneously, intraperitonally or intravenously.

In another embodiment of the invention, a polynucleotide is administered/delivered as "naked" DNA, for example as described in Ulmer et al., *Science 259*:1745-1749, 1993 and reviewed by Cohen, Science 259:1691-1692, 1993. The uptake of naked DNA may be increased by coating the DNA onto biodegradable beads, which are efficiently transported into the cells. In a preferred embodiment, the composition is delivered intradermally. In particular, the composition is delivered by means of a gene gun (particularly particle bombardment) administration techniques which involve coating the vector on to a bead (eg gold) which are then administered under high pressure into the epidermis; such as, for example, as described in Haynes et al, J Biotechnology 44: 37-42 (1996).

In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide, are accelerated to high speed within a helium gas jet generated by a hand held device, propelling the particles into a target tissue of interest, typically the skin. The particles are preferably gold beads of a 0.4 - 4.0 μm, more preferably 0.6 - 2.0 μm diameter and the DNA conjugate coated onto these and then encased in a cartridge or cassette for placing into the "gene gun".

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

It is possible for the immunogen component comprising the nucleotide sequence encoding the antigenic peptide, to be administered on a once off basis or to be administered repeatedly, for example, between 1 and 7 times, preferably between 1 and 4 times, at intervals between about 1 day and about 18 months. However, this treatment regime will be significantly varied depending upon the size of the patient, the disease which is being treated/protected against, the amount of nucleotide sequence administered, the route of administration, and other factors which would be apparent to a skilled medical practitioner.

It is therefore another aspect of the present invention to provide for the use of a protein or a DNA encoding said protein, as described herein, in the manufacture of an immunogenic composition for eliciting an immune response in a patient. Preferably the immune response is to be elicited by sequential administration of i) the said protein followed by the said DNA sequence; or ii) the said DNA sequence followed by the said protein. More preferably the DNA sequence is coated onto biodegradable beads or delivered via a particle bombardment approach. Still more preferably the protein ios adjuvanted, preferably with a TH-1 inducing adjuvant, preferably with a CpG/QS21 based adjuvant formulation.

The vectors which comprise the nucleotide sequences encoding antigenic peptides are administered in such amount as will be prophylactically or therapeutically effective. The quantity to be administered, is generally in the range of one picogram to 16 milligram, preferably 1 picogram to 10 micrograms for particle-mediated delivery, and 10 micrograms to 16 milligram for other routes of nucleotide per dose. The exact quantity may vary considerably depending on the weight of the patient being immunised and the route of administration.

Suitable techniques for introducing the naked polynucleotide or vector into a patient also include topical application with an appropriate vehicle. The nucleic acid may be administered topically to the skin, or to mucosal surfaces for example by intranasal, oral, intravaginal or intrarectal administration. The naked polynucleotide or vector may be present together with a pharmaceutically acceptable excipient, such as phosphate buffered saline (PBS). DNA uptake may be further facilitated by use of facilitating agents such as bupivacaine, either separately or included in the DNA formulation. Other methods of administering the nucleic acid directly to a recipient include ultrasound, electrical stimulation, electroporation and microseeding which is described in US 5,697,901.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the nucleic acid to be administered can be altered.

The fusion proteins and encoding polypeptides according to the invention can also be formulated as a phamaceutical/immunogenic composition, e.g. as a vaccine. Accordingly therefore, the present invention also provides for a pharmaceutical/immunogenic composition comprising a fusion protein of the present invention in a pharmaceutically acceptable excipient. Accordingly there is also provided a process for the preparation of an immunogenic composition according to the present invention, comprising admixing the fusion protein of the invention or the encoding polynucleotide with a suitable adjuvant, diluent or other pharmaceutically acceptable carrier.

The fusion proteins of the present invention are provided preferably at least 80% pure more preferably 90% pure as visualised by SDS PAGE. Preferably the proteins appear as a single band by SDS PAGE.

Vaccine preparation is generally described in Vaccine Design ("The subunit and adjuvant approach" (eds. Powell M.F. & Newman M.J). (1995) Plenum Press New York). Encapsulation within liposomes is described by Fullerton, US Patent 4,235,877.

The fusion proteins of the present invention and encoding polynucleotides are preferably adjuvanted in the vaccine formulation of the invention. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatised polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that induces an immune response predominantly of the Th1 type. High levels of Th1-type cytokines (*e.g.*, IFN-$\gamma$, TNF$\alpha$, IL-2 and IL-12) tend to favor the induction of cell mediated immune responses to an administered antigen. In contrast, high levels of Th2-type cytokines (e.g., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, *Ann. Rev. Immunol. 7*: 145-173, 1989.

Preferred TH-1 inducing adjuvants are selected from the group of adjuvants comprising: 3D-MPL, QS21, a mixture of QS21 and cholesterol, and a CpG oligonucleotide or a mixture of two or more said adjuvants. Certain preferred adjuvants for eliciting a predominantly Th1-type response include, for example, a combination of monophosphoryl lipid A, preferably 3-de-O-acylated monophosphoryl lipid A, together with an aluminum salt. MPL® adjuvants are available from Corixa Corporation (Seattle, WA; see, for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., *Science 273*:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins . Other preferred formulations include more than one saponin in the adjuvant combinations of the present invention, for example combinations of at least two of the following group comprising QS21, QS7, Quil A, β-escin, or digitonin.

Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol$^R$ to increase viscosity, or may be formulated in a dry powder form with a powder excipient such as lactose.

In one preferred embodiment, the adjuvant system includes the combination of a monophosphoryl lipid A and a saponin derivative, such as the combination of QS21 and 3D-MPL® adjuvant, as described in WO 94/00153, or a less reactogenic composition where the QS21 is quenched with cholesterol, as described in WO 96/33739. Other preferred formulations comprise an oil-in-water emulsion and tocopherol. Another particularly preferred adjuvant formulation employing QS21, 3D-MPL® adjuvant and tocopherol in an oil-in-water emulsion is described in WO 95/17210.

Another enhanced adjuvant system involves the combination of a CpG-containing oligonucleotide and a saponin derivative particularly the combination of CpG and QS21 as disclosed in WO 00/09159 and in WO 00/62800. Preferably the formulation additionally comprises an oil in water emulsion and tocopherol.

In a yet further embodiment the present invention provides an immunogenic composition comprising a fusion protein according to the invention, and further comprising D3-MPL, a saponin preferably QS21 and a CpG oligonucleotide, optionally formulated in an oil in water emulsion.

Additional illustrative adjuvants for use in the pharmaceutical compositions of the invention include Montanide ISA 720 (Seppic, France), SAF (Chiron, California, United States), ISCOMS (CSL), MF-59 (Chiron), the SBAS series of adjuvants (e.g., SBAS-2 or SBAS-4, available from SmithKline Beecham, Rixensart, Belgium), Detox (Enhanzyn® (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula (I):

$HO(CH_2CH_2O)_n$-A-R, wherein, n is 1-50, A is a bond or -C(O)-, R is $C_{1-50}$ alkyl or Phenyl $C_{1-50}$ alkyl. One embodiment of the present invention consists of a vaccine formulation comprising a polyoxyethylene ether of general formula (I), wherein n is between 1 and 50, preferably 4-24, most preferably 9; the R component is $C_{1-50}$, preferably $C_4$-$C_{20}$ alkyl and most preferably $C_{12}$ alkyl, and A is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethytene-4-lauryl ether, polyoxyethylene-35-lauryl ether, and polyoxyethylene-23-lauryl ether. Polyoxyethylene ethers such as polyoxyethylene lauryl ether are described in the Merck index (12$^{th}$ edition: entry 7717). These adjuvant molecules are described in WO 99/52549. The polyoxyethylene ether according to the general formula (I) above may, if desired, be combined with another adjuvant. For example, a preferred adjuvant combination is preferably with CpG as described in the pending UK patent application GB 9820956.2.

It is an embodiment of the invention that the antigens, including nucleic acid vector, of the invention be utilised with immunostimulatory agent. Preferably the immunostimulatory agent is administered at the same time as the antigens of the invention and in preferred embodiments are formulated together. It is another embodiment of the invention that the antigen and immunostimulatory agent (or vice versa) are administered sequentially to the same or adjacent sites, separated in time by periods of between 0-100 hours. Such immunostimulatory agents include but are not limited to: synthetic imidazoquinolines such as imiquimod [S-26308, R-837], (Harrison, et al., Vaccine 19: 1820-1826, 2001; and resiquimod [S-28463, R-848] (Vasilakos, et al., Cellular immunology 204: 64-74, 2000.; Schiff bases of carbonyls and amines that are constitutively expressed on antigen presenting cell and T-cell surfaces, such as tucaresol (Rhodes, J. et al., Nature 377: 71-75, 1995), cytokine, chemokine and co-stimulatory molecules as either protein or peptide, including for example pro-inflammatory cytokines such as Interferon, GM-CSF, IL-1 alpha, IL-1 beta, TGF- alpha and TGF - beta, Th1 inducers such as interferon gamma, IL-2, IL-12, IL-15, IL-18 and IL-21, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, other immunostimulatory targeting ligands such as CTLA-4 and L-selectin, apoptosis stimulating proteins and peptides such as Fas, (49), synthetic lipid based adjuvants, such as vaxfectin, (Reyes et al., Vaccine 19: 3778-3786, 2001) squalene, alpha- tocopherol, polysorbate 80, DOPC and cholesterol, endotoxin, [LPS], (Beutler, B., Current Opinion in Microbiology 3: 23-30, 2000); CpG oligo- and di-nucleotides (Sato, Y. et al., Science 273 (5273): 352-354, 1996; Hemmi, H. et al., Nature 408: 740-745, 2000) and other potential ligands that trigger Toll receptors to produce Th1-inducing cytokines, such as synthetic Mycobacterial lipoproteins, Mycobacterial protein p19, peptidoglycan, teichoic acid and lipid A.

Other suitable adjuvant include CT (cholera toxin, subunites A and B) and LT (heat labile enterotoxin from E. coli, subunites A and B), heat shock protein family (HSPs), and LLO (listeriolysin O; WO 01/72329).

Where the immunostimulatory agent is a protein, the agent may be administered either as a protein or as a polynucleotide encoding the protein.

Other suitable delivery systems include microspheres wherein the antigenic material is incorporated into or conjugated to biodegradable polymers/microspheres sothat the antigenic material can be mixed with a suitable pharmaceutical carrier and used as a vaccine. The term "microspheres" is generally employed to describe colloidal particles which are substantially spherical and have a diameter in the range 10 nm to 2 mm. Microspheres made from a very wide range of natural and synthetic polymers have found use in a variety of biomedical applications. This delivery system is especially advantageous for proteins having short half-lives in vivo requiring multiple treatments to provide efficacy, or being unstable in biological fluids or not fully absorbed from the gastrointestinal tract because of their relatively high molecular weights. Several polymers have been described as a matrix for protein release. Suitable polymers include gelatin, collagen, alginates, dextran. Preferred delivery systems include biodegradable poly(DL-lactic acid) (PLA), poly (lactide-co-glycolide) (PLG), poly(glycolic acid) (PGA), poly(ε-caprolactone) (PCL), and copolymers poly(DL-lactic-co-glycolic acid) (PLGA). Other preferred systems include heterogeneous hydrogels such as poly(ether ester) multiblock copolymers, containing repeating blocks based on hydrophilic poly-(ethylene glycol) (PEG) and hydrophobic poly(butylene terephtalate) (PBT), or poly(ehtykene glycol)-terephtalate/poly(-butylene terephtalate) (PEGT/PBT) (Sohier et al. Eur. J. Pharm and Biopharm, 2003, 55, 221-228). Systems are preferred which provide a sustained release for 1 to 3 months such as PLGA, PLA and PEGT/PBT.

It is possible for the immunogenic or vaccine composition to be administered on a once off basis or, preferably, to be administered repeatedly, as many times as necessary, for example, between 1 and 7 times, preferably between 1 and 4 times, at intervals between about 1 day and about 18 months, preferably one month. This may be optionally followed by dosing at regular intervals of between 1 and 12 months for a period up to the remainder of the patient's life. In a preferred embodiment the patient receives the antigen in different forms in a "prime boost" regime. Thus for example the antigen, the fusion protein, is first administered as a protein adjuvant base formulation and then subsequently administered as a DNA based vaccine. This administration mode is preferred. The preferred adjuvant is a combination of a CpG-containing oligonucleotide and a saponin derivative, particularly the combination of CpG and QS21 as disclosed in WO 00/09159 and in WO 00/62800. The uptake of naked DNA may be increased by coating the DNA onto biodegradable beads, which are efficiently transported into the cells. Alternatively the DNA can be delivered via a particle bombardment approach, for example, gas-driven particle acceleration with devices such as those manufactured by Powderject Pharmaceuticals PLC (Oxford, UK) and Powderject Vaccines Inc. (Madison, WI) as taught herein. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In another preferred embodiment, the DNA based vaccine will be administered first, followed by the protein adjuvant base formulation. Still another embodiment will concern the delivery of the DNA construct by means of specialised delivery vectors, preferably by the means of viral system, most preferably by the means of adenoviral-based systems. Other suitable viral-based systems of DNA delivery include retroviral, lentiviral, adeno-associated viral, herpes viral and vaccinia-viral based systems.

In another preferred embodiment, the protein adjuvant base formulation and DNA based vaccine may be co-administered at adjacent or overlapping sites. Dependent upon the nature of the DNA vaccine formulation, this can be achieved by mixing the DNA and protein adjuvant formulations prior to administration or by simultaneously administration of the DNA and protein adjuvant formulation.

The treatment regime will be significantly varied depending upon the size and species of patient concerned, the amount of nucleic acid vaccine and / or protein composition administered, the route of administration, the potency and dose of any adjuvant compounds used and other factors which would be apparent to a skilled medical practitioner.

Within further aspects, the present invention provides methods for stimulating an immune response in a patient, preferably a T cell response in a human patient, comprising administering a pharmaceutical composition described herein. The patient may be afflicted with lung or colon cancer or colorectal cancer or breast cancer, in which case the methods provide treatment for the disease, or patient considered at risk for such a disease may be treated prophylactically.

Within further aspects, the present invention provides methods for inhibiting the development of a cancer in a patient, comprising administering to a patient a pharmaceutical composition as recited above. The patient may be afflicted with, for example, sarcoma, prostate, ovarian, bladder, lung, colon, colorectal or breast cancer, in which case the methods provide treatment for the disease, or patient considered at risk for such a disease may be treated prophylactically.

The present invention further provides, within other aspects, methods for removing tumour cells from a biological sample, comprising contacting a biological sample with T cells that specifically react with a polypeptide of the present invention, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a polypeptide of the present invention, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.
The present invention further provides methods for inhibiting the development of a cancer in a patient, comprising the steps of: (a) incubating CD4+ and/or CD8+ T cells isolated from a patient with one or more of: (i) a polypeptide disclosed herein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient. Proliferated cells may, but need not, be cloned prior to administration to the patient.

According to another embodiment of this invention, an immunogenic composition described herein is delivered to a host via antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver *(i.e.,* matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, *Nature* 392:245-251, 1998) and have been shown to be effective as a physiological adjuvant for eliciting prophylactic or therapeutic antitumor immunity (see Timmerman and Levy, *Ann. Rev. Med. 50*:507-529, 1999). In general, dendritic cells may be identified based on their typical shape (stellate *in situ,* with marked cytoplasmic processes (dendrites) visible *in vitro),* their ability to take up, process and present antigens with high efficiency and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (see Zitvogel et al., *Nature Med. 4*:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNF$\alpha$ to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNF$\alpha$, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fey receptor and mannose receptor. The mature phenotype is typically characterized by a lower expression of these markers, but a high expression of cell surface molecules responsible for T cell activation such as class I and class II MHC, adhesion molecules (*e.g.*, CD54 and CD11) and costimulatory molecules (*e.g.*, CD40, CD80, CD86 and 4-1 BB).

APCs may generally be transfected with a polynucleotide of the invention (or portion or other variant thereof) such that the encoded polypeptide, or an immunogenic portion thereof, is expressed on the cell surface. Such transfection may take place *ex vivo,* and a pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., *Immunology and cell Biology 75*:456-460, 1997. Antigen loading of dendritic cells may be achieved by incubating dendritic cells or progenitor cells with the tumor polypeptide, DNA (naked or within a plasmid vector) or RNA; or with antigen-expressing recombinant bacterium or viruses (e.g., vaccinia, fowlpox, adenovirus or lentivirus vectors). Prior to loading, the polypeptide may be covalently conjugated to an immunological partner that provides T cell help (e.g., a carrier molecule). Alternatively, a dendritic cell may be pulsed with a non-conjugated immunological partner, separately or in the presence of the polypeptide.

**Definitions**

Also provided by the invention are methods for the analysis of character sequences or strings, particularly genetic sequences or encoded protein sequences. Preferred methods of sequence analysis include, for example, methods of sequence homology analysis, such as identity and similarity analysis, DNA, RNA and protein structure analysis, sequence assembly, cladistic analysis, sequence motif analysis, open reading frame determination, nucleic acid base calling, codon usage analysis, nucleic acid base trimming, and sequencing chromatogram peak analysis.

A computer based method is provided for performing homology identification. This method comprises the steps of: providing a first polynucleotide sequence comprising the sequence of a polynucleotide of the invention in a computer readable medium; and comparing said first polynucleotide sequence to at least one second polynucleotide or polypeptide sequence to identify homology. A computer based method is also provided for performing homology identification, said method comprising the steps of: providing a first polypeptide sequence comprising the sequence of a polypeptide of the invention in a computer readable medium; and comparing said first polypeptide sequence to at least one second polynucleotide or polypeptide sequence to identify homology.

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in *(Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data,* Part I, Griffin, A.M., and Griffin, H.G., eds., Humana Press, New Jersey, 1994; *Sequence Analysis in Molecular Biology,* von Heine, G., Academic Press, 1987; and *Sequence Analysis Primer,* Gribskov, M. and Devereux, J., eds., M Stockton Press, New York, 1991; and Carillo, H., and Lipman, D., SIAM *J. Applied Math., 48:* 1073 (1988). Methods to determine identity are designed to give the largest match between the sequences tested. Moreover, methods to determine identity are codified in publicly available computer programs. Computer program methods to determine identity between two sequences include, but are not limited to, the GAP program in the GCG program package (Devereux, J., et al., *Nucleic Acids Research 12(1):* 387 (1984)), BLASTP, BLASTN (Altschul, S.F. et al., *J. Molec. Biol. 215*: 403-410 (1990), and FASTA( Pearson and Lipman Proc. Natl. Acad. Sci. USA 85; 2444-2448 (1988). The BLAST family of programs is publicly available from NCBI and other sources *(BLAST Manual,* Altschul, S., *et al.,* NCBI NLM NIH Bethesda, MD 20894; Altschul, S., *et al., J. Mol. Biol. 215*: 403-410 (1990). The well known Smith Waterman algorithm may also be used to determine identity.

Parameters for polypeptide sequence comparison include the following:

Algorithm: Needleman and Wunsch, J. Mol Biol. 48: 443-453 (1970)
Comparison matrix: BLOSSUM62 from Henikoff and Henikoff,
Proc. Natl. Acad. Sci. USA. 89:10915-10919 (1992)
Gap Penalty: 8
Gap Length Penalty: 2
A program useful with these parameters is publicly available as the "gap" program from Genetics Computer Group, Madison WI. The aforementioned parameters are the default parameters for peptide comparisons (along with no penalty for end gaps).

Parameters for polynucleotide comparison include the following:

Algorithm: Needleman and Wunsch, J. Mol Biol. 48: 443-453 (1970)
Comparison matrix: matches = +10, mismatch = 0
Gap Penalty: 50
Gap Length Penalty: 3

Available as: The "gap" program from Genetics Computer Group, Madison WI. These are the default parameters for nucleic acid comparisons.

A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to any of the reference sequences of SEQ ID NO: 9 to SEQ ID NO:16, wherein said polynucleotide sequence may be identical to any the reference sequences of SEQ ID NO:9 to SEQ ID NO:16 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in any of SEQ ID NO:9 to SEQ ID N0:16 by the integer defining the percent identity divided by 100 and then subtracting that product from said total number of nucleotides in any of SEQ ID NO:9 to SEQ ID NO:16, or:

$$\mathbf{n_n \leq x_n - (x_n \bullet y),}$$

wherein $\mathbf{n}_n$ is the number of nucleotide alterations, $\mathbf{x}_n$ is the total number of nucleotides in any of SEQ ID NO:9 to SEQ ID NO:16, y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x}_n$ and y is rounded down to the nearest integer prior to subtracting it from $\mathbf{x}_n$. Alterations of polynucleotide sequences encoding the polypeptides of any of SEQ ID NO:1 to SEQ ID NO:8 may create nonsense, missense or frameshift mutations in this coding sequence and thereby alter the polypeptide encoded by the polynucleotide following such alterations.

By way of example, a polynucleotide sequence of the present invention may be identical to any of the reference sequences of SEQ ID NO:9 to SEQ ID N0:16, that is it may be 100% identical, or it may include up to a certain integer number of nucleic acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of nucleic acids in any of SEQ ID NO:9 to SEQ ID NO:16 by the integer defining the percent identity divided by 100 and then subtracting that product from said total number of nucleic acids in any of SEQ ID N0:9-to SEQ ID NO:16, or:

$$\mathbf{n_n \leq x_n - (x_n \bullet y),}$$

wherein $\mathbf{n}_n$ is the number of nucleic acid alterations, $\mathbf{x}_n$ is the total number of nucleic acids in any of SEQ ID NO:9 to SEQ ID N0:16, **y** is, for instance 0.70 for 70%, 0.80 for 80%, 0.85 for 85% etc., • is the symbol for the multiplication operator, and wherein any non-integer product of $x_n$ and y is rounded down to the nearest integer prior to subtracting it from $x_n$.

(2) Polypeptide embodiments further include an isolated polypeptide comprising a polypeptide having at least a 50,60, 70, 80, 85, 90, 95, 97 or 100% identity to the polypeptide reference sequence of any of SEQ ID NO:1 to SEQ ID NO:8, wherein said polypeptide sequence may be identical to any of the reference sequence of SEQ ID NO: to SEQ ID NO:8 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in any of SEQ ID NO:1 to SEQ ID NO:8 by the integer defining the percent identity divided by 100 and then subtracting that product from said total number of amino acids in any of SEQ ID NO:1 to SEQ ID NO:8, or:

$$n_a \leq x_a - (x_a \bullet y),$$

wherein $n_a$ is the number of amino acid alterations, $x_a$ is the total number of amino acids in SEQ ID NO:2, y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $x_a$ and $y$ is rounded down to the nearest integer prior to subtracting it from $x_a$.

By way of example, a polypeptide sequence of the present invention may be identical to the reference sequence of any of SEQ ID N0:1 to SEQ ID NO:8, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in any of SEQ ID N0:1 to SEQ ID NO:8 by the integer defining the percent identity divided by 100 and then subtracting that product from said total number of amino acids in any of SEQ ID NO:1 to SEQ ID NO:8, or:

$$n_a \leq x_a - (x_a \bullet y),$$

wherein $n_a$ is the number of amino acid alterations, $x_a$ is the total number of amino acids in any of SEQ ID NO:1 to SEQ ID NO:8, $y$ is, for instance 0.70 for 70%, 0.80 for 80%, 0.85 for 85% etc., and • is the symbol for the multiplication operator, and wherein any non-integer product of $x_a$ and $y$ is rounded down to the nearest integer prior to subtracting it from $x_a$.

**Figure legends**

Figure 1: Sequence information for C-LytA. Each repeat has been defined on the basis of both multiple sequence alignment and secondary structure prediction using the following alignment programs: 1) MatchBox (Depiereux E et al. (1992) Comput Applic Biosci 8:501-9); 2) ClustalW (Thompson JD et al. (1994) Nucl Acid Res 22:4673-80); 3) Block-Maker (Henikoff S et al (1995) Gene 163:gc17-26)
Figure 2: CPC and native Constructs (SEQ ID NOs. 27-36)
Figure 3: Schematic structure of CPC-p501 His fusion protein expressed in *S. cerevisiae*
Figure 4: Primary structure of CPC-P501 His fusion protein (SEQ ID NO.41)
Figure 5: Nucleotide sequence of CPC P501 His(pRIT15201) (SEQ ID NO.42)
Figure 6: Cloning strategy for generation of plasmid pRIT 15201
Figure 7: Plasmid map of pRIT15201
Figure 8: Comparative expression of CPC P501 and P501 in *S.cerevisiae* strain DC5
Figure 9: Production of CPC-P501S HIS (Y1796) at small scale. Fig. 9A represents the antigen productivity as estimated by SDS-PAGE with silver staining; Fig. 9B represents the antigen productivity as estimated by western blot.
Figure 10: Purification scheme of CPC-P501-His produced by Y1796.
Figure 11: Pattern of CPC P501 His purified protein (4-12% Novex Nu-Page polyacrylamide precasted gels).
Figure 12: Native full-length P501 S sequence (SEQ ID NO: 17)
Figure 13: Sequence of the CPC-P501 S expression cassette of JNW735 (SEQ ID NO:18)
Figure 14: Two codon optimised P501S sequences (SEa ID NO:19-20)
Figure 15: Re-engineered codon optimised sequence 19 (SEQ ID NO:21)
Figure 16: Re-engineered codon optimised sequence 20 (SEQ ID NO:22)
Figure 17: The starting sequence for the optimisation of CPC (SEQ ID NO:23)
Figure 18: Representative codon optimised CPC sequences (SEQ ID NO:24-25)

Figure 19: Engineered CPC codon optimised sequence (SEQ ID NO:26)
Figure 20: P501S CPC fusion candidate constructs and sequences (SEQ ID NOs. 37-40 & 45-48)
Figure 21: Western blot analysis of CHO cells following transient transfection with P501 S (JNW680), CPC-P501S (JNW735) and empty vector control.
Figure 22: Anti-P501S antibody responses following immunisation at day0, 21 & 42 with pVAC-P501S (JNW680, mice B1-9) or Empty vector (pVAC, mice A1-6). A pre-bleed was taken at day -1. Subsequently bleeds were taken at day 28 and day 49 (mice A1-3, B1-3) and day 56 (mice A4-6, B4-9). All sera was tested at 1/100 dilution. The results for the pVAC immunised mice were averaged. The results for the individual pVAC-P501 S immunised mice are shown. As a positive control, sera from Adeno-P501 S immunised mice (Corixa Corp, diluted 1/100) is included.
Figure 23: Peptide library screen using C57BL/6 mice immunised at day 0, 21, 42, and 70 with pVAC-P501 S (JNW680). All peptides were used at a final concentration of 50μg/ml. Peptides 1-50 are overlapping 15-20mers obtained from Corixa. Peptides 51-70 are predicted 8-9mer Kb and Db epitopes and were ordered from Mimotopes (UK). Samples 71-72 and 73-78 are DMSO controls and no peptide controls respectively. Graph A shows the IFN-γ responses whilst Graph B shows the IL-2 responses. Peptides selected for use in subsequent immunoassays are shown in black.
Figure 24: Cellular responses by ELISPOT at day 77 following PMID immunisation at day 0, 21, 42, and 70 with pVAC-P501S (JNW680, B6-9) and pVAC empty (A4-6). Peptide 18, 22 & 48 were used at 50μg/ml. CPC-P501 S protein was used at 20μg/ml. Graph A shows the IFN-γ responses whilst Graph B shows the IL-2 responses.
Figure 25: Comparison of P501 S and CPC-P501 S. Cellular responses were measured by IL-2 ELISPOT using peptide 22 (10μg/ml) at day 28. Mice were immunised by PMID at day 0 and 21 with pVAC empty (control), pVAC-P501 S (JNW680) and CPC-P501 S (JNW735).
Figure 26: Immune response (lymphoproliferation on spleen cells) following protein immunisation with CPC-P501 S.
Figure 27: Evaluation of the immune response to different CPC-P501 S constructs. Cellular responses were measured by IL-2 ELISPOT at day 28. Mice were immunised by PMID at day 0 and 21 with p7313-ie empty (control), JNW735 and CPC-P501S constructs (JNW770, 771 and 773)
Figure 28: MUC-1 CPC sequences (SEQ ID NOs. 49 & 50)
Figure 29: ss-CPC-MUC-1 sequences (SEQ ID NOs. 51 & 52)

The invention will be further described by reference to the following examples:

## EXAMPLE I: Preparation of the recombinant Yeast strain Y1796 expressing P501 Fusion Protein containing a C-LytA-P2-C-LytA (CPC) as fusion partner

### 1. - Protein design

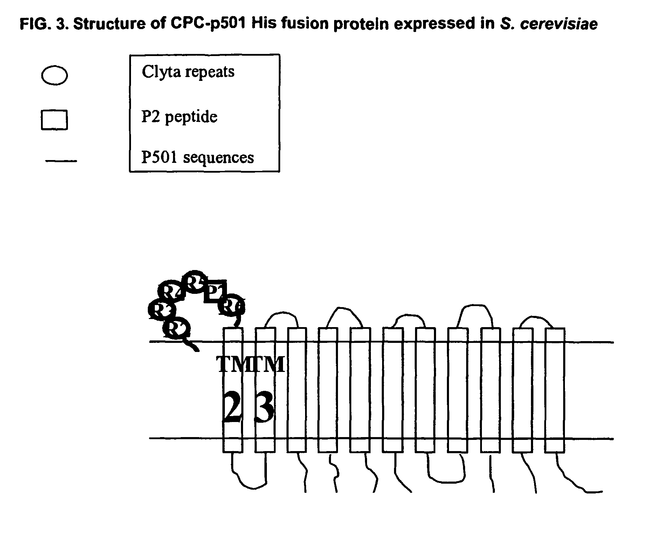

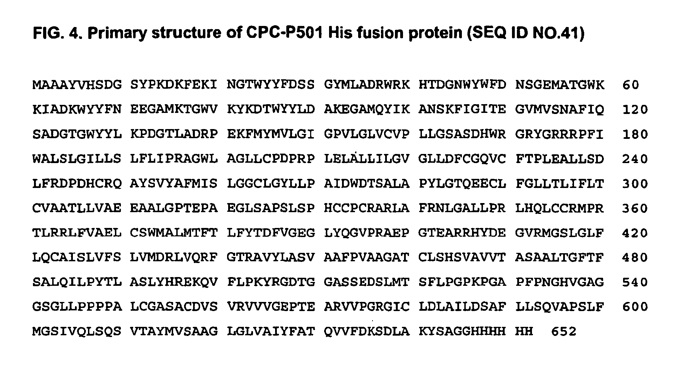

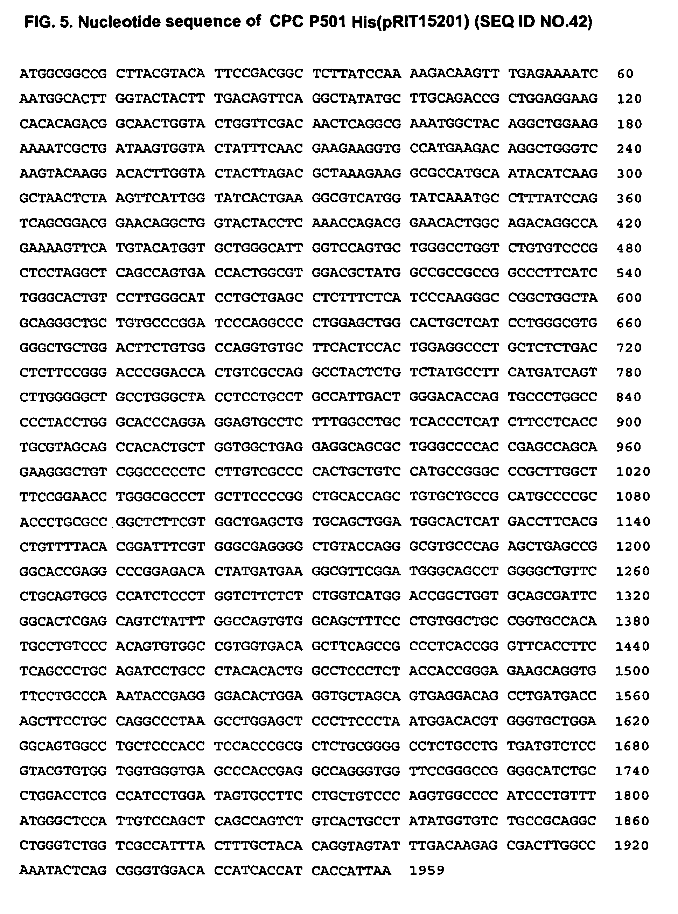

The structure of the fusion protein C-P2-C-p501 (alternatively named CPC-P501) to be expressed in *S. cerevisiae* is depicted in figure 3. This fusion contains the C-terminal region of gene LytA (residues 187 to 306), in which the P2 fragment of tetanus toxin (residues 830-843) has been inserted. The P2 fragment is placed between the residues 277 and 278 of C-Lyt-A. The C-lytA fragment containing the P2 insertion is followed by P501 (residues amino acid 51 to 553) and by the His tail.
The primary structure of the resulting fusion protein has the sequence described in figure 4 and the coding sequence corresponding to the above protein design is in figure 5.

### 2. - Cloning strategy for the generation of a yeast plasmid expressing CPC-P501 (51-553)-His fusion protein

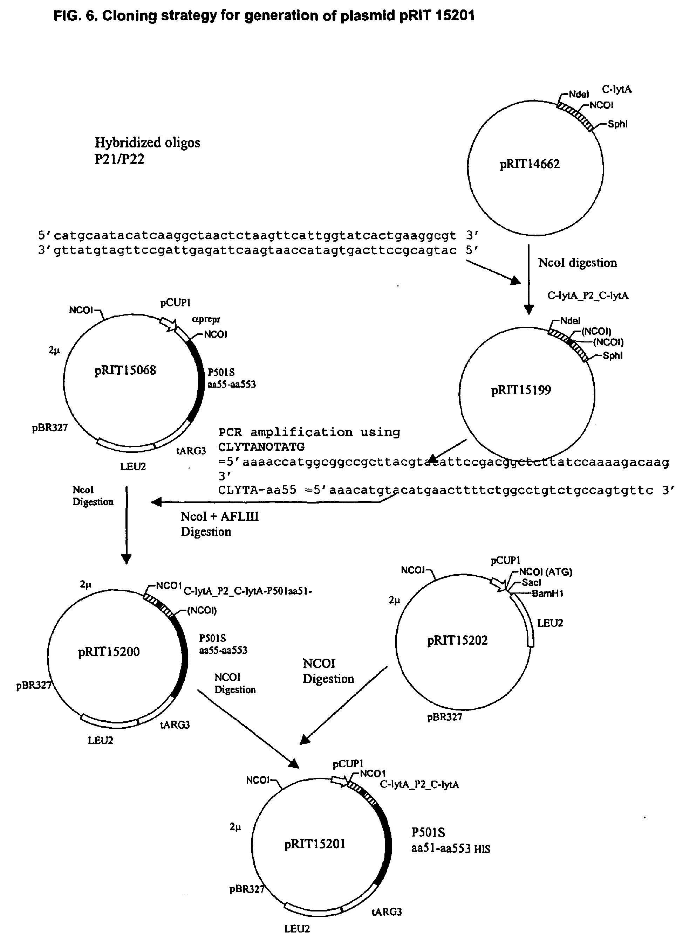

- The starting material is the yeast vector pRIT15068 (UK patent application 0015619.0).
- This vector contains the yeast Cup1 promoter, the yeast alpha prepro signal coding sequence and the coding sequence corresponding to residues 55 to 553 of P501S followed by His tail.
- The cloning strategy outlined in figure 6 include the following steps:

a) The first step is the insertion of P2 sequence (codon-optimised for yeast expression) in frame, inside the C-lytA coding sequence. The C-lytA coding sequence is harbored by plasmid pRIT 14662 (PCT/EP99/00660). The insertion is done using an adaptor formed by two complementary oligonucleotides named P21 and P22 into the plasmid pRIT 14662 previously open by Ncol
The sequence of P21 and P22 is:

P21 5' catgcaatacatcaaggctaactctaagttcattggtatcactgaaggcgt 3'
P22 3' gttatgtagttccgattgagattcaagtaaccatagtgacttccgcagtac 5'

After ligation and transformation of *E. coli* and transformant characterization, the plasmid named pRIT15199 is obtained.

b) The second step is the preparation of C-lytA-P2-C-lytA DNA fragment by PCR amplification. The amplification is performed using pRIT15199 as template and the oligonucleotides named C-LytANOTATG and C-LytA-aa55. The sequence of both oligonucleotides being:

C-LytANOTATG
=5'aaaaccatggcggccgcttacgtacattccgacggctcttatccaaaagacaag 3'
C-LytA-aa55 =5'aaacatgtacatgaacttttctggcctgtctgccagtgttc 3'

The amplified fragment is treated with the restriction enzymes NcoI and Afl III to generate the respective cohesive ends.

c) The next step is the ligation of the above fragment with vector pRIT15068 (largest fragment obtained after NcoI treatment) to generate the complete fusion protein coding sequence. After ligation and *E. coli* transformation the plasmid named pRIT15200 is obtained. In this plasmid the remaining unique Nco1 site contains the ATG coding for the start codon.

d) In the next step a NcoI fragment containing the CUP1 promoter and a portion of 2μ plasmid sequences is prepared from plasmid PRIT 15202. Plasmid pRIT 15202 is a yeast 2μ derivative containing the CUP1 promoter with an NcoI site at ATG ( ATG sequence: AAACC ATG )

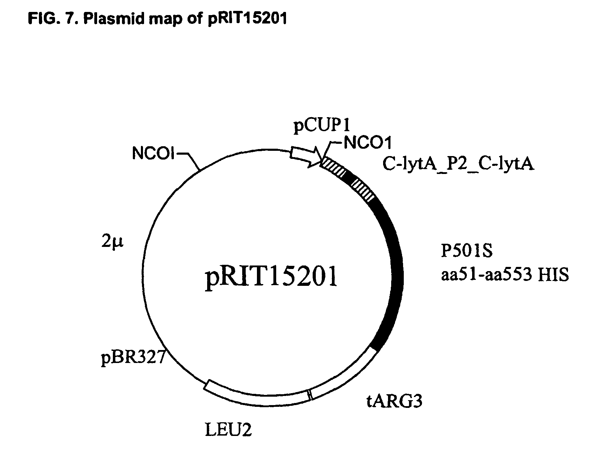

e) The NcoI fragment isolated from pRIT 15202 is ligated to pRIT15200, previously open with NcoI, in the righ orientation, in such a way the pCUP1 promoter is at the 5' side of the coding sequence. This results in the generation of a final expression plasmid named pRIT15201 (see figure 7).

**3. - Preparation of the recombinant yeast strain Y1796 (RIX4440)**

The plasmid pRIT 15201 is used to transform the *S. cerevisiae* strain DC5 (ATCC 20820). After selection and characterisation of the yeast transformants containing the plasmid pRIT 15201 a recombinant yeast strain named Y1796 expressing CPC-P501-His fusion protein is obtained. The protein after reduction and carboxyamidation, is isolated and purified by affinity chromatography (IMAC) followed by anion exchange chromatography (Q Sepharose FF).

**Example II**

In analogous fashion proteins constructs as depicted in figure 2 may be expressed utilising the corresponding DNA sequences shown therein. In particular, yeast strain SC333 (construct 2) corresponds to Y1796 strain but expressing $P501_{55-553}$ devoid of the CPC fusion partner. Yeast strain Y1800 (construct 3) corresponds to Y1796 strain but additionally comprises the native sequence signal for P501 S (aa1-aa34), while yeast strain Y1802 (construct 4) comprises the alpha pre signal sequence upstream CPC-P501S sequence. Yeast strain Y1790 (construct 5) is expressing a P501S construct devoid of CPC and having the alpha prepro signal sequence.

**Example III. Preparation of purified CPC-P501**

**1. - Production of CPC-P501S HIS (Y1796) at small scale**

For Y1796, in minimal medium supplemented with histidine, expression is induced in log phase by addition of $CuSO_4$ ranging from 100 to 500 μM, and culture is maintained at 30°. Cells are harvested after 8 or 24H induction. Copper is added just before use and not mixed with medium in advance.

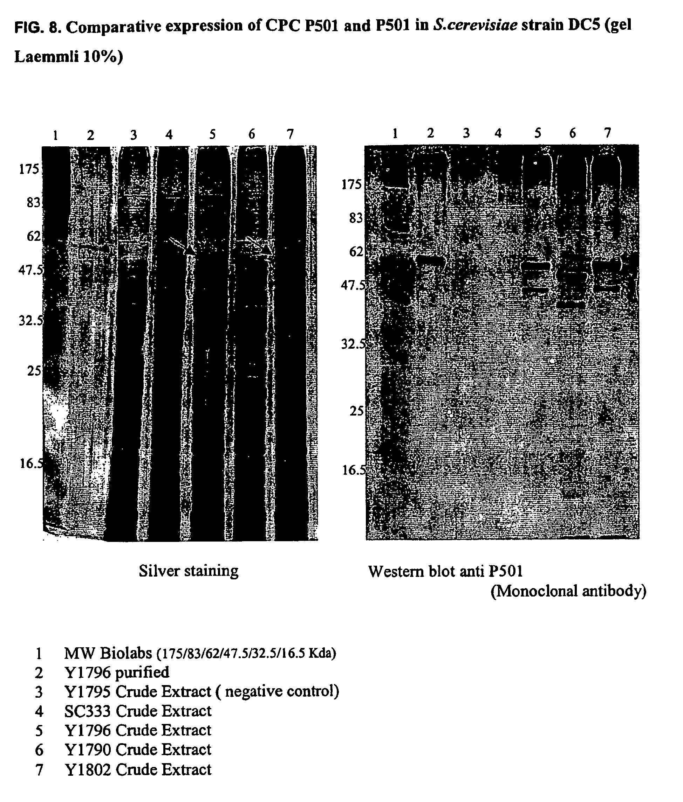

For SDS PAGE analysis, yeast cells extraction is performed in citrate phosphate buffer pH4.0 + 130 mM NaCl. Extraction is performed with glass beads for small cell quantity and with French press for higher cells quantity, and then mixed with sample buffer and SDS-PAGE analysed. Results of comparative analysis on SDS PAGE of the different constructs are depicted in figure 8 and summariosed in Table 2 below.

As shown in Table 1 below, the level of expression of the culture is much higher for Y1796 strain as compared to the expression level of parent strain SC333, a strain expressing the corresponding P501S-His devoid of CPC partner. Likewise, the presence of a signal sequence (alpha pre) does not affect the results discussed above: the level of expression of the culture is much higher for Y1802 strain as compared to the expression level of corresponding strain Y1790, a strain expressing the corresponding P501S-His devoid of CPC partner.

**Table 2**

| Recombinant Strain | Plasmid | Promotor | Signal sequence | Fusion Partner | P501 aa sequences | Expressio n level |
|---|---|---|---|---|---|---|
| SC333 | Ma333 | CUP1 | - | - | 55-553-His | ⊖ND |
| Y1796 | pRIT 15201 | CUP 1 | - | CPC | 51-553- His | +++ |
| Y1802 | pRIT 15219 | CUP 1 | α pre | CPC | 51-553- His | ++++ |
| Y1790 | pRIT 15068 | CUP 1 | α prepro | - | 55-553- His | + |
| CPC = clyta P2 clyta<br>ND= not detectable, even in western blot<br>+ = detectable in western blot<br>+++ / ++++ = detectable in western blot and visible in silver stained gels | | | | | | |

**2. - Fermentation ofY1796 (RIX4440) at larger scale**

100μl of the working seed are spread on solid medium and grown for approximately 24h at 30°C. This solid pre-culture is then used to inoculate a liquid pre-culture in shake flasks.

This liquid pre-culture is grown for 20h at 30°C and transferred into a 20L fermenter. The fed-batch fermentation includes a growth phase of about 44h and an induction phase of about 22h.

The carbon source (glucose) was supplemented to the culture by a continuous feeding. The residual glucose concentration was maintained very low (≤50mg/L) in order to minimise the ethanol production by fermentation. This was realised by limiting the development of the micro-organism by limited glucose feed rate.

At the end of the growth phase, CUP1 promoter is induced by adding $CuSO_4$ in order to produce the antigen.

The absence of contaminations was checked by inoculating $10^6$ cells into standard TSB and THI vials supplemented with nystatine and incubated respectively for 14 days at 20-25°C and at 30-35°C. No growth was observed as expected.

**3. - <u>Antigen characterisation and productivity</u>**

Cell homogenates were prepared by French pressing of fermentation samples harvested at different times during the induction phase and analysed by SDS-PAGE and Western Blot. It was shown that the major part of the protein of interest was located in the insoluble fraction obtained from the cell homogenate after centrifugation. The SDS-PAGE and Western Blot analyses shown in the Figures below were realised on the pellets obtained after centrifugation of these cell homogenates.

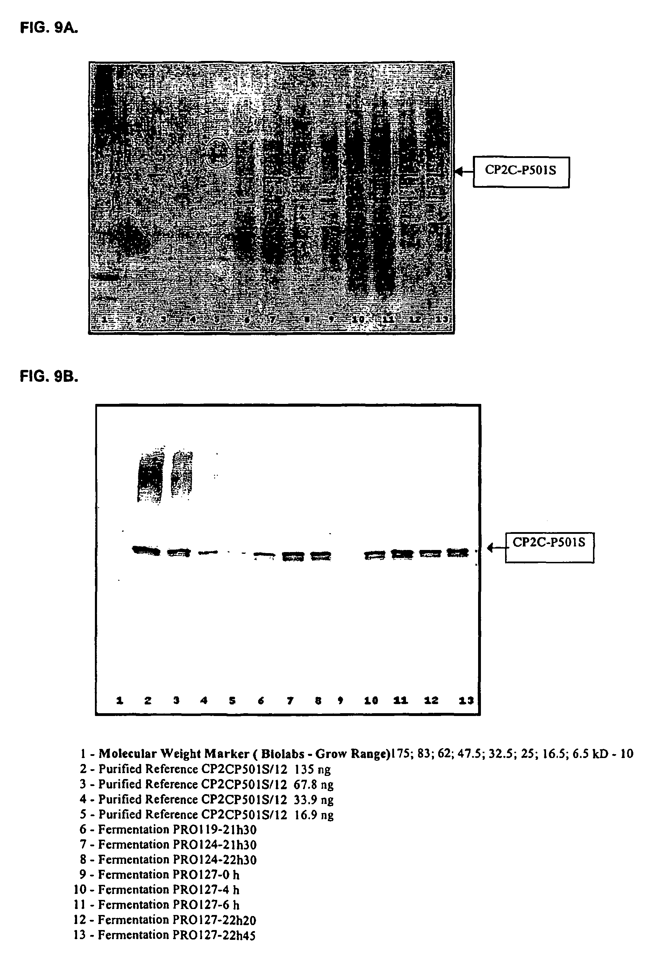

Figures 8 A and B show a kinetics of the antigen production during the induction phase for culture PR0127. It appears that no antigen expression occurred during the growth phase. The specific antigen productivity seems to increase from the beginning of the induction phase up to 6h and then remained quite stable up to the end. But the volumetric productivity increased by a factor 1.5 to 2 due to biomass accumulation observed during the same period of time. The antigen productivity was estimated at about 500 mg per litre of fermentation broth by comparing purified reference of the antigen and crude extracts on SDS-PAGE with silver staining (figure 9A) and WB analyses using an anti-P501 S antibody (a murine ascite directed against P501S aa439-aa459 used at a dilution of 1/1000) (figure 9B).

**<u>Example IV. Purification of CPC-P501 (51-553)-His fusion protein produced by Y1796</u>**

After the cell breakage, the protein is associated with the pellet fraction. A carbamido-methylation of the molecule has been introduced in the process in order to cope with the oxidative aggregation of the molecule with itself or with host cell protein contaminants through covalent bridging with disulphide bonds. The use of detergents has also been required to manage the hydrophobic character of this protein (12 trans-membrane domains predicted).

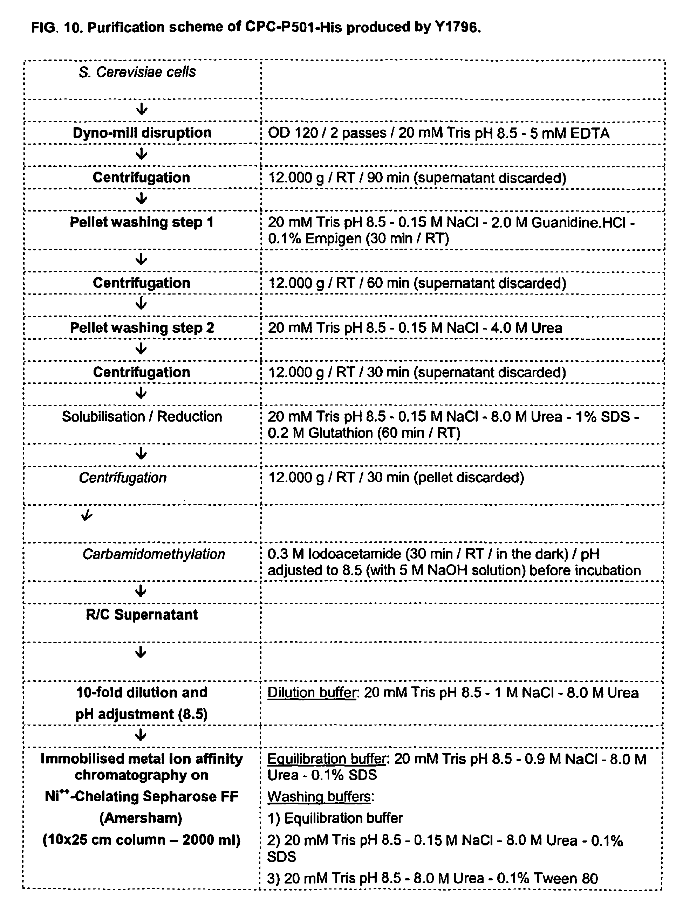

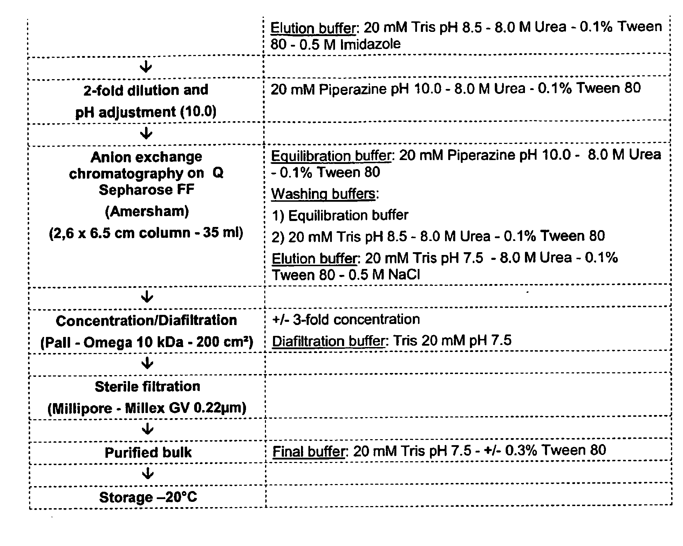

The purification protocol, developed for the scale of 1 L of culture OD (optical density) 120, is described in figure 10. All the operations are performed at room temperature (RT).

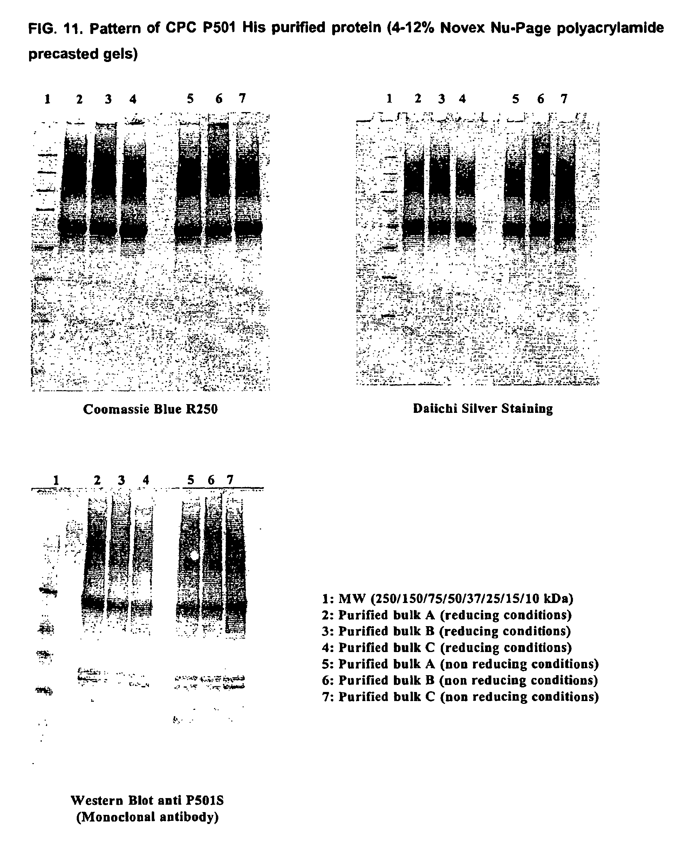

According to DOC TCA BCA protein assay, the global purification yield is 30 - 70 mg of purified antigen / L of culture OD 120. The yield is linked to the level of expression of the culture and is higher as compared to the purification yield of parent strain expressing unfused P501 S-His.
The protein assay is performed as followed: proteins are first precipitated using TCA (trichloroacetic acid) in the presence of DOC (deoxycholate) then dissolved in a alcaline medium in the presence of SDS. The proteins then react with BCA (bicinchoninic acid) (Pierce) to form a soluble purple complex presenting a high adsorbance at 562 nm, which is proportional to the amount of proteins present in the sample.
SDS-PAGE analysis of 3 purified bulks (figure 11) shows no difference in reducing and non reducing conditions (cf. lanes 2, 3 and 4 versus lanes 5, 6 and 7). The pattern consists of a major band at 70 kDa, a smear of higher MW and faint degradation bands. All the bands are detected by a specific anti P501 S monoclonal antibody.

## Example V. Vaccine preparation using CPC- P501S His protein

The protein of Example 3 or 4 can be formulated into a vaccine containing QS21 and 3D-MPL in an oil in water emulsion.

### 1. - Vaccine preparation:

The antigen produced as shown in Example 1 to 3 a C-LytA - P2 - P501 S His. As an adjuvant, the formulation comprises a mixture of 3 de -O-acylated monophosphoryl lipid A (3D-MPL) and QS21 in an oil/water emulsion. The adjuvant system SBAS2 has been previously described WO 95/17210.

**3D-MPL:** is an immunostimulant derived from the lipopolysaccharide (LPS) of the Gram-negative bacterium *Salmonella minnesota.* MPL has been deacylated and is lacking a phosphate group on the lipid A moiety. This chemical treatment dramatically reduces toxicity while preserving the immunostimulant properties (Ribi, 1986). Ribi Immunochemistry produces and supplies MPL to SB-Biologicals.
Experiments performed at Smith Kline Beecham Biologicals have shown that
3D-MPL combined with various vehicles strongly enhances both the humoral and a TH1 type of cellular immunity.

**QS21:** is a natural saponin molecule extracted from the bark of the South American tree Quillaja saponaria Molina. A purification technique developed to separate the individual saponins from the crude extracts of the bark, permitted the isolation of the particular saponin, QS21, which is a triterpene glycoside demonstrating stronger adjuvant activity and lower toxicity as compared with the parent component. QS21 has been shown to activate MHC class I restricted CTLs to several subunit Ags, as well as to stimulate Ag specific lymphocytic proliferation (Kensil, 1992). Aquila (formally Cambridge Biotech Corporation) produces and supplies QS21 to SB-Biologicals.
Experiments performed at SmithKline Beecham Biologicals have demonstrated a clear synergistic effect of combinations of MPL and QS21 in the induction of both humoral and TH1 type cellular immune responses.

**The oil/water emulsion** is composed an organic phase made of of 2 oils (a tocopherol and squalene), and an aqueous phase of PBS containing Tween 80 as emulsifier. The emulsion comprised 5% squalene 5% tocopherol 0.4% Tween 80 and had an average particle size of 180 nm and is known as SB62 (see WO 95/17210).
Experiments performed at SmithKline Beecham Biologicals have proven that the adjunction of this O/W emulsion to 3D-MPL/QS21 (SBAS2) further increases the immunostimulant properties of the latter against various subunit antigens.

### 2. - Preparation of emulsion SB62 (2 fold concentrate):

Tween 80 is dissolved in phosphate buffered saline (PBS) to give a 2% solution in the PBS. To provide 100 ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M110S microfluidics machine. The resulting oil droplets have a size of approximately 180 nm.

### 3. - Formulations:

A typical formulation containing 3D-MPL and QS21 in an oil/water emulsion is performed as follows: 20μg - 25 μg C-LytA P2-P501S are diluted in 10 fold concentrated of PBS pH 6.8 and $H_2O$ before consecutive addition of SB62 (50μl), MPL (20μg), QS21 (20μg), optionally comprising CpG oligonucleotide (100 μg) and 1 μg/ml thiomersal as preservative. The amount of each component may vary as necessary. All incubations are carried out at room temperature with agitation.

## Example VI. Codon-optimised P501S sequences

### 1. - Generation of the control recombinant plasmids:

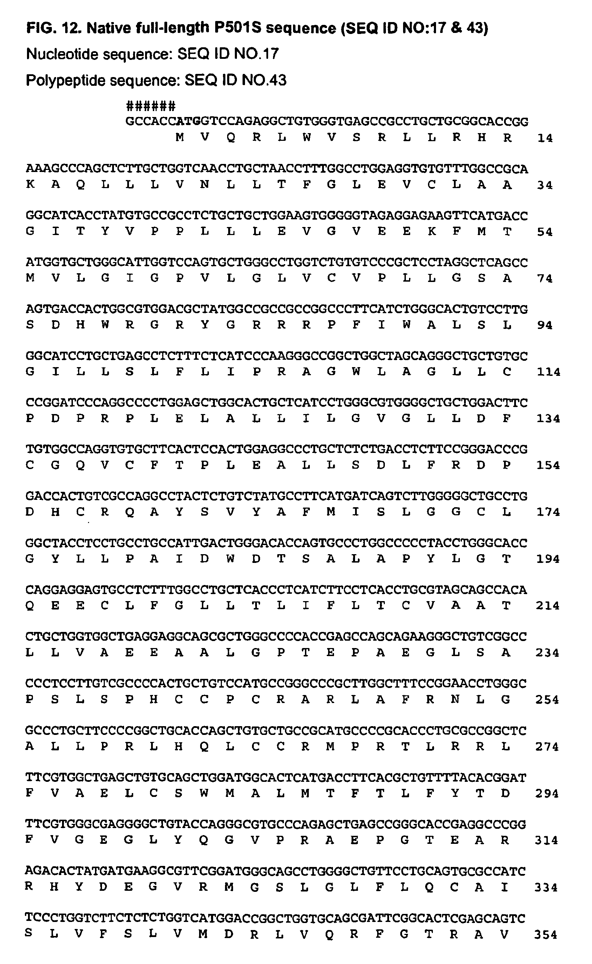

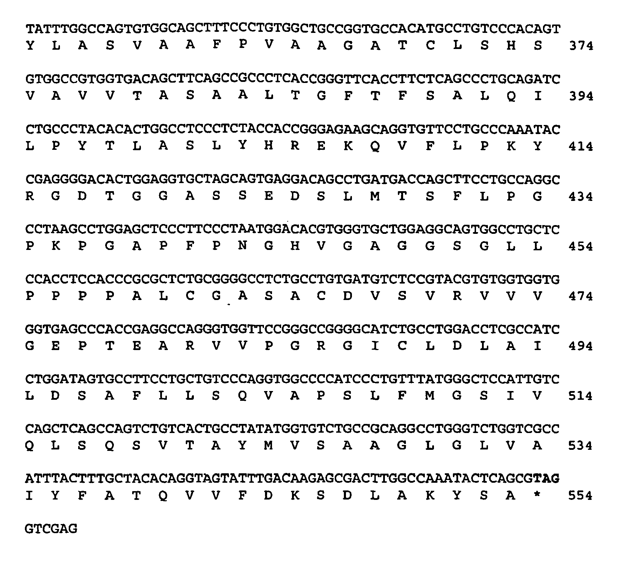

Full-length P501S sequence was cloned into pVAC (Thomsen, Immunology, 1998; 95:510P105), generating expression plasmid JNW680. SEQ ID NO:17 represents human P501S expression cassette in the plasmid JNW680 and is illustrated in Figure 12. The protein sequence of SEQ ID NO:17 is shown in single letter format, the start and stop codons being shown in bold. The Kozak sequence is denoted by the hash symbols. The codon usage index of the human P501 S sequence (SEQ ID NO:17) is 0.618, as calculated by the SynGene programme.

SynGene programme

Basically, the codons are assigned using a statistical method to give synthetic gene having a codon frequency closer to that found naturally in highly expressed *E.coli* and human genes.

SynGene is an updated version of the Visual Basic program called Calcgene, written by R. S. Hale and G Thompson (Protein Expression and Purification Vol. 12 pp.185-188 (1998). For each amino acid residue in the original sequence, a codon was assigned based on the probability of it appearing in highly expressed *E. coli* genes. Details of the Calcgene program, which works under Microsoft Windows 3.1, can be obtained from the authors. Because the program applies a statistical method to assign codons to the synthetic gene, not all resulting codons are the most frequently used in the target organism. Rather, the proportion of frequently and infrequently used codons of the target organism is reflected in the synthetic sequence by assigning codons in the correct proportions. However, as there is no hard-and-fast rule assigning a particular codon to a particular position in the sequence, each time it is run the program will produce a different synthetic gene - although each will have the same codon usage pattern and each will encode the same amino acid sequence. If the program is run several times for a given amino acid sequence and a given target organism, several different nucleotide sequences will be produced which may differ in the number, type and position of restriction sites, intron splice signals etc., some of which may be undesirable. The skilled artisan will be able to select an appropriate sequence for use in expression of the polypeptide on the basis of these features.

Furthermore, since the codons are randomly assigned on a statistical basis, it is possible (although perhaps unlikely) that two or more codons which are relatively rarely used in the target organism might be clustered in close proximity. It is believed that such clusters may upset the machinery of translation and result in particularly low expression rates, so the algorithm for choosing the codons in the optimized gene excludes any codons with an RSCU value of less than 0.2 for highly expressed genes in order to prevent any rare codon clusters being fortuitously selected. The distribution of the remaining codons is then allocated according to the frequencies for highly expressed *E. coli* to give an overall distribution within the synthetic gene that is typical such genes (coefficient = 0.85) and also for highly expressed human genes (coefficient = 0.50).
Syngene (Peter Ertl, unpublished), an updated version of the Calcgene program, allows exclusion of rare codons to be optional, and is also used to allocate codons according to the codon frequency pattern of highly expressed human genes.

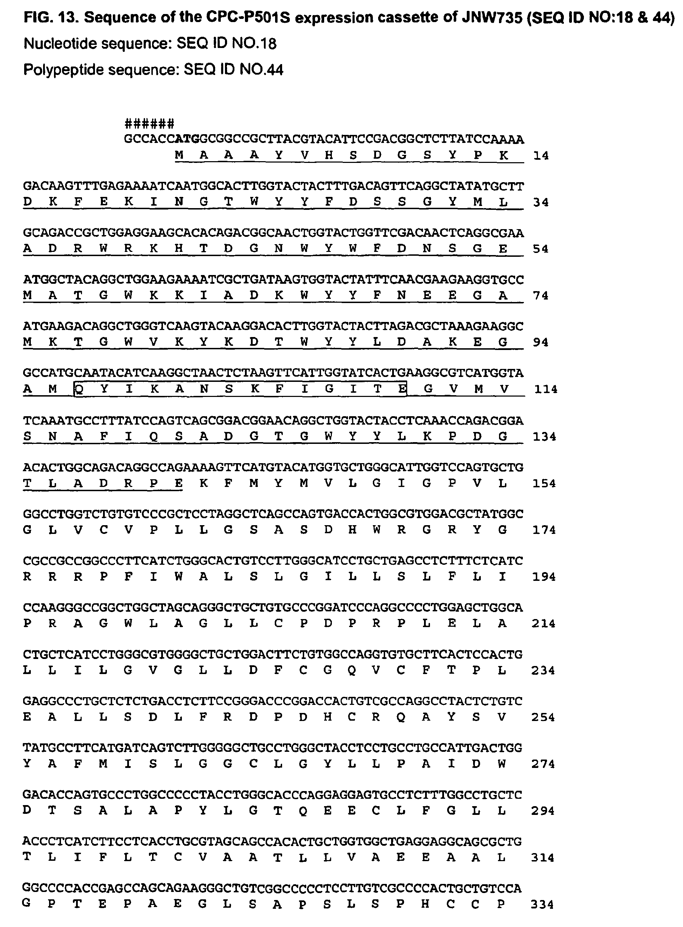

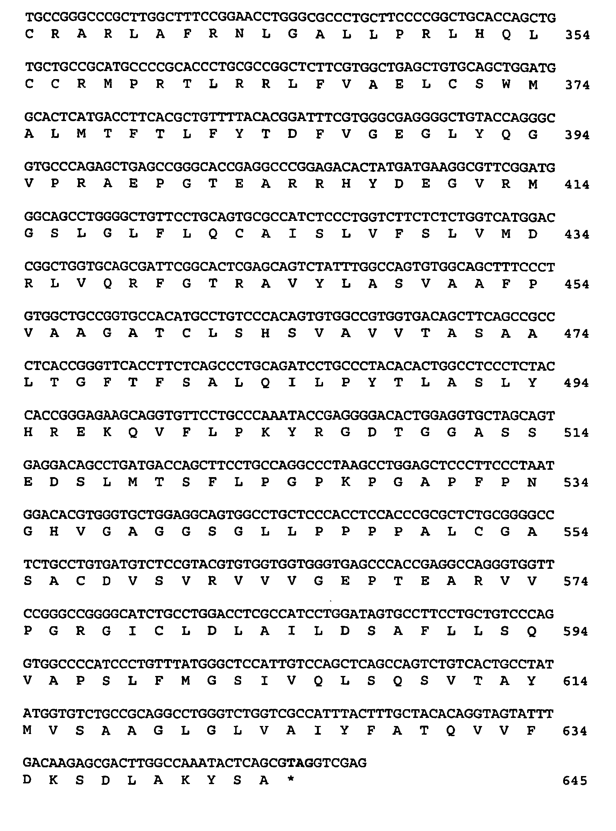

The sequence of the CPC-P501 S cassette cloned from the vector pRIT15201 (see Figure 7) into pVAC, thereby generating plasmid JNW735, is set forth in SEQ ID NO:18 and is illustrated in Figure 13. This sequence is identical to the pRIT15201 sequence with the exception of the removal of the His tag and the addition of a Kozak sequence (GCCACC) and appropriate restriction enzyme sites. The amino acid sequence of SEQ ID NO:18 is shown in single letter format, the start and stop codons are shown in bold. The boxed residues are the P2 helper epitope of tetanus toxoid. The underlined residues are the Clyta purification tag. The Kozak sequence is denoted by the hash symbols.

### 2. - Generation of the recombinant plasmids with P501S codon optimised sequences:

Although the codon coefficient index (CI) of P501S native sequence is already high (0.618), it is possible increase the CI value further. This will have two potential benefits - to improve the antigen expression and/or immunogenicity and to reduce the possibility for recombination between the P501 S vector and genomic sequences.

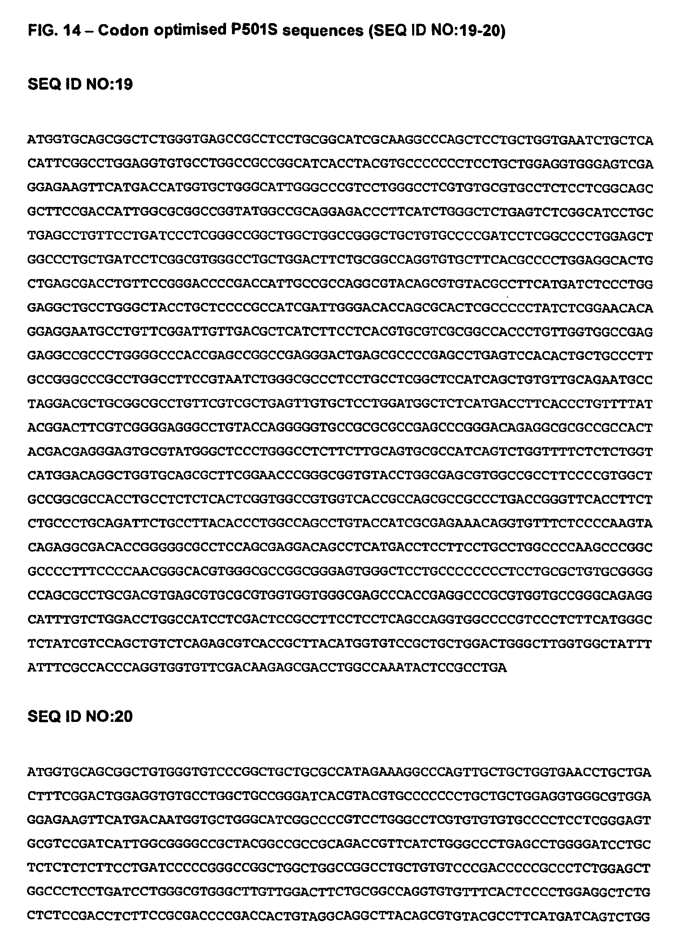

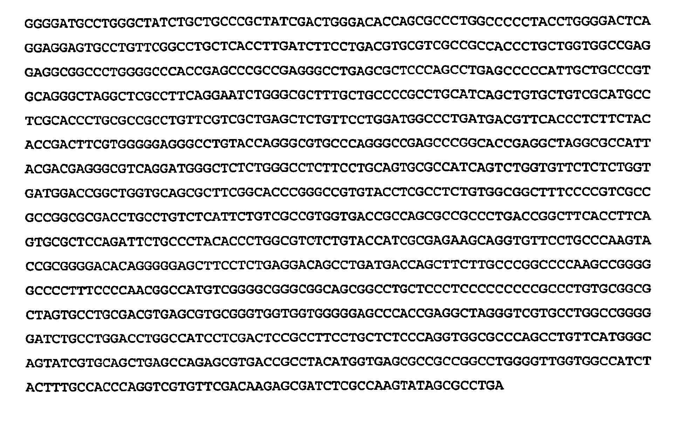

Using the Syngene programme, a selection (SEQ ID N0:19 to SEQ ID NO:20) of codon optimised sequences was obtained (Figure 14). Table 3 below shows a comparison of the codon coefficient index for the starting P501 S sequence and the two representative codon optimised sequences, selected on the basis of a suitable restriction enzyme site profile and a good CI index.

Table 3 - Comparison of the codon coefficient indices of two codon optimised P501 S genes

| Sequence | Codon coefficient index (CI) |
|---|---|
| **P501 S** | 0.618 |

(continued)

| Sequence | Codon coefficient index (CI) |
|---|---|
| **SEQ ID NO:19** | 0.725 |
| **SEQ ID NO:20** | 0.755 |

### 3. Further evaluation of the codon-optimised sequences

#### Sequence SEQ ID NO:19

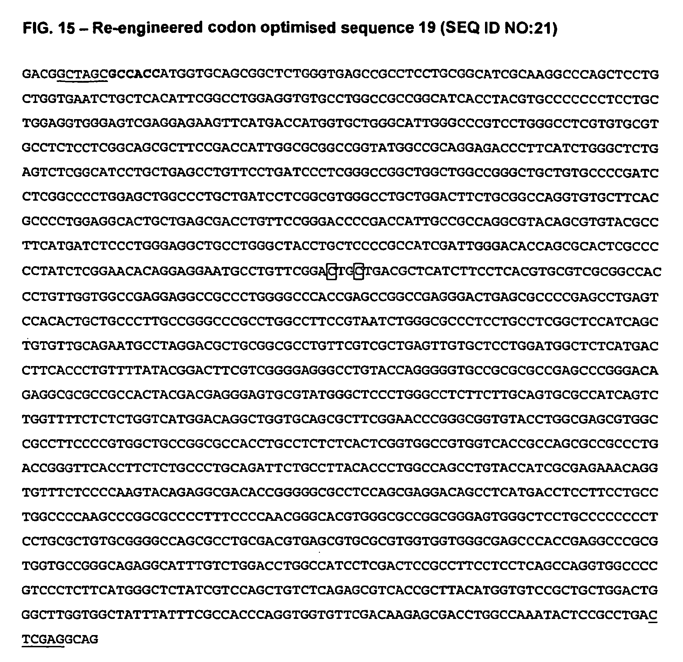

Although SEQ ID NO: 19 has a good CI index (0.725), it contains a doublet of rare codons at amino acids position 202 and 203. These codons were manually substituted with more frequent codons by changing the DNA sequence from TTGTTG to CTGCTG. To facilitate cloning and expression, restriction enzyme sites and a Kozak sequence were added. The final engineered sequence (SEQ ID NO:21) is shown in Figure 15. The Syngene programme was used to fragment this sequence into oligonucleotides with a minimum overlap of 19-20 bases. Therefore, Figure 15 shows the re-engineered P501S codon optimised SEQ ID NO. 19. Restriction enzyme sites are underlined, Kozak sequence is bolded, re-engineered DNA sequence to remove a rare codon doublet is boxed.

Using a two-step PCR protocol, the overlapping primers generated by the Syngene programme were first assembled using a PCR Assembly protocol (detailed below). The assembly reaction generates a diverse population of fragments. The correct full-length fragment was recovered/amplified using the PCR recovery protocol and the terminal primers. The resulting PCR fragment was excised from an agarose gel, purified, restricted with Nhel and Xhol and cloned into pVAC. Positive clones were identified by restriction enzyme analysis and confirmed by double-stranded sequencing. This generates plasmid JNW766, which, due to the error-prone nature of the PCR process, contained a single silent mutation (C to T at position 360 of SEQ ID NO: 21).

1. Assembly reaction - PCR conditions, generic protocol

Reaction mix (total volume = 50μl):

- 1 x Reaction buffer (Pfx or Proofstart)
- 1μl Oligo pool (equal mix of all overlapping oligos)
- 0.5mM dNTPs
- DNA polymerase (Pfx or Proofstart, 2.5-5U)
- +/-1mM $MgSO_4$
- +/-1x enhancer solution (Pfx enhancer or Proofstart buffer Q)

1. 94°C for 120s (Proofstart only)
2. 94°C for 30s
3. 40°C for 120s
4. 72°C for 10s
5. 94°C for 15s
6. 40°C for 30s
7. 72°C for 20s + 3s/cycle
8. Cycle to step 5, 25 times
9. Hold at 4°C

2. Recovery reaction - PCR conditions (generic protocol)

Reaction mix (total volume = 50μl):

- 1 x Reaction buffer (Pfx or Proofstart)
- 5-10μl assembly reaction mix
- 0.3-0.75mM dNTPs
- 50pmol primer (5' terminal primer, sense orientation)
- 50pmol primer (3' terminal primer, anti-sense orientation)
- DNA polymerase (Pfx or Proofstart, 2.5-5U)
- +/-1 mM $MgSO_4$

- +/-1x enhancer solution (Pfx enhancer or Proofstart buffer Q)

1. 94°C 120s (Proofstart only)
2. 94°C 45s
3. 60°C 30s
4. 72°C 120s
5. Cycle to step 2, 25 times
6. 72°C 240s
7. Hold at 4°C

**Sequence SEQ ID NO:20**

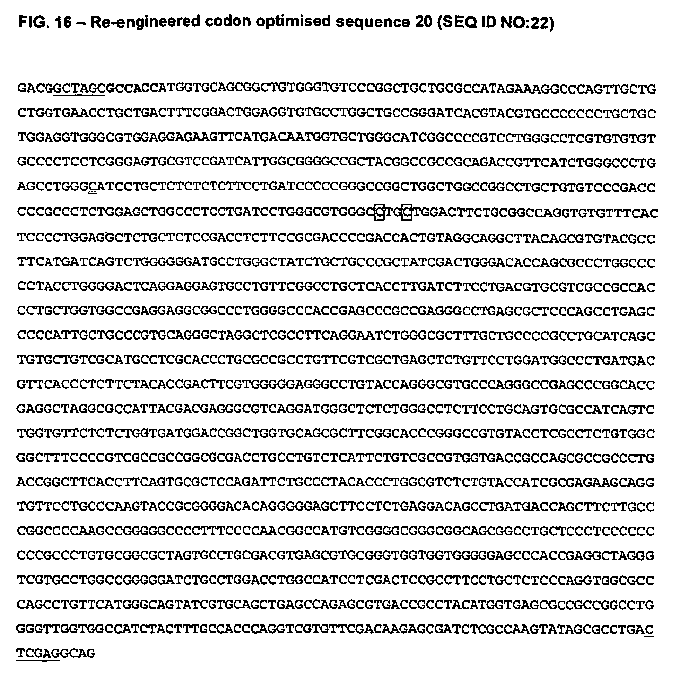

Although SEQ ID NO: 20 has a very good CI index (0.755), it was noticed that it contained a doublet of rare codons at amino acids position 131 and 132. These codons were manually substituted with more frequent codons by changing the DNA sequence from TTGTTG to CTGCTG. To facilitate cloning, an internal BamHI site was removed by mutating G to C (see the double-underlined nucleotide in Figure 16). To facilitate cloning and expression, restriction enzyme sites and a Kozak sequence were added. The final engineered sequence (SEQ ID NO:22) is shown in Figure 16. The Syngene programme was used to fragment this sequence into oligonucleotides with a minimum overlap of 19-20 bases.

Figure 16 therefore shows the re-engineered P501S codon optimised sequence 20 (SEQ ID NO:22). Restriction enzyme sites are underlined, Kozak sequence is bolded, re-engineered DNA sequence to remove a rare codon doublet is boxed and a silent point mutation to remove a BamHI site is double-underlined.

Using a similar two-step PCR protocol to the one described above, full-length P501S fragment was amplified and cloned into pVAC. Positive clones were identified by restriction enzyme analysis and confirmed by double-stranded sequencing. This generates plasmid JNW764. The sequence of the P501S coding cassette is shown in Figure 16 (SEQ ID NO: 22).

**DNA Sequence similarity**

Pair distances following alignment by the ClustalV (weighted) method are shown in Table 3 below. Table 4 below shows percent similarity between the starting human P501S sequence and the two codon optimised sequences SEQ ID NO:21 and 22 selected for further investigation. The data confirms that the codon optimised DNA sequences are approximately 80% similar to the original P501 S sequence.

Table 4

| SEQ ID NO: | % similarity with starting P501S sequence |
|---|---|
| **21** | 79.6 |
| **22** | 79.4 |

**Example VII. Codon-optimised CPC sequences**

**1.- Approach**

Since the original CPC sequence was originally designed for optimal expression in yeast, this section describes the process of codon optimising for human expression.

**2.- Sequence design**

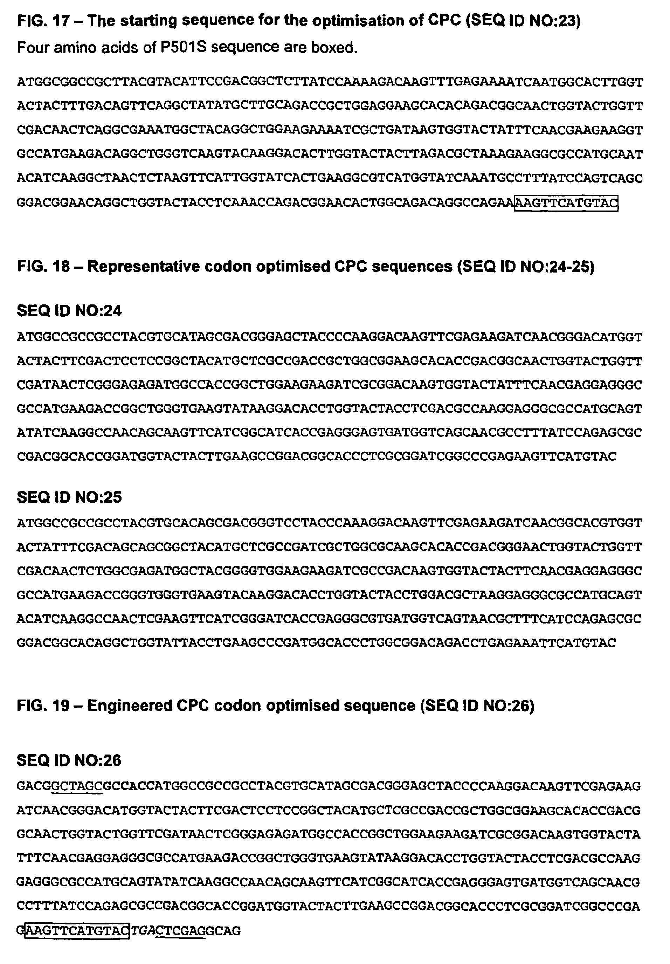

The starting sequence for the optimisation of CPC is shown in Figure 17 (SEQ ID NO: 23). This is derived entirely from the pRIT15201 and contains the entire coding sequence of CPC plus four amino acids of P501S to facilitate downstream cloning. Using the Syngene programme, a selection of codon optimised sequences were obtained, from which representative sequences are shown in Figure 18 (SEQ ID NO: 24-25). Table 5 below shows a comparison of the codon coefficient index for the starting CPC sequence and the two representative codon optimised sequences.

Table 5. Codon coefficient indices for two CPC optimised sequences

| Sequence | Codon coefficient index (CI) |
|---|---|
| **Original CPC = SEQ ID NO:23** | 0.506 |
| **SEQ ID NO:24** | 0.809 |
| **SEQ ID NO:25** | 0.800 |

In addition to the codon optimisation, all sequences were also screened for restriction enzyme cloning sites. On the basis of the highest CI value and a favourable restriction enzyme site profile, SEQ ID NO: 24 was selected for construction. To facilitate cloning and expression, 5' and 3' cloning sites were added and a Kozak sequence (GCCACC) was inserted 5' of the initiating ATG start codon. This engineered sequence is shown in Figure 19 (SEQ ID NO:26). This sequence includes four amino aicds of P501 S (boxed), restriction enzyme cloning sites (Nhel and Xhol, underlined), a Kozak sequence (Bold), a stop codon (italicised) and 4bp of flanking irrelevant DNA to facilitate cloning.

The Syngene programme was used to fragment this sequence into 50-60-mer oligonucleotides with a minimum overlap of 18-20 bases.

Using a similar two-step PCR protocol to the one described above, the correct fragment was recovered/amplified and cloned into pVAC. Positive clones were identified by restriction enzyme analysis and sequence verified generating vector JNW759.

**4.- DNA similarity**

Pair Distances following alignment ClustalV (Weighted) are shown in Table 6 below. The table shows percent similarity at the DNA level between the starting sequence of CPC and the codon optimised sequence and confirms that the codon optimised sequences are approximately 80% similar to the original CPC sequence.

Table 6

| Sequence SEQ ID NO: | % similarity with starting CPC sequence |
|---|---|
| **24** | 80.2 |
| **25** | 81.6 |

**Example VIII. Construction of the P501S fusion candidate**

All the candidates shown in the schematic below are codon optimised and constructed using overlapping PCR methodologies from plasmids JNW764 and JNW759 as templates (SEQ ID NO: 22 and SEQ ID NO: 26 respectively), and cloned into the expression vector p7313 ie.

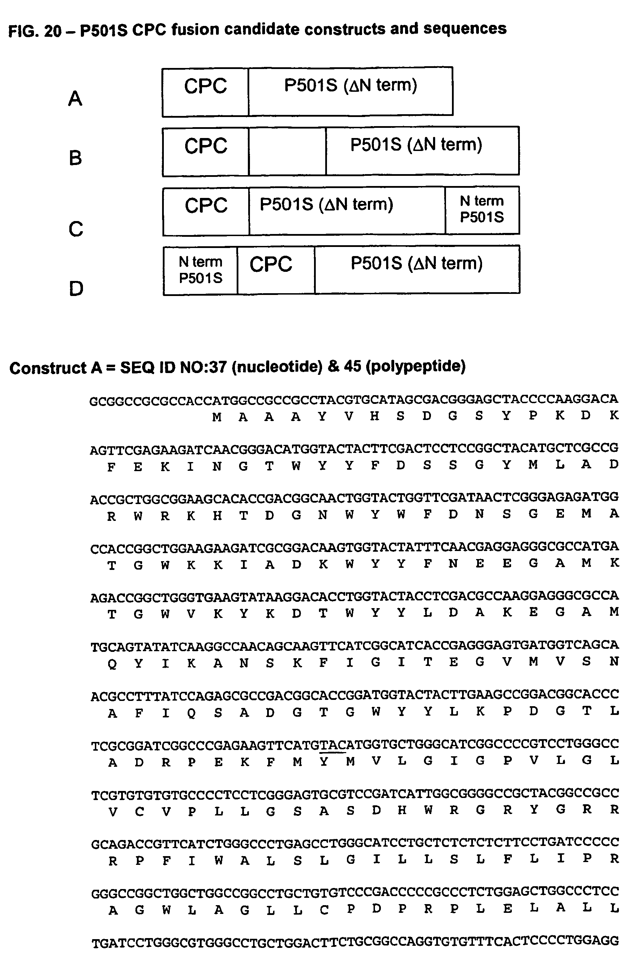

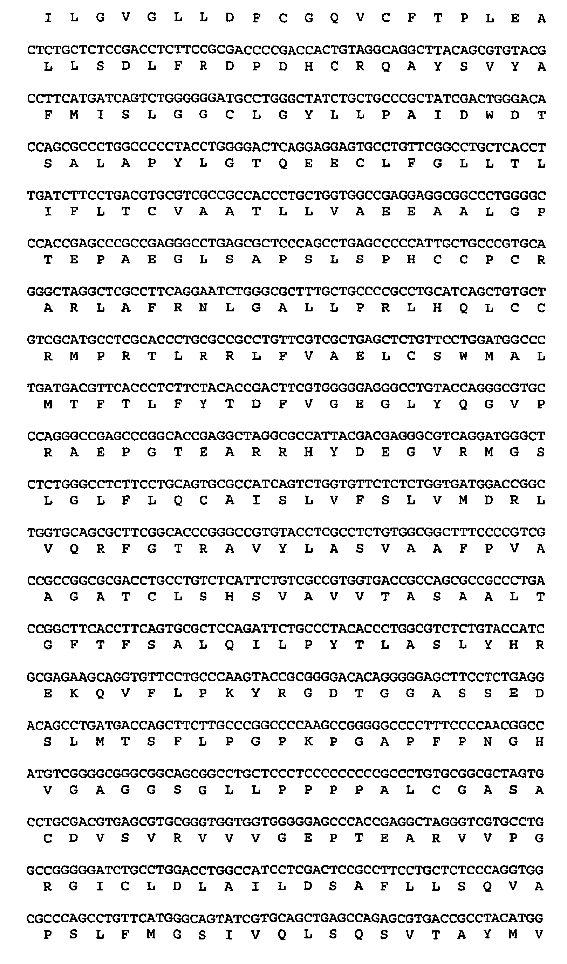

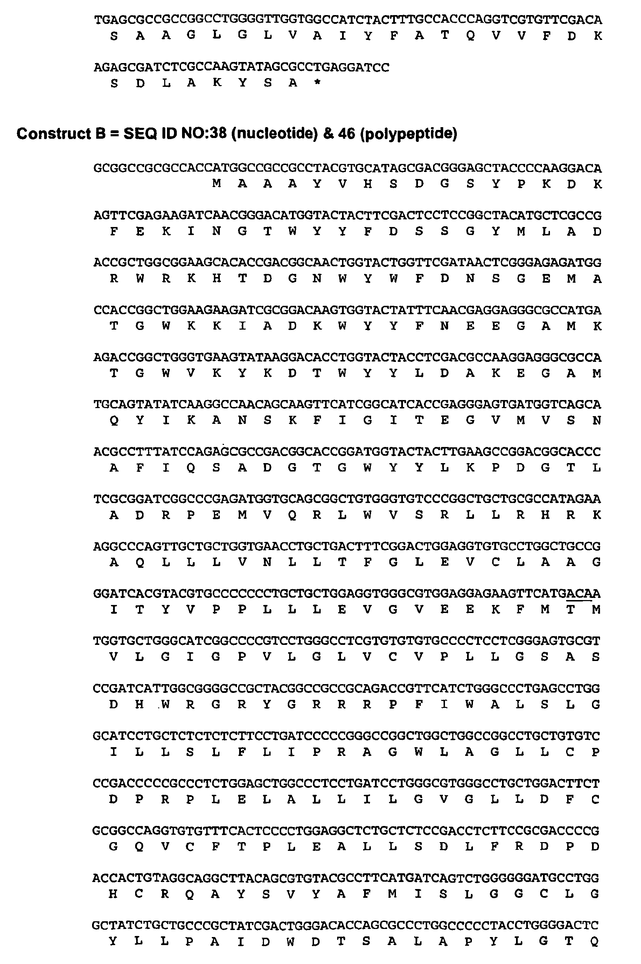

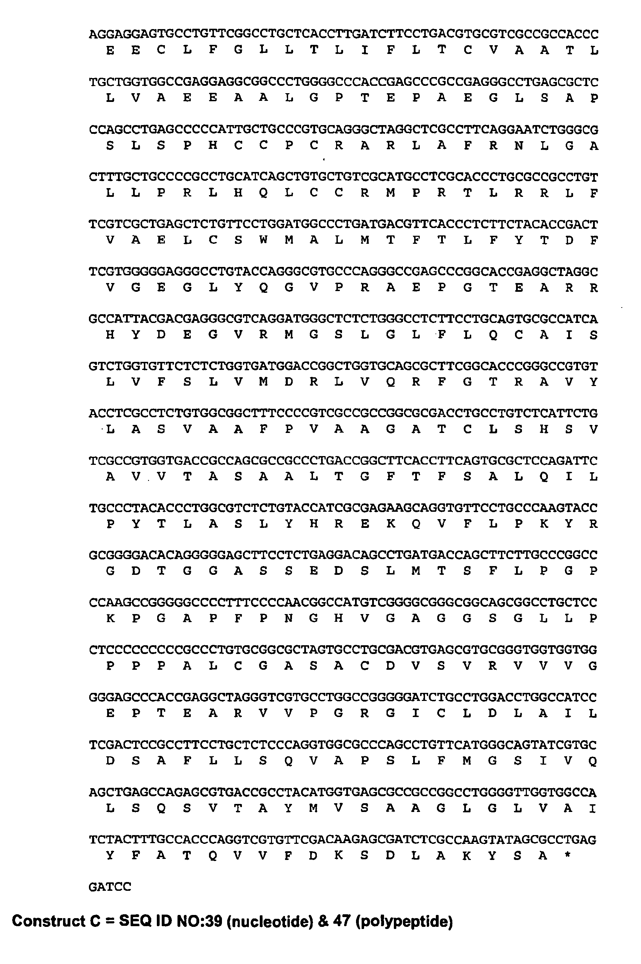

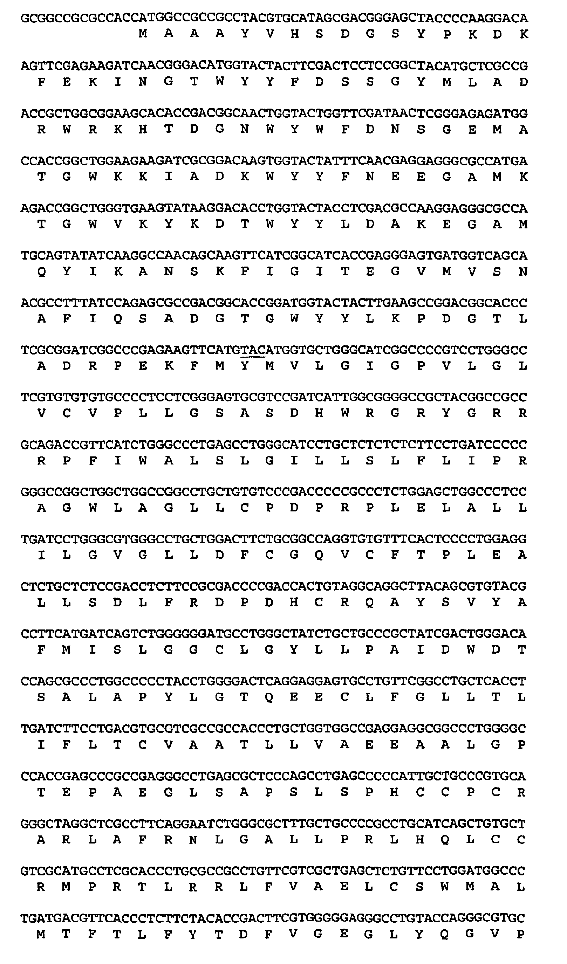

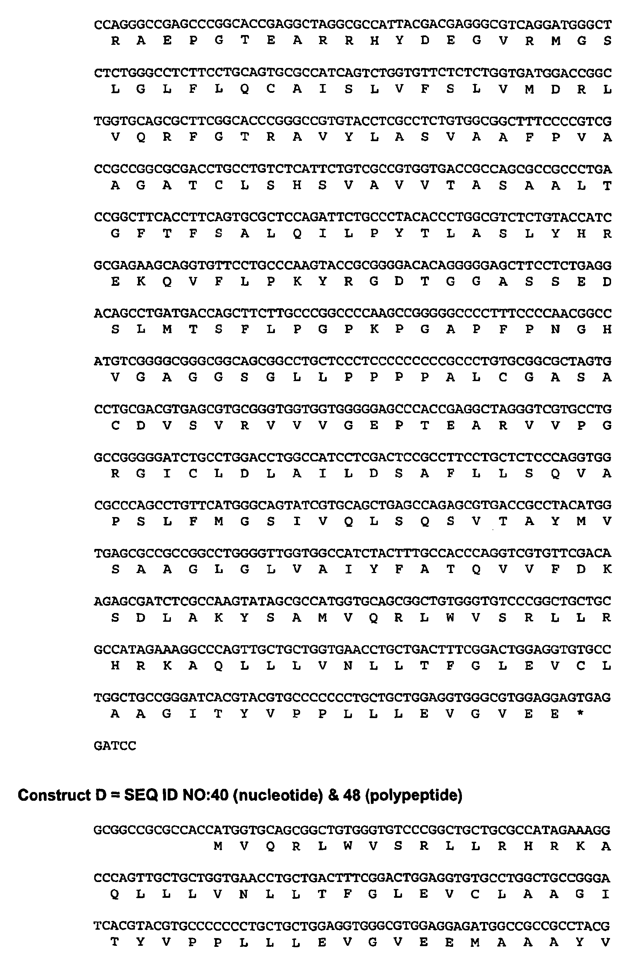

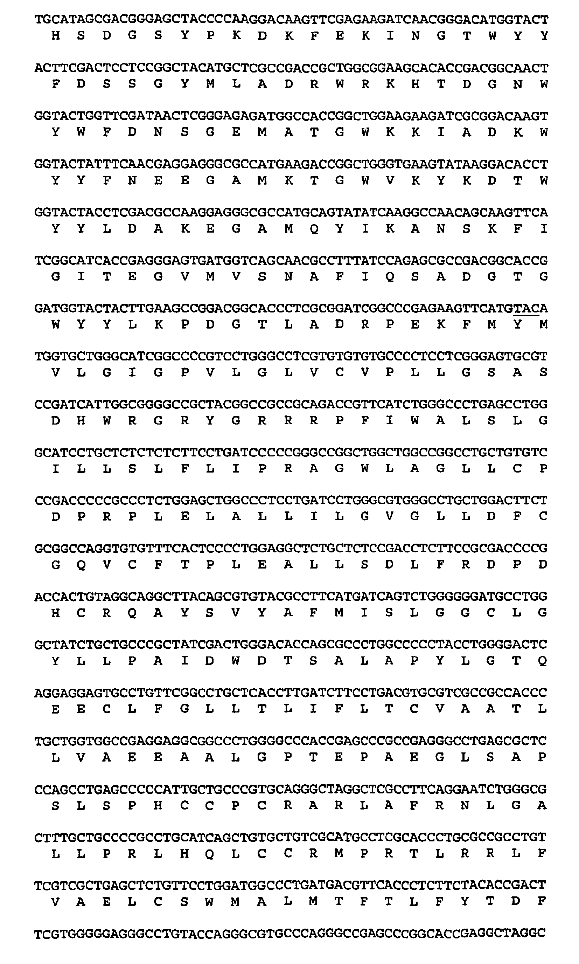

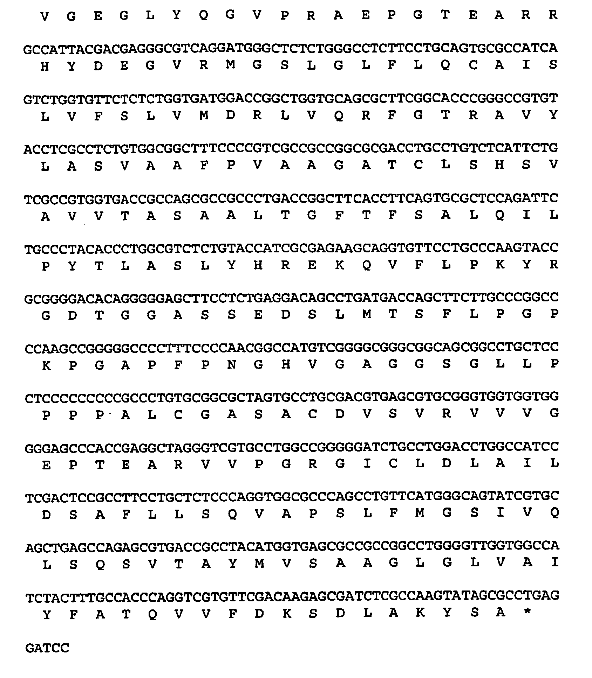

The four candidates shown schematically below are based upon CPC-P501S. Codon optimised CPC-P501S is construct A. Candidates B, C, D also include the sequence encoding the N terminal 50 amino acids of P501S, positioned either at the N terminus of CPC-P501S (construct D), the C terminus of CPC-P501S (construct C), or between CPC and P501S (construct B). A schematic representation of the constructs is given in Figure 20.

The nucleotide and protein sequence for each of the four constructs is shown in SEQ ID NO: 37-40 for the nucleotide sequences, and SEQ ID NO. 45-48 for the corresponding polypeptide sequences. In constructs A, C and D, the underlined codon preferentially encodes tyrosine (either TAC or TAT) but the nucleotide sequence may be altered to encode threonine (either ACA, ACC, ACG or ACT). In construct B, the underlined codon preferentially encodes threonine (either ACA, ACC, ACG or ACT), but the nucleotide sequence may be altered to encode tyrosine (either TAC or TAT). In all constructs, the coding sequence is flanked by appropriate restriction enzyme cloning sites (in this case, Notl and BamHl), and a Kozak sequence immediately upstream of the initiating ATG. Table 7 below shows the plasmid identification for the constructs detailed above:

**Table 7**

| Construct | Amino acid at underlined codon | Sequence of codon | Plasmid ID |
|---|---|---|---|
| A | Tyrosine | TAC | JNW771 |
| B | Threonine | ACA | JNW773 |
| B | Tyrosine | TAC | JNW770 |

(continued)

| Construct | Amino acid at underlined codon | Sequence of codon | Plasmid ID |
|---|---|---|---|
| C | Tyrosine | TAC | JNW777 |
| D | Tyrosine | TAC | JNW769 |

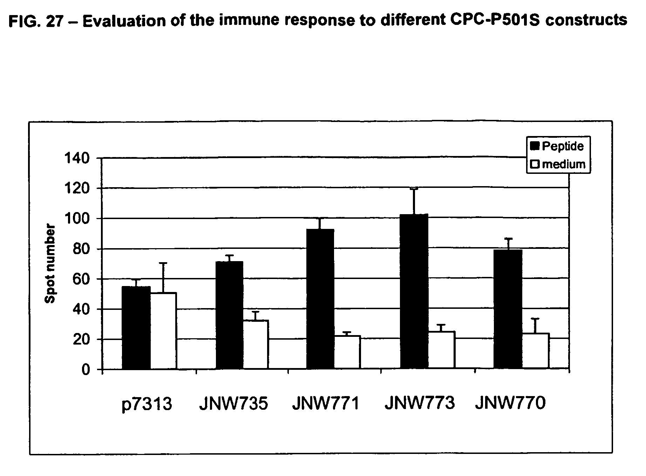

The cellular responses following immunisation with p7313-ie (empty vector), pVAC-P501 S (JNW735), JNW770, JNW771 and JNW773 were assessed by ELISPOT following a primary immunisation by PMID at day 0 and three boosts at day 21, 42 and 70. Assays were carried out 7 days post boost. Figure 27 shows that good IL-2 ELISPOT responses were detected in mice immunised with JNW770, JNW771 and JNW773.

**Example IX. Immunogenicity experiments using particle-mediated intra-dermal delivery (PMID) studies**

Full-length P501 S, when delivered by particle mediated intra-dermal delivery (PMID), generates good antibody & cellular responses. These data demonstrate that the PMID is a very effective delivery route. Furthermore, comparison of P501 S and CPC-P501 S confirms that CPC-P501S induces a stronger immune response as determined by peptide ELISPOT.

**1.- Materials & Methods**

**1.1. Cutaneous gene gun immunisation**

Plasmid DNA was precipitated onto 2μm diameter gold beads using calcium chloride and spermidine. Loaded beads were coated onto Tefzel tubing as described (Eisenbraum et al, 1993; Pertmer et al, 1996). Particle bombardment was performed using the Accell gene delivery system (PCT WO 95/19799). For each plasmid, female C57BU6 mice were immunised on days 0, 21, 42 and 70. Each administration consisted of two bombardments with DNA/gold, providing a total dose of approximately 4-5 μg of plasmid.

**1.2. ELISPOT assays for T cell responses to the P501S gene product**

a) Preparation of splenocytes

Spleens were obtained from immunised animals at 7-14 days post boost. Spleens were processed by grinding between glass slides to produce a cell suspension. Red blood cells were lysed by ammonium chloride treatment and debris was removed to leave a fine suspension of splenocytes. Cells were resuspended at a concentration of $8x10^8$/ml in RPMI complete media for use in ELISPOT assays.

b) Screening of peptide library

A peptide library covering a majority of the P501 S sequence was obtained from Corixa Corp. The library contained fifty 15-20mer peptides overlapping by 4-11 amino acids peptides. The peptides are numbered 1-50. In addition, a prediction programme (H-G. Rammensee, et al.: Immunogenetics, 1999, 50: 213-219) (http://syfpeithi.bmi-heidelberg.com/) was used to predict putative Kb and Db epitopes from the P501 S sequence. The ten best epitopes for Kb and Db were ordered from Mimotopes (UK) and included in the library (peptides 51-70). For screening of the peptide library, peptides were used at a final concentration of 50μg/ml (approx. 25-50μM) in IFNy and IL-2 ELISPOTS using the protocol described below. For IFNγ ELISPOTS, IL-2 was added to the assays at 10ng/ml. Splenocytes used for the screening were taken at day 84 from C57BU6 mice immunised at day 0, 21, 42 and 70. Three peptides were identified from the library screen - Peptides 18 (HCRQAYSVYAFMISLGGCLG), 22 (GLSAPSLSPHCCPCRARLAF) and 48 (VCLAAGITYVPPLLLEVGV). These peptides were subsequently used in the ELISPOT assays

c) ELISPOT assay

Plates were coated with 15μg/ml (in PBS) rat anti mouse IFNy or rat anti mouse IL-2 (Pharmingen). Plates were coated overnight at +4°C. Before use the plates were washed three times with PBS. Splenocytes were added to the plates at $4x10^5$ cells/well. Peptides identified in the library screen were re-ordered from Genemed Synthesis and used at a final concentration of 50μg/ml. CPC-P501S protein (GSKBio) was used in the assay at 20μg/ml. ELISPOT assays were carried out in the presence of either IL-2 (10ng/ml), IL-7 (10ng/ml) or no cytokine. Total volume in each

well was 200μl. Plates containing peptide stimulated cells were incubated for 16 hours in a humidified 37°C incubator.

e) Development of ELISPOT assay plates.

Cells were removed from the plates by washing once with water (with 10 minute soak to ensure lysis of cells) and three times with PBS. Biotin conjugated rat anti mouse IFNg or IL-2 (Phamingen) was added at 1μg/ml in PBS. Plates were incubated with shaking for 2 hours at room temperature. Plates were then washed three times with PBS before addition of Streptavidin alkaline phosphatase (Caltag) at 1/1000 dilution. Following three washes in PBS spots were revealed by incubation with BCICP substrate (Biorad) for 15-45 mins. Substrate was washed off using water and plates were allowed to dry. Spots were enumerated using an image analysis system devised by Brian Hayes, Asthma Cell Biology unit, GSK.

**1.3. ELISA assay for antibodies to the P501 S gene product**

Serum samples were obtained from the animals by venepuncture on days -1, 28, 49 and 56, and assayed for the presence of anti-P501S antibodies. ELISA was performed using Nunc Maxisorp plates coated overnight at 4°C with 0.5μg/ml of CPC-P501S protein (GSKBio) in sodium bicarbonate buffer. After washing with TBS-Tween (Tris-buffered saline, pH 7.4 containing 0.05 % of Tween 20) the plates were blocked with Blocking buffer (3% BSA in TBS-Tween buffer) for 2hrs at room temperature. All sera were incubated at 1:100 dilution for 1 hr at RT in Blocking buffer. Antibody binding was detected using HRP-conjugated rabbit anti-mouse immunoglobulins (#P0260, Dako) at 1:2000 dilution in Blocking buffer. Plates were washed again and bound conjugate detected using Fast OPD colour reagents (Sigma, UK). The reaction was stopped by the addition of 3M sulphuric acid, and the OPD product quantitated by measuring the absorbance at 490nm.

**1.4. Transient transfection assays**

Human P501 S expression from various DNA constructs was analysed by transient transfection of the plasmids into CHO (Chinese hamster ovary) cells followed by Western blotting on total cell protein. Transient transfections were performed with the Transfectam reagent (Promega) according to the manufacturer's guidelines. In brief, 24-well tissue culture plates were seeded with $5x10^4$ CHO cells per well in 1 ml DMEM complete medium (DMEM, 10% FCS, 2mM L-glutamine, penicillin 100IU/ml, streptomycin 100μg/ml) and incubated for 16 hours at 37°C. 0.5μg DNA was added to 25μl of 0.3M NaCl (sufficient for one well) and 2μl of Transfectam was added to 25μl of Milli-Q. The DNA and Transfectam solutions were mixed gently and incubated at room temperature for 15 minutes. During this incubation step, the cells were washed once in PBS and covered with 150μl of serum free medium (DMEM, 2mM L-glutamine). The DNA-Transfectam solution was added drop wise to the cells, the plate gentle shaken and incubated at 37°C for 4-6 hours. 500μl of DMEM complete medium was added and the cells incubated for a further 48-72 hours at 37°C.

**2. Western blot analysis of CHO cells transiently transfected with P501S plasmids**

The transiently transfected CHO cells were washed with PBS and treated with a Versene (1:5000)/0.025% trypsin solution to transfer the cells into suspension. Following trypsinisation, the CHO cells were pelleted and resuspended in 50μl of PBS. An equal volume of 2x NP40 lysis buffer was added and the cells incubated on ice for 30 minutes. 100μl of 2x TRIS-Glycine SDS sample buffer (Invitrogen) containing 50mM DTT was added and the solution heated to 95°C for 5 minutes. 1-20μl of sample was loaded onto a 4-20% TRIS-Glycine Gel 1.5mm (Invitrogen) and electrophoresed at constant voltage (125V) for 90 minutes in 1x TRIS-Glycine buffer (Invitrogen). A pre-stained broad range marker (New England Biolabs, #P7708S) was used to size the samples. Following electrophoresis, the samples were transferred to Immobilon-P PVDF membrane (Millipore), pre-wetted in methanol, using an Xcell III Blot Module (Invitrogen), 1x Transfer buffer (Invitrogen) containing 20% methanol and a constant voltage of 25V for 90 minutes. The membrane was blocked overnight at 4°C in TBS-Tween (Tris-buffered saline, pH 7.4 containing 0.05 % of Tween 20) containing 3% dried skimmed milk (Marvel). The primary antibody (10E3) was diluted 1:1000 and incubated with the membrane for 1 hour at room temperature. Following extensive washing in TBS-Tween, the secondary antibody (HRP-conjugated rabbit anti-mouse immunoglobulins (#P0260, Dako)) was diluted 1:2000 in TBS-Tween containing 3% dried skimmed milk and incubated with the membrane for one hour at room temperature. Following extensive washing, the membrane was incubated with Supersignal West Pico Chemiluminescent substrate (Pierce) for 5 minutes. Excess liquid was removed and the membrane sealed between two sheets of cling film, and exposed to Hyperfilm ECL film (Amersham-PharmaciaBiotech) for 1-30 minutes.

### 3. Generation of the Full-length human P501S expression cassette

The starting point for the construction of a P501S expression cassette was the plasmid pcDNA3.1-P501 S (Corixa Corp), which has a pcDNA3.1 backbone (Invitrogen) containing a full-length human P501 S cDNA cassette cloned between the EcoRl and Notl sites. This vector is also termed JNW673. The presence of P501S was confirmed by fluorescent sequencing. The sequence of the cDNA cassette is given by the NCBI/Genbank sequence (accession number AY033593). Human P501 S was PCR amplified from JNW673 template DNA, restricted with Xbal and Sall and cloned into the Nhel/Xhol sites of pVAC generating vector JNW680. The correct orientation of the fragment relative to the CMV promoter was confirmed by PCR and by DNA sequencing. The sequence of the expression cassette is shown in Figure 12 (SEQ ID NO: 17).
To construct a CPC-P501 S expression cassette, CPC-P501 S was PCR amplified from the vector pRIT15201 (see Figure 7), restricted with Xbal and Sall and cloned into the Nhel and Xhol sites of pVAC, generating plasmid JNW735. The correct orientation was confirmed by PCR and sequencing. The sequence of the CPC-P501 S expression cassette is shown in Figure 13 (SEQ ID N0:18).

### 4. Expression of human P501S from plasmids JNW680 and JNW735

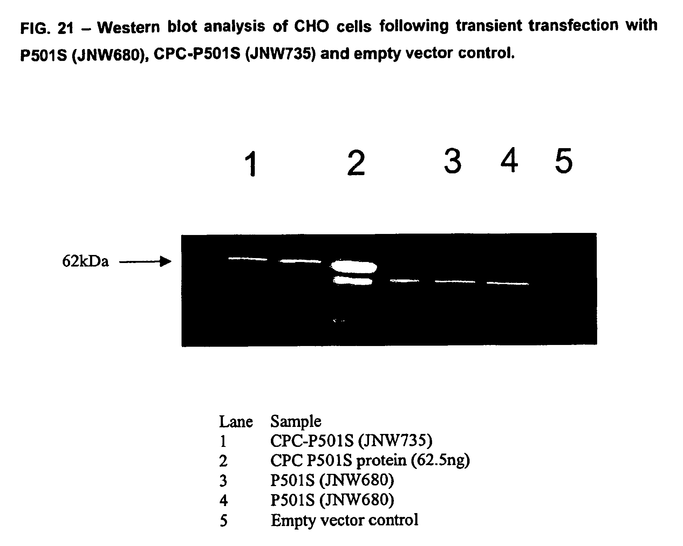

The P501S expression plasmids were transiently transfected into CHO cells and a total cell lysate prepared as described in methods. A Western blot of a total cell lysate identified single bands of approximately 55kDa and 62kDa for samples transfected with JNW680 and JNW735 respectively (Figure 21). This is consistent with the predicted molecular weights of 59.3kDa and 63.3kDa for P501 S and CPC-P501 S respectively. The addition of the CPC tag does not adversely affect the expression of P501 S.

### 5. Results

#### 5.1. Antibody responses to human P501S following PMID Immunisation

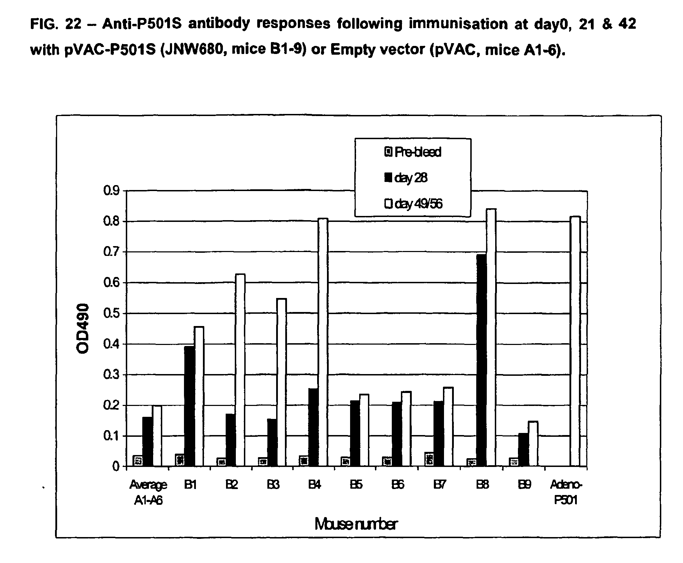

The antibody responses following immunisation with pVAC (empty vector) and pVAC-P501S (JNW680) were assessed by ELISA following a primary immunisation by PMID at day 0 and three boosts at day 21 and day 42 and day 70. Figure 22 shows the antibody responses from sera taken at day -1, day 28 and day 49 (mice A1-3, B1-3) and day 56 (mice A4-6, B4-9). Whilst there were some non-specific responses to the pVAC empty vector, specific responses to the P501 S construct were seen in 5 of 9 mice.

#### 5.2. Identification of novel T cell epitopes from human P501S in C57BU6 mice by screening of a P501S peptide library

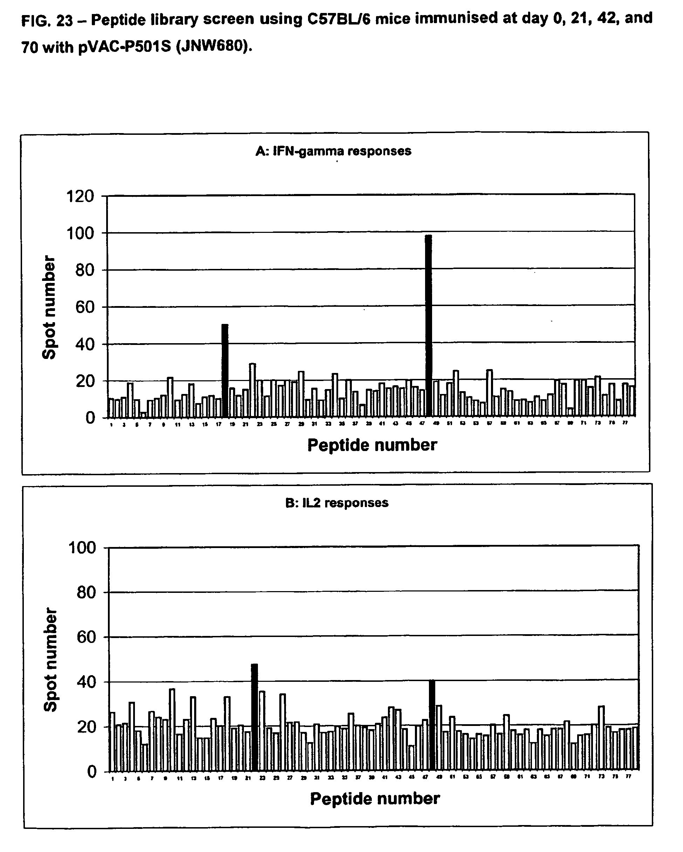

Following immunisation with JNW680 (pVAC-P501S) by PMID at day 0 and three boosts at day 21 and day 42 and day 70, ELISPOT assays were carried out at day 84. Peptides from the P501 S library were tested at 50$\mu$g/ml final concentration. From this initial screen, three peptides were found to stimulate IFNy and/or IL-2 secretion. Peptides 18, 22 and 48 (Figure 23). These peptides were used in subsequent cellular assays.

#### 5.3. Cellular responses to pVAC-P501S (JNW680) following PMID immunisation

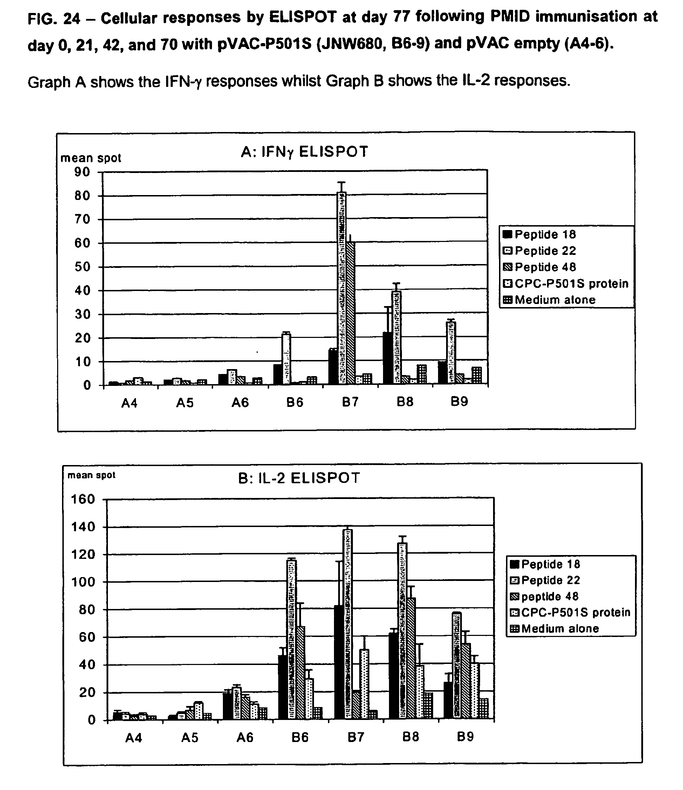

The cellular responses following immunisation with pVAC (empty vector) and pVAC-P501 S were assessed by ELISPOT following a primary immunisation by PMID at day 0 and three boosts at day 21, 42 and 70. Assays were carried out 7 days post boost. Two different assay conditions were used: 1) Peptides 18, 22 and 48 identified in the peptide library screen used at 50$\mu$g/ml final concentration and 2) CPC-P501S protein used at 20$\mu$g/ml final concentration. Figure 24A shows that whilst there were no P501 S-specfic responses to the empty vector (A4-6), the pVAC-P501 S construct induced specific IFN-$\gamma$ responses to Peptides 18 and 22 in all mice (B6-9) whilst one mouse (B7) also showed an IFN-$\gamma$ response to Peptide 48. Figure 24B shows that all mice showed specific IL-2 responses to Peptides 18, 22 and 48. Furthermore, pVAC-P501 S immunised mice (B6-9) also showed moderate IL-2 responses to CPC-P501S, whereas the empty vector immunised mice (A4-6) showed no responses.

#### 5.4. Comparison of cellular responses to P501S and CPC-P501S following PMID immunisation.

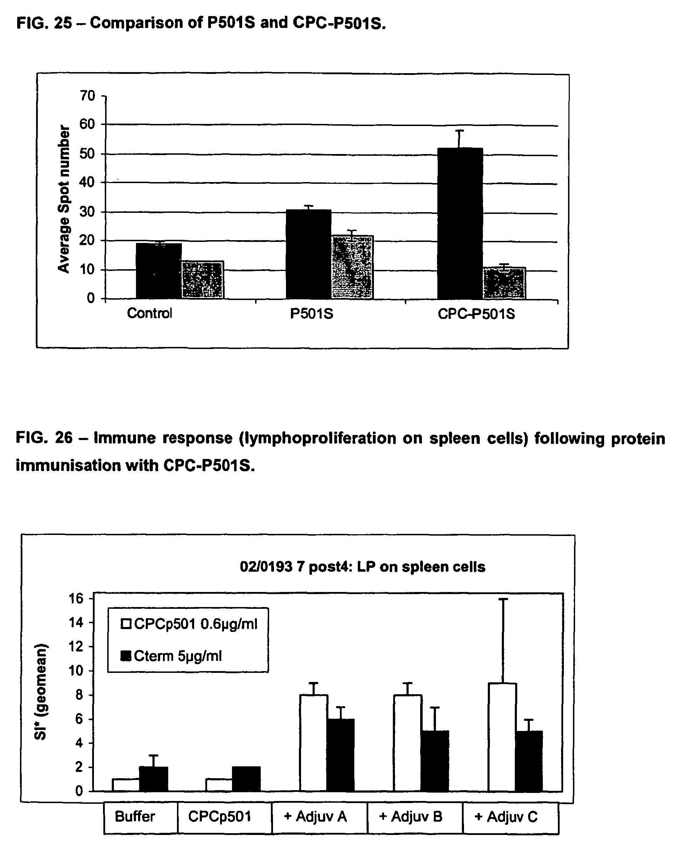

The cellular responses following immunisation with pVAC (empty vector), pVAC-P501S (JNW680) and CPC-P501S (JNW735) were assessed by ELISPOT following a primary immunisation by PMID at day 0 and boosts at day 21 and 42. Assays were carried out 7 days post boost. Two different assay conditions were used: 1) Peptides 18, 22 and 48 identified in the peptide library screen used at 50μg/ml final concentration and 2) CPC-P501 S protein used at 20μg/ml final concentration. Figure 25 shows that at day 28, CPC-P501 S induced good IL-2 responses to 10μg/ml of peptide 22, whilst there were no P501 S-specific responses to either the empty vector or the pVAC-P501 S. These results were also seen using CPC-P501 S protein to re-stimulated the splenocytes. At day 49 (post 2nd boost), the responses induced by P501 S and CPC-P501 S were equivalent. These data suggest that the addition of the CPC tag improves the kinetics and/or magnitude of the response to P501 S.

**Example IX. Immunogenicity experiments in mice usin4 P501S Protein + adjuvant studies**

**1. Design and adjuvant formulation**

The immune response induced by vaccination using the recombinant purified CPC-P501 S protein formulated in adjuvants is characterized in experiments performed in mice.
Groups of 5 to 10, eight weeks old female C57BL6 mice are vaccinated, 2-6 times intramuscularly at 2 weeks intervals with 10μg of the CPC-P501S protein formulated in different adjuvant systems. The volume administered corresponds to 1/10th of a human dose (50 μl).
The serology (total lg response) and cellular response (T cell lymphoproliferation and cytokine production) are analyzed on spleen cells, 6-14 days after the last vaccination using standard protocols as described in Gérard, c. et al, 2001, Vaccine 19, 2583-2589.
The data of one representative experiment is shown. It included 5 groups of eight C57Bl/6 female mice which received 4 intramuscular injections of CPC P501 (10μg) + adjuvant (A, B, C) at days 0, 14, 28, 42. Example V provides an experimental protocol of how to carry out the formulations. Briefly the adjuvant formulations are as follows (quantities given for one dose of 100μl)):

- **Adjuvant A:** QS21 (10μg), MPL (10μg) and CpG7909 (100 μg) made according to the method disclosed in WO 00/62800;
- **Adjuvant B:** formulation of QS21 (20μg), MPL (20μg), CpG7909 (100 μg) and 50 μf SB62 oil-in-water emulsion (WO 95/17210);
- **Adjuvant C:** formulation of QS21 (10μg), MPL (10μg), CpG7909 (100 μg) and 10 μl SB62 oil-in-water emulsion (WO 99/12565).

**2. Serology**

The total lg response induced by vaccination was measured by ELISA using either the CPC-P501 or RA12 -P501 (C term, which is a truncated form of the P501 protein corresponding to the C terminus of the protein fused at its N terminus, to a TB derived protein RA12 - Ra12 is derived from MTB32A antigen described in Skeiky et al., Infection and Immun. (1999) 67:3998-4007).
The adjuvanted CPC-P501 S proteins give a good antibody response after vaccination.

**3. Cellular response**

**3.1. Lymphoproliferation**

7 days after the latest vaccine, lymphoproliferation was performed on spleen cells individually. 2.10e5 spleen cells were plated in quadruplicate, in 96 well microplate, in RPMI medium containing 1% normal mice serum. After 72 hours of re-stimulation with either the immunogen (CPC-P501) or the truncated protein (RA12 P501) at different concentration , 1 μCi 3H thymidine (Amersham 5Ci/ml) was added. After 16 hours, cells were harvested onto filter plates. Incorporated radioactivity was counted in a β counter. Results are expressed in CPM or as stimulation indexes* (geomean CPM in cultures with antigen / geomean CPM in cultures without antigen).
Re-stimulation with ConA (2μg/ml) as positive control was included as positive control.
As shown in Figure 26, a P501 specific lymphoproliferation is seen in the spleen of all groups of mice receiving the adjuvanted protein after in vitro re-stimulation with either the immunogen or another P501 protein made in another expression system (*E coli*), indicating that T cells have been primed in vivo by the vaccination.

**3.2. IFNg production measured by intracellular staining of spleen cells**

Bone Marrow Dendritic Cells (BMDC) obtained after culture of mouse PBL for 7 days in the presence of GMCSF.. 7 days after the latest vaccine, spleen or PBL are collected and a cell suspension prepared. 10e6 cells (1 pool per group) were incubated +/-18hrs with 10e5 BMDC pulsed overnight with 10μg/ml of either the CPCp501 protein or the RA12. After a treatment with the 2.4.G.2 antibody, spleen cells were stained with fluorescent anti CD4 and CD8 antibodies. (anti CD4-APC and an anti CDBPerCP). After a permeabilization and fixation step, cells were stained with a fluorescent anti IFNg-FITC antibody.

In mice vaccinated with CPC P501 in different adjuvant, both CD4 and CD8 T cells are shown to produce IFNg in response to DC pulsed with either the immunogen and the C-term p501 made in *E coli* ( as shown by intracellular straining of spleen and PBLs). There is an increase of 4-10X in the % of cells making this cytokine in the groups receiving the adjuvanted CPC-P501 S compared to the protein alone, and between 0.1 to 10% of CD4 or CD8 T cells are shown to produce IFNg.

In conclusion, these data allow to conclude that the adjuvanted CPC-P501 protein is immunogenic in mice. Both a P501 specific humoral and cellular responses including IFNg production by CD4 and CD8 T cells can be detected after several intramuscular vaccination with CPC P501 in adjuvants.

**<u>Example X. CPC-MUC-1 constructs and sequences</u>**

CPC sequence is taken from nucleotide SEQ ID NO. 28.
MUC1 sequence is available from Genbank database (accession number NM_002456).

**1. MUC1-CPC construct**

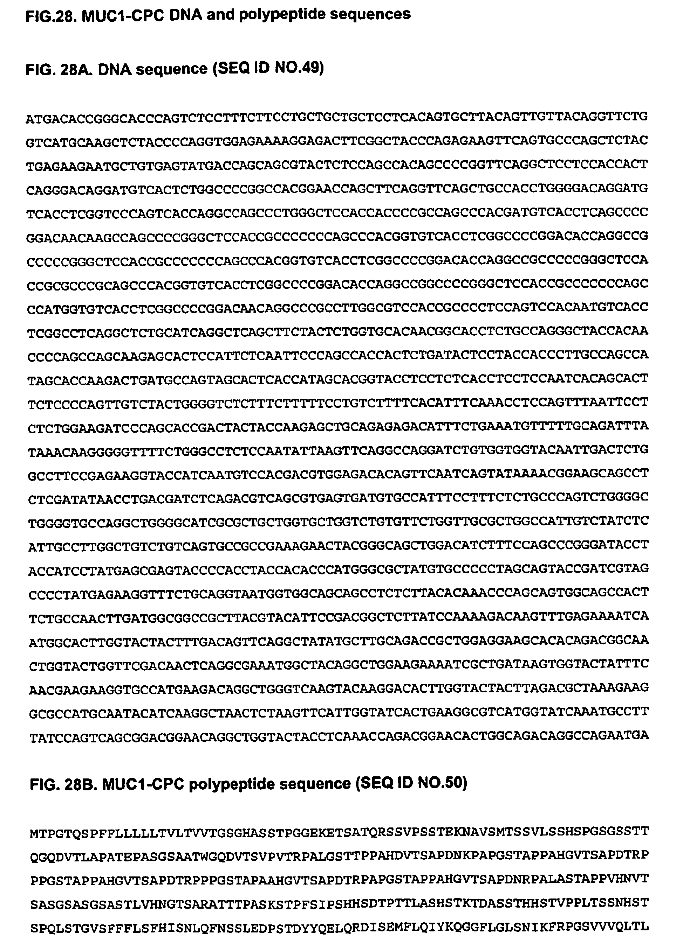

Due to the presence of a signal sequence in MUC1 that is cleaved post-translationally, the CPC motif was placed at the C-terminus. The resulting MUC1-CPC DNA sequence is depicted in SEQ ID NO. xx (figure 28A) and the corresponding MUC1-CPC protein sequence in SEQ ID NO. yy (figure 28B).

**2. ss-CPC-MUC1 construct**

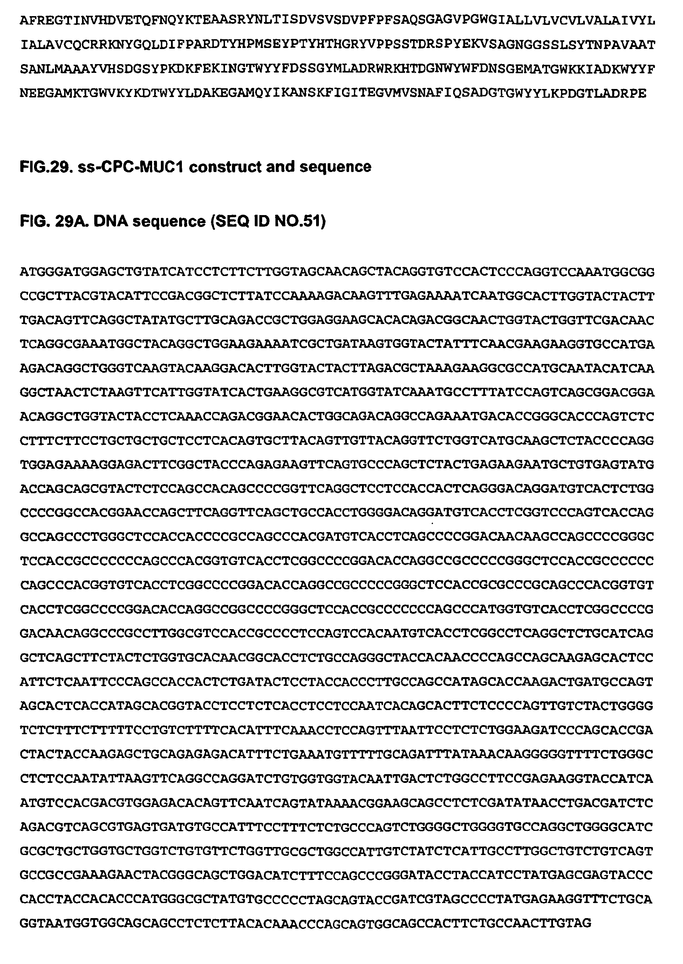

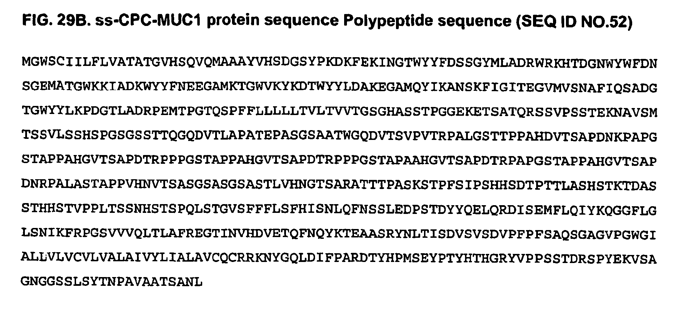

Due to the presence of a signal sequence in MUC1 that is cleaved post-translationally, the MUC1 signal sequence was replaced by a heterologous leader sequence (from the human immunoglobulin heavy chain) and the CPC motif was inserted between the heterologous leader sequence and the MUC1 sequence, generating a sequence termed ss-CPC-MUC1 as depicted in figure 29.

## Claims

1. A fusion partner protein comprising a choline binding domain and a heterologous promiscuous T helper epitope.

2. A fusion partner protein according to claim 1 wherein the choline binding domain is the C terminus of LytA or a derivative thereof in which the derivative of the C-terminus of LytA retains both the capability of acting as an immunological partner and an expression enhancer.

3. A fusion partner protein according to claim 2 wherein the C-LytA or derivative thereof comprises at least four repeats of any of SEQ ID NO:1 to 6.

4. A fusion partner protein according to any of claims 1 to 3, wherein the choline binding domain is selected from the group comprising:

   a) the C-terminal domain of LytA as set forth in SEQ ID NO:7; or
   b) the sequence of SEQ ID NO:8; or
   c) a peptide sequence comprising an amino acid sequence having at least 85% identity, preferably at least 90% identity, more preferably at least 95% identity, most preferably at least 97-99% identity, to any of SEQ ID NO: 1 to 6; or
   d) a peptide sequence comprising an amino acid sequence having at least 15, 20, 30, 40, 50 or 100 contiguous amino acids from the amino acid sequence of SEQ ID NO:7 or SEO ID NO:8.

5. A fusion protein comprising a fusion partner protein as claimed in any of claims 1 to 4 and a heterologous protein.

6. A fusion protein as claimed in claim 5 wherein the heterologous protein is chemically conjugated to the fusion partner.

7. A fusion protein as claimed in claim 5 or 6 wherein the heterologous protein is derived from an organism selected from the following group: Human Immunodeficiency virus HIV-1, human herpes simplex viruses, cytomegalovirus, Rotavirus, Epstein Barr virus, Varicella Zoster Virus, from a hepatitis virus such as hepatitis B virus, hepatitis A virus, _ hepatitis C virus and hepatitis E virus, from Respiratory Syncytial virus, parainfluenza virus, measles virus, mumps virus, human papilloma viruses, flaviviruses or Influenza virus, from *Neisseria spp, Moraxella spp, Bordetella spp; Mycobacterium spp.,* including *M. tuberculosis; Escherichia spp,* including *enterotoxic E. coli; Salmonella spp,; Listeria spp; Helicobacter spp; Staphylococcus spp.,* including *S. aureus, S. epidermidis;; Borrelia spp; Chlamydia spp.,* including *C. trachomatis, C. pneumoniae; Plasmodium spp.,* including *P. falciparum; Toxoplasma spp., Candida spp.*

8. A fusion protein as claimed in claim 5 or 6 wherein the heterologous protein is a tumour associated protein or tissue specific protein or immunogenic fragment thereof.

9. A fusion protein as claimed in claim 8 wherein the heterologous protein or fragment thereof is selected from MAGE 1, MAGE 3, MAGE 4, PRAME, BAGE, LAGE 1, LAGE 2, SAGE, HAGE, XAGE, PSA, PAP, PSCA, prostein, P501S, HASH2, Cripto, B726, NY-BR1.1, P510, MUC-1, Prostase, STEAP, tyrosinase, telomerase, survivin, CASB616, P53, or her 2 neu.

10. A fusion protein as claimed in any of claims 6 to 9 further comprising an affinity tag of at least 4 histidine residues.

11. A nucleic acid sequence encoding a protein of claim 1 to 10.

12. An expression vector comprising a nucleic acid sequence of claim 11.

13. A host cell transformed with a nucleic acid sequence of claim 11 or with an expression vector of claim 12.

**14.** An immunogenic composition comprising a protein as claimed in any of claim 1 to 10 or a DNA sequence as claimed in claim 11 and a pharmaceutically acceptable excipient.

**15.** An immunogenic composition as claimed in claim 14 which additionally comprises a TH-1 inducing adjuvant.

**16.** An immunogenic composition as claimed in claim 15 in which the TH-1 inducing adjuvant is selected from the group of adjuvants comprising: 3D-MPL, QS21, a mixture of QS21 and cholesterol, a CpG oligonucleotide or a mixture of two or more of said adjuvants.

**17.** A process for the preparation of a immunogenic composition as claimed in any of claims 14 to 16, comprising admixing the fusion protein of any of claims 6 to 10 or the encoding polynucleotide of claim 11 with a suitable adjuvant, diluent or other pharmaceutically acceptable carrier.

**18.** A process for producing a fusion protein of any of claims 1 to 10 comprising culturing a host cell of claim 13 under conditions sufficient for the production of said fusion protein and recovering the fusion protein from the culture medium.

**19.** A protein of any of claims 1 to 10 or a DNA sequence of claim 11 for use in medicine.

**20.** Use of a protein as claimed in any of claim 1 to 10 or a DNA sequence of claim 11 in the manufacture of an immunogenic composition for eliciting an immune response in a patient.

**21.** Use according to claim 20, wherein said immune response is to be elicited by sequential administration of i) the said protein followed by the said DNA sequence; or ii) the said DNA sequence followed by the said protein.

**22.** Use according to claim 21 wherein said DNA sequence is coated onto biodegradable beads or delivered via a particle bombardment approach.

**23.** Use according to claim 21 or claim 22 wherein said protein is adjuvanted.

**24.** Use of a protein as claimed in any of claim 1 to 10 or a DNA sequence of claim 11 in the manufacture of an immunogenic composition for immunotherapeutically treating a patient suffering from or susceptible to cancer.

**25.** Use according to claim 24 wherein said cancer is prostate cancer, colon cancer, lung cancer, breast cancer or melanoma.

## Patentansprüche

**1.** Fusionspartnerprotein, das eine Cholin-Bindungsdomäne und ein heterologes promiskes T-Helferepitop umfaßt.

**2.** Fusionspartnerprotein gemäß Anspruch 1, worin die Cholin-Bindungsdomäne der C-Terminus von LytA oder ein Derivat davon ist, worin das Derivat des C-Terminus von LytA sowohl die Fähigkeit zur Funktion als immunologischer Partner als auch als Expressionsverstärker bewahrt.

**3.** Fusionspartnerprotein gemäß Anspruch 2, worin das C-LytA oder Derivat davon wenigstens vier Repeats aus einem beliebigen aus SEQ ID NO: 1 bis 6 umfaßt.

**4.** Fusionspartnerprotein gemäß einem der Ansprüche 1 bis 3, worin die Cholin-Bindungsdomäne aus der Gruppe ausgewählt ist, die folgendes umfaßt:

a) die C-terminale Domäne von LytA wie in SEQ ID NO: 7 dargestellt; oder
b) die Sequenz von SEQ ID NO: 8; oder
c) eine Peptidsequenz, die eine Aminosäuresequenz mit wenigstens 85 % Identität, bevorzugt wenigstens 90 % Identität, besonders bevorzugt wenigstens 95 % Identität, am meisten bevorzugt wenigstens 97-99 % Identität mit einem beliebigen aus SEQ ID NO: 1 bis 6 umfaßt; oder
d) eine Peptidsequenz, die eine Aminosäuresequenz mit wenigstens 15, 20, 30, 40, 50 oder 100 zusammenhängenden Aminosäuren aus der Aminosäuresequenz von SEQ ID NO: 7 oder SEQ ID NO: 8 umfaßt.

**5.** Fusionsprotein, das ein Fusionspartnerprotein gemäß einem der Ansprüche 1 bis 4 und ein heterologes Protein umfaßt.

**6.** Fusionsprotein gemäß Anspruch 5, worin das heterologe Protein chemisch an den Fusionspartner konjugiert ist.

**7.** Fusionsprotein gemäß Anspruch 5 oder 6, worin das heterologe Protein aus einem Organismus stammt, der aus der folgenden Gruppe ausgewählt ist: humanes Immundefizienzvirus HIV-1, humane Herpes simplex-Viren, Cytomegalovirus, Rotavirus, Epstein-Barr-Virus, Varicella Zoster-Virus, aus einem Hepatitisvirus wie Hepatitis B-Virus, Hepatitis A-Virus, Hepatitis C-Virus und Hepatitis E-Virus, aus respiratorischem Synzytialvirus, Parainfluenzavirus, Masernvirus, Mumpsvirus, humane Papillomaviren, Flaviviren oder Influenzavirus, aus Neisseria spp., Moraxella spp., Bordetella spp., Mycobaterium spp., einschließlich M. tuberculosis; Escherichia spp., einschließlich enterotoxisches E. coli; Salmonella spp.; Listeria spp.; Helicobacter spp.; Staphylococcus spp.; einschließlich S. aureus, S. epidermidis; Borrelia spp.; Chlamydia spp., einschließlich C. trachomatis, C. pneumoniae; Plasmodium spp., einschließlich P. falciparum; Toxoplasma spp., Candida spp.

**8.** Fusionsprotein gemäß Anspruch 5 oder 6, worin das heterologe Protein ein Tumor-assoziiertes Protein oder gewebespezifisches Protein oder immunogenes Fragment davon ist.

**9.** Fusionsprotein gemäß Anspruch 8, worin das heterologe Protein oder Fragment davon ausgewählt ist aus MAGE 1, MAGE 3, MAGE 4, PRAME, BAGE, LAGE 1, LAGE 2, SAGE, HAGE, XAGE, PSA, PAP, PSCA, Prostein, P501S, HASH2, Cripto, B726, NY-BR1.1, P510, MUC-1, Prostase, STEAP, Tyrosinase, Telomerase, Survivin, CASB616, P53 oder her 2 neu.

**10.** Fusionsprotein gemäß einem der Ansprüche 6 bis 9, das ferner einen Affinitätsmarker mit wenigstens 4 Histidinresten umfaßt.

**11.** Nukleinsäuresequenz, die ein Protein gemäß Anspruch 1 bis 10 codiert.

**12.** Expressionsvektor, der eine Nukleinsäuresequenz gemäß Anspruch 11 umfaßt.

**13.** Wirtszelle, die mit einer Nukleinsäuresequenz gemäß Anspruch 11 oder mit einem Expressionsvektor gemäß Anspruch 12 transformiert ist.

**14.** Immunogene Zusammensetzung, die ein Protein gemäß einem der Ansprüche 1 bis 10 oder eine DNA-Sequenz gemäß Anspruch 11 und einen pharmazeutisch akzeptablen Exzipienten umfaßt.

**15.** Immunogene Zusammensetzung gemäß Anspruch 14, die zusätzlich einen TH-1-induzierenden Hilfsstoff umfaßt.

**16.** Immunogene Zusammensetzung gemäß Anspruch 15, worin der TH-1-induzierende Hilfsstoff aus der Gruppe von Hilfsstoffen ausgewählt ist, die 3D-MPL, QS21, eine Mischung aus QS21 und Cholesterol, ein CpG-Oligonukleotid oder eine Mischung aus zwei oder mehreren der Hilfsstoffe umfaßt.

**17.** Verfahren zur Herstellung einer immunogenen Zusammensetzung gemäß einem der Ansprüche 14 bis 16, das das Vermischen des Fusionsproteins gemäß einem der Ansprüche 6 bis 10 oder des codierenden Polynukleotids gemäß Anspruch 11 mit einem geeigneten Hilfsstoff, Verdünnungsmittel oder anderen pharmazeutisch akzeptablen Träger umfaßt.

**18.** Verfahren zur Herstellung eines Fusionsproteins gemäß einem der Ansprüche 1 bis 10, das das Kultivieren einer Wirtszelle gemäß Anspruch 13 unter Bedingungen, die ausreichend zur Herstellung des Fusionsproteins sind, und das Gewinnen des Fusionsproteins aus dem Kulturmedium umfaßt.

**19.** Protein gemäß einem der Ansprüche 1 bis 10 oder DNA-Sequenz gemäß Anspruch 11 zur Verwendung in der Medizin.

**20.** Verwendung eines Proteins gemäß einem der Ansprüche 1 bis 10 oder einer DNA-Sequenz gemäß Anspruch 11 in der Herstellung einer immunogenen Zusammensetzung zum Hervorrufen einer Immunreaktion in einem Patienten.

**21.** Verwendung gemäß Anspruch 20, worin die Immunreaktion durch aufeinanderfolgende Verabreichung i) des Proteins, gefolgt von der DNA-Sequenz; oder ii) der DNA-Sequenz, gefolgt vom Protein hervorgerufen wird.

**22.** Verwendung gemäß Anspruch 21, worin die DNA-Sequenz auf biologisch abbaubaren Perlen aufgetragen ist oder über einen Partikelbombardierungsansatz übertragen wird.

**23.** Verwendung gemäß Anspruch 21 oder 22, worin das Protein mit Hilfsstoff versetzt ist.

**24.** Verwendung eines Proteins gemäß einem der Ansprüche 1 bis 10 oder einer DNA-Sequenz gemäß Anspruch 11 in der Herstellung einer immunogenen Zusammensetzung zur immuntherapeutischen Behandlung eines Patienten, der an Krebs leidet oder dafür anfällig ist.

**25.** Verwendung gemäß Anspruch 24, worin der Krebs Prostatakrebs, Darmkrebs, Lungenkrebs, Brustkrebs oder Melanom ist.

## Revendications

**1.** Protéine partenaire de fusion comprenant un domaine de liaison à la choline et un épitope de lymphocyte T auxiliaire multivalent.

**2.** Protéine partenaire de fusion selon la revendication 1, dans laquelle le domaine de liaison à la choline est l'extrémité C-terminale de LytA ou un dérivé de celle-ci, où le dérivé de l'extrémité C-terminale de LytA conserve la capacité d'agir à la fois en tant que partenaire immunogène et stimulateur de l'expression.

**3.** Protéine partenaire de fusion selon la revendication 2, dans laquelle le C-LytA ou un dérivé de celle-ci comprend au moins quatre répétitions de l'une quelconque des SEQ ID N° 1 à 6.

**4.** Protéine partenaire de fusion selon l'une quelconque des revendications 1 à 3, dans laquelle le domaine de liaison à la choline est sélectionné dans le groupe consistant en :

a) le domaine C-terminal de LytA tel que représenté par SEQ ID N°7 ; ou
b) la séquence de SEQ ID N° 8 ; ou
c) une séquence peptidique comprenant une séquences d'acides aminés ayant au moins 85 % d'identité, de préférence au moins 90 % d'identité, de manière plus préférée au moins 95 % d'identité, et de la manière la plus préférée entre toutes au moins 97-99 % d'identité, avec l'une quelconque des SEQ ID N° 1 à 6 ; ou
d) une séquence peptidique comprenant une séquence d'acides aminés ayant au moins 15, 20, 30, 40, 50 ou 100 acides aminés contigus de la séquence d'acides aminés de SEQ ID N° 7 ou SEQ ID N° 8.

**5.** Protéine de fusion comprenant une protéine partenaire de fusion selon l'une quelconque des revendications 1 à 4, et une protéine hétérologue.

**6.** Protéine de fusion selon la revendication 5, dans laquelle la protéine hétérologue est chimiquement conjuguée au partenaire de fusion.

**7.** Protéine de fusion selon la revendication 5 ou 6, dans laquelle la protéine hétérologue est dérivée d'un organisme sélectionné dans le groupe suivant: virus de l'immunodéficience humaine HIV-1, virus herpès simplex humains, cytomégalovirus, rotavirus, virus d'Epstein-Barr, virus varicelle-zona, à partir d'un virus de l'hépatite tel que le virus de l'hépatite B, virus de l'hépatite A, virus de l'hépatite C et virus de l'hépatite E, à partir d'un virus respiratoire syncytial, virus parainfluenza, virus de la rougeole, virus des oreillons, des virus du papillome humain, des flavivirus ou virus de la grippe, à partir des *espèces Neisseria, espèces Moraxella, espèces Bordetella ; espèces Mycobacterium ,* comprenant *M. tuberculosis; des espèces Escherichia,* comprenant *E.* coli *entérotoxique; espèces Salmonella; espèces Listeria; espèces Helicobacter; espèces Staphylococcus;* comprenant *S. aureus, S. epidermidis ; espèces Borrelia ; espèces Chlamydia,* comprenant *C. trachomatis, C. pneumoniae ; espèces Plasmodium,* comprenant *P. falciparum ; espèces Toxoplasma, espèces Candida.*

**8.** Protéine de fusion selon la revendication 5 ou 6, dans laquelle la protéine hétérologue est une protéine associée à une tumeur ou une protéine spécifique à un tissu ou un fragment immunogène de celle-ci.

**9.** Protéine de fusion selon la revendication 8, dans laquelle la protéine hétérologue, ou un fragment de celle-ci, est sélectionnée parmi MAGE 1, MAGE 3, MAGE 4, PRAME, BAGE, LAGE 1, LAGE 2, SAGE, HAGE, XAGE, PSA, PAP, PSCA, prostéine, P501S, HASH2, Cripto, B726, NY-BR1.1, P510, MUC-1, Prostase, STEAP, tyrosinase, télomérase, survivine, CASB616, P53, ou her 2 neu.

**10.** Protéine de fusion selon l'une quelconque des revendications 6 à 9 comprenant en outre un marqueur d'affinité d'au moins 4 résidus histidine.

**11.** Séquence d'acide nucléique codant pour une protéine selon la revendication 1 à 10.

**12.** Vecteur d'expression comprenant une séquence d'acide nucléique selon la revendication 11.

**13.** Cellule hôte transformée avec une séquence d'acide nucléique selon la revendication 11 ou avec un vecteur d'expression selon la revendication 12.

**14.** Composition immunogène comprenant une protéine selon l'une quelconque des revendications 1 à 10 ou une séquence ADN selon la revendication 11 et un excipient pharmaceutiquement acceptable.

**15.** Composition immunogène selon la revendication 14 qui comprend en outre un adjuvant induisant TH-1.

**16.** Composition immunogène selon la revendication 15, dans laquelle l'adjuvant induisant TH-1 est sélectionné parmi le groupe d'adjuvants comprenant : 3D-MPL, QS21, un mélange de QS21 et de cholestérol, un oligonucléotide CpG ou un mélange de deux ou plusieurs desdits adjuvants.

**17.** Procédé pour la préparation d'une composition immunogène selon l'une quelconque des revendications 14 à 16, comprenant un mélange de la protéine de fusion selon l'une quelconque des revendications 6 à 10, ou du polynucléotide codant selon la revendication 11, avec un adjuvant approprié, un diluant ou autre transporteur pharmaceutiquement acceptable.

**18.** Procédé de production d'une protéine de fusion selon l'une quelconque des revendications 1 à 10 comprenant la mise en culture d'une cellule hôte selon la revendication 13 dans des conditions suffisantes pour produire ladite protéine de fusion et récupérer la protéine de fusion dans le milieu de culture.

**19.** Protéine selon l'une quelconque des revendications 1 à 10 ou une séquence ADN selon la revendication 11 destinée à une utilisation en médecine.

**20.** Utilisation d'une protéine selon l'une quelconque des revendications 1 à 10 ou une séquence ADN selon la revendication 11 dans la fabrication d'une composition immunogène pour provoquer une réponse immunitaire chez un patient.

**21.** Utilisation selon la revendication 20, dans laquelle ladite réponse immunitaire doit être provoquée par une administration séquentielle de i) ladite protéine suivie par ladite séquence ADN ; ou ii) ladite séquence ADN suivie par ladite protéine.

**22.** Utilisation selon la revendication 21, dans laquelle ladite séquence ADN est enrobée sur des microsphères biodégradables ou est délivrée par une approche de bombardement particulaire.

**23.** Utilisation selon la revendication 21 ou la revendication 22, dans laquelle ladite protéine est associée à un adjuvant.

**24.** Utilisation d'une protéine selon l'une quelconque des revendications 1 à 10 ou une séquence ADN selon la revendication 11 dans la fabrication d'une composition immunogène pour un traitement d'immunothérapie chez un patient présentant un cancer ou étant susceptible de présenter un cancer.

**25.** Utilisation selon la revendication 24, dans laquelle ledit cancer est le cancer de la prostate, le cancer du côlon, le cancer du sein ou le mélanome.

**Fig. 1 – Sequence information for C-LytA.**

SEQ ID NO:1 – amino acid sequence of C-LytA repeat 1

GWQKNDTGYWYVHSD 15

SEQ ID NO:2 – amino acid sequence of C-LytA repeat 2

GSYPKDKFEKINGTWYYFDSS 21

SEQ ID NO:3 – amino acid sequence of C-LytA repeat 3

GYMLADRWRKHTDGNWYWFDNS 22

SEQ ID NO:4 – amino acid sequence of C-LytA repeat 4

GEMATGWKKIADKWYYFNEE 20

SEQ ID NO:5 – amino acid sequence of C-LytA repeat 5

GAMKTGWVKYKDTWYYLDAKE 21

SEQ ID NO:6 – amino acid sequence of C-LytA repeat 6

GAMVSNAFIQSADGTGWYYLKPD 23

SEQ ID NO:7 – amino acid sequence of C-LytA cholin-binding domain

GWQKNDTGYW YVHSDGSYPK DKFEKINGTW YYFDSSGYML ADRWRKHTDG NWYWFDNSGE 60
MATGWKKIAD KWYYFNEEGA MKTGWVKYKD TWYYLDAKEG AMVSNAFIQS ADGTGWYYLK 120
PDGTLADRPE FTVEPDGLIT VK 142

SEQ ID NO:8 – amino acid sequence of C-LytA domain from truncated repeat 1 to repeat 6 (as part of our constructs shown in figure 2)

YVHSD**GSYPKDKFEKINGTWYYFDS**SGYMLADRWRKHTDGNWYWFDNS**GEMATGWKKIADKWYYFNEE**GAMKT
GWVKYKDTWYYLDAKE**GAMVSNAFIQSADGTGWYYLKPD**

SEQ ID NO:9 – DNA sequence encoding the amino acid sequence of SEQ ID NO:1

ggctggcaga agaatgacac tggctactgg tacgtacatt cagac

SEQ ID NO:10 – DNA sequence encoding the amino acid sequence of SEQ ID NO:2

ggctcttatc caaaagacaa gtttgagaaa atcaatggca cttggtacta ctttgacagt tca

SEQ ID NO:11 – DNA sequence encoding the amino acid sequence of SEQ ID NO:3

ggctatatgc ttgcagaccg ctggaggaag cacacagacg gcaactggta ctggttcgac aactca

SEQ ID NO:12 – DNA sequence encoding the amino acid sequence of SEQ ID NO:4

ggcgaaatgg ctacaggctg gaagaaaatc gctgataagt ggtactattt caacgaagaa

SEQ ID NO:13 – DNA sequence encoding the amino acid sequence of SEQ ID NO:5

Ggtgccatga agacaggctg ggtcaagtac aaggacactt ggtactactt agacgctaaa gaa

SEQ ID NO:14 – DNA sequence encoding the amino acid sequence of SEQ ID NO:6

Ggcgccatgg tatcaaatgc ctttatccag tcagcggacg gaacaggctg gtactacctc
aaaccagac

SEQ ID NO:15 – DNA sequence encoding the amino acid sequence of SEQ ID NO:7

ggctggcaga agaatgacac tggctactgg tacgtacatt cagacggctc ttatccaaaa 60
gacaagtttg agaaaatcaa tggcacttgg tactactttg acagttcagg ctatatgctt 120
gcagaccgct ggaggaagca cacagacggc aactggtact ggttcgacaa ctcaggcgaa 180
atggctacag gctggaagaa aatcgctgat aagtggtact atttcaacga agaaggtgcc 240
atgaagacag gctgggtcaa gtacaaggac acttggtact acttagacgc taaagaaggc 300
gccatggtat caaatgcctt tatccagtca gcggacggaa caggctggta ctacctcaaa 360
ccagacggaa cactggcaga caggccagaa ttcacagtag agccagatgg cttgattaca 420
gtaaaataa 429

SEQ ID NO:16 – DNA sequence encoding the amino acid sequence of SEQ ID NO:8

TACGTACATTCCGACGGCTCTTATCCAAAAGACAAGTTTGAGAAAATCAATGGCACTTGGTACTACTTTGACA
GTTCAGGCTATATGCTTGCAGACCGCTGGAGGAAGCACACAGACGGCAACTGGTACTGGTTCGACAACTCAGG
CGAAATGGCTACAGGCTGGAAGAAAATCGCTGATAAGTGGTACTATTTCAACGAAGAAGGTGCCATGAAGACA
GGCTGGGTCAAGTACAAGGACACTTGGTACTACTTAGACGCTAAAGAAGGCGCCATGGTATCAAATGCCTTTA
TCCAGTCAGCGGACGGAACAGGCTGGTACTACCTCAAACCAGAC

## FIG. 2. CPC and native Constructs

Construct 1 – coding sequence of CPC-P501$_{51\text{-}553}$ (see plasmid of figure 7 -Y1796)

**Protein sequence (SEQ ID NO:27)**

R1 R2 R3 R4

MAAAYVHSD**GSYPKDKFEKINGTWYYFD**SSGYMLADRWRKHTDGNWYWFDNS**GEMATG**

R5 P2 R6

**WKKIADKWYYFNEE**GAMKTGWVKYKDTWYYLDAKE**GA**MQYIKANSKFIGITEGV**MVSNAFIQS**
**ADGTGWYYLKPD**GTLADRPEKFMYMVLGIGPVLGLVCVPLLGSASDHWRGRYGRRRPFIWALSL
GILLSLFLIPRAGWLAGLLCPDPRPLELALLILGVGLLDFCGQVCFTPLEALLSDLFRDPDHCRQAYSV
YAFMISLGGCLGYLLPAIDWDTSALAPYLGTQEECLFGLLTLIFLTCVAATLLVAEEAALGPTEPAEG
LSAPSLSPHCCPCRARLAFRNLGALLPRLHQLCCRMPRTLRRLFVAELCSWMALMTFTLFYTDFVGE
GLYQGVPRAEPGTEARRHYDEGVRMGSLGLFLQCAISLVFSLVMDRLVQRFGTRAVYLASVAAFPV
AAGATCLSHSVAVVTASAALTGFTFSALQILPYTLASLYHREKQVFLPKYRGDTGGASSEDSLMTSF
LPGPKPGAPFPNGHVGAGGSGLLPPPPALCGASACDVSVRVVVGEPTEARVVPGRGICLDLAILDSAF
LLSQVAPSLFMGSIVQLSQSVTAYMVSAAGLGLVAIYFATQVVFDKSDLAKYSAGGHHHHHH

| | | |
|---|---|---|
| R1 (plain): aa5-9 (fragment) | **R4 (bold)**: aa53-72 | P2 (underline): 97-110 |
| **R2 (bold)**: aa10-30 | R5 (plain): aa73-93 | |
| R3 (plain): aa31-52 | **R6a (bold)**: aa94-95 | **R6b (bold)**: 113-133 |

**Nucleotide sequence (SEQ ID NO:28)**

ATGgcggccgct**TACGTACATTCCGACGGCTCTTATCCAAAAGACAAGTTTGAGAAAATCAATGGCACTTGGT**
**ACTACTTTGACAGTTCAGGCTATATGCTTGCAGACCGCTGGAGGAAGCACACAGACGGCAACTGGTACTGGTT**
**CGACAACTCAGGCGAAATGGCTACAGGCTGGAAGAAAATCGCTGATAAGTGGTACTATTTCAACGAAGAAGGT**
**GCCATGAAGACAGGCTGGGTCAAGTACAAGGACACTTGGTACTACTTAGACGCTAAAGAAGGCGCC**atgcaat
acatcaaggctaactctaagttcattggtatcactgaaggcgtc**ATGGTATCAAATGCCTTTATCCAGTCAGC**
**GGACGGAACAGGCTGGTACTACCTCAAACCAGAC**GGAACACTGGCAGACAGGCCAGAAaagttcatgtaCatg
GTGCTGGGCATTGGTCCAGTGCTGGGCCTGGTCTGTGTCCCGCTCCTAGGCTCAGCCAGTGACCACTGGCGTG
GACGCTATGGCCGCCGCCGGCCCTTCATCTGGGCACTGTCCTTGGGCATCCTGCTGAGCCTCTTTCTCATCCC
AAGGGCCGGCTGGCTAGCAGGGCTGCTGTGCCCGGATCCCAGGCCCCTGGAGCTGGCACTGCTCATCCTGGGC
GTGGGGCTGCTGGACTTCTGTGGCCAGGTGTGCTTCACTCCACTGGAGGCCCTGCTCTCTGACCTCTTCCGGG
ACCCGGACCACTGTCGCCAGGCCTACTCTGTCTATGCCTTCATGATCAGTCTTGGGGGCTGCCTGGGCTACCT
CCTGCCTGCCATTGACTGGGACACCAGTGCCCTGGCCCCCTACCTGGGCACCCAGGAGGAGTGCCTCTTTGGC
CTGCTCACCCTCATCTTCCTCACCTGCGTAGCAGCCACACTGCTGGTGGCTGAGGAGGCAGCGCTGGGCCCCA
CCGAGCCAGCAGAAGGGCTGTCGGCCCCCTCCTTGTCGCCCCACTGCTGTCCATGCCGGGCCCGCTTGGCTTT

CCGGAACCTGGGCGCCCTGCTTCCCCGGCTGCACCAGCTGTGCTGCCGCATGCCCCGCACCCTGCGCCGGCTC
TTCGTGGCTGAGCTGTGCAGCTGGATGGCACTCATGACCTTCACGCTGTTTTACACGGATTTCGTGGCGAGG
GGCTGTACCAGGGCGTGCCCAGAGCTGAGCCGGGCACCGAGGCCCGGAGACACTATGATGAAGGCGTTCGGAT
GGGCAGCCTGGGGCTGTTCCTGCAGTGCGCCATCTCCCTGGTCTTCTCTCTGGTCATGGACCGGCTGGTGCAG
CGATTCGGCACTCGAGCAGTCTATTTGGCCAGTGTGGCAGCTTTCCCTGTGGCTGCCGGTGCCACATGCCTGT
CCCACAGTGTGGCCGTGGTGACAGCTTCAGCCGCCCTCACCGGGTTCACCTTCTCAGCCCTGCAGATCCTGCC
CTACACACTGGCCTCCCTCTACCACCGGGAGAAGCAGGTGTTCCTGCCCAAATACCGAGGGGACACTGGAGGT
GCTAGCAGTGAGGACAGCCTGATGACCAGCTTCCTGCCAGGCCCTAAGCCTGGAGCTCCCTTCCCTAATGGAC
ACGTGGGTGCTGGAGGCAGTGGCCTGCTCCCACCTCCACCCGCGCTCTGCGGGGCCTCTGCCTGTGAtGTCTC
CGTACGTGTGGTGGTGGGTGAGCCCACCGAGGCCAGGGTGGTTCCGGGCCGGGGCATCTGCCTGGACCTCGCC
ATCCTGGATAGTGCCTTCCTGCTGTCCCAGGTGGCCCCATCCCTGTTTATGGGCTCCATTGTCCAGCTCAGCC
AGTCTGTCACTGCCTATATGGTGTCTGCCGCAGGCCTGGGTCTGGTCGCCATTTACTTTGCTACACAGGTAGT
ATTTGACAAGAGCGACTTGGCCAAATACTCAGCGggtggacaccatcaccatcaccattaa

Construct 2 – Coding sequence of $P501_{55-553}$ HIS (control) (yeast strain SC333)

**Protein sequence (SEQ ID NO:29)**

MVLGIGPVLG LVCVPLLGSA SDHWRGRYGR RRPFIWALSL GILLSLFLIP RAGWLAGLLC 60
PDPRPLELAL LILGVGLLDF CGQVCFTPLE ALLSDLFRDP DHCRQAYSVY AFMISLGGCL 120
GYLLPAIDWD TSALAPYLGT QEECLFGLLT LIFLTCVAAT LLVAEEAALG PTEPAEGLSA 180
PSLSPHCCPC RARLAFRNLG ALLPRLHQLC CRMPRTLRRL FVAELCSWMA LMTFTLFYTD 240
FVGEGLYQGV PRAEPGTEAR RHYDEGVRMG SLGLFLQCAI SLVFSLVMDR LVQRFGTRAV 300
YLASVAAFPV AAGATCLSHS VAVVTASAAL TGFTFSALQI LPYTLASLYH REKQVFLPKY 360
RGDTGGASSE DSLMTSFLPG PKPGAPFPNG HVGAGGSGLL PPPPALCGAS ACDVSVRVVV 420
GEPTEARVVP GRGICLDLAI LDSAFLLSQV APSLFMGSIV QLSQSVTAYM VSAAGLGLVA 480
IYFATQVVFD KSDLAKYSAG GHHHHHH 507

**Nucleotide sequence (SEQ ID NO:30)**

atgGTGCTGG GCATTGGTCC AGTGCTGGGC CTGGTCTGTG TCCCGCTCCT AGGCTCAGCC 60
AGTGACCACT GGCGTGGACG CTATGGCCGC CGCCGGCCCT TCATCTGGGC ACTGTCCTTG 120
GGCATCCTGC TGAGCCTCTT TCTCATCCCA AGGGCCGGCT GGCTAGCAGG GCTGCTGTGC 180
CCGGATCCCA GGCCCCTGGA GCTGGCACTG CTCATCCTGG GCGTGGGGCT GCTGGACTTC 240
TGTGGCCAGG TGTGCTTCAC TCCACTGGAG GCCCTGCTCT CTGACCTCTT CCGGGACCCG 300
GACCACTGTC GCCAGGCCTA CTCTGTCTAT GCCTTCATGA TCAGTCTTGG GGGCTGCCTG 360
GGCTACCTCC TGCCTGCCAT TGACTGGGAC ACCAGTGCCC TGGCCCCCTA CCTGGGCACC 420
CAGGAGGAGT GCCTCTTTGG CCTGCTCACC CTCATCTTCC TCACCTGCGT AGCAGCCACA 480
CTGCTGGTGG CTGAGGAGGC AGCGCTGGGC CCCACCGAGC CAGCAGAAGG GCTGTCGGCC 540
CCCTCCTTGT CGCCCCACTG CTGTCCATGC CGGGCCCGCT TGGCTTTCCG GAACCTGGGC 600

GCCCTGCTTC CCCGGCTGCA CCAGCTGTGC TGCCGCATGC CCCGCACCCT GCGCCGGCTC 660
TTCGTGGCTG AGCTGTGCAG CTGGATGGCA CTCATGACCT TCACGCTGTT TTACACGGAT 720
TTCGTGGGCG AGGGGCTGTA CCAGGGCGTG CCCAGAGCTG AGCCGGGCAC CGAGGCCCGG 780
AGACACTATG ATGAAGGCGT TCGGATGGGC AGCCTGGGGC TGTTCCTGCA GTGCGCCATC 840
TCCCTGGTCT TCTCTCTGGT CATGGACCGG CTGGTGCAGC GATTCGGCAC TCGAGCAGTC 900
TATTTGGCCA GTGTGGCAGC TTTCCCTGTG GCTGCCGGTG CCACATGCCT GTCCCACAGT 960
GTGGCCGTGG TGACAGCTTC AGCCGCCCTC ACCGGGTTCA CCTTCTCAGC CCTGCAGATC 1020
CTGCCCTACA CACTGGCCTC CCTCTACCAC CGGGAGAAGC AGGTGTTCCT GCCCAAATAC 1080
CGAGGGGACA CTGGAGGTGC TAGCAGTGAG GACAGCCTGA TGACCAGCTT CCTGCCAGGC 1140
CCTAAGCCTG GAGCTCCCTT CCCTAATGGA CACGTGGGTG CTGGAGGCAG TGGCCTGCTC 1200
CCACCTCCAC CCGCGCTCTG CGGGGCCTCT GCCTGTGAtG TCTCCGTACG TGTGGTGGTG 1260
GGTGAGCCCA CCGAGGCCAG GGTGGTTCCG GGCCGGGGCA TCTGCCTGGA CCTCGCCATC 1320
CTGGATAGTG CCTTCCTGCT GTCCCAGGTG GCCCCATCCC TGTTTATGGG CTCCATTGTC 1380
CAGCTCAGCC AGTCTGTCAC TGCCTATATG GTGTCTGCCG CAGGCCTGGG TCTGGTCGCC 1440
ATTTACTTTG CTACACAGGT AGTATTTGAC AAGAGCGACT TGGCCAAATA CTCAGCGggt 1500
ggacaccatc accatcacca ttaa 1524

Construct 3 - Coding sequence of natssP501$_{1-34}$ P501$_{51-553}$ HIS (yeast strain Y1800)

**Protein sequence (SEQ ID NO:31)**

R1 R2

MAAVQRLWVSRLLRHRKAQLLLVNLLTFGLEVCLAAAYVHSDGSYPKDKFEKINGTW

R3 R4 R5

YYFDSSGYMLADRWRKHTDGNWYWFDNSGEMATGWKKIADKWYYFNEEGAMKTGWVK

P2 R6

YKDTWYYLDAKEGAMQYIKANSKFIGITEGVMVSNAFIQSADGTGWYYLKPDGTLADRPEKFMY
MVLGIGPVLGLVCVPLLGSASDHWRGRYGRRRPFIWALSLGILLSLFLIPRAGWLAGLLCPDPRPLEL
ALLILGVGLLDFCGQVCFTPLEALLSDLFRDPDHCRQAYSVYAFMISLGGCLGYLLPAIDWDTSALAP
YLGTQEECLFGLLTLIFLTCVAATLLVAEEAALGPTEPAEGLSAPSLSPHCCPCRARLAFRNLGALLPR
LHQLCCRMPRTLRRLFVAELCSWMALMTFTLFYTDFVGEGLYQGVPRAEPGTEARRHYDEGVRMG
SLGLFLQCAISLVFSLVMDRLVQRFGTRAVYLASVAAFPVAAGATCLSHSVAVVTASAALTGFTFSA
LQILPYTLASLYHREKQVFLPKYRGDTGGASSEDSLMTSFLPGPKPGAPFPNGHVGAGGSGLLPPPPA
LCGASACDVSVRVVVGEPTEARVVPGRGICLDLAILDSAFLLSQVAPSLFMGSIVQLSQSVTAYMVS
AAGLGLVAIYFATQVVFDKSDLAKYSAGGHHHHHH

| | | |
|---|---|---|
| R1 (plain): aa38-42 (fragment) | **R4 (bold)**: aa77-106 | P2 (underline): 130-143 |
| **R2 (bold)**: aa43-64 | R5 (plain): aa107-126 | |
| R3 (plain): aa65-76 | **R6a (bold)**: aa127-128 | **R6b (bold)**: aa146-166 |

natss stands for native signal sequence

**Nucleotide sequence (SEQ ID NO:32)**

ATGgcGGCCGTGCAGAGGCTATGGGTATCGAGACTGCTAAGACACCGCAAAGCTCAGTTGTTGTTGGTTAACT
TGTTGACCTTCGGGCTGGAAGTCTGTTTGGCggccgct**TACGTACATTCCGACGGCTCTTATCCAAAAGACAA**
**GTTTGAGAAAATCAATGGCACTTGGTACTACTTTGACAGTTCAGGCTATATGCTTGCAGACCGCTGGAGGAAG**
**CACACAGACGGCAACTGGTACTGGTTCGACAACTCAGGCGAAATGGCTACAGGCTGGAAGAAAATCGCTGATA**
**AGTGGTACTATTTCAACGAAGAAGGTGCCATGAAGACAGGCTGGGTCAAGTACAAGGACACTTGGTACTACTT**
**AGACGCTAAAGAAGGCGCC**atgcaatacatcaaggctaactctaagttcattggtatcactgaaggcgtc**ATG**
**GTATCAAATGCCTTTATCCAGTCAGCGGACGGAACAGGCTGGTACTACCTCAAACCAGAC**GGAACACTGGCAG
ACAGGCCAGAAaagttcatgtaCatgGTGCTGGGCATTGGTCCAGTGCTGGGCCTGGTCTGTGTCCCGCTCCT
AGGCTCAGCCAGTGACCACTGGCGTGGACGCTATGGCCGCCGCCGGCCCTTCATCTGGGCACTGTCCTTGGGC
ATCCTGCTGAGCCTCTTTCTCATCCCAAGGGCCGGCTGGCTAGCAGGGCTGCTGTGCCCGGATCCCAGGCCCC
TGGAGCTGGCACTGCTCATCCTGGGCGTGGGGCTGCTGGACTTCTGTGGCCAGGTGTGCTTCACTCCACTGGA
GGCCCTGCTCTCTGACCTCTTCCGGGACCCGGACCACTGTCGCCAGGCCTACTCTGTCTATGCCTTCATGATC
AGTCTTGGGGGCTGCCTGGGCTACCTCCTGCCTGCCATTGACTGGGACACCAGTGCCCTGGCCCCCTACCTGG
GCACCCAGGAGGAGTGCCTCTTTGGCCTGCTCACCCTCATCTTCCTCACCTGCGTAGCAGCCACACTGCTGGT
GGCTGAGGAGGCAGCGCTGGGCCCCACCGAGCCAGCAGAAGGGCTGTCGGCCCCCTCCTTGTCGCCCCACTGC
TGTCCATGCCGGGCCCGCTTGGCTTTCCGGAACCTGGGCGCCCTGCTTCCCCGGCTGCACCAGCTGTGCTGCC
GCATGCCCCGCACCCTGCGCCGGCTCTTCGTGGCTGAGCTGTGCAGCTGGATGGCACTCATGACCTTCACGCT
GTTTTACACGGATTTCGTGGGCGAGGGGCTGTACCAGGGCGTGCCCAGAGCTGAGCCGGGCACCGAGGCCCGG
AGACACTATGATGAAGGCGTTCGGATGGGCAGCCTGGGGCTGTTCCTGCAGTGCGCCATCTCCCTGGTCTTCT
CTCTGGTCATGGACCGGCTGGTGCAGCGATTCGGCACTCGAGCAGTCTATTTGGCCAGTGTGGCAGCTTTCCC
TGTGGCTGCCGGTGCCACATGCCTGTCCCACAGTGTGGCCGTGGTGACAGCTTCAGCCGCCCTCACCGGGTTC
ACCTTCTCAGCCCTGCAGATCCTGCCCTACACACTGGCCTCCCTCTACCACCGGGAGAAGCAGGTGTTCCTGC
CCAAATACCGAGGGGACACTGGAGGTGCTAGCAGTGAGGACAGCCTGATGACCAGCTTCCTGCCAGGCCCTAA
GCCTGGAGCTCCCTTCCCTAATGGACACGTGGGTGCTGGAGGCAGTGGCCTGCTCCCACCTCCACCCGCGCTC
TGCGGGGCCTCTGCCTGTGAtGTCTCCGTACGTGTGGTGGTGGGTGAGCCCACCGAGGCCAGGGTGGTTCCGG
GCCGGGGCATCTGCCTGGACCTCGCCATCCTGGATAGTGCCTTCCTGCTGTCCCAGGTGGCCCCATCCCTGTT
TATGGGCTCCATTGTCCAGCTCAGCCAGTCTGTCACTGCCTATATGGTGTCTGCCGCAGGCCTGGGTCTGGTC
GCCATTTACTTTGCTACACAGGTAGTATTTGACAAGAGCGACTTGGCCAAATACTCAGCGggtggacaccatc
accatcaccattaa

Construct 4 - Coding sequence of alphapreCPC-P501$_{51-553}$ HIS (yeast strain Y1802)

**Protein sequence (SEQ ID NO:33)**

Alpha-pre signal R1 R2 R3

**MAARFPSIFTAVLFAASSALAAA**YVHSDG**SYPKDKFEKINGTWYYFDS**SGYMLADRWRKHTDGNWYWFD

R4 R5 P2

NSG**EMATGWKKIADKWYYFNEE**GAMKTGWVKYKDTWYYLDAKEGAMQYIKANSKFIGITEGV**MVSNAFI**

R6

QSADGTGWYYLKPDGTLADRPEKFMYMVLGIGPVLGLVCVPLLGSASDHWRGRYGRRRPFIWALSLGILLSLF
LIPRAGWLAGLLCPDPRPLELALLILGVGLLDFCGQVCFTPLEALLSDLFRDPDHCRQAYSVYAFMISLGGCL
GYLLPAIDWDTSALAPYLGTQEECLFGLLTLIFLTCVAATLLVAEEAALGPTEPAEGLSAPSLSPHCCPCRAR
LAFRNLGALLPRLHQLCCRMPRTLRRLFVAELCSWMALMTFTLFYTDFVGEGLYQGVPRAEPGTEARRHYDEG
VRMGSLGLFLQCAISLVFSLVMDRLVQRFGTRAVYLASVAAFPVAAGATCLSHSVAVVTASAALTGFTFSALQ
ILPYTLASLYHREKQVFLPKYRGDTGGASSEDSLMTSFLPGPKPGAPFPNGHVGAGGSGLLPPPALCGASAC
DVSVRVVVGEPTEARVVPGRGICLDLAILDSAFLLSQVAPSLFMGSIVQLSQSVTAYMVSAAGLGLVAIYFAT
QVVFDKSDLAKYSAGGHHHHHH

**Alpha-pre signal (bold)**: aa4-22

R1 (plain): aa24-28 (fragment) | **R4 (bold)**: aa72-91 | P2 (underline): 116-129

**R2 (bold)**: aa29-49 | R5 (plain): aa92-112

R3 (plain): aa50-71 | **R6a (bold)**: aa113-114 | **R6b (bold)**: aa132-152

Alphapre stands for alpha pre signal sequence

**Nucleotide sequence (SEQ ID NO:34)**

**TACGTACATTCCGACGGCTCTTATCCAAAAGACAAGTTTGAGAAAATCAATGGCACTTGGTACTACTTTGACA**
**GTTCAGGCTATATGCTTGCAGACCGCTGGAGGAAGCACACAGACGGCAACTGGTACTGGTTCGACAACTCAGG**
**CGAAATGGCTACAGGCTGGAAGAAAATCGCTGATAAGTGGTACTATTTCAACGAAGAAGGTGCCATGAAGACA**
**GGCTGGGTCAAGTACAAGGACACTTGGTACTACTTAGACGCTAAAGAAGGCGCC**atgcaatacatcaaggcta
actctaagttcattggtatcactgaaggcgtc**ATGGTATCAAATGCCTTTATCCAGTCAGCGGACGGAACAGG**
**CTGGTACTACCTCAAACCAGAC**GGAACACTGGCAGACAGGCCAGAA

**ATGgcGGCCAGATTTCCTTCAATTTTTACTGCAGTTTTATTCGCAGCATCCTCCGCATTAGC**ggccgct**TACG**
**TACATTCCGACGGCTCTTATCCAAAAGACAAGTTTGAGAAAATCAATGGCACTTGGTACTACTTTGACAGTTC**
**AGGCTATATGCTTGCAGACCGCTGGAGGAAGCACACAGACGGCAACTGGTACTGGTTCGACAACTCAGGCGAA**
**ATGGCTACAGGCTGGAAGAAAATCGCTGATAAGTGGTACTATTTCAACGAAGAAGGTGCCATGAAGACAGGCT**
**GGGTCAAGTACAAGGACACTTGGTACTACTTAGACGCTAAAGAAGGCGCC**atgcaatacatcaaggctaactc
taagttcattggtatcactgaaggcgtc**ATGGTATCAAATGCCTTTATCCAGTCAGCGGACGGAACAGGCTGG**
**TACTACCTCAAACCAGAC**GGAACACTGGCAGACAGGCCAGAAgctggtattacttacgttccaccattgttgt
tggaagttggtgttgaagaaaagttcatgtaCatgGTGCTGGGCATTGGTCCAGTGCTGGGCCTGGTCTGTGT
CCCGCTCCTAGGCTCAGCCAGTGACCACTGGCGTGGACGCTATGGCCGCCGCCGGCCCTTCATCTGGGCACTG
TCCTTGGGCATCCTGCTGAGCCTCTTTCTCATCCCAAGGGCCGGCTGGCTAGCAGGGCTGCTGTGCCCGGATC
CCAGGCCCCTGGAGCTGGCACTGCTCATCCTGGGCGTGGGGCTGCTGGACTTCTGTGGCCAGGTGTGCTTCAC
TCCACTGGAGGCCCTGCTCTCTGACCTCTTCCGGGACCCGGACCACTGTCGCCAGGCCTACTCTGTCTATGCT
TCATGATCAGTCTTGGGGGCTGCCTGGGCTACCTCCTGCCTGCCATTGACTGGGACACCAGTGCCCTGGCCCC
CTACCTGGGCACCCAGGAGGAGTGCCTCTTTGGCCTGCTCACCCTCATCTTCCTCACCTGCGTAGCAGCCACA

CTGCTGGTGGCTGAGGAGGCAGCGCTGGGCCCCACCGAGCCAGCAGAAGGGCTGTCGGCCCCCTCCTTGTCGC
CCCACTGCTGTCCATGCCGGGCCCGCTTGGCTTTCCGGAACCTGGGCGCCCTGCTTCCCCGGCTGCACCAGCT
GTGCTGCCGCATGCCCCGCACCCTGCGCCGGCTCTTCGTGGCTGAGCTGTGCAGCTGGATGGCACTCATGACC
TTCACGCTGTTTTACACGGATTTCGTGGGCGAGGGGCTGTACCAGGGCGTGCCCAGAGCTGAGCCGGGCACCG
AGGCCCGGAGACACTATGATGAAGGCGTTCGGATGGGCAGCCTGGGGCTGTTCCTGCAGTGCGCCATCTCCCT
GGTCTTCTCTCTGGTCATGGACCGGCTGGTGCAGCGATTCGGCACTCGAGCAGTCTATTTGGCCAGTGTGGCA
GCTTTCCCTGTGGCTGCCGGTGCCACATGCCTGTCCCACAGTGTGGCCGTGGTGACAGCTTCAGCCGCCCTCA
CCGGGTTCACCTTCTCAGCCCTGCAGATCCTGCCCTACACACTGGCCTCCCTCTACCACCGGGAGAAGCAGGT
GTTCCTGCCCAAATACCGAGGGGACACTGGAGGTGCTAGCAGTGAGGACAGCCTGATGACCAGCTTCCTGCCA
GGCCCTAAGCCTGGAGCTCCCTTCCCTAATGGACACGTGGGTGCTGGAGGCAGTGGCCTGCTCCCACCTCCAC
CCGCGCTCTGCGGGGCCTCTGCCTGTGAtGTCTCCGTACGTGTGGTGGTGGGTGAGCCCACCGAGGCCAGGGT
GGTTCCGGGCCGGGGCATCTGCCTGGACCTCGCCATCCTGGATAGTGCCTTCCTGCTGTCCCAGGTGGCCCCA
TCCCTGTTTATGGGCTCCATTGTCCAGCTCAGCCAGTCTGTCACTGCCTATATGGTGTCTGCCGCAGGCCTGG
GTCTGGTCGCCATTTACTTTGCTACACAGGTAGTATTTGACAAGAGCGACTTGGCCAAATACTCAGCGggtgg
acaccatcaccatcaccattaa

Construct 5 - Coding sequence of alphaprepro-P501$_{51-553}$ HIS (in plasmid pRIT 15068 and yeast strain Y1790)

**Protein sequence (SEQ ID NO:35)**

```
MSFLNFTAVL FAASSALAAP VNTTTEDETA QIPAEAVIGY SDLEGDFDVA VLPFSNSTNN  60
GLLFINTTIA SIAAKEEGVS LEKREAEAMV LGIGPVLGLV CVPLLGSASD HWRGRYGRRR  120
PFIWALSLGI LLSLFLIPRA GWLAGLLCPD PRPLELALLI LGVGLLDFCG QVCFTPLEAL  180
LSDLFRDPDH CRQAYSVYAF MISLGGCLGY LLPAIDWDTS ALAPYLGTQE ECLFGLLTLI  240
FLTCVAATLL VAEEAALGPT EPAEGLSAPS LSPHCCPCRA RLAFRNLGAL LPRLHQLCCR  300
MPRTLRRLFV AELCSWMALM TFTLFYTDFV GEGLYQGVPR AEPGTEARRH YDEGVRMGSL  360
GLFLQCAISL VFSLVMDRLV QRFGTRAVYL ASVAAFPVAA GATCLSHSVA VVTASAALTG  420
FTFSALQILP YTLASLYHRE KQVFLPKYRG DTGGASSEDS LMTSFLPGPK PGAPFPNGHV  480
GAGGSGLLPP PPALCGASAC DVSVRVVVGE PTEARVVPGR GICLDLAILD SAFLLSQVAP  540
SLFMGSIVQL SQSVTAYMVS AAGLGLVAIY FATQVVFDKS DLAKYSAGGH HHHHH  595
```

**Nucleotide sequence (SEQ ID NO:36)**

```
ATGAGTTTCC TCAATTTTAC TGCAGTTTTA TTCGCAGCAT CCTCCGCATT AGCTGCTCCA  60
GTCAACACTA CAACAGAAGA TGAAACGGCA CAAATTCCGG CTGAAGCTGT CATCGGTTAC  120
TCAGATTTAG AAGGGGATTT CGATGTTGCT GTTTTGCCAT TTTCCAACAG CACAAATAAC  180
GGGTTATTGT TTATAAATAC TACTATTGCC AGCATTGCTG CTAAAGAAGA AGGGGTATCT  240
CTCGAGAAAA GAGAGGCTGA AGCCatgGTG CTGGGCATTG GTCCAGTGCT GGGCCTGGTC  300
TGTGTCCCGC TCCTAGGCTC AGCCAGTGAC CACTGGCGTG GACGCTATGG CCGCCGCCGG  360
```

CCCTTCATCT GGGCACTGTC CTTGGGCATC CTGCTGAGCC TCTTTCTCAT CCCAAGGGCC 420
GGCTGGCTAG CAGGGCTGCT GTGCCCGGAT CCCAGGCCCC TGGAGCTGGC ACTGCTCATC 480
CTGGGCGTGG GGCTGCTGGA CTTCTGTGGC CAGGTGTGCT TCACTCCACT GGAGGCCCTG 540
CTCTCTGACC TCTTCCGGGA CCCGGACCAC TGTCGCCAGG CCTACTCTGT CTATGCCTTC 600
ATGATCAGTC TTGGGGGCTG CCTGGGCTAC CTCCTGCCTG CCATTGACTG GGACACCAGT 660
GCCCTGGCCC CCTACCTGGG CACCCAGGAG GAGTGCCTCT TTGGCCTGCT CACCCTCATC 720
TTCCTCACCT GCGTAGCAGC CACACTGCTG GTGGCTGAGG AGGCAGCGCT GGGCCCCACC 780
GAGCCAGCAG AAGGGCTGTC GGCCCCCTCC TTGTCGCCCC ACTGCTGTCC ATGCCGGGCC 840
CGCTTGGCTT TCCGGAACCT GGGCGCCCTG CTTCCCCGGC TGCACCAGCT GTGCTGCCGC 900
ATGCCCCGCA CCCTGCGCCG GCTCTTCGTG GCTGAGCTGT GCAGCTGGAT GGCACTCATG 960
ACCTTCACGC TGTTTTACAC GGATTTCGTG GGCGAGGGGC TGTACCAGGG CGTGCCCAGA 1020
GCTGAGCCGG GCACCGAGGC CCGGAGACAC TATGATGAAG GCGTTCGGAT GGGCAGCCTG 1080
GGGCTGTTCC TGCAGTGCGC CATCTCCCTG GTCTTCTCTC TGGTCATGGA CCGGCTGGTG 1140
CAGCGATTCG GCACTCGAGC AGTCTATTTG GCCAGTGTGG CAGCTTTCCC TGTGGCTGCC 1200
GGTGCCACAT GCCTGTCCCA CAGTGTGGCC GTGGTGACAG CTTCAGCCGC CCTCACCGGG 1260
TTCACCTTCT CAGCCCTGCA GATCCTGCCC TACACACTGG CCTCCCTCTA CCACCGGGAG 1320
AAGCAGGTGT TCCTGCCCAA ATACCGAGGG GACACTGGAG GTGCTAGCAG TGAGGACAGC 1380
CTGATGACCA GCTTCCTGCC AGGCCCTAAG CCTGGAGCTC CCTTCCCTAA TGGACACGTG 1440
GGTGCTGGAG GCAGTGGCCT GCTCCCACCT CCACCCGCGC TCTGCGGGGC CTCTGCCTGT 1500
GAtGTCTCCG TACGTGTGGT GGTGGGTGAG CCCACCGAGG CCAGGGTGGT TCCGGGCCGG 1560
GGCATCTGCC TGGACCTCGC CATCCTGGAT AGTGCCTTCC TGCTGTCCCA GGTGGCCCCA 1620
TCCCTGTTTA TGGGCTCCAT TGTCCAGCTC AGCCAGTCTG TCACTGCCTA TATGGTGTCT 1680
GCCGCAGGCC TGGGTCTGGT CGCCATTTAC TTTGCTACAC AGGTAGTATT TGACAAGAGC 1740
GACTTGGCCA AATACTCAGC Gggtggacac catcaccatc accattaa 1788

**FIG. 3. Structure of CPC-p501 His fusion protein expressed in *S. cerevisiae***

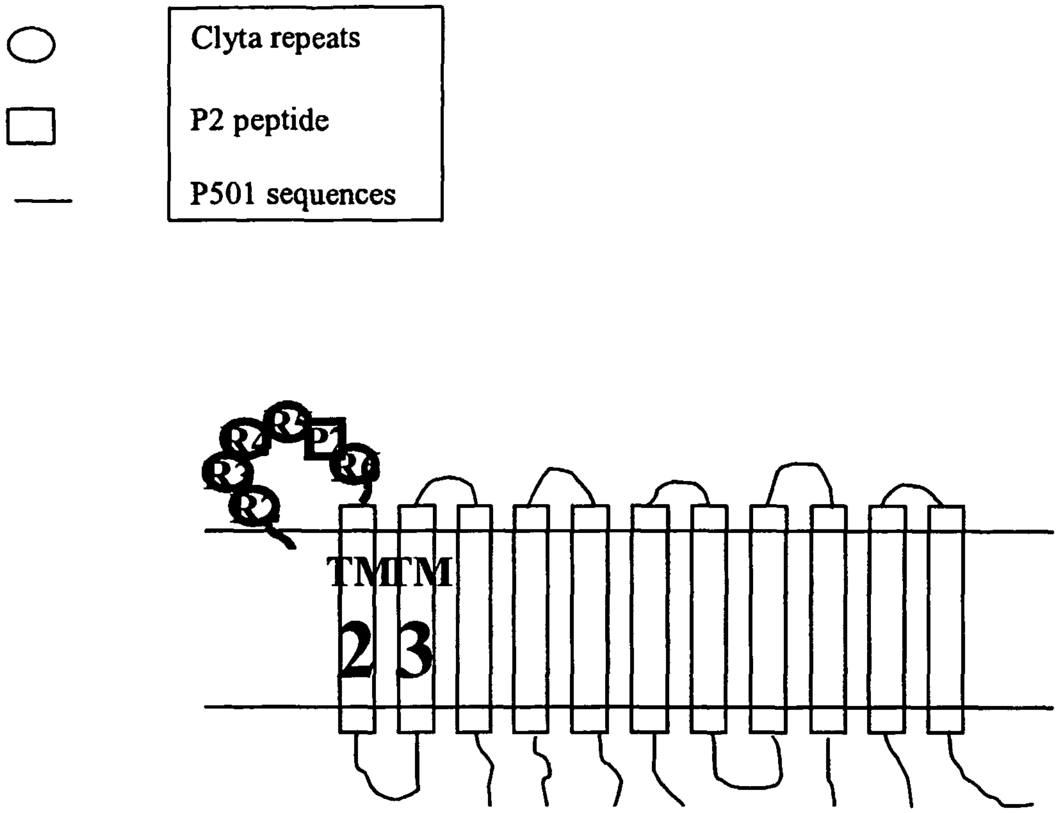

**FIG. 4. Primary structure of CPC-P501 His fusion protein (SEQ ID NO.41)**

```
MAAAYVHSDG SYPKDKFEKI NGTWYYFDSS GYMLADRWRK HTDGNWYWFD NSGEMATGWK  60
KIADKWYYFN EEGAMKTGWV KYKDTWYYLD AKEGAMQYIK ANSKFIGITE GVMVSNAFIQ  120
SADGTGWYYL KPDGTLADRP EKFMYMVLGI GPVLGLVCVP LLGSASDHWR GRYGRRRPFI  180
WALSLGILLS LFLIPRAGWL AGLLCPDPRP LELALLILGV GLLDFCGQVC FTPLEALLSD  240
LFRDPDHCRQ AYSVYAFMIS LGGCLGYLLP AIDWDTSALA PYLGTQEECL FGLLTLIFLT  300
CVAATLLVAE EAALGPTEPA EGLSAPSLSP HCCPCRARLA FRNLGALLPR LHQLCCRMPR  360
TLRRLFVAEL CSWMALMTFT LFYTDFVGEG LYQGVPRAEP GTEARRHYDE GVRMGSLGLF  420
LQCAISLVFS LVMDRLVQRF GTRAVYLASV AAFPVAAGAT CLSHSVAVVT ASAALTGFTF  480
SALQILPYTL ASLYHREKQV FLPKYRGDTG GASSEDSLMT SFLPGPKPGA PFPNGHVGAG  540
GSGLLPPPPA LCGASACDVS VRVVVGEPTE ARVVPGRGIC LDLAILDSAF LLSQVAPSLF  600
MGSIVQLSQS VTAYMVSAAG LGLVAIYFAT QVVFDKSDLA KYSAGGHHHH HH  652
```

## FIG. 5. Nucleotide sequence of CPC P501 His(pRIT15201) (SEQ ID NO.42)

ATGGCGGCCG CTTACGTACA TTCCGACGGC TCTTATCCAA AAGACAAGTT TGAGAAAATC 60
AATGGCACTT GGTACTACTT TGACAGTTCA GGCTATATGC TTGCAGACCG CTGGAGGAAG 120
CACACAGACG GCAACTGGTA CTGGTTCGAC AACTCAGGCG AAATGGCTAC AGGCTGGAAG 180
AAAATCGCTG ATAAGTGGTA CTATTTCAAC GAAGAAGGTG CCATGAAGAC AGGCTGGGTC 240
AAGTACAAGG ACACTTGGTA CTACTTAGAC GCTAAAGAAG GCGCCATGCA ATACATCAAG 300
GCTAACTCTA AGTTCATTGG TATCACTGAA GGCGTCATGG TATCAAATGC CTTTATCCAG 360
TCAGCGGACG GAACAGGCTG GTACTACCTC AAACCAGACG GAACACTGGC AGACAGGCCA 420
GAAAAGTTCA TGTACATGGT GCTGGGCATT GGTCCAGTGC TGGGCCTGGT CTGTGTCCCG 480
CTCCTAGGCT CAGCCAGTGA CCACTGGCGT GGACGCTATG GCCGCCGCCG GCCCTTCATC 540
TGGGCACTGT CCTTGGGCAT CCTGCTGAGC CTCTTTCTCA TCCCAAGGGC CGGCTGGCTA 600
GCAGGGCTGC TGTGCCCGGA TCCCAGGCCC CTGGAGCTGG CACTGCTCAT CCTGGGCGTG 660
GGGCTGCTGG ACTTCTGTGG CCAGGTGTGC TTCACTCCAC TGGAGGCCCT GCTCTCTGAC 720
CTCTTCCGGG ACCCGGACCA CTGTCGCCAG GCCTACTCTG TCTATGCCTT CATGATCAGT 780
CTTGGGGGCT GCCTGGGCTA CCTCCTGCCT GCCATTGACT GGGACACCAG TGCCCTGGCC 840
CCCTACCTGG GCACCCAGGA GGAGTGCCTC TTTGGCCTGC TCACCCTCAT CTTCCTCACC 900
TGCGTAGCAG CCACACTGCT GGTGGCTGAG GAGGCAGCGC TGGGCCCCAC CGAGCCAGCA 960
GAAGGGCTGT CGGCCCCCTC CTTGTCGCCC CACTGCTGTC CATGCCGGGC CCGCTTGGCT 1020
TTCCGGAACC TGGGCGCCCT GCTTCCCCGG CTGCACCAGC TGTGCTGCCG CATGCCCCGC 1080
ACCCTGCGCC GGCTCTTCGT GGCTGAGCTG TGCAGCTGGA TGGCACTCAT GACCTTCACG 1140
CTGTTTTACA CGGATTTCGT GGGCGAGGGG CTGTACCAGG GCGTGCCCAG AGCTGAGCCG 1200
GGCACCGAGG CCCGGAGACA CTATGATGAA GGCGTTCGGA TGGGCAGCCT GGGGCTGTTC 1260
CTGCAGTGCG CCATCTCCCT GGTCTTCTCT CTGGTCATGG ACCGGCTGGT GCAGCGATTC 1320
GGCACTCGAG CAGTCTATTT GGCCAGTGTG GCAGCTTTCC CTGTGGCTGC CGGTGCCACA 1380
TGCCTGTCCC ACAGTGTGGC CGTGGTGACA GCTTCAGCCG CCCTCACCGG GTTCACCTTC 1440
TCAGCCCTGC AGATCCTGCC CTACACACTG GCCTCCCTCT ACCACCGGGA GAAGCAGGTG 1500
TTCCTGCCCA AATACCGAGG GGACACTGGA GGTGCTAGCA GTGAGGACAG CCTGATGACC 1560
AGCTTCCTGC CAGGCCCTAA GCCTGGAGCT CCCTTCCCTA ATGGACACGT GGGTGCTGGA 1620
GGCAGTGGCC TGCTCCCACC TCCACCCGCG CTCTGCGGGG CCTCTGCCTG TGATGTCTCC 1680
GTACGTGTGG TGGTGGGTGA GCCCACCGAG GCCAGGGTGG TTCCGGGCCG GGGCATCTGC 1740
CTGGACCTCG CCATCCTGGA TAGTGCCTTC CTGCTGTCCC AGGTGGCCCC ATCCCTGTTT 1800
ATGGGCTCCA TTGTCCAGCT CAGCCAGTCT GTCACTGCCT ATATGGTGTC TGCCGCAGGC 1860
CTGGGTCTGG TCGCCATTTA CTTTGCTACA CAGGTAGTAT TTGACAAGAG CGACTTGGCC 1920
AAATACTCAG CGGGTGGACA CCATCACCAT CACCATTAA 1959

**FIG. 6. Cloning strategy for generation of plasmid pRIT 15201**

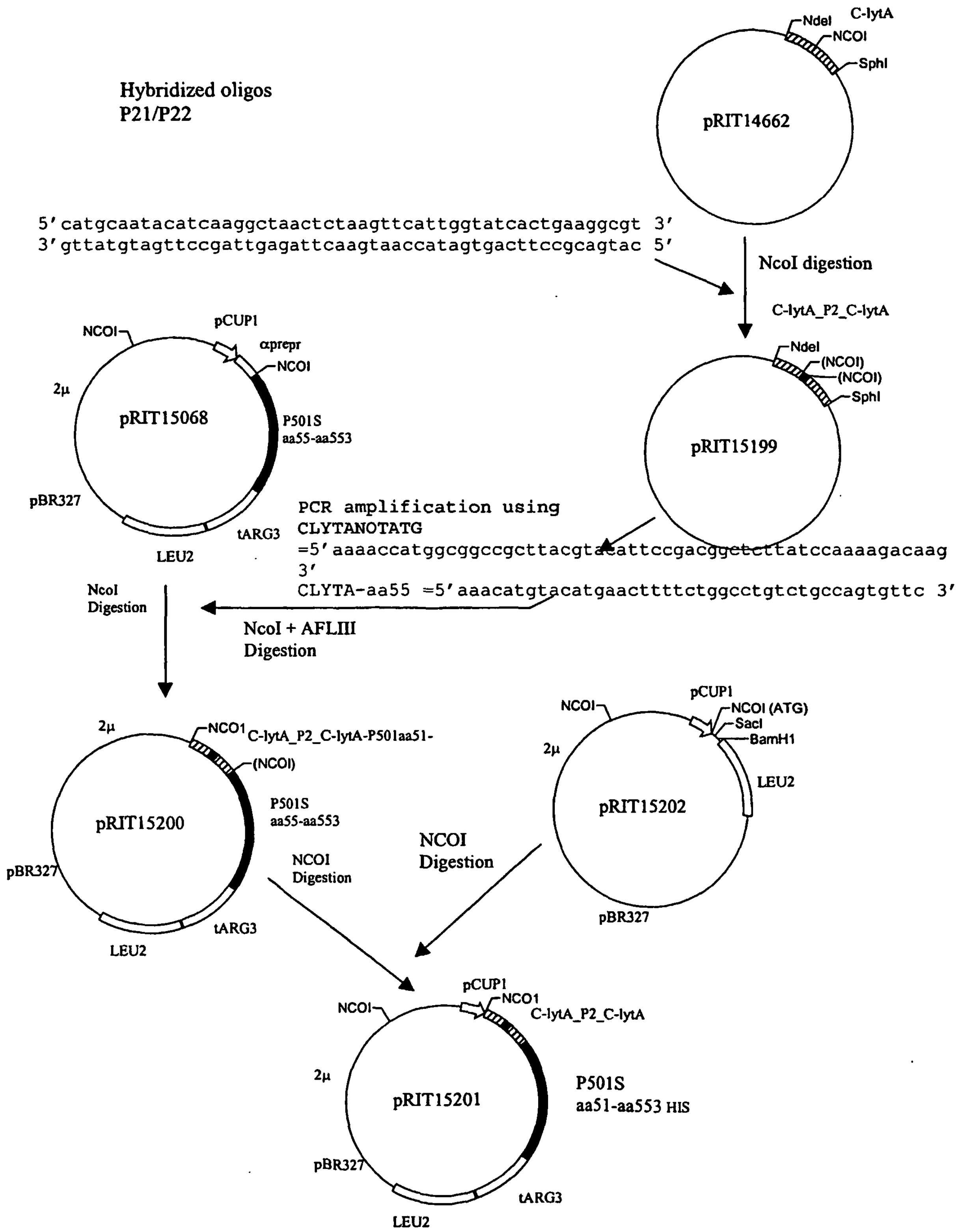

**FIG. 7. Plasmid map of pRIT15201**

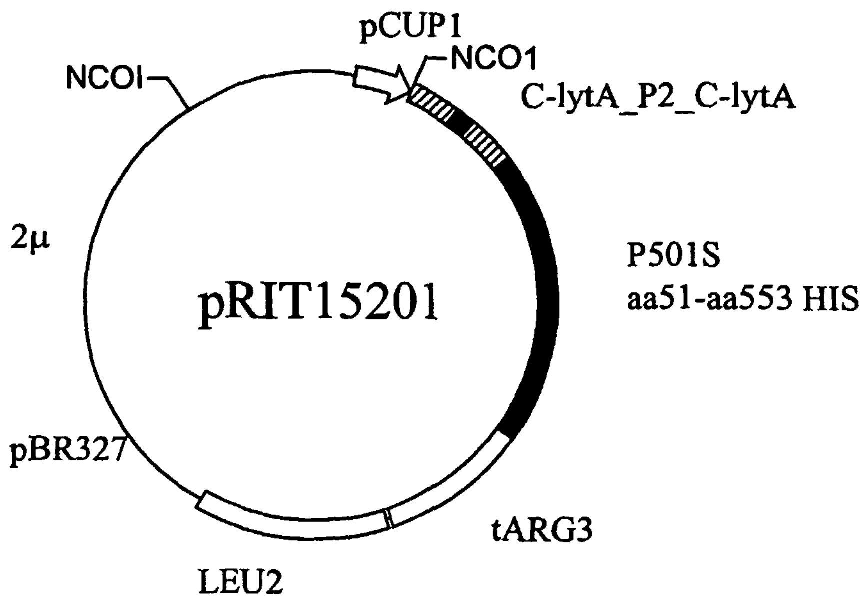

**FIG. 8. Comparative expression of CPC P501 and P501 in *S.cerevisiae* strain DC5 (gel Laemmli 10%)**

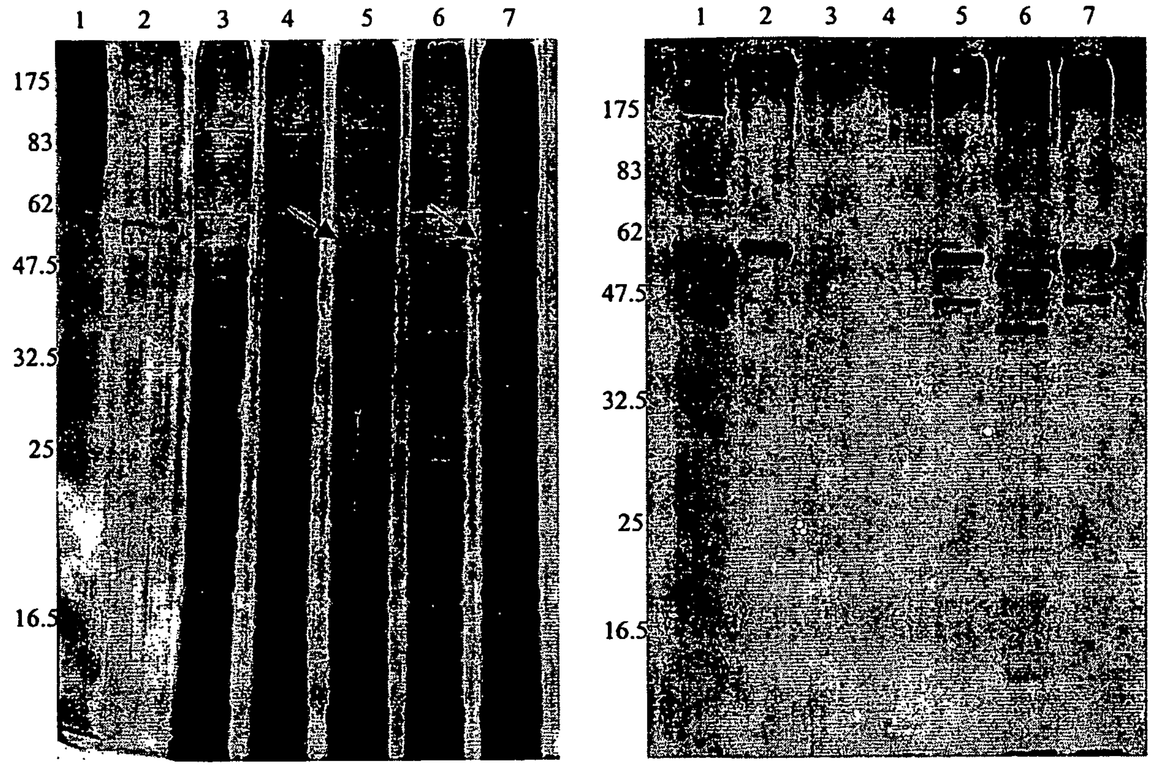


Silver staining

Western blot anti P501
(Monoclonal antibody)

1 MW Biolabs (175/83/62/47.5/32.5/16.5 Kda)
2 Y1796 purified
3 Y1795 Crude Extract ( negative control)
4 SC333 Crude Extract
5 Y1796 Crude Extract
6 Y1790 Crude Extract
7 Y1802 Crude Extract

FIG. 9A.

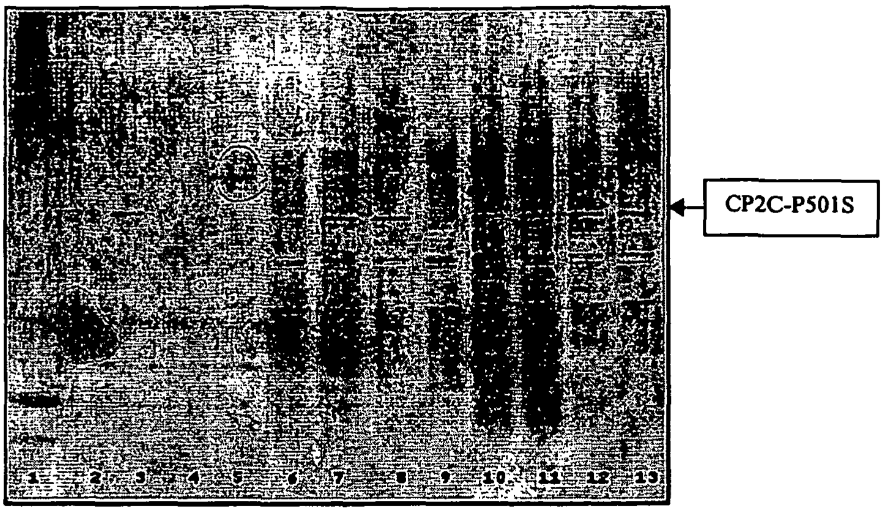


FIG. 9B.

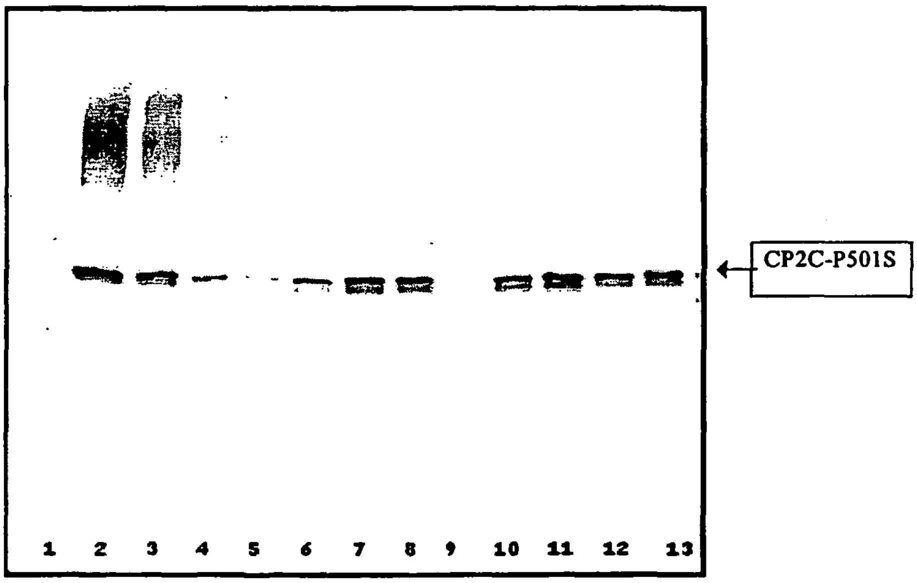


1 - **Molecular Weight Marker ( Biolabs - Grow Range)**175; 83; 62; 47.5; 32.5; 25; 16.5; 6.5 kD - 10
2 - Purified Reference CP2CP501S/12 135 ng
3 - Purified Reference CP2CP501S/12 67.8 ng
4 - Purified Reference CP2CP501S/12 33.9 ng
5 - Purified Reference CP2CP501S/12 16.9 ng
6 - Fermentation PRO119-21h30
7 - Fermentation PRO124-21h30
8 - Fermentation PRO124-22h30
9 - Fermentation PRO127-0 h
10 - Fermentation PRO127-4 h
11 - Fermentation PRO127-6 h
12 - Fermentation PRO127-22h20
13 - Fermentation PRO127-22h45

**FIG. 10. Purification scheme of CPC-P501-His produced by Y1796.**

| | |
|---|---|
| *S. Cerevisiae cells* | |
| ↓ | |
| **Dyno-mill disruption** | OD 120 / 2 passes / 20 mM Tris pH 8.5 - 5 mM EDTA |
| ↓ | |
| **Centrifugation** | 12.000 g / RT / 90 min (supernatant discarded) |
| ↓ | |
| **Pellet washing step 1** | 20 mM Tris pH 8.5 - 0.15 M NaCl - 2.0 M Guanidine.HCl - 0.1% Empigen (30 min / RT) |
| ↓ | |
| **Centrifugation** | 12.000 g / RT / 60 min (supernatant discarded) |
| ↓ | |
| **Pellet washing step 2** | 20 mM Tris pH 8.5 - 0.15 M NaCl - 4.0 M Urea |
| ↓ | |
| **Centrifugation** | 12.000 g / RT / 30 min (supernatant discarded) |
| ↓ | |
| Solubilisation / Reduction | 20 mM Tris pH 8.5 - 0.15 M NaCl - 8.0 M Urea - 1% SDS - 0.2 M Glutathion (60 min / RT) |
| ↓ | |
| *Centrifugation* | 12.000 g / RT / 30 min (pellet discarded) |
| ↓ | |
| *Carbamidomethylation* | 0.3 M Iodoacetamide (30 min / RT / in the dark) / pH adjusted to 8.5 (with 5 M NaOH solution) before incubation |
| ↓ | |
| **R/C Supernatant** | |
| ↓ | |
| **10-fold dilution and pH adjustment (8.5)** | Dilution buffer: 20 mM Tris pH 8.5 - 1 M NaCl - 8.0 M Urea |
| ↓ | |
| **Immobilised metal ion affinity chromatography on $Ni^{++}$-Chelating Sepharose FF (Amersham) (10x25 cm column – 2000 ml)** | Equilibration buffer: 20 mM Tris pH 8.5 - 0.9 M NaCl - 8.0 M Urea - 0.1% SDS<br>Washing buffers:<br>1) Equilibration buffer<br>2) 20 mM Tris pH 8.5 - 0.15 M NaCl - 8.0 M Urea - 0.1% SDS<br>3) 20 mM Tris pH 8.5 - 8.0 M Urea - 0.1% Tween 80 |

| | |
|---|---|
| | Elution buffer: 20 mM Tris pH 8.5 - 8.0 M Urea - 0.1% Tween 80 - 0.5 M Imidazole |
| ↓ | |
| **2-fold dilution and pH adjustment (10.0)** | 20 mM Piperazine pH 10.0 - 8.0 M Urea - 0.1% Tween 80 |
| ↓ | |
| **Anion exchange chromatography on Q Sepharose FF (Amersham) (2,6 x 6.5 cm column - 35 ml)** | Equilibration buffer: 20 mM Piperazine pH 10.0 - 8.0 M Urea - 0.1% Tween 80<br>Washing buffers:<br>1) Equilibration buffer<br>2) 20 mM Tris pH 8.5 - 8.0 M Urea - 0.1% Tween 80<br>Elution buffer: 20 mM Tris pH 7.5 - 8.0 M Urea - 0.1% Tween 80 - 0.5 M NaCl |
| ↓ | |
| **Concentration/Diafiltration (Pall - Omega 10 kDa - 200 cm²)** | +/- 3-fold concentration<br>Diafiltration buffer: Tris 20 mM pH 7.5 |
| ↓ | |
| **Sterile filtration (Millipore - Millex GV 0.22µm)** | |
| ↓ | |
| **Purified bulk** | Final buffer: 20 mM Tris pH 7.5 - +/- 0.3% Tween 80 |
| ↓ | |
| **Storage –20°C** | |

FIG. 11. Pattern of CPC P501 His purified protein (4-12% Novex Nu-Page polyacrylamide precasted gels)

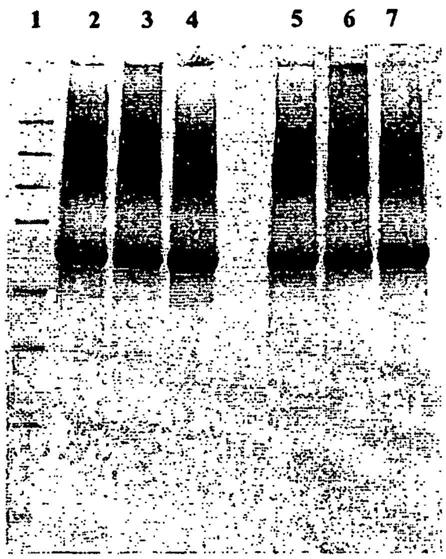


Coomassie Blue R250

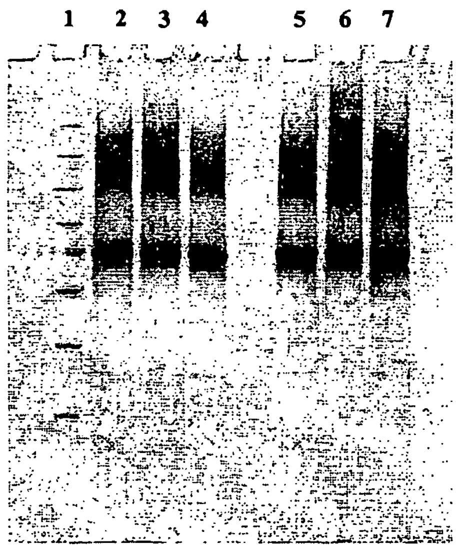


Daiichi Silver Staining

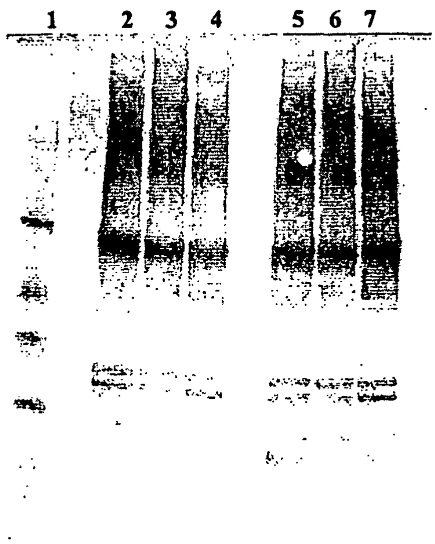


Western Blot anti P501S
(Monoclonal antibody)

1: MW (250/150/75/50/37/25/15/10 kDa)
2: Purified bulk A (reducing conditions)
3: Purified bulk B (reducing conditions)
4: Purified bulk C (reducing conditions)
5: Purified bulk A (non reducing conditions)
6: Purified bulk B (non reducing conditions)
7: Purified bulk C (non reducing conditions)

**FIG. 12. Native full-length P501S sequence (SEQ ID NO:17 & 43)**

Nucleotide sequence: SEQ ID NO.17

Polypeptide sequence: SEQ ID NO.43

```
                      ######
                      GCCACCATGGTCCAGAGGCTGTGGGTGAGCCGCCTGCTGCGGCACCGG
                            M  V  Q  R  L  W  V  S  R  L  L  R  H  R   14

AAAGCCCAGCTCTTGCTGGTCAACCTGCTAACCTTTGGCCTGGAGGTGTGTTTGGCCGCA
K  A  Q  L  L  L  V  N  L  L  T  F  G  L  E  V  C  L  A  A   34

GGCATCACCTATGTGCCGCCTCTGCTGCTGGAAGTGGGGGTAGAGGAGAAGTTCATGACC
G  I  T  Y  V  P  P  L  L  L  E  V  G  V  E  E  K  F  M  T   54

ATGGTGCTGGGCATTGGTCCAGTGCTGGGCCTGGTCTGTGTCCCGCTCCTAGGCTCAGCC
M  V  L  G  I  G  P  V  L  G  L  V  C  V  P  L  L  G  S  A   74

AGTGACCACTGGCGTGGACGCTATGGCCGCCGCCGGCCCTTCATCTGGGCACTGTCCTTG
S  D  H  W  R  G  R  Y  G  R  R  R  P  F  I  W  A  L  S  L   94

GGCATCCTGCTGAGCCTCTTTCTCATCCCAAGGGCCGGCTGGCTAGCAGGGCTGCTGTGC
G  I  L  L  S  L  F  L  I  P  R  A  G  W  L  A  G  L  L  C   114

CCGGATCCCAGGCCCCTGGAGCTGGCACTGCTCATCCTGGGCGTGGGGCTGCTGGACTTC
P  D  P  R  P  L  E  L  A  L  L  I  L  G  V  G  L  L  D  F   134

TGTGGCCAGGTGTGCTTCACTCCACTGGAGGCCCTGCTCTCTGACCTCTTCCGGGACCCG
C  G  Q  V  C  F  T  P  L  E  A  L  L  S  D  L  F  R  D  P   154

GACCACTGTCGCCAGGCCTACTCTGTCTATGCCTTCATGATCAGTCTTGGGGGCTGCCTG
D  H  C  R  Q  A  Y  S  V  Y  A  F  M  I  S  L  G  G  C  L   174

GGCTACCTCCTGCCTGCCATTGACTGGGACACCAGTGCCCTGGCCCCCTACCTGGGCACC
G  Y  L  L  P  A  I  D  W  D  T  S  A  L  A  P  Y  L  G  T   194

CAGGAGGAGTGCCTCTTTGGCCTGCTCACCCTCATCTTCCTCACCTGCGTAGCAGCCACA
Q  E  E  C  L  F  G  L  L  T  L  I  F  L  T  C  V  A  A  T   214

CTGCTGGTGGCTGAGGAGGCAGCGCTGGGCCCCACCGAGCCAGCAGAAGGGCTGTCGGCC
L  L  V  A  E  E  A  A  L  G  P  T  E  P  A  E  G  L  S  A   234

CCCTCCTTGTCGCCCCACTGCTGTCCATGCCGGGCCCGCTTGGCTTTCCGGAACCTGGGC
P  S  L  S  P  H  C  C  P  C  R  A  R  L  A  F  R  N  L  G   254

GCCCTGCTTCCCCGGCTGCACCAGCTGTGCTGCCGCATGCCCCGCACCCTGCGCCGGCTC
A  L  L  P  R  L  H  Q  L  C  C  R  M  P  R  T  L  R  R  L   274

TTCGTGGCTGAGCTGTGCAGCTGGATGGCACTCATGACCTTCACGCTGTTTTACACGGAT
F  V  A  E  L  C  S  W  M  A  L  M  T  F  T  L  F  Y  T  D   294

TTCGTGGGCGAGGGGCTGTACCAGGGCGTGCCCAGAGCTGAGCCGGGCACCGAGGCCCGG
F  V  G  E  G  L  Y  Q  G  V  P  R  A  E  P  G  T  E  A  R   314

AGACACTATGATGAAGGCGTTCGGATGGGCAGCCTGGGGCTGTTCCTGCAGTGCGCCATC
R  H  Y  D  E  G  V  R  M  G  S  L  G  L  F  L  Q  C  A  I   334

TCCCTGGTCTTCTCTCTGGTCATGGACCGGCTGGTGCAGCGATTCGGCACTCGAGCAGTC
S  L  V  F  S  L  V  M  D  R  L  V  Q  R  F  G  T  R  A  V   354
```

TATTTGGCCAGTGTGGCAGCTTTCCCTGTGGCTGCCGGTGCCACATGCCTGTCCCACAGT
Y L A S V A A F P V A A G A T C L S H S 374

GTGGCCGTGGTGACAGCTTCAGCCGCCCTCACCGGGTTCACCTTCTCAGCCCTGCAGATC
V A V V T A S A A L T G F T F S A L Q I 394

CTGCCCTACACACTGGCCTCCCTCTACCACCGGGAGAAGCAGGTGTTCCTGCCCAAATAC
L P Y T L A S L Y H R E K Q V F L P K Y 414

CGAGGGGACACTGGAGGTGCTAGCAGTGAGGACAGCCTGATGACCAGCTTCCTGCCAGGC
R G D T G G A S S E D S L M T S F L P G 434

CCTAAGCCTGGAGCTCCCTTCCCTAATGGACACGTGGGTGCTGGAGGCAGTGGCCTGCTC
P K P G A P F P N G H V G A G G S G L L 454

CCACCTCCACCCGCGCTCTGCGGGGCCTCTGCCTGTGATGTCTCCGTACGTGTGGTGGTG
P P P P A L C G A S A C D V S V R V V V 474

GGTGAGCCCACCGAGGCCAGGGTGGTTCCGGGCCGGGGCATCTGCCTGGACCTCGCCATC
G E P T E A R V V P G R G I C L D L A I 494

CTGGATAGTGCCTTCCTGCTGTCCCAGGTGGCCCCATCCCTGTTTATGGGCTCCATTGTC
L D S A F L L S Q V A P S L F M G S I V 514

CAGCTCAGCCAGTCTGTCACTGCCTATATGGTGTCTGCCGCAGGCCTGGGTCTGGTCGCC
Q L S Q S V T A Y M V S A A G L G L V A 534

ATTTACTTTGCTACACAGGTAGTATTTGACAAGAGCGACTTGGCCAAATACTCAGCG**TAG**
I Y F A T Q V V F D K S D L A K Y S A * 554

GTCGAG

**FIG. 13. Sequence of the CPC-P501S expression cassette of JNW735 (SEQ ID NO:18 & 44)**

Nucleotide sequence: SEQ ID NO.18

Polypeptide sequence: SEQ ID NO.44

```
          ######
          GCCACCATGGCGGCCGCTTACGTACATTCCGACGGCTCTTATCCAAAA
                M  A  A  A  Y  V  H  S  D  G  S  Y  P  K     14

GACAAGTTTGAGAAAATCAATGGCACTTGGTACTACTTTGACAGTTCAGGCTATATGCTT
D  K  F  E  K  I  N  G  T  W  Y  Y  F  D  S  S  G  Y  M  L     34

GCAGACCGCTGGAGGAAGCACACAGACGGCAACTGGTACTGGTTCGACAACTCAGGCGAA
A  D  R  W  R  K  H  T  D  G  N  W  Y  W  F  D  N  S  G  E     54

ATGGCTACAGGCTGGAAGAAAATCGCTGATAAGTGGTACTATTTCAACGAAGAAGGTGCC
M  A  T  G  W  K  K  I  A  D  K  W  Y  Y  F  N  E  E  G  A     74

ATGAAGACAGGCTGGGTCAAGTACAAGGACACTTGGTACTACTTAGACGCTAAAGAAGGC
M  K  T  G  W  V  K  Y  K  D  T  W  Y  Y  L  D  A  K  E  G     94

GCCATGCAATACATCAAGGCTAACTCTAAGTTCATTGGTATCACTGAAGGCGTCATGGTA
A  M  Q  Y  I  K  A  N  S  K  F  I  G  I  T  E  G  V  M  V     114

TCAAATGCCTTTATCCAGTCAGCGGACGGAACAGGCTGGTACTACCTCAAACCAGACGGA
S  N  A  F  I  Q  S  A  D  G  T  G  W  Y  Y  L  K  P  D  G     134

ACACTGGCAGACAGGCCAGAAAAGTTCATGTACATGGTGCTGGGCATTGGTCCAGTGCTG
T  L  A  D  R  P  E  K  F  M  Y  M  V  L  G  I  G  P  V  L     154

GGCCTGGTCTGTGTCCCGCTCCTAGGCTCAGCCAGTGACCACTGGCGTGGACGCTATGGC
G  L  V  C  V  P  L  L  G  S  A  S  D  H  W  R  G  R  Y  G     174

CGCCGCCGGCCCTTCATCTGGGCACTGTCCTTGGGCATCCTGCTGAGCCTCTTTCTCATC
R  R  R  P  F  I  W  A  L  S  L  G  I  L  L  S  L  F  L  I     194

CCAAGGGCCGGCTGGCTAGCAGGGCTGCTGTGCCCGGATCCCAGGCCCCTGGAGCTGGCA
P  R  A  G  W  L  A  G  L  L  C  P  D  P  R  P  L  E  L  A     214

CTGCTCATCCTGGGCGTGGGGCTGCTGGACTTCTGTGGCCAGGTGTGCTTCACTCCACTG
L  L  I  L  G  V  G  L  L  D  F  C  G  Q  V  C  F  T  P  L     234

GAGGCCCTGCTCTCTGACCTCTTCCGGGACCCGGACCACTGTCGCCAGGCCTACTCTGTC
E  A  L  L  S  D  L  F  R  D  P  D  H  C  R  Q  A  Y  S  V     254

TATGCCTTCATGATCAGTCTTGGGGGCTGCCTGGGCTACCTCCTGCCTGCCATTGACTGG
Y  A  F  M  I  S  L  G  G  C  L  G  Y  L  L  P  A  I  D  W     274

GACACCAGTGCCCTGGCCCCCTACCTGGGCACCCAGGAGGAGTGCCTCTTTGGCCTGCTC
D  T  S  A  L  A  P  Y  L  G  T  Q  E  E  C  L  F  G  L  L     294

ACCCTCATCTTCCTCACCTGCGTAGCAGCCACACTGCTGGTGGCTGAGGAGGCAGCGCTG
T  L  I  F  L  T  C  V  A  A  T  L  L  V  A  E  E  A  A  L     314

GGCCCCACCGAGCCAGCAGAAGGGCTGTCGGCCCCCTCCTTGTCGCCCCACTGCTGTCCA
G  P  T  E  P  A  E  G  L  S  A  P  S  L  S  P  H  C  C  P     334
```

TGCCGGGCCCGCTTGGCTTTCCGGAACCTGGGCGCCCTGCTTCCCCGGCTGCACCAGCTG
C R A R L A F R N L G A L L P R L H Q L 354

TGCTGCCGCATGCCCCGCACCCTGCGCCGGCTCTTCGTGGCTGAGCTGTGCAGCTGGATG
C C R M P R T L R R L F V A E L C S W M 374

GCACTCATGACCTTCACGCTGTTTTACACGGATTTCGTGGGCGAGGGGCTGTACCAGGGC
A L M T F T L F Y T D F V G E G L Y Q G 394

GTGCCCAGAGCTGAGCCGGGCACCGAGGCCCGGAGACACTATGATGAAGGCGTTCGGATG
V P R A E P G T E A R R H Y D E G V R M 414

GGCAGCCTGGGGCTGTTCCTGCAGTGCGCCATCTCCCTGGTCTTCTCTCTGGTCATGGAC
G S L G L F L Q C A I S L V F S L V M D 434

CGGCTGGTGCAGCGATTCGGCACTCGAGCAGTCTATTTGGCCAGTGTGGCAGCTTTCCCT
R L V Q R F G T R A V Y L A S V A A F P 454

GTGGCTGCCGGTGCCACATGCCTGTCCCACAGTGTGGCCGTGGTGACAGCTTCAGCCGCC
V A A G A T C L S H S V A V V T A S A A 474

CTCACCGGGTTCACCTTCTCAGCCCTGCAGATCCTGCCCTACACACTGGCCTCCCTCTAC
L T G F T F S A L Q I L P Y T L A S L Y 494

CACCGGGAGAAGCAGGTGTTCCTGCCCAAATACCGAGGGGACACTGGAGGTGCTAGCAGT
H R E K Q V F L P K Y R G D T G G A S S 514

GAGGACAGCCTGATGACCAGCTTCCTGCCAGGCCCTAAGCCTGGAGCTCCCTTCCCTAAT
E D S L M T S F L P G P K P G A P F P N 534

GGACACGTGGGTGCTGGAGGCAGTGGCCTGCTCCCACCTCCACCCGCGCTCTGCGGGGCC
G H V G A G G S G L L P P P P A L C G A 554

TCTGCCTGTGATGTCTCCGTACGTGTGGTGGTGGGTGAGCCCACCGAGGCCAGGGTGGTT
S A C D V S V R V V V G E P T E A R V V 574

CCGGGCCGGGGCATCTGCCTGGACCTCGCCATCCTGGATAGTGCCTTCCTGCTGTCCCAG
P G R G I C L D L A I L D S A F L L S Q 594

GTGGCCCCATCCCTGTTTATGGGCTCCATTGTCCAGCTCAGCCAGTCTGTCACTGCCTAT
V A P S L F M G S I V Q L S Q S V T A Y 614

ATGGTGTCTGCCGCAGGCCTGGGTCTGGTCGCCATTTACTTTGCTACACAGGTAGTATTT
M V S A A G L G L V A I Y F A T Q V V F 634

GACAAGAGCGACTTGGCCAAATACTCAGCGTAGGTCGAG
D K S D L A K Y S A * 645

## FIG. 14 – Codon optimised P501S sequences (SEQ ID NO:19-20)

### SEQ ID NO:19

ATGGTGCAGCGGCTCTGGGTGAGCCGCCTCCTGCGGCATCGCAAGGCCCAGCTCCTGCTGGTGAATCTGCTCA
CATTCGGCCTGGAGGTGTGCCTGGCCGCCGGCATCACCTACGTGCCCCCCCTCCTGCTGGAGGTGGGAGTCGA
GGAGAAGTTCATGACCATGGTGCTGGGCATTGGGCCCGTCCTGGGCCTCGTGTGCGTGCCTCTCCTCGGCAGC
GCTTCCGACCATTGGCGCGGCCGGTATGGCCGCAGGAGACCCTTCATCTGGGCTCTGAGTCTCGGCATCCTGC
TGAGCCTGTTCCTGATCCCTCGGGCCGGCTGGCTGGCCGGGCTGCTGTGCCCCGATCCTCGGCCCCTGGAGCT
GGCCCTGCTGATCCTCGGCGTGGGCCTGCTGGACTTCTGCGGCCAGGTGTGCTTCACGCCCCTGGAGGCACTG
CTGAGCGACCTGTTCCGGGACCCCGACCATTGCCGCCAGGCGTACAGCGTGTACGCCTTCATGATCTCCCTGG
GAGGCTGCCTGGGCTACCTGCTCCCCGCCATCGATTGGGACACCAGCGCACTCGCCCCCTATCTCGGAACACA
GGAGGAATGCCTGTTCGGATTGTTGACGCTCATCTTCCTCACGTGCGTCGCGGCCACCCTGTTGGTGGCCGAG
GAGGCCGCCCTGGGGCCCACCGAGCCGGCCGAGGGACTGAGCGCCCCGAGCCTGAGTCCACACTGCTGCCCTT
GCCGGGCCCGCCTGGCCTTCCGTAATCTGGGCGCCCTCCTGCCTCGGCTCCATCAGCTGTGTTGCAGAATGCC
TAGGACGCTGCGGCGCCTGTTCGTCGCTGAGTTGTGCTCCTGGATGGCTCTCATGACCTTCACCCTGTTTTAT
ACGGACTTCGTCGGGGAGGGCCTGTACCAGGGGGTGCCGCGCGCCGAGCCCGGGACAGAGGCGCGCCGCCACT
ACGACGAGGGAGTGCGTATGGGCTCCCTGGGCCTCTTCTTGCAGTGCGCCATCAGTCTGGTTTTCTCTCTGGT
CATGGACAGGCTGGTGCAGCGCTTCGGAACCCGGGCGGTGTACCTGGCGAGCGTGGCCGCCTTCCCCGTGGCT
GCCGGCGCCACCTGCCTCTCTCACTCGGTGGCCGTGGTCACCGCCAGCGCCGCCCTGACCGGGTTCACCTTCT
CTGCCCTGCAGATTCTGCCTTACACCCTGGCCAGCCTGTACCATCGCGAGAAACAGGTGTTTCTCCCCAAGTA
CAGAGGCGACACCGGGGGCGCCTCCAGCGAGGACAGCCTCATGACCTCCTTCCTGCCTGGCCCCAAGCCCGGC
GCCCCTTTCCCCAACGGGCACGTGGGCGCCGGCGGGAGTGGGCTCCTGCCCCCCCCTCCTGCGCTGTGCGGGG
CCAGCGCCTGCGACGTGAGCGTGCGCGTGGTGGTGGGCGAGCCCACCGAGGCCCGCGTGGTGCCGGGCAGAGG
CATTTGTCTGGACCTGGCCATCCTCGACTCCGCCTTCCTCCTCAGCCAGGTGGCCCCGTCCCTCTTCATGGGC
TCTATCGTCCAGCTGTCTCAGAGCGTCACCGCTTACATGGTGTCCGCTGCTGGACTGGGCTTGGTGGCTATTT
ATTTCGCCACCCAGGTGGTGTTCGACAAGAGCGACCTGGCCAAATACTCCGCCTGA

### SEQ ID NO:20

ATGGTGCAGCGGCTGTGGGTGTCCCGGCTGCTGCGCCATAGAAAGGCCCAGTTGCTGCTGGTGAACCTGCTGA
CTTTCGGACTGGAGGTGTGCCTGGCTGCCGGGATCACGTACGTGCCCCCCCTGCTGCTGGAGGTGGGCGTGGA
GGAGAAGTTCATGACAATGGTGCTGGGCATCGGCCCCGTCCTGGGCCTCGTGTGTGTGCCCCTCCTCGGGAGT
GCGTCCGATCATTGGCGGGGCCGCTACGGCCGCCGCAGACCGTTCATCTGGGCCCTGAGCCTGGGGATCCTGC
TCTCTCTCTTCCTGATCCCCCGGGCCGGCTGGCTGGCCGGCCTGCTGTGTCCCGACCCCCGCCCTCTGGAGCT
GGCCCTCCTGATCCTGGGCGTGGGCTTGTTGGACTTCTGCGGCCAGGTGTGTTTCACTCCCCTGGAGGCTCTG
CTCTCCGACCTCTTCCGCGACCCCGACCACTGTAGGCAGGCTTACAGCGTGTACGCCTTCATGATCAGTCTGG

GGGGATGCCTGGGCTATCTGCTGCCCGCTATCGACTGGGACACCAGCGCCCTGGCCCCCTACCTGGGGACTCA
GGAGGAGTGCCTGTTCGGCCTGCTCACCTTGATCTTCCTGACGTGCGTCGCCGCCACCCTGCTGGTGGCCGAG
GAGGCGGCCCTGGGGCCCACCGAGCCCGCCGAGGGCCTGAGCGCTCCCAGCCTGAGCCCCCATTGCTGCCCGT
GCAGGGCTAGGCTCGCCTTCAGGAATCTGGGCGCTTTGCTGCCCCGCCTGCATCAGCTGTGCTGTCGCATGCC
TCGCACCCTGCGCCGCCTGTTCGTCGCTGAGCTCTGTTCCTGGATGGCCCTGATGACGTTCACCCTCTTCTAC
ACCGACTTCGTGGGGGAGGGCCTGTACCAGGGCGTGCCCAGGGCCGAGCCCGGCACCGAGGCTAGGCGCCATT
ACGACGAGGGCGTCAGGATGGGCTCTCTGGGCCTCTTCCTGCAGTGCGCCATCAGTCTGGTGTTCTCTCTGGT
GATGGACCGGCTGGTGCAGCGCTTCGGCACCCGGGCCGTGTACCTCGCCTCTGTGGCGGCTTTCCCCGTCGCC
GCCGGCGCGACCTGCCTGTCTCATTCTGTCGCCGTGGTGACCGCCAGCGCCGCCCTGACCGGCTTCACCTTCA
GTGCGCTCCAGATTCTGCCCTACACCCTGGCGTCTCTGTACCATCGCGAGAAGCAGGTGTTCCTGCCCAAGTA
CCGCGGGGACACAGGGGGAGCTTCCTCTGAGGACAGCCTGATGACCAGCTTCTTGCCCGGCCCCAAGCCGGGG
GCCCCTTTCCCCAACGGCCATGTCGGGGCGGGCGGCAGCGGCCTGCTCCCTCCCCCCCCCGCCCTGTGCGGCG
CTAGTGCCTGCGACGTGAGCGTGCGGGTGGTGGTGGGGGAGCCCACCGAGGCTAGGGTCGTGCCTGGCCGGGG
GATCTGCCTGGACCTGGCCATCCTCGACTCCGCCTTCCTGCTCTCCCAGGTGGCGCCCAGCCTGTTCATGGGC
AGTATCGTGCAGCTGAGCCAGAGCGTGACCGCCTACATGGTGAGCGCCGCCGGCCTGGGGTTGGTGGCCATCT
ACTTTGCCACCCAGGTCGTGTTCGACAAGAGCGATCTCGCCAAGTATAGCGCCTGA

**FIG. 15 – Re-engineered codon optimised sequence 19 (SEQ ID NO:21)**

GACGGCTAGCGCCACCATGGTGCAGCGGCTCTGGGTGAGCCGCCTCCTGCGGCATCGCAAGGCCCAGCTCCTG
CTGGTGAATCTGCTCACATTCGGCCTGGAGGTGTGCCTGGCCGCCGGCATCACCTACGTGCCCCCCCTCCTGC
TGGAGGTGGGAGTCGAGGAGAAGTTCATGACCATGGTGCTGGGCATTGGGCCCGTCCTGGGCCTCGTGTGCGT
GCCTCTCCTCGGCAGCGCTTCCGACCATTGGCGCGGCCGGTATGGCCGCAGGAGACCCTTCATCTGGGCTCTG
AGTCTCGGCATCCTGCTGAGCCTGTTCCTGATCCCTCGGGCCGGCTGGCTGGCCGGGCTGCTGTGCCCCGATC
CTCGGCCCCTGGAGCTGGCCCTGCTGATCCTCGGCGTGGGCCTGCTGGACTTCTGCGGCCAGGTGTGCTTCAC
GCCCCTGGAGGCACTGCTGAGCGACCTGTTCCGGGACCCCGACCATTGCCGCCAGGCGTACAGCGTGTACGCC
TTCATGATCTCCCTGGGAGGCTGCCTGGGCTACCTGCTCCCCGCCATCGATTGGGACACCAGCGCACTCGCCC
CCTATCTCGGAACACAGGAGGAATGCCTGTTCGGACTGCTGACGCTCATCTTCCTCACGTGCGTCGCGGCCAC
CCTGTTGGTGGCCGAGGAGGCCGCCCTGGGGCCCACCGAGCCGGCCGAGGGACTGAGCGCCCCGAGCCTGAGT
CCACACTGCTGCCCTTGCCGGGCCCGCCTGGCCTTCCGTAATCTGGGCGCCCTCCTGCCTCGGCTCCATCAGC
TGTGTTGCAGAATGCCTAGGACGCTGCGGCGCCTGTTCGTCGCTGAGTTGTGCTCCTGGATGGCTCTCATGAC
CTTCACCCTGTTTTATACGGACTTCGTCGGGGAGGGCCTGTACCAGGGGGTGCCGCGCGCCGAGCCCGGGACA
GAGGCGCGCCGCCACTACGACGAGGGAGTGCGTATGGGCTCCCTGGGCCTCTTCTTGCAGTGCGCCATCAGTC
TGGTTTTCTCTCTGGTCATGGACAGGCTGGTGCAGCGCTTCGGAACCCGGGCGGTGTACCTGGCGAGCGTGGC
CGCCTTCCCCGTGGCTGCCGGCGCCACCTGCCTCTCTCACTCGGTGGCCGTGGTCACCGCCAGCGCCGCCCTG
ACCGGGTTCACCTTCTCTGCCCTGCAGATTCTGCCTTACACCCTGGCCAGCCTGTACCATCGCGAGAAACAGG
TGTTTCTCCCCAAGTACAGAGGCGACACCGGGGGCGCCTCCAGCGAGGACAGCCTCATGACCTCCTTCCTGCC
TGGCCCCAAGCCCGGCGCCCCTTTCCCCAACGGGCACGTGGGCGCCGGCGGGAGTGGGCTCCTGCCCCCCCCT
CCTGCGCTGTGCGGGGCCAGCGCCTGCGACGTGAGCGTGCGCGTGGTGGTGGGCGAGCCCACCGAGGCCCGCG
TGGTGCCGGGCAGAGGCATTTGTCTGGACCTGGCCATCCTCGACTCCGCCTTCCTCCTCAGCCAGGTGGCCCC
GTCCCTCTTCATGGGCTCTATCGTCCAGCTGTCTCAGAGCGTCACCGCTTACATGGTGTCCGCTGCTGGACTG
GGCTTGGTGGCTATTTATTTCGCCACCCAGGTGGTGTTCGACAAGAGCGACCTGGCCAAATACTCCGCCTGAC
TCGAGGCAG

**FIG. 16 – Re-engineered codon optimised sequence 20 (SEQ ID NO:22)**

GACGGCTAGCGCCACCATGGTGCAGCGGCTGTGGGTGTCCCGGCTGCTGCGCCATAGAAAGGCCCAGTTGCTG
CTGGTGAACCTGCTGACTTTCGGACTGGAGGTGTGCCTGGCTGCCGGGATCACGTACGTGCCCCCCCTGCTGC
TGGAGGTGGGCGTGGAGGAGAAGTTCATGACAATGGTGCTGGGCATCGGCCCCGTCCTGGGCCTCGTGTGTGT
GCCCCTCCTCGGGAGTGCGTCCGATCATTGGCGGGGCCGCTACGGCCGCCGCAGACCGTTCATCTGGGCCCTG
AGCCTGGGCATCCTGCTCTCTCTCTTCCTGATCCCCCGGGCCGGCTGGCTGGCCGGCCTGCTGTGTCCCGACC
CCCGCCCTCTGGAGCTGGCCCTCCTGATCCTGGGCGTGGGCGTGCTGGACTTCTGCGGCCAGGTGTGTTTCAC
TCCCCTGGAGGCTCTGCTCTCCGACCTCTTCCGCGACCCCGACCACTGTAGGCAGGCTTACAGCGTGTACGCC
TTCATGATCAGTCTGGGGGGATGCCTGGGCTATCTGCTGCCCGCTATCGACTGGGACACCAGCGCCCTGGCCC
CCTACCTGGGGACTCAGGAGGAGTGCCTGTTCGGCCTGCTCACCTTGATCTTCCTGACGTGCGTCGCCGCCAC
CCTGCTGGTGGCCGAGGAGGCGGCCCTGGGGCCCACCGAGCCCGCCGAGGGCCTGAGCGCTCCCAGCCTGAGC
CCCCATTGCTGCCCGTGCAGGGCTAGGCTCGCCTTCAGGAATCTGGGCGCTTTGCTGCCCCGCCTGCATCAGC
TGTGCTGTCGCATGCCTCGCACCCTGCGCCGCCTGTTCGTCGCTGAGCTCTGTTCCTGGATGGCCCTGATGAC
GTTCACCCTCTTCTACACCGACTTCGTGGGGGAGGGCCTGTACCAGGGCGTGCCCAGGGCCGAGCCCGGCACC
GAGGCTAGGCGCCATTACGACGAGGGCGTCAGGATGGGCTCTCTGGGCCTCTTCCTGCAGTGCGCCATCAGTC
TGGTGTTCTCTCTGGTGATGGACCGGCTGGTGCAGCGCTTCGGCACCCGGGCCGTGTACCTCGCCTCTGTGGC
GGCTTTCCCCGTCGCCGCCGGCGCGACCTGCCTGTCTCATTCTGTCGCCGTGGTGACCGCCAGCGCCGCCCTG
ACCGGCTTCACCTTCAGTGCGCTCCAGATTCTGCCCTACACCCTGGCGTCTCTGTACCATCGCGAGAAGCAGG
TGTTCCTGCCCAAGTACCGCGGGGACACAGGGGGAGCTTCCTCTGAGGACAGCCTGATGACCAGCTTCTTGCC
CGGCCCCAAGCCGGGGGCCCCTTTCCCCAACGGCCATGTCGGGGCGGGCGGCAGCGGCCTGCTCCCTCCCCCC
CCCGCCCTGTGCGGCGCTAGTGCCTGCGACGTGAGCGTGCGGGTGGTGGTGGGGGAGCCCACCGAGGCTAGGG
TCGTGCCTGGCCGGGGGATCTGCCTGGACCTGGCCATCCTCGACTCCGCCTTCCTGCTCTCCCAGGTGGCGCC
CAGCCTGTTCATGGGCAGTATCGTGCAGCTGAGCCAGAGCGTGACCGCCTACATGGTGAGCGCCGCCGGCCTG
GGGTTGGTGGCCATCTACTTTGCCACCCAGGTCGTGTTCGACAAGAGCGATCTCGCCAAGTATAGCGCCTGAC
TCGAGGCAG

**FIG. 17 – The starting sequence for the optimisation of CPC (SEQ ID NO:23)**

Four amino acids of P501S sequence are boxed.

ATGGCGGCCGCTTACGTACATTCCGACGGCTCTTATCCAAAAGACAAGTTTGAGAAAATCAATGGCACTTGGT
ACTACTTTGACAGTTCAGGCTATATGCTTGCAGACCGCTGGAGGAAGCACACAGACGGCAACTGGTACTGGTT
CGACAACTCAGGCGAAATGGCTACAGGCTGGAAGAAAATCGCTGATAAGTGGTACTATTTCAACGAAGAAGGT
GCCATGAAGACAGGCTGGGTCAAGTACAAGGACACTTGGTACTACTTAGACGCTAAAGAAGGCGCCATGCAAT
ACATCAAGGCTAACTCTAAGTTCATTGGTATCACTGAAGGCGTCATGGTATCAAATGCCTTTATCCAGTCAGC
GGACGGAACAGGCTGGTACTACCTCAAACCAGACGGAACACTGGCAGACAGGCCAGAAAAGTTCATGTAC

**FIG. 18 – Representative codon optimised CPC sequences (SEQ ID NO:24-25)**

**SEQ ID NO:24**

ATGGCCGCCGCCTACGTGCATAGCGACGGGAGCTACCCCAAGGACAAGTTCGAGAAGATCAACGGGACATGGT
ACTACTTCGACTCCTCCGGCTACATGCTCGCCGACCGCTGGCGGAAGCACACCGACGGCAACTGGTACTGGTT
CGATAACTCGGGAGAGATGGCCACCGGCTGGAAGAAGATCGCGGACAAGTGGTACTATTTCAACGAGGAGGGC
GCCATGAAGACCGGCTGGGTGAAGTATAAGGACACCTGGTACTACCTCGACGCCAAGGAGGGCGCCATGCAGT
ATATCAAGGCCAACAGCAAGTTCATCGGCATCACCGAGGGAGTGATGGTCAGCAACGCCTTTATCCAGAGCGC
CGACGGCACCGGATGGTACTACTTGAAGCCGGACGGCACCCTCGCGGATCGGCCCGAGAAGTTCATGTAC

**SEQ ID NO:25**

ATGGCCGCCGCCTACGTGCACAGCGACGGGTCCTACCCAAAGGACAAGTTCGAGAAGATCAACGGCACGTGGT
ACTATTTCGACAGCAGCGGCTACATGCTCGCCGATCGCTGGCGCAAGCACACCGACGGGAACTGGTACTGGTT
CGACAACTCTGGCGAGATGGCTACGGGGTGGAAGAAGATCGCCGACAAGTGGTACTACTTCAACGAGGAGGGC
GCCATGAAGACCGGGTGGGTGAAGTACAAGGACACCTGGTACTACCTGGACGCTAAGGAGGGCGCCATGCAGT
ACATCAAGGCCAACTCGAAGTTCATCGGGATCACCGAGGGCGTGATGGTCAGTAACGCTTTCATCCAGAGCGC
GGACGGCACAGGCTGGTATTACCTGAAGCCCGATGGCACCCTGGCGGACAGACCTGAGAAATTCATGTAC

**FIG. 19 – Engineered CPC codon optimised sequence (SEQ ID NO:26)**

**SEQ ID NO:26**

GACGGCTAGCGCCACCATGGCCGCCGCCTACGTGCATAGCGACGGGAGCTACCCCAAGGACAAGTTCGAGAAG
ATCAACGGGACATGGTACTACTTCGACTCCTCCGGCTACATGCTCGCCGACCGCTGGCGGAAGCACACCGACG
GCAACTGGTACTGGTTCGATAACTCGGGAGAGATGGCCACCGGCTGGAAGAAGATCGCGGACAAGTGGTACTA
TTTCAACGAGGAGGGCGCCATGAAGACCGGCTGGGTGAAGTATAAGGACACCTGGTACTACCTCGACGCCAAG
GAGGGCGCCATGCAGTATATCAAGGCCAACAGCAAGTTCATCGGCATCACCGAGGGAGTGATGGTCAGCAACG
CCTTTATCCAGAGCGCCGACGGCACCGGATGGTACTACTTGAAGCCGGACGGCACCCTCGCGGATCGGCCCGA
GAAGTTCATGTAC*TGA*CTCGAGGCAG

**FIG. 20 – P501S CPC fusion candidate constructs and sequences**

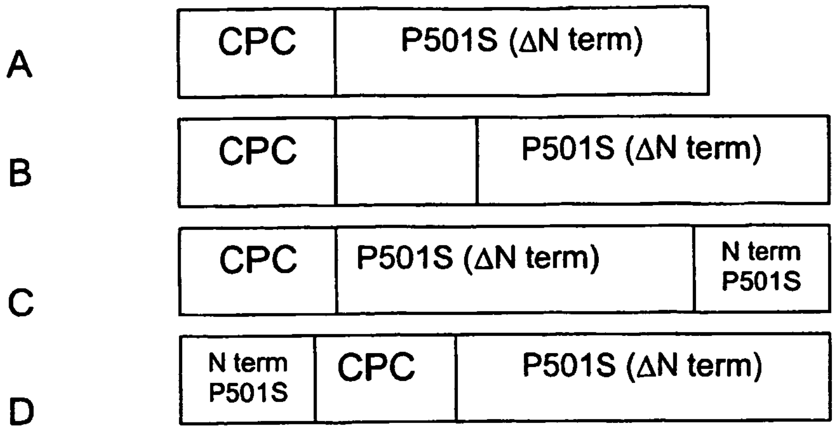


**Construct A = SEQ ID NO:37 (nucleotide) & 45 (polypeptide)**

```
GCGGCCGCGCCACCATGGCCGCCGCCTACGTGCATAGCGACGGGAGCTACCCCAAGGACA
              M  A  A  Y  V  H  S  D  G  S  Y  P  K  D  K

AGTTCGAGAAGATCAACGGGACATGGTACTACTTCGACTCCTCCGGCTACATGCTCGCCG
  F  E  K  I  N  G  T  W  Y  Y  F  D  S  S  G  Y  M  L  A  D

ACCGCTGGCGGAAGCACACCGACGGCAACTGGTACTGGTTCGATAACTCGGGAGAGATGG
  R  W  R  K  H  T  D  G  N  W  Y  W  F  D  N  S  G  E  M  A

CCACCGGCTGGAAGAAGATCGCGGACAAGTGGTACTATTTCAACGAGGAGGGCGCCATGA
  T  G  W  K  K  I  A  D  K  W  Y  Y  F  N  E  E  G  A  M  K

AGACCGGCTGGGTGAAGTATAAGGACACCTGGTACTACCTCGACGCCAAGGAGGGCGCCA
  T  G  W  V  K  Y  K  D  T  W  Y  Y  L  D  A  K  E  G  A  M

TGCAGTATATCAAGGCCAACAGCAAGTTCATCGGCATCACCGAGGGAGTGATGGTCAGCA
  Q  Y  I  K  A  N  S  K  F  I  G  I  T  E  G  V  M  V  S  N

ACGCCTTTATCCAGAGCGCCGACGGCACCGGATGGTACTACTTGAAGCCGGACGGCACCC
  A  F  I  Q  S  A  D  G  T  G  W  Y  Y  L  K  P  D  G  T  L

TCGCGGATCGGCCCGAGAAGTTCATGTACATGGTGCTGGGCATCGGCCCCGTCCTGGGCC
  A  D  R  P  E  K  F  M  Y  M  V  L  G  I  G  P  V  L  G  L

TCGTGTGTGTGCCCCTCCTCGGGAGTGCGTCCGATCATTGGCGGGGCCGCTACGGCCGCC
  V  C  V  P  L  L  G  S  A  S  D  H  W  R  G  R  Y  G  R  R

GCAGACCGTTCATCTGGGCCCTGAGCCTGGGCATCCTGCTCTCTCTCTTCCTGATCCCCC
  R  P  F  I  W  A  L  S  L  G  I  L  L  S  L  F  L  I  P  R

GGGCCGGCTGGCTGGCCGGCCTGCTGTGTCCCGACCCCCGCCCTCTGGAGCTGGCCCTCC
  A  G  W  L  A  G  L  L  C  P  D  P  R  P  L  E  L  A  L  L

TGATCCTGGGCGTGGGCCTGCTGGACTTCTGCGGCCAGGTGTGTTTCACTCCCCTGGAGG
```

I L G V G L L D F C G Q V C F T P L E A

CTCTGCTCTCCGACCTCTTCCGCGACCCCGACCACTGTAGGCAGGCTTACAGCGTGTACG
L L S D L F R D P D H C R Q A Y S V Y A

CCTTCATGATCAGTCTGGGGGATGCCTGGGCTATCTGCTGCCCGCTATCGACTGGGACA
F M I S L G G C L G Y L L P A I D W D T

CCAGCGCCCTGGCCCCCTACCTGGGGACTCAGGAGGAGTGCCTGTTCGGCCTGCTCACCT
S A L A P Y L G T Q E E C L F G L L T L

TGATCTTCCTGACGTGCGTCGCCGCCACCCTGCTGGTGGCCGAGGAGGCGGCCCTGGGGC
I F L T C V A A T L L V A E E A A L G P

CCACCGAGCCCGCCGAGGGCCTGAGCGCTCCCAGCCTGAGCCCCCATTGCTGCCCGTGCA
T E P A E G L S A P S L S P H C C P C R

GGGCTAGGCTCGCCTTCAGGAATCTGGGCGCTTTGCTGCCCCGCCTGCATCAGCTGTGCT
A R L A F R N L G A L L P R L H Q L C C

GTCGCATGCCTCGCACCCTGCGCCGCCTGTTCGTCGCTGAGCTCTGTTCCTGGATGGCCC
R M P R T L R R L F V A E L C S W M A L

TGATGACGTTCACCCTCTTCTACACCGACTTCGTGGGGGAGGGCCTGTACCAGGGCGTGC
M T F T L F Y T D F V G E G L Y Q G V P

CCAGGGCCGAGCCCGGCACCGAGGCTAGGCGCCATTACGACGAGGGCGTCAGGATGGGCT
R A E P G T E A R R H Y D E G V R M G S

CTCTGGGCCTCTTCCTGCAGTGCGCCATCAGTCTGGTGTTCTCTCTGGTGATGGACCGGC
L G L F L Q C A I S L V F S L V M D R L

TGGTGCAGCGCTTCGGCACCCGGGCCGTGTACCTCGCCTCTGTGGCGGCTTTCCCCGTCG
V Q R F G T R A V Y L A S V A A F P V A

CCGCCGGCGCGACCTGCCTGTCTCATTCTGTCGCCGTGGTGACCGCCAGCGCCGCCCTGA
A G A T C L S H S V A V V T A S A A L T

CCGGCTTCACCTTCAGTGCGCTCCAGATTCTGCCCTACACCCTGGCGTCTCTGTACCATC
G F T F S A L Q I L P Y T L A S L Y H R

GCGAGAAGCAGGTGTTCCTGCCCAAGTACCGCGGGGACACAGGGGGAGCTTCCTCTGAGG
E K Q V F L P K Y R G D T G G A S S E D

ACAGCCTGATGACCAGCTTCTTGCCCGGCCCCAAGCCGGGGGCCCCTTTCCCCAACGGCC
S L M T S F L P G P K P G A P F P N G H

ATGTCGGGGCGGGCGGCAGCGGCCTGCTCCCTCCCCCCCCCGCCCTGTGCGGCGCTAGTG
V G A G G S G L L P P P P A L C G A S A

CCTGCGACGTGAGCGTGCGGGTGGTGGTGGGGGAGCCCACCGAGGCTAGGGTCGTGCCTG
C D V S V R V V V G E P T E A R V V P G

GCCGGGGGATCTGCCTGGACCTGGCCATCCTCGACTCCGCCTTCCTGCTCTCCCAGGTGG
R G I C L D L A I L D S A F L L S Q V A

CGCCCAGCCTGTTCATGGGCAGTATCGTGCAGCTGAGCCAGAGCGTGACCGCCTACATGG
P S L F M G S I V Q L S Q S V T A Y M V

TGAGCGCCGCCGGCCTGGGGTTGGTGGCCATCTACTTTGCCACCCAGGTCGTGTTCGACA
S A A G L G L V A I Y F A T Q V V F D K

AGAGCGATCTCGCCAAGTATAGCGCCTGAGGATCC
S D L A K Y S A *

## Construct B = SEQ ID NO:38 (nucleotide) & 46 (polypeptide)

GCGGCCGCGCCACCATGGCCGCCGCCTACGTGCATAGCGACGGGAGCTACCCCAAGGACA
M A A A Y V H S D G S Y P K D K

AGTTCGAGAAGATCAACGGGACATGGTACTACTTCGACTCCTCCGGCTACATGCTCGCCG
F E K I N G T W Y Y F D S S G Y M L A D

ACCGCTGGCGGAAGCACACCGACGGCAACTGGTACTGGTTCGATAACTCGGGAGAGATGG
R W R K H T D G N W Y W F D N S G E M A

CCACCGGCTGGAAGAAGATCGCGGACAAGTGGTACTATTTCAACGAGGAGGGCGCCATGA
T G W K K I A D K W Y Y F N E E G A M K

AGACCGGCTGGGTGAAGTATAAGGACACCTGGTACTACCTCGACGCCAAGGAGGGCGCCA
T G W V K Y K D T W Y Y L D A K E G A M

TGCAGTATATCAAGGCCAACAGCAAGTTCATCGGCATCACCGAGGGAGTGATGGTCAGCA
Q Y I K A N S K F I G I T E G V M V S N

ACGCCTTTATCCAGAGCGCCGACGGCACCGGATGGTACTACTTGAAGCCGGACGGCACCC
A F I Q S A D G T G W Y Y L K P D G T L

TCGCGGATCGGCCCGAGATGGTGCAGCGGCTGTGGGTGTCCCGGCTGCTGCGCCATAGAA
A D R P E M V Q R L W V S R L L R H R K

AGGCCCAGTTGCTGCTGGTGAACCTGCTGACTTTCGGACTGGAGGTGTGCCTGGCTGCCG
A Q L L L V N L L T F G L E V C L A A G

GGATCACGTACGTGCCCCCCCTGCTGCTGGAGGTGGGCGTGGAGGAGAAGTTCATGACAA
I T Y V P P L L L E V G V E E K F M T M

TGGTGCTGGGCATCGGCCCCGTCCTGGGCCTCGTGTGTGTGCCCCTCCTCGGGAGTGCGT
V L G I G P V L G L V C V P L L G S A S

CCGATCATTGGCGGGGCCGCTACGGCCGCCGCAGACCGTTCATCTGGGCCCTGAGCCTGG
D H W R G R Y G R R R P F I W A L S L G

GCATCCTGCTCTCTCTCTTCCTGATCCCCCGGGCCGGCTGGCTGGCCGGCCTGCTGTGTC
I L L S L F L I P R A G W L A G L L C P

CCGACCCCCGCCCTCTGGAGCTGGCCCTCCTGATCCTGGGCGTGGGCCTGCTGGACTTCT
D P R P L E L A L L I L G V G L L D F C

GCGGCCAGGTGTGTTTCACTCCCCTGGAGGCTCTGCTCTCCGACCTCTTCCGCGACCCCG
G Q V C F T P L E A L L S D L F R D P D

ACCACTGTAGGCAGGCTTACAGCGTGTACGCCTTCATGATCAGTCTGGGGGGATGCCTGG
H C R Q A Y S V Y A F M I S L G G C L G

GCTATCTGCTGCCCGCTATCGACTGGGACACCAGCGCCCTGGCCCCCTACCTGGGGACTC
Y L L P A I D W D T S A L A P Y L G T Q

```
AGGAGGAGTGCCTGTTCGGCCTGCTCACCTTGATCTTCCTGACGTGCGTCGCCGCCACCC
  E  E  C  L  F  G  L  L  T  L  I  F  L  T  C  V  A  A  T  L

TGCTGGTGGCCGAGGAGGCGGCCCTGGGGCCCACCGAGCCCGCCGAGGGCCTGAGCGCTC
  L  V  A  E  E  A  A  L  G  P  T  E  P  A  E  G  L  S  A  P

CCAGCCTGAGCCCCCATTGCTGCCCGTGCAGGGCTAGGCTCGCCTTCAGGAATCTGGGCG
  S  L  S  P  H  C  C  P  C  R  A  R  L  A  F  R  N  L  G  A

CTTTGCTGCCCCGCCTGCATCAGCTGTGCTGTCGCATGCCTCGCACCCTGCGCCGCCTGT
  L  L  P  R  L  H  Q  L  C  C  R  M  P  R  T  L  R  R  L  F

TCGTCGCTGAGCTCTGTTCCTGGATGGCCCTGATGACGTTCACCCTCTTCTACACCGACT
  V  A  E  L  C  S  W  M  A  L  M  T  F  T  L  F  Y  T  D  F

TCGTGGGGGAGGGCCTGTACCAGGGCGTGCCCAGGGCCGAGCCCGGCACCGAGGCTAGGC
  V  G  E  G  L  Y  Q  G  V  P  R  A  E  P  G  T  E  A  R  R

GCCATTACGACGAGGGCGTCAGGATGGGCTCTCTGGGCCTCTTCCTGCAGTGCGCCATCA
  H  Y  D  E  G  V  R  M  G  S  L  G  L  F  L  Q  C  A  I  S

GTCTGGTGTTCTCTCTGGTGATGGACCGGCTGGTGCAGCGCTTCGGCACCCGGGCCGTGT
  L  V  F  S  L  V  M  D  R  L  V  Q  R  F  G  T  R  A  V  Y

ACCTCGCCTCTGTGGCGGCTTTCCCCGTCGCCGCCGGCGCGACCTGCCTGTCTCATTCTG
  L  A  S  V  A  A  F  P  V  A  A  G  A  T  C  L  S  H  S  V

TCGCCGTGGTGACCGCCAGCGCCGCCCTGACCGGCTTCACCTTCAGTGCGCTCCAGATTC
  A  V  V  T  A  S  A  A  L  T  G  F  T  F  S  A  L  Q  I  L

TGCCCTACACCCTGGCGTCTCTGTACCATCGCGAGAAGCAGGTGTTCCTGCCCAAGTACC
  P  Y  T  L  A  S  L  Y  H  R  E  K  Q  V  F  L  P  K  Y  R

GCGGGGACACAGGGGGAGCTTCCTCTGAGGACAGCCTGATGACCAGCTTCTTGCCCGGCC
  G  D  T  G  G  A  S  S  E  D  S  L  M  T  S  F  L  P  G  P

CCAAGCCGGGGGCCCCTTTCCCCAACGGCCATGTCGGGGCGGGCGGCAGCGGCCTGCTCC
  K  P  G  A  P  F  P  N  G  H  V  G  A  G  G  S  G  L  L  P

CTCCCCCCCCCGCCCTGTGCGGCGCTAGTGCCTGCGACGTGAGCGTGCGGGTGGTGGTGG
  P  P  P  A  L  C  G  A  S  A  C  D  V  S  V  R  V  V  V  G

GGGAGCCCACCGAGGCTAGGGTCGTGCCTGGCCGGGGGATCTGCCTGGACCTGGCCATCC
  E  P  T  E  A  R  V  V  P  G  R  G  I  C  L  D  L  A  I  L

TCGACTCCGCCTTCCTGCTCTCCCAGGTGGCGCCCAGCCTGTTCATGGGCAGTATCGTGC
  D  S  A  F  L  L  S  Q  V  A  P  S  L  F  M  G  S  I  V  Q

AGCTGAGCCAGAGCGTGACCGCCTACATGGTGAGCGCCGCCGGCCTGGGGTTGGTGGCCA
  L  S  Q  S  V  T  A  Y  M  V  S  A  A  G  L  G  L  V  A  I

TCTACTTTGCCACCCAGGTCGTGTTCGACAAGAGCGATCTCGCCAAGTATAGCGCCTGAG
  Y  F  A  T  Q  V  V  F  D  K  S  D  L  A  K  Y  S  A  *

GATCC
```

**Construct C = SEQ ID NO:39 (nucleotide) & 47 (polypeptide)**

```
GCGGCCGCGCCACCATGGCCGCCGCCTACGTGCATAGCGACGGGAGCTACCCCAAGGACA
              M  A  A  A  Y  V  H  S  D  G  S  Y  P  K  D  K

AGTTCGAGAAGATCAACGGGACATGGTACTACTTCGACTCCTCCGGCTACATGCTCGCCG
 F  E  K  I  N  G  T  W  Y  Y  F  D  S  S  G  Y  M  L  A  D

ACCGCTGGCGGAAGCACACCGACGGCAACTGGTACTGGTTCGATAACTCGGGAGAGATGG
 R  W  R  K  H  T  D  G  N  W  Y  W  F  D  N  S  G  E  M  A

CCACCGGCTGGAAGAAGATCGCGGACAAGTGGTACTATTTCAACGAGGAGGGCGCCATGA
 T  G  W  K  K  I  A  D  K  W  Y  Y  F  N  E  E  G  A  M  K

AGACCGGCTGGGTGAAGTATAAGGACACCTGGTACTACCTCGACGCCAAGGAGGGCGCCA
 T  G  W  V  K  Y  K  D  T  W  Y  Y  L  D  A  K  E  G  A  M

TGCAGTATATCAAGGCCAACAGCAAGTTCATCGGCATCACCGAGGGAGTGATGGTCAGCA
 Q  Y  I  K  A  N  S  K  F  I  G  I  T  E  G  V  M  V  S  N

ACGCCTTTATCCAGAGCGCCGACGGCACCGGATGGTACTACTTGAAGCCGGACGGCACCC
 A  F  I  Q  S  A  D  G  T  G  W  Y  Y  L  K  P  D  G  T  L

TCGCGGATCGGCCCGAGAAGTTCATGTACATGGTGCTGGGCATCGGCCCCGTCCTGGGCC
 A  D  R  P  E  K  F  M  Y  M  V  L  G  I  G  P  V  L  G  L

TCGTGTGTGTGCCCCTCCTCGGGAGTGCGTCCGATCATTGGCGGGGCCGCTACGGCCGCC
 V  C  V  P  L  L  G  S  A  S  D  H  W  R  G  R  Y  G  R  R

GCAGACCGTTCATCTGGGCCCTGAGCCTGGGCATCCTGCTCTCTCTCTTCCTGATCCCCC
 R  P  F  I  W  A  L  S  L  G  I  L  L  S  L  F  L  I  P  R

GGGCCGGCTGGCTGGCCGGCCTGCTGTGTCCCGACCCCCGCCCTCTGGAGCTGGCCCTCC
 A  G  W  L  A  G  L  L  C  P  D  P  R  P  L  E  L  A  L  L

TGATCCTGGGCGTGGGCCTGCTGGACTTCTGCGGCCAGGTGTGTTTCACTCCCCTGGAGG
 I  L  G  V  G  L  L  D  F  C  G  Q  V  C  F  T  P  L  E  A

CTCTGCTCTCCGACCTCTTCCGCGACCCCGACCACTGTAGGCAGGCTTACAGCGTGTACG
 L  L  S  D  L  F  R  D  P  D  H  C  R  Q  A  Y  S  V  Y  A

CCTTCATGATCAGTCTGGGGGGATGCCTGGGCTATCTGCTGCCCGCTATCGACTGGGACA
 F  M  I  S  L  G  G  C  L  G  Y  L  L  P  A  I  D  W  D  T

CCAGCGCCCTGGCCCCCTACCTGGGGACTCAGGAGGAGTGCCTGTTCGGCCTGCTCACCT
 S  A  L  A  P  Y  L  G  T  Q  E  E  C  L  F  G  L  L  T  L

TGATCTTCCTGACGTGCGTCGCCGCCACCCTGCTGGTGGCCGAGGAGGCGGCCCTGGGGC
 I  F  L  T  C  V  A  A  T  L  L  V  A  E  E  A  A  L  G  P

CCACCGAGCCCGCCGAGGGCCTGAGCGCTCCCAGCCTGAGCCCCCATTGCTGCCCGTGCA
 T  E  P  A  E  G  L  S  A  P  S  L  S  P  H  C  C  P  C  R

GGGCTAGGCTCGCCTTCAGGAATCTGGGCGCTTTGCTGCCCCGCCTGCATCAGCTGTGCT
 A  R  L  A  F  R  N  L  G  A  L  L  P  R  L  H  Q  L  C  C

GTCGCATGCCTCGCACCCTGCGCCGCCTGTTCGTCGCTGAGCTCTGTTCCTGGATGGCCC
 R  M  P  R  T  L  R  R  L  F  V  A  E  L  C  S  W  M  A  L

TGATGACGTTCACCCTCTTCTACACCGACTTCGTGGGGGAGGGCCTGTACCAGGGCGTGC
 M  T  F  T  L  F  Y  T  D  F  V  G  E  G  L  Y  Q  G  V  P
```

CCAGGGCCGAGCCCGGCACCGAGGCTAGGCGCCATTACGACGAGGGCGTCAGGATGGGCT
R A E P G T E A R R H Y D E G V R M G S

CTCTGGGCCTCTTCCTGCAGTGCGCCATCAGTCTGGTGTTCTCTCTGGTGATGGACCGGC
L G L F L Q C A I S L V F S L V M D R L

TGGTGCAGCGCTTCGGCACCCGGGCCGTGTACCTCGCCTCTGTGGCGGCTTTCCCCGTCG
V Q R F G T R A V Y L A S V A A F P V A

CCGCCGGCGCGACCTGCCTGTCTCATTCTGTCGCCGTGGTGACCGCCAGCGCCGCCCTGA
A G A T C L S H S V A V V T A S A A L T

CCGGCTTCACCTTCAGTGCGCTCCAGATTCTGCCCTACACCCTGGCGTCTCTGTACCATC
G F T F S A L Q I L P Y T L A S L Y H R

GCGAGAAGCAGGTGTTCCTGCCCAAGTACCGCGGGGACACAGGGGGAGCTTCCTCTGAGG
E K Q V F L P K Y R G D T G G A S S E D

ACAGCCTGATGACCAGCTTCTTGCCCGGCCCCAAGCCGGGGGCCCCTTTCCCCAACGGCC
S L M T S F L P G P K P G A P F P N G H

ATGTCGGGGCGGGCGGCAGCGGCCTGCTCCCTCCCCCCCCCGCCCTGTGCGGCGCTAGTG
V G A G G S G L L P P P P A L C G A S A

CCTGCGACGTGAGCGTGCGGGTGGTGGTGGGGGAGCCCACCGAGGCTAGGGTCGTGCCTG
C D V S V R V V V G E P T E A R V V P G

GCCGGGGGATCTGCCTGGACCTGGCCATCCTCGACTCCGCCTTCCTGCTCTCCCAGGTGG
R G I C L D L A I L D S A F L L S Q V A

CGCCCAGCCTGTTCATGGGCAGTATCGTGCAGCTGAGCCAGAGCGTGACCGCCTACATGG
P S L F M G S I V Q L S Q S V T A Y M V

TGAGCGCCGCCGGCCTGGGGTTGGTGGCCATCTACTTTGCCACCCAGGTCGTGTTCGACA
S A A G L G L V A I Y F A T Q V V F D K

AGAGCGATCTCGCCAAGTATAGCGCCATGGTGCAGCGGCTGTGGGTGTCCCGGCTGCTGC
S D L A K Y S A M V Q R L W V S R L L R

GCCATAGAAAGGCCCAGTTGCTGCTGGTGAACCTGCTGACTTTCGGACTGGAGGTGTGCC
H R K A Q L L L V N L L T F G L E V C L

TGGCTGCCGGGATCACGTACGTGCCCCCCCTGCTGCTGGAGGTGGGCGTGGAGGAGTGAG
A A G I T Y V P P L L L E V G V E E *

GATCC

## Construct D = SEQ ID NO:40 (nucleotide) & 48 (polypeptide)

GCGGCCGCGCCACCATGGTGCAGCGGCTGTGGGTGTCCCGGCTGCTGCGCCATAGAAAGG
M V Q R L W V S R L L R H R K A

CCCAGTTGCTGCTGGTGAACCTGCTGACTTTCGGACTGGAGGTGTGCCTGGCTGCCGGGA
Q L L L V N L L T F G L E V C L A A G I

TCACGTACGTGCCCCCCCTGCTGCTGGAGGTGGGCGTGGAGGAGATGGCCGCCGCCTACG
T Y V P P L L L E V G V E E M A A A Y V

TGCATAGCGACGGGAGCTACCCCAAGGACAAGTTCGAGAAGATCAACGGGACATGGTACT
H S D G S Y P K D K F E K I N G T W Y Y

ACTTCGACTCCTCCGGCTACATGCTCGCCGACCGCTGGCGGAAGCACACCGACGGCAACT
F D S S G Y M L A D R W R K H T D G N W

GGTACTGGTTCGATAACTCGGGAGAGATGGCCACCGGCTGGAAGAAGATCGCGGACAAGT
Y W F D N S G E M A T G W K K I A D K W

GGTACTATTTCAACGAGGAGGGCGCCATGAAGACCGGCTGGGTGAAGTATAAGGACACCT
Y Y F N E E G A M K T G W V K Y K D T W

GGTACTACCTCGACGCCAAGGAGGGCGCCATGCAGTATATCAAGGCCAACAGCAAGTTCA
Y Y L D A K E G A M Q Y I K A N S K F I

TCGGCATCACCGAGGGAGTGATGGTCAGCAACGCCTTTATCCAGAGCGCCGACGGCACCG
G I T E G V M V S N A F I Q S A D G T G

GATGGTACTACTTGAAGCCGGACGGCACCCTCGCGGATCGGCCCGAGAAGTTCATGTACA
W Y Y L K P D G T L A D R P E K F M Y M

TGGTGCTGGGCATCGGCCCCGTCCTGGGCCTCGTGTGTGTGCCCCTCCTCGGGAGTGCGT
V L G I G P V L G L V C V P L L G S A S

CCGATCATTGGCGGGGCCGCTACGGCCGCCGCAGACCGTTCATCTGGGCCCTGAGCCTGG
D H W R G R Y G R R R P F I W A L S L G

GCATCCTGCTCTCTCTCTTCCTGATCCCCCGGGCCGGCTGGCTGGCCGGCCTGCTGTGTC
I L L S L F L I P R A G W L A G L L C P

CCGACCCCCGCCCTCTGGAGCTGGCCCTCCTGATCCTGGGCGTGGGCCTGCTGGACTTCT
D P R P L E L A L L I L G V G L L D F C

GCGGCCAGGTGTGTTTCACTCCCCTGGAGGCTCTGCTCTCCGACCTCTTCCGCGACCCCG
G Q V C F T P L E A L L S D L F R D P D

ACCACTGTAGGCAGGCTTACAGCGTGTACGCCTTCATGATCAGTCTGGGGGGATGCCTGG
H C R Q A Y S V Y A F M I S L G G C L G

GCTATCTGCTGCCCGCTATCGACTGGGACACCAGCGCCCTGGCCCCCTACCTGGGGACTC
Y L L P A I D W D T S A L A P Y L G T Q

AGGAGGAGTGCCTGTTCGGCCTGCTCACCTTGATCTTCCTGACGTGCGTCGCCGCCACCC
E E C L F G L L T L I F L T C V A A T L

TGCTGGTGGCCGAGGAGGCGGCCCTGGGGCCCACCGAGCCCGCCGAGGGCCTGAGCGCTC
L V A E E A A L G P T E P A E G L S A P

CCAGCCTGAGCCCCCATTGCTGCCCGTGCAGGGCTAGGCTCGCCTTCAGGAATCTGGGCG
S L S P H C C P C R A R L A F R N L G A

CTTTGCTGCCCCGCCTGCATCAGCTGTGCTGTCGCATGCCTCGCACCCTGCGCCGCCTGT
L L P R L H Q L C C R M P R T L R R L F

TCGTCGCTGAGCTCTGTTCCTGGATGGCCCTGATGACGTTCACCCTCTTCTACACCGACT
V A E L C S W M A L M T F T L F Y T D F

TCGTGGGGGAGGGCCTGTACCAGGGCGTGCCCAGGGCCGAGCCCGGCACCGAGGCTAGGC

V G E G L Y Q G V P R A E P G T E A R R

GCCATTACGACGAGGGCGTCAGGATGGGCTCTCTGGGCCTCTTCCTGCAGTGCGCCATCA
H Y D E G V R M G S L G L F L Q C A I S

GTCTGGTGTTCTCTCTGGTGATGGACCGGCTGGTGCAGCGCTTCGGCACCCGGGCCGTGT
L V F S L V M D R L V Q R F G T R A V Y

ACCTCGCCTCTGTGGCGGCTTTCCCCGTCGCCGCCGGCGCGACCTGCCTGTCTCATTCTG
L A S V A A F P V A A G A T C L S H S V

TCGCCGTGGTGACCGCCAGCGCCGCCCTGACCGGCTTCACCTTCAGTGCGCTCCAGATTC
A V V T A S A A L T G F T F S A L Q I L

TGCCCTACACCCTGGCGTCTCTGTACCATCGCGAGAAGCAGGTGTTCCTGCCCAAGTACC
P Y T L A S L Y H R E K Q V F L P K Y R

GCGGGGACACAGGGGGAGCTTCCTCTGAGGACAGCCTGATGACCAGCTTCTTGCCCGGCC
G D T G G A S S E D S L M T S F L P G P

CCAAGCCGGGGGCCCCTTTCCCCAACGGCCATGTCGGGGCGGGCGGCAGCGGCCTGCTCC
K P G A P F P N G H V G A G G S G L L P

CTCCCCCCCCCGCCCTGTGCGGCGCTAGTGCCTGCGACGTGAGCGTGCGGGTGGTGGTGG
P P P A L C G A S A C D V S V R V V V G

GGGAGCCCACCGAGGCTAGGGTCGTGCCTGGCCGGGGGATCTGCCTGGACCTGGCCATCC
E P T E A R V V P G R G I C L D L A I L

TCGACTCCGCCTTCCTGCTCTCCCAGGTGGCGCCCAGCCTGTTCATGGGCAGTATCGTGC
D S A F L L S Q V A P S L F M G S I V Q

AGCTGAGCCAGAGCGTGACCGCCTACATGGTGAGCGCCGCCGGCCTGGGGTTGGTGGCCA
L S Q S V T A Y M V S A A G L G L V A I

TCTACTTTGCCACCCAGGTCGTGTTCGACAAGAGCGATCTCGCCAAGTATAGCGCCTGAG
Y F A T Q V V F D K S D L A K Y S A *

GATCC

**FIG. 21 – Western blot analysis of CHO cells following transient transfection with P501S (JNW680), CPC-P501S (JNW735) and empty vector control.**

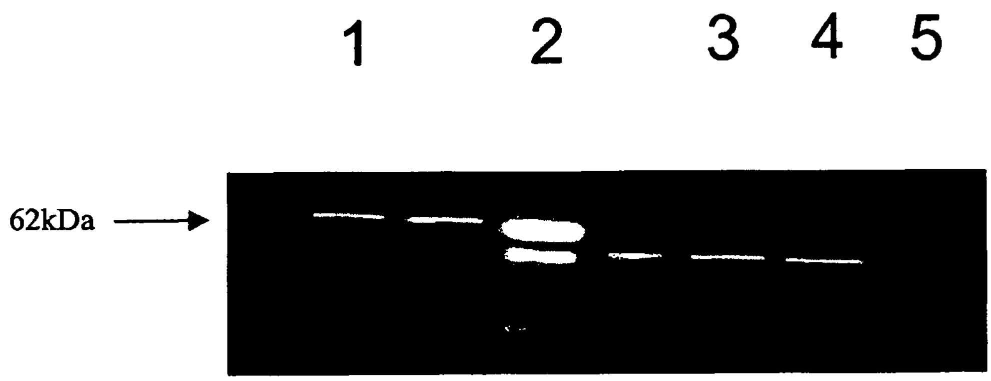


| Lane | Sample |
|---|---|
| 1 | CPC-P501S (JNW735) |
| 2 | CPC P501S protein (62.5ng) |
| 3 | P501S (JNW680) |
| 4 | P501S (JNW680) |
| 5 | Empty vector control |

**FIG. 22 – Anti-P501S antibody responses following immunisation at day0, 21 & 42 with pVAC-P501S (JNW680, mice B1-9) or Empty vector (pVAC, mice A1-6).**

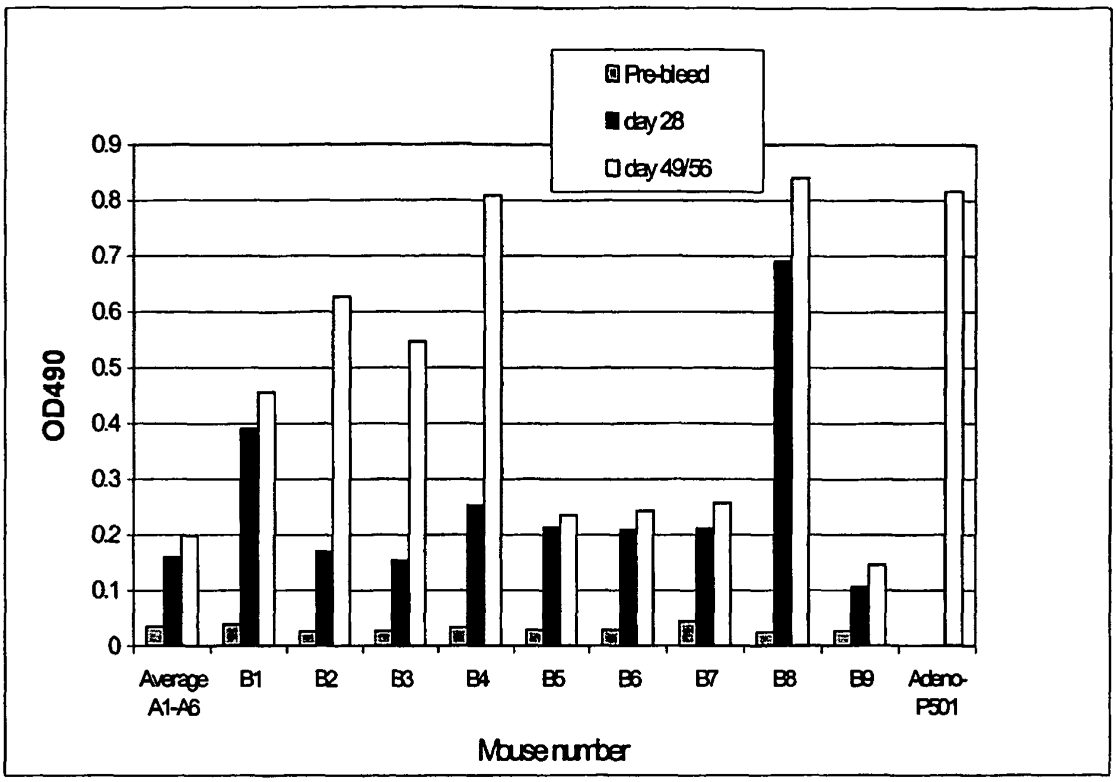

**FIG. 23 - Peptide library screen using C57BL/6 mice immunised at day 0, 21, 42, and 70 with pVAC-P501S (JNW680).**

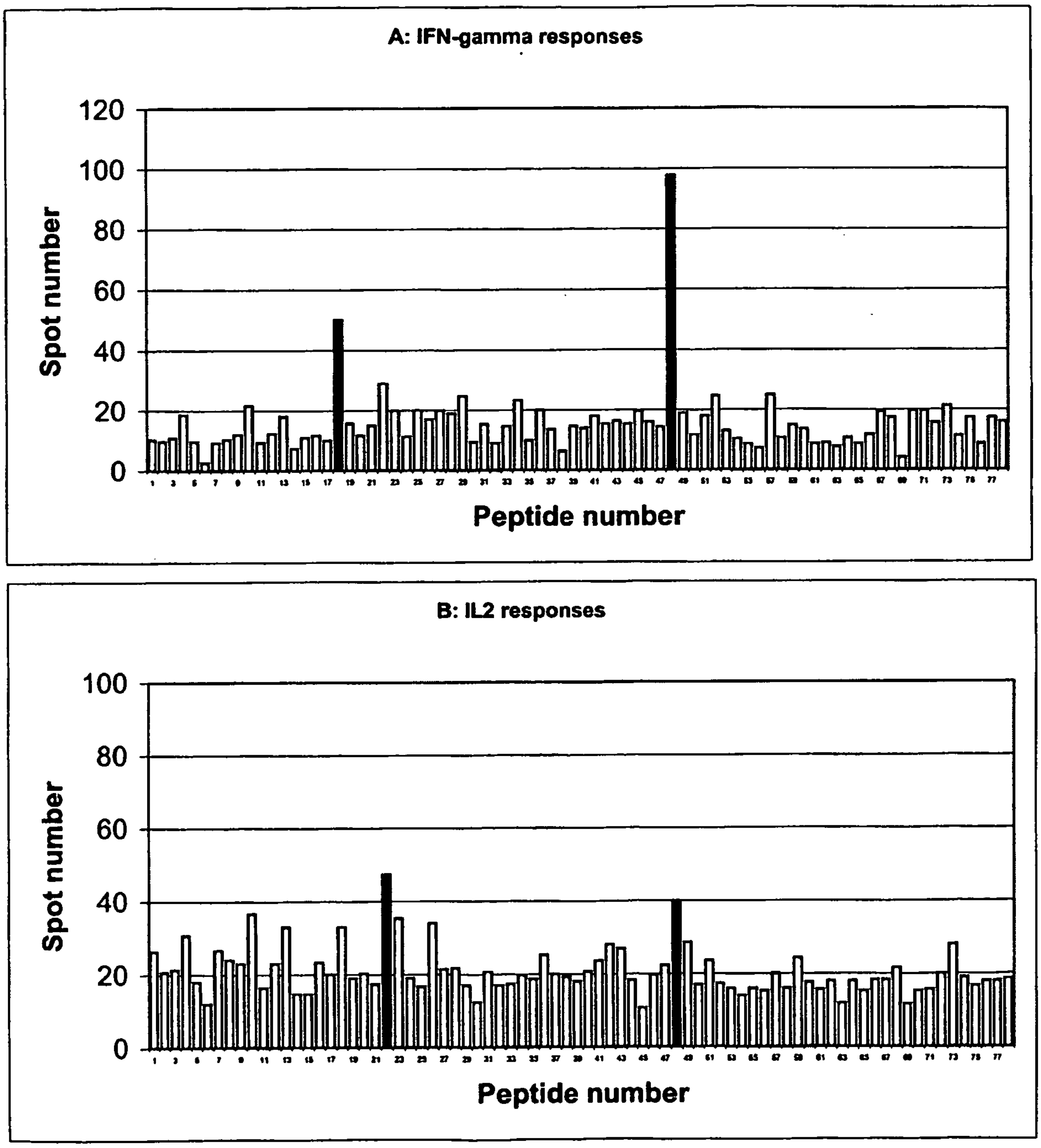

**FIG. 24 – Cellular responses by ELISPOT at day 77 following PMID immunisation at day 0, 21, 42, and 70 with pVAC-P501S (JNW680, B6-9) and pVAC empty (A4-6).**

Graph A shows the IFN-γ responses whilst Graph B shows the IL-2 responses.

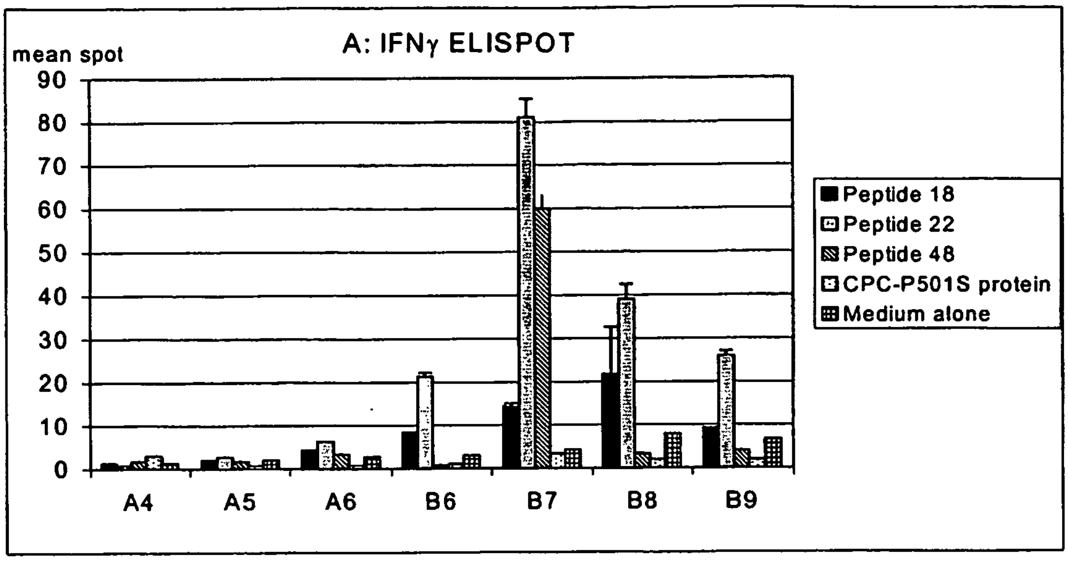


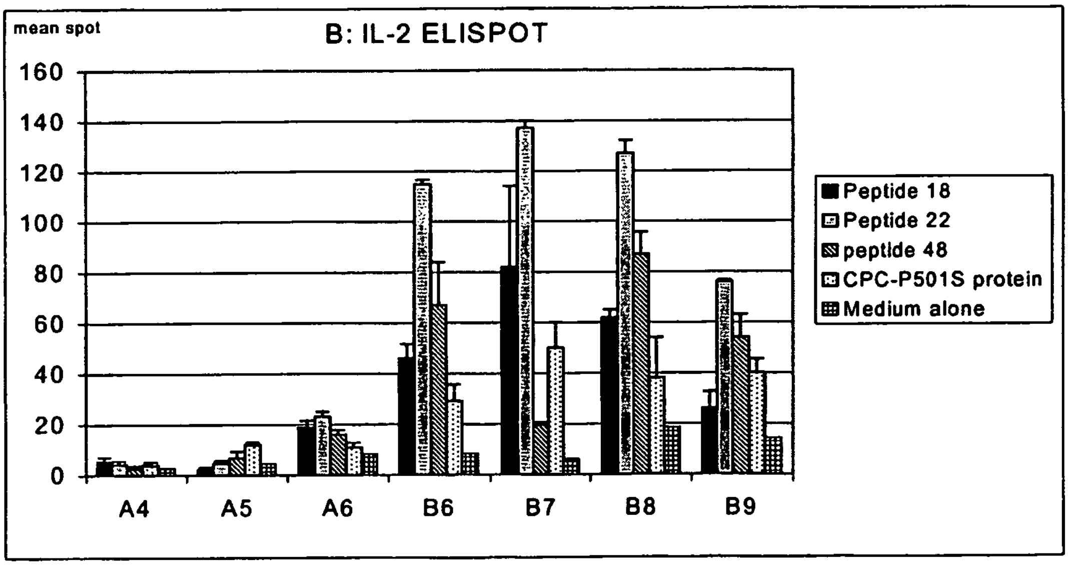

**FIG. 25 – Comparison of P501S and CPC-P501S.**

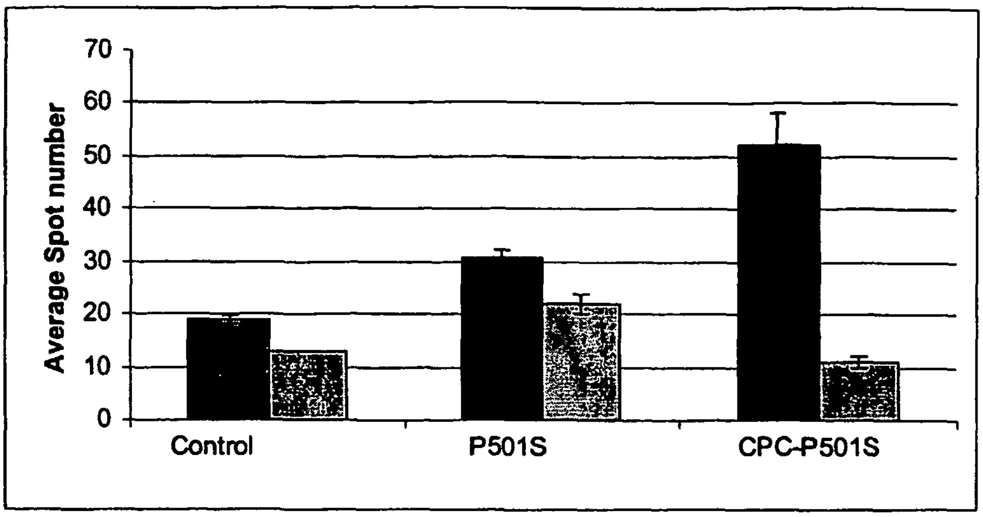


**FIG. 26 – Immune response (lymphoproliferation on spleen cells) following protein immunisation with CPC-P501S.**

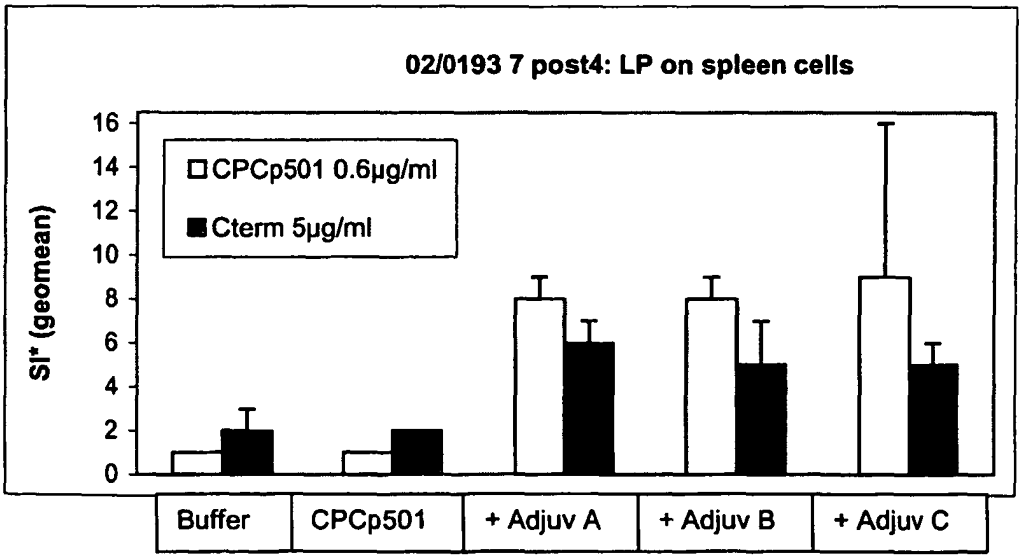

**FIG. 27 – Evaluation of the immune response to different CPC-P501S constructs**

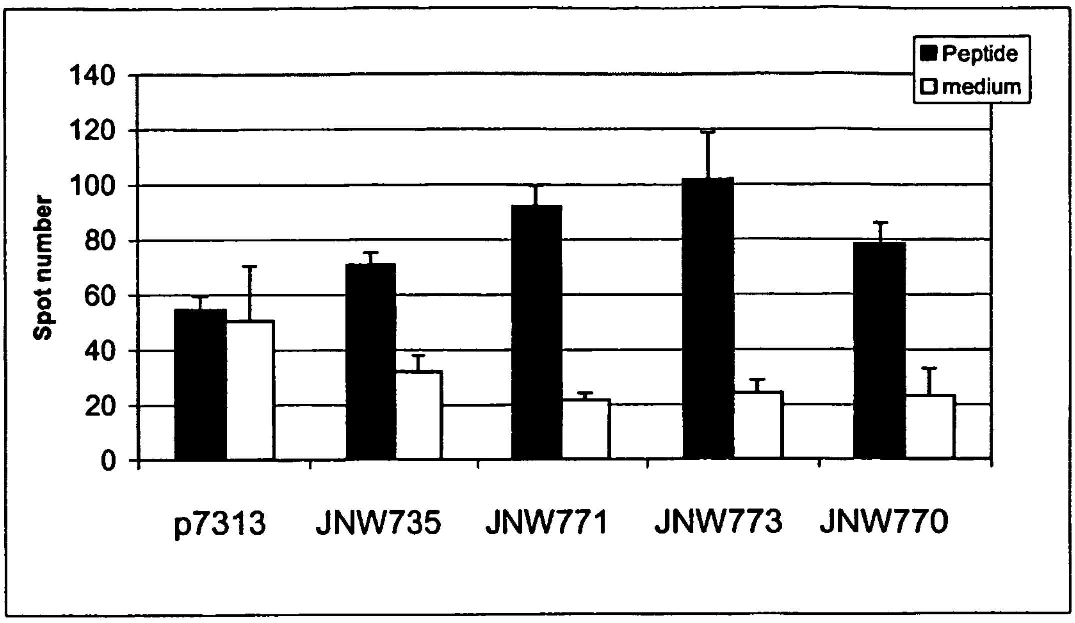

## FIG.28. MUC1-CPC DNA and polypeptide sequences

### FIG. 28A. DNA sequence (SEQ ID NO.49)

ATGACACCGGGCACCCAGTCTCCTTTCTTCCTGCTGCTGCTCCTCACAGTGCTTACAGTTGTTACAGGTTCTG
GTCATGCAAGCTCTACCCCAGGTGGAGAAAAGGAGACTTCGGCTACCCAGAGAAGTTCAGTGCCCAGCTCTAC
TGAGAAGAATGCTGTGAGTATGACCAGCAGCGTACTCTCCAGCCACAGCCCCGGTTCAGGCTCCTCCACCACT
CAGGGACAGGATGTCACTCTGGCCCCGGCCACGGAACCAGCTTCAGGTTCAGCTGCCACCTGGGGACAGGATG
TCACCTCGGTCCCAGTCACCAGGCCAGCCCTGGGCTCCACCACCCCGCCAGCCCACGATGTCACCTCAGCCCC
GGACAACAAGCCAGCCCCGGGCTCCACCGCCCCCCCAGCCCACGGTGTCACCTCGGCCCCGGACACCAGGCCG
CCCCCGGGCTCCACCGCCCCCCCAGCCCACGGTGTCACCTCGGCCCCGGACACCAGGCCGCCCCCGGGCTCCA
CCGCGCCCGCAGCCCACGGTGTCACCTCGGCCCCGGACACCAGGCCGGCCCCGGGCTCCACCGCCCCCCCAGC
CCATGGTGTCACCTCGGCCCCGGACAACAGGCCCGCCTTGGCGTCCACCGCCCCTCCAGTCCACAATGTCACC
TCGGCCTCAGGCTCTGCATCAGGCTCAGCTTCTACTCTGGTGCACAACGGCACCTCTGCCAGGGCTACCACAA
CCCCAGCCAGCAAGAGCACTCCATTCTCAATTCCCAGCCACCACTCTGATACTCCTACCACCCTTGCCAGCCA
TAGCACCAAGACTGATGCCAGTAGCACTCACCATAGCACGGTACCTCCTCTCACCTCCTCCAATCACAGCACT
TCTCCCCAGTTGTCTACTGGGGTCTCTTTCTTTTTCCTGTCTTTTCACATTTCAAACCTCCAGTTTAATTCCT
CTCTGGAAGATCCCAGCACCGACTACTACCAAGAGCTGCAGAGAGACATTTCTGAAATGTTTTTGCAGATTTA
TAAACAAGGGGGTTTTCTGGGCCTCTCCAATATTAAGTTCAGGCCAGGATCTGTGGTGGTACAATTGACTCTG
GCCTTCCGAGAAGGTACCATCAATGTCCACGACGTGGAGACACAGTTCAATCAGTATAAAACGGAAGCAGCCT
CTCGATATAACCTGACGATCTCAGACGTCAGCGTGAGTGATGTGCCATTTCCTTTCTCTGCCCAGTCTGGGGC
TGGGGTGCCAGGCTGGGGCATCGCGCTGCTGGTGCTGGTCTGTGTTCTGGTTGCGCTGGCCATTGTCTATCTC
ATTGCCTTGGCTGTCTGTCAGTGCCGCCGAAAGAACTACGGGCAGCTGGACATCTTTCCAGCCCGGGATACCT
ACCATCCTATGAGCGAGTACCCCACCTACCACACCCATGGGCGCTATGTGCCCCCTAGCAGTACCGATCGTAG
CCCCTATGAGAAGGTTTCTGCAGGTAATGGTGGCAGCAGCCTCTCTTACACAAACCCAGCAGTGGCAGCCACT
TCTGCCAACTTGATGGCGGCCGCTTACGTACATTCCGACGGCTCTTATCCAAAAGACAAGTTTGAGAAAATCA
ATGGCACTTGGTACTACTTTGACAGTTCAGGCTATATGCTTGCAGACCGCTGGAGGAAGCACACAGACGGCAA
CTGGTACTGGTTCGACAACTCAGGCGAAATGGCTACAGGCTGGAAGAAAATCGCTGATAAGTGGTACTATTTC
AACGAAGAAGGTGCCATGAAGACAGGCTGGGTCAAGTACAAGGACACTTGGTACTACTTAGACGCTAAAGAAG
GCGCCATGCAATACATCAAGGCTAACTCTAAGTTCATTGGTATCACTGAAGGCGTCATGGTATCAAATGCCTT
TATCCAGTCAGCGGACGGAACAGGCTGGTACTACCTCAAACCAGACGGAACACTGGCAGACAGGCCAGAATGA

### FIG. 28B. MUC1-CPC polypeptide sequence (SEQ ID NO.50)

MTPGTQSPFFLLLLLTVLTVVTGSGHASSTPGGEKETSATQRSSVPSSTEKNAVSMTSSVLSSHSPGSGSSTT
QGQDVTLAPATEPASGSAATWGQDVTSVPVTRPALGSTTPPAHDVTSAPDNKPAPGSTAPPAHGVTSAPDTRP
PPGSTAPPAHGVTSAPDTRPPPGSTAPAAHGVTSAPDTRPAPGSTAPPAHGVTSAPDNRPALASTAPPVHNVT
SASGSASGSASTLVHNGTSARATTTPASKSTPFSIPSHHSDTPTTLASHSTKTDASSTHHSTVPPLTSSNHST
SPQLSTGVSFFFLSFHISNLQFNSSLEDPSTDYYQELQRDISEMFLQIYKQGGFLGLSNIKFRPGSVVVQLTL

AFREGTINVHDVETQFNQYKTEAASRYNLTISDVSVSDVPFPFSAQSGAGVPGWGIALLVLVCVLVALAIVYL
IALAVCQCRRKNYGQLDIFPARDTYHPMSEYPTYHTHGRYVPPSSTDRSPYEKVSAGNGGSSLSYTNPAVAAT
SANLMAAAYVHSDGSYPKDKFEKINGTWYYFDSSGYMLADRWRKHTDGNWYWFDNSGEMATGWKKIADKWYYF
NEEGAMKTGWVKYKDTWYYLDAKEGAMQYIKANSKFIGITEGVMVSNAFIQSADGTGWYYLKPDGTLADRPE

## FIG.29. ss-CPC-MUC1 construct and sequence

## FIG. 29A. DNA sequence (SEQ ID NO.51)

ATGGGATGGAGCTGTATCATCCTCTTCTTGGTAGCAACAGCTACAGGTGTCCACTCCCAGGTCCAAATGGCGG
CCGCTTACGTACATTCCGACGGCTCTTATCCAAAAGACAAGTTTGAGAAAATCAATGGCACTTGGTACTACTT
TGACAGTTCAGGCTATATGCTTGCAGACCGCTGGAGGAAGCACACAGACGGCAACTGGTACTGGTTCGACAAC
TCAGGCGAAATGGCTACAGGCTGGAAGAAAATCGCTGATAAGTGGTACTATTTCAACGAAGAAGGTGCCATGA
AGACAGGCTGGGTCAAGTACAAGGACACTTGGTACTACTTAGACGCTAAAGAAGGCGCCATGCAATACATCAA
GGCTAACTCTAAGTTCATTGGTATCACTGAAGGCGTCATGGTATCAAATGCCTTTATCCAGTCAGCGGACGGA
ACAGGCTGGTACTACCTCAAACCAGACGGAACACTGGCAGACAGGCCAGAAATGACACCGGGCACCCAGTCTC
CTTTCTTCCTGCTGCTGCTCCTCACAGTGCTTACAGTTGTTACAGGTTCTGGTCATGCAAGCTCTACCCCAGG
TGGAGAAAAGGAGACTTCGGCTACCCAGAGAAGTTCAGTGCCCAGCTCTACTGAGAAGAATGCTGTGAGTATG
ACCAGCAGCGTACTCTCCAGCCACAGCCCCGGTTCAGGCTCCTCCACCACTCAGGGACAGGATGTCACTCTGG
CCCCGGCCACGGAACCAGCTTCAGGTTCAGCTGCCACCTGGGGACAGGATGTCACCTCGGTCCCAGTCACCAG
GCCAGCCCTGGGCTCCACCACCCCGCCAGCCCACGATGTCACCTCAGCCCCGGACAACAAGCCAGCCCCGGGC
TCCACCGCCCCCCCAGCCCACGGTGTCACCTCGGCCCCGGACACCAGGCCGCCCCCGGGCTCCACCGCCCCCC
CAGCCCACGGTGTCACCTCGGCCCCGGACACCAGGCCGCCCCCGGGCTCCACCGCGCCCGCAGCCCACGGTGT
CACCTCGGCCCCGGACACCAGGCCGGCCCCGGGCTCCACCGCCCCCCAGCCCATGGTGTCACCTCGGCCCCG
GACAACAGGCCCGCCTTGGCGTCCACCGCCCCTCCAGTCCACAATGTCACCTCGGCCTCAGGCTCTGCATCAG
GCTCAGCTTCTACTCTGGTGCACAACGGCACCTCTGCCAGGGCTACCACAACCCCAGCCAGCAAGAGCACTCC
ATTCTCAATTCCCAGCCACCACTCTGATACTCCTACCACCCTTGCCAGCCATAGCACCAAGACTGATGCCAGT
AGCACTCACCATAGCACGGTACCTCCTCTCACCTCCTCCAATCACAGCACTTCTCCCCAGTTGTCTACTGGGG
TCTCTTTCTTTTTCCTGTCTTTTCACATTTCAAACCTCCAGTTTAATTCCTCTCTGGAAGATCCCAGCACCGA
CTACTACCAAGAGCTGCAGAGAGACATTTCTGAAATGTTTTTGCAGATTTATAAACAAGGGGGTTTTCTGGGC
CTCTCCAATATTAAGTTCAGGCCAGGATCTGTGGTGGTACAATTGACTCTGGCCTTCCGAGAAGGTACCATCA
ATGTCCACGACGTGGAGACACAGTTCAATCAGTATAAAACGGAAGCAGCCTCTCGATATAACCTGACGATCTC
AGACGTCAGCGTGAGTGATGTGCCATTTCCTTTCTCTGCCCAGTCTGGGGCTGGGGTGCCAGGCTGGGGCATC
GCGCTGCTGGTGCTGGTCTGTGTTCTGGTTGCGCTGGCCATTGTCTATCTCATTGCCTTGGCTGTCTGTCAGT
GCCGCCGAAAGAACTACGGGCAGCTGGACATCTTTCCAGCCCGGGATACCTACCATCCTATGAGCGAGTACCC
CACCTACCACACCCATGGGCGCTATGTGCCCCCTAGCAGTACCGATCGTAGCCCCTATGAGAAGGTTTCTGCA
GGTAATGGTGGCAGCAGCCTCTCTTACACAAACCCAGCAGTGGCAGCCACTTCTGCCAACTTGTAG

**FIG. 29B. ss-CPC-MUC1 protein sequence Polypeptide sequence (SEQ ID NO.52)**

MGWSCIILFLVATATGVHSQVQMAAAYVHSDGSYPKDKFEKINGTWYYFDSSGYMLADRWRKHTDGNWYWFDN
SGEMATGWKKIADKWYYFNEEGAMKTGWVKYKDTWYYLDAKEGAMQYIKANSKFIGITEGVMVSNAFIQSADG
TGWYYLKPDGTLADRPEMTPGTQSPFFLLLLLTVLTVVTGSGHASSTPGGEKETSATQRSSVPSSTEKNAVSM
TSSVLSSHSPGSGSSTTQGQDVTLAPATEPASGSAATWGQDVTSVPVTRPALGSTTPPAHDVTSAPDNKPAPG
STAPPAHGVTSAPDTRPPPGSTAPPAHGVTSAPDTRPPPGSTAPAAHGVTSAPDTRPAPGSTAPPAHGVTSAP
DNRPALASTAPPVHNVTSASGSASGSASTLVHNGTSARATTTPASKSTPFSIPSHHSDTPTTLASHSTKTDAS
STHHSTVPPLTSSNHSTSPQLSTGVSFFFLSFHISNLQFNSSLEDPSTDYYQELQRDISEMFLQIYKQGGFLG
LSNIKFRPGSVVVQLTLAFREGTINVHDVETQFNQYKTEAASRYNLTISDVSVSDVPFPFSAQSGAGVPGWGI
ALLVLVCVLVALAIVYLIALAVCQCRRKNYGQLDIFPARDTYHPMSEYPTYHTHGRYVPPSSTDRSPYEKVSA
GNGGSSLSYTNPAVAATSANL